# EUROPEAN PATENT SPECIFICATION

(11) **EP 4 144 741 B1**
(45) Date of publication and mention of the grant of the patent: **23.07.2025**
(21) Application number: 21796464.2
(22) Date of filing: 29.04.2021
(51) Int. Cl.: C07D 519/00, C07D 487/04, C07D 487/06, C07D 498/16, A61P 9/00, A61K 31/519

(54) **PYRIMIDINE-BASED TRICYCLIC COMPOUND AND USE THEREOF**
TRICYCLISCHE VERBINDUNG AUF PYRIMIDINBASIS UND VERWENDUNG DAVON
COMPOSÉ TRICYCLIQUE À BASE DE PYRIMIDINE ET SON UTILISATION

(30) Priority: 30.04.2020 CN 202010361013; 12.04.2021 CN 202110390228
(43) Date of publication of application: 08.03.2023
(73) Proprietor: CHIA TAI TIANQING PHARMACEUTICAL GROUP CO., LTD., Lianyungang, Jiangsu 222062 (CN)
(72) Inventor: LUO, Yunfu, Shanghai 200131 (CN); ZHANG, Guoli, Shanghai 200131 (CN); LI, Shaolong, Shanghai 200131 (CN); GE, Weizhi, Shanghai 200131 (CN); CHEN, Shuhui, Shanghai 200131 (CN)
(74) Representative: Hoffmann Eitle
(86) International application number: PCT/CN2021/091073
(87) International publication number: WO 2021/219088

(56) References cited:
- WO-A1-2012/004258
- WO-A1-2013/004785
- CN-A- 103 906 752
- CN-A- 106 459 090
- CN-A- 107 964 018
- CN-A- 108 727 340

## Description

The present application claims priority to Chinese Patent Application No. CN202010361013.7 filed on Apr. 30, 2020 and Chinese Patent Application No. CN202110390228.6 filed on Apr. 12, 2021.

### TECHNICAL FIELD

The present application relates to a pyrimidine-based tricyclic compound and use thereof for preparing a medicament for treating related diseases. The present application specifically relates to compounds of formula (I) and formula (II), and stereoisomers thereof or pharmaceutically acceptable salts thereof.

### BACKGROUND

Soluble guanylate cyclase (sGC), a heterodimer composed of α and β subunits, is widely found in the cytosol of mammals. Soluble guanylate cyclase is a key signal transduction enzyme in the NO-sGC-cGMP signaling pathway. sGC catalyzes the conversion of guanosine triphosphate (GTP) to cyclic guanosine monophosphate (cGMP) upon being *in vivo* activated. cGMP is an important secondary messenger molecule. It triggers a series of cascade reactions downstream by activating various effector molecules downstream, such as cGMP-dependent protein kinase G and cGMP-gated ion channels. It serves important physiological functions in the gastrointestinal system, the cardiovascular system and the central nervous system, such as promoting vasodilation and the relaxation of smooth muscles, inhibiting platelet aggregation, vascular remodeling, apoptosis and inflammation, and taking part in neurotransmission. Under pathophysiological conditions, the NO/cGMP system may be suppressed, which may lead to, for example, hypertension, platelet activation, increased cell proliferation, endothelial dysfunction, arteriosclerosis, angina pectoris, heart failure, myocardial infarction, thrombosis, strokes, sexual dysfunction, etc. In recent two years, studies have shown that abnormality in the sGC-mediated signaling pathway is also closely related to the occurrence of fibrotic diseases such as chronic kidney diseases and systemic sclerosis. sGC stimulators are known from WO 2012/004258 and WO 2013/004785.

sGC stimulators have a dual mechanism of action: they can directly activate the sGC-cGMP signaling pathway without depending on NO but necessarily on Fe²⁺-containing heme prosthetic groups; they can also heighten sGC's sensitivity to endogenous NO to produce a synergistic effect with NO. sGC stimulators are therefore heme-dependent and NO-independent. By stimulating sGC to generate more cGMP, a variety of important physiological processes can be regulated: to promote the relaxation of vascular smooth muscles, to inhibit platelet aggregation, etc. In addition, by activating sGC, other signaling pathways such as TGF-β can also be regulated to produce anti-fibrosis and anti-tumor effects. sGC stimulators can therefore be used as potential treatments for cardiovascular diseases (heart failure, pulmonary hypertension, angina pectoris, and myocardial infarction) and fibrotic diseases (renal fibrosis, and systemic sclerosis).

In response to the currently unmet market and clinical needs for such soluble guanylate cyclase stimulators, the present application provides a class of new compounds. Such compounds can be used as stimulators of soluble guanylate cyclase. They have excellent *in vitro* stimulating activity for soluble guanylate cyclase and have good pharmacokinetic properties.

### BRIEF SUMMARY

The present application provides a compound of formula (II), a stereoisomer thereof or a pharmaceutically acceptable salt thereof, 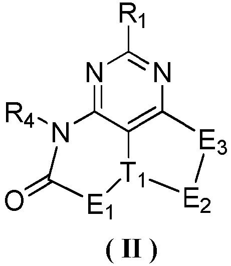 wherein,
R₁ is selected from the group consisting of 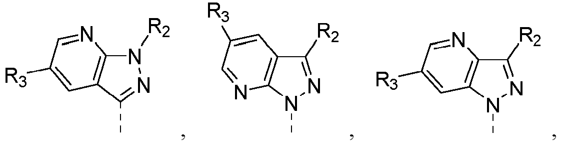 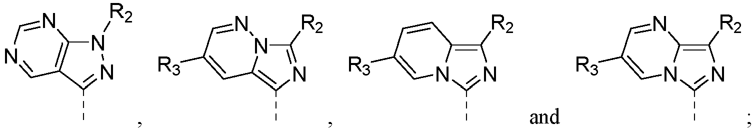
each R₂ is independently selected from the group consisting of 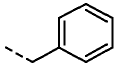 and 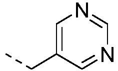 substituted with 1 or 2 R_{d} and C₁₋₃ alkyl substituted with 1, 2 or 3 R_{d};
each R₃ is independently selected from the group consisting of H and halogen;
R₄ is selected from the group consisting of H and C₁₋₃ alkyl;
E₁ is selected from -(CH₂)ₘ-;
m is selected from the group consisting of 0, 1 and 2;
E₂ is selected from the group consisting of -(CH₂)ₙ-, -(CH₂)ₚC(O)-, -O(CH₂)_{q}-, -O(CH₂)ᵣC(O)-, -CH₂CH=CH- and -(CH₂)ₛNHC(O)-, wherein each of the CH₂ optionally substituted with 1 or 2 R_{b};
E₃ is selected from the group consisting of a single bond, NR_{c} and O;
n is selected from the group consisting of 1, 2 and 3;
p is selected from the group consisting of 0, 1 and 2;
q is selected from the group consisting of 1 and 2;
r is selected from the group consisting of 1 and 2;
s is selected from the group consisting of 1 and 2;
T₁ is selected from the group consisting of N and CRₐ;
each Rₐ is independently selected from the group consisting of H, OH, -OC(=O)NHEt, -CO₂Et, -NHCO₂CH₃, - C(=O)NH(CH₂)₂OCH₃ and C₁₋₃ alkyl;
each R_{b} is independently selected from the group consisting of F and CH₃;
each R_{c} is independently selected from the group consisting of H and CH₃;
each R_{d} is independently selected from the group consisting of halogen and CF₃.

In some embodiments of the present application, each of the Rₐ described above is independently selected from the group consisting of H, OH, -OC(=O)NHEt, -CO₂Et, -C(=O)NH(CH₂)₂OCH₃, -NHCO₂CH₃ and CH₃, and the other variables are as defined in the present application.

In some embodiments of the present application, each of the R_{d} described above is independently selected from the group consisting of F and CF₃, and the other variables are as defined in the present application.

In some embodiments of the present application, each of the R₂ described above is independently selected from the group consisting of 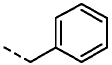 and 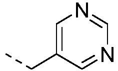 substituted with 1 or 2 R_{d} and C₁₋₃ alkyl substituted with 1, 2 or 3 R_{d}, and the other variables are as defined in the present application.

In some embodiments of the present application, each of the R₂ described above is independently selected from the group consisting of 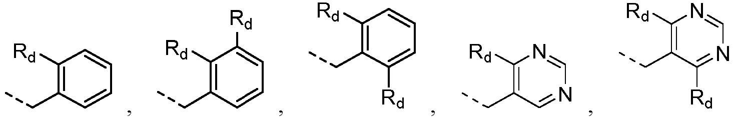 and 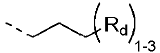 , and the other variables are as defined in the present application.

In some embodiments of the present application, each of the R₂ described above is selected from the group consisting of 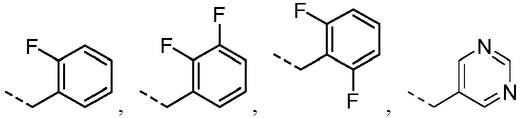 and 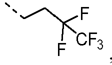 , and the other variables are as defined in the present application.

In some embodiments of the present application, each of the R₂ described above is selected from the group consisting of 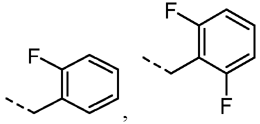 and 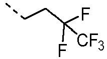 , and the other variables are as defined in the present application.

In some embodiments of the present application, each of the R₃ described above is selected from the group consisting of H and F, and the other variables are as defined in the present application.

In some embodiments of the present application, R₁ described above is selected from the group consisting of 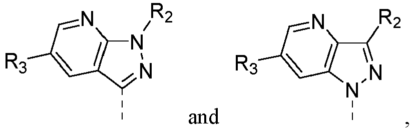 and the other variables are as defined in the present application.

In some embodiments of the present application, the R₁ described above is selected from the group consisting of 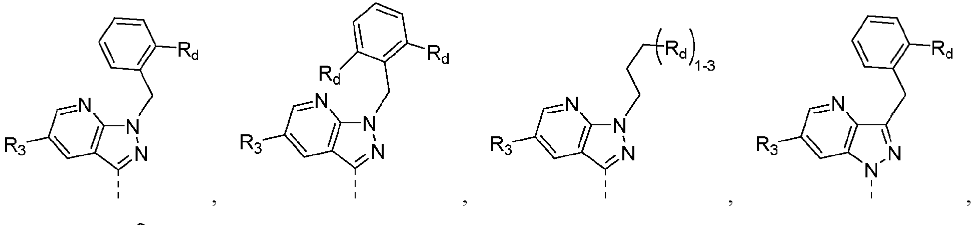 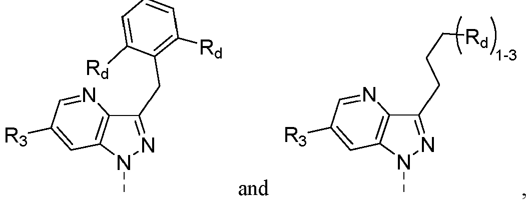 and the other variables are as defined in the present application.

In some embodiments of the present application, the R₁ described above is selected from the group consisting of 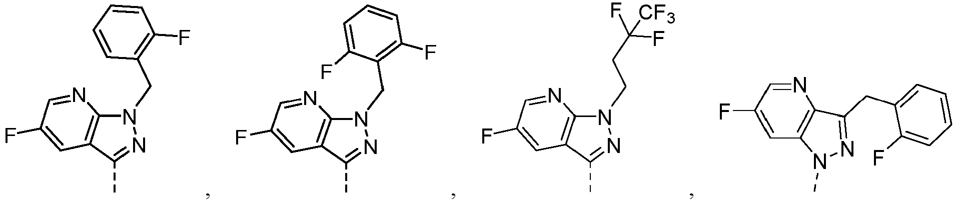 and 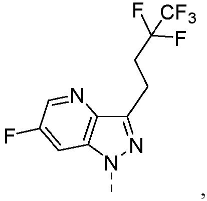 and the other variables are as defined in the present application.

In some embodiments of the present application, the E₁ described above is selected from the group consisting of a single bond, -CH₂- and -(CH₂)₂-, and the other variables are as defined in the present application.

In some embodiments of the present application, the E₂ described above is selected from the group consisting of - CH₂-, -(CH₂)₂-, -(CH₂)₃-, -CH₂CH=CH-, -CH₂CO-, -(CH₂)₂CO-, -O(CH₂)₂-, -OCH₂C(O)- and -CH₂NHC(O)-, wherein each of the CH₂ is optionally substituted with 1 or 2 R_{b}, and the other variables are as defined in the present application.

In some embodiments of the present application, the E₂ described above is selected from the group consisting of - CH₂-, -(CH₂)₂-, -CF₂CH₂-, -(CH₂)₃-, -CH₂CH=CH-, -CH₂CO-, -CO-, -C(CH₃)₂CO-, -CF₂CO-, -(CH₂)₂CO-, - O(CH₂)₂-, -OCH₂C(O)- and -CH₂NHC(O)-, and the other variables are as defined in the present application.

In some embodiments of the present application, the E₃ described above is selected from the group consisting of a single bond, NH, N(CH₃) and O, and the other variables are as defined in the present application.

In some embodiments of the present application, the T₁ described above is selected from the group consisting of N, CH, C(OH), C(OC(=O)NHEt), C(CO₂Et), C(NHCO₂CH₃), C[C(=O)NH(CH₂)₂OCH₃] and C(CH₃), and the other variables are as defined in the present application.

In some embodiments of the present application, the structural unit 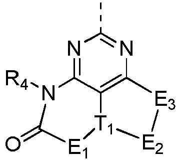 described above is selected from the group consisting of 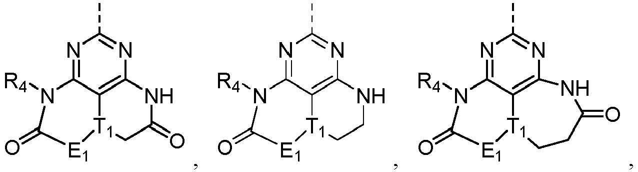 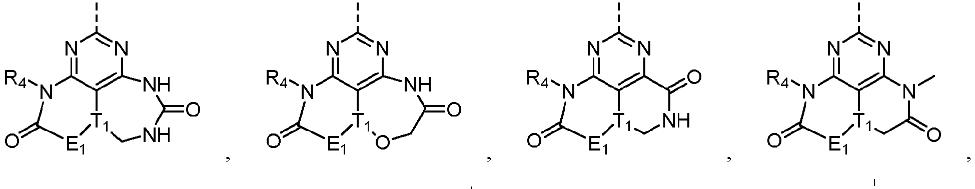 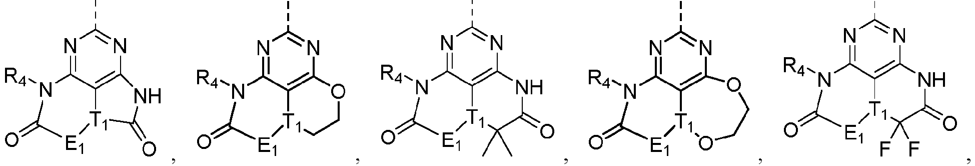 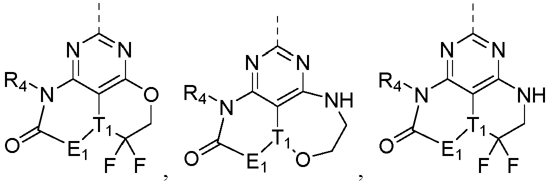 and 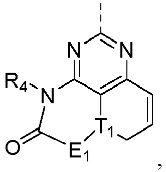 and the other variables are as defined in the present application.

In some embodiments of the present application, the structural unit 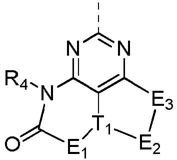 described above is selected from the group consisting of 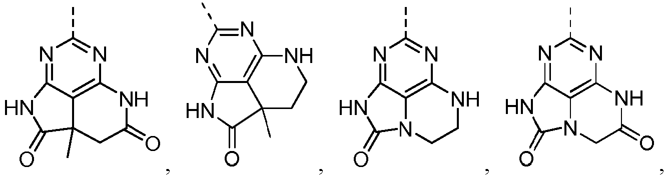 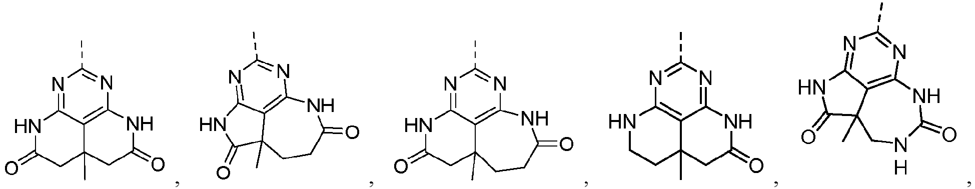 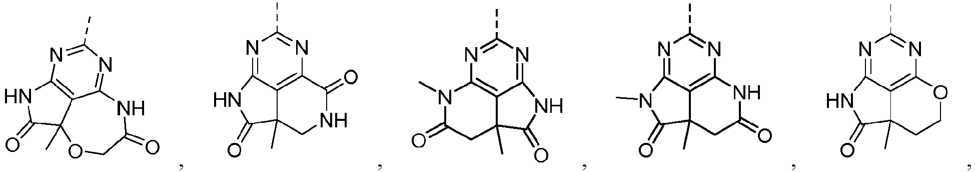 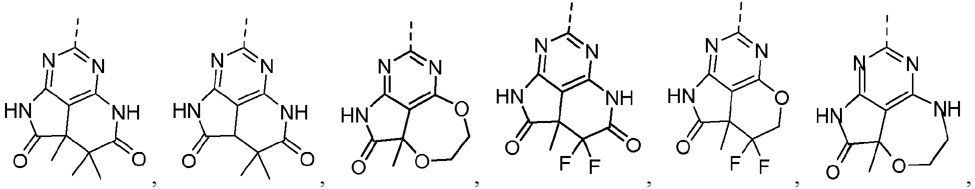 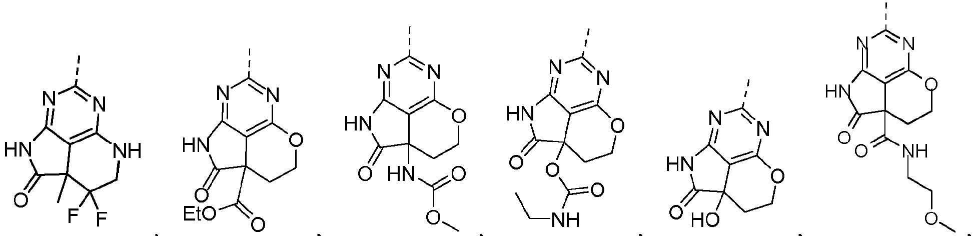 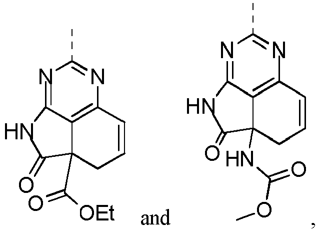 and the other variables are as defined in the present application.

The present application provides a compound of formula (I), a stereoisomer thereof or a pharmaceutically acceptable salt thereof, 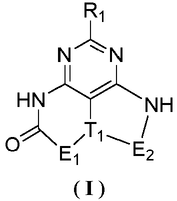 wherein,
R₁ is selected from the group consisting of 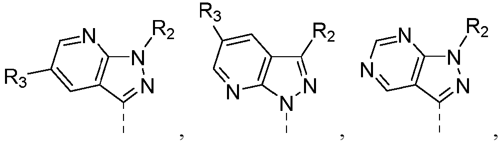 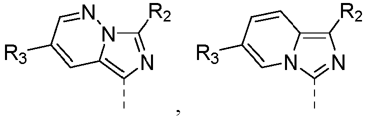 and 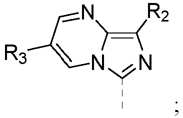
each R₂ is independently selected from the group consisting of 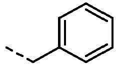 and 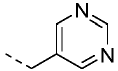 containing 1 or 2 substituents and C₁₋₃ alkyl containing 1, 2 or 3 substituents, wherein the substituents are selected from the group consisting of halogen and CF₃;
each R₃ is independently selected from the group consisting of H and halogen;
E₁ is selected from -(CH₂)ₘ-;
m is selected from the group consisting of 0, 1 and 2;
E₂ is selected from the group consisting of -(CH₂)ₙ- and -(CH₂)ₚC(O)-;
each n is selected from the group consisting of 1, 2 and 3;
each p is selected from the group consisting of 0, 1 and 2;
T₁ is selected from the group consisting of N and CRₐ;
Rₐ is selected from the group consisting of H and C₁₋₃ alkyl.

In some embodiments of the present application, each of the R₂ described above is independently selected from the group consisting of 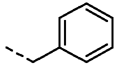 and 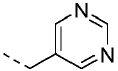 containing 1 or 2 substituents and C₁₋₃ alkyl containing 1, 2 or 3 substituents, wherein the substituents are selected from the group consisting of F and CF₃, and the other variables are as defined in the present application.

In some embodiments of the present application, each of the R₂ described above is selected from the group consisting of 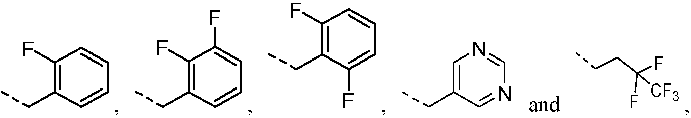 and the other variables are as defined in the present application.

In some embodiments of the present application, each of the R₃ described above is selected from the group consisting of H and F, and the other variables are as defined in the present application.

In some embodiments of the present application, R₁ described above is selected from the group consisting of 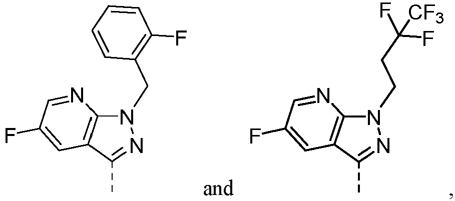 and the other variables are as defined in the present application.

In some embodiments of the present application, the E₁ described above is selected from the group consisting of a single bond, -CH₂- and -(CH₂)₂-, and the other variables are as defined in the present application.

In some embodiments of the present application, the E₂ described above is selected from the group consisting of - CH₂-, -(CH₂)₂-, -(CH₂)₃-, -CH₂CO- and -(CH₂)₂CO-, and the other variables are as defined in the present application.

In some embodiments of the present application, the T₁ described above is selected from the group consisting of N and -C(CH₃)-, and the other variables are as defined in the present application.

In some embodiments of the present application, the structural unit 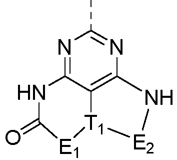 described above is selected from the group consisting of 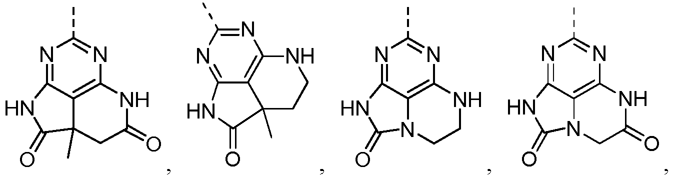 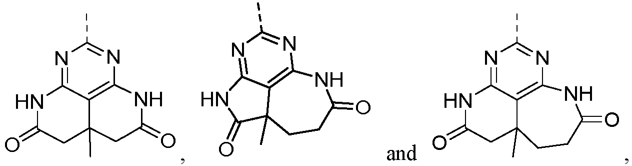 and the other variables are as defined in the present application.

The present application provides a compound of formula (II), a stereoisomer thereof or a pharmaceutically acceptable salt thereof, 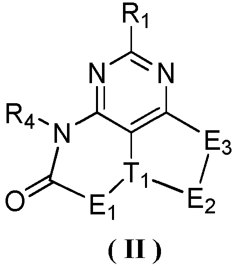 wherein,
R₁ is selected from the group consisting of 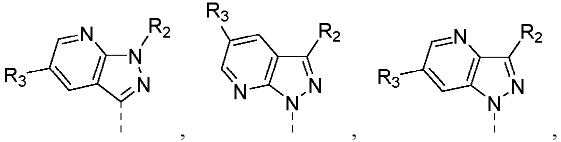 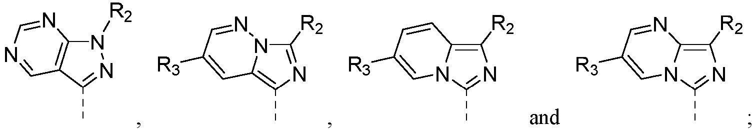
each R₂ is independently selected from the group consisting of 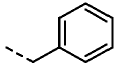 and 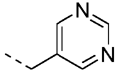 substituted with 1 or 2 R_{d} and C₁₋₃ alkyl substituted with 1, 2 or 3 R_{d};
each R₃ is independently selected from the group consisting of H and halogen;
R₄ is selected from the group consisting of H and C₁₋₃ alkyl;
E₁ is selected from -(CH₂)ₘ-;
m is selected from the group consisting of 0, 1 and 2;
E₂ is selected from the group consisting of -(CH₂)ₙ-, -(CH₂)ₚC(O)-, -O(CH₂)_{q}-, -O(CH₂)ᵣC(O)- and - (CH₂)ₛNHC(O)-, wherein the CH₂ is optionally substituted with 1 or 2 R_{b};
E₃ is selected from the group consisting of a single bond, NR_{c} and O;
n is selected from the group consisting of 1, 2 and 3;
p is selected from the group consisting of 0, 1 and 2;
q is selected from the group consisting of 1 and 2;
r is selected from the group consisting of 1 and 2;
s is selected from the group consisting of 1 and 2;
T₁ is selected from the group consisting of N and CRₐ;
each Rₐ is independently selected from the group consisting of H, OH, -OC(=O)NHEt, -CO₂Et, -NHCO₂CH₃ and C₁₋₃ alkyl;
each R_{b} is independently selected from the group consisting of F and CH₃;
each R_{c} is independently selected from the group consisting of H and CH₃;
each R_{d} is independently selected from the group consisting of halogen and CF₃.

In some embodiments of the present application, each of the Rₐ described above is independently selected from the group consisting of H, OH, -OC(=O)NHEt, -CO₂Et, -NHCO₂CH₃ and CH₃, and the other variables are as defined in the present application.

In some embodiments of the present application, each of the R_{d} described above is independently selected from the group consisting of F and CF₃, and the other variables are as defined in the present application.

In some embodiments of the present application, each of the R₂ described above is independently selected from the group consisting of 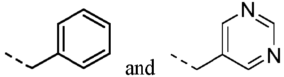 substituted with 1 or 2 R_{d} and C₁₋₃ alkyl substituted with 1, 2 or 3 R_{d}, and the other variables are as defined in the present application.

In some embodiments of the present application, each of the R₂ described above is selected from the group consisting of 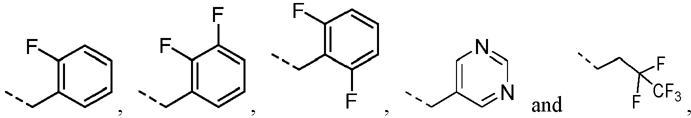 and the other variables are as defined in the present application.

In some embodiments of the present application, each of the R₃ described above is selected from the group consisting of H and F, and the other variables are as defined in the present application.

In some embodiments of the present application, the R₁ described above is selected from the group consisting of 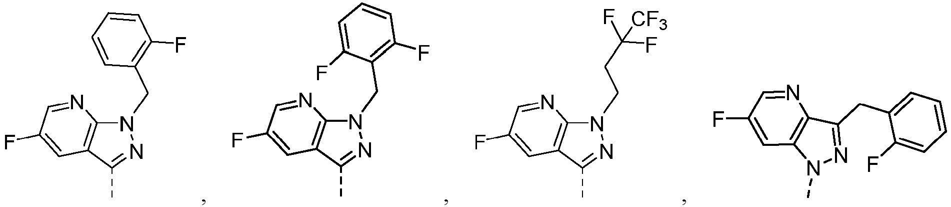 and 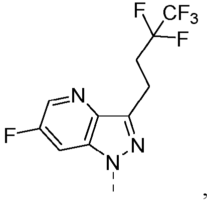 and the other variables are as defined in the present application.

In some embodiments of the present application, the E₁ described above is selected from the group consisting of a single bond, -CH₂- and -(CH₂)₂-, and the other variables are as defined in the present application.

In some embodiments of the present application, the E₂ described above is selected from the group consisting of - CH₂-, -(CH₂)₂-, -(CH₂)₃-, -CH₂CO-, -(CH₂)₂CO-, -O(CH₂)₂-, -OCH₂C(O)- and -CH₂NHC(O)-, wherein each of the CH₂ is optionally substituted with 1 or 2 R_{b}, and the other variables are as defined in the present application.

In some embodiments of the present application, the E₂ described above is selected from the group consisting of - CH₂-, -(CH₂)₂-, -CF₂CH₂-, -(CH₂)₃-, -CH₂CO-, -CO-, -C(CH₃)₂CO-, -CF₂CO-, -(CH₂)₂CO-, -O(CH₂)₂-, - OCH₂C(O)- and -CH₂NHC(O)-, and the other variables are as defined in the present application.

In some embodiments of the present application, the E₃ described above is selected from the group consisting of a single bond, NH, N(CH₃) and O, and the other variables are as defined in the present application.

In some embodiments of the present application, the T₁ described above is selected from the group consisting of N, CH, C(OH), C(OC(=O)NHEt), C(CO₂Et), C(NHCO₂CH₃) and C(CH₃), and the other variables are as defined in the present application.

In some embodiments of the present application, the structural unit 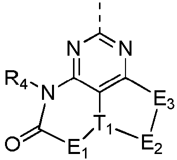 described above is selected from the group consisting of 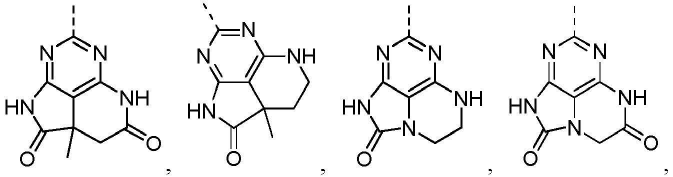 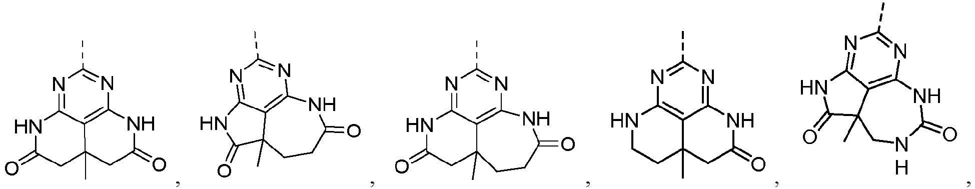 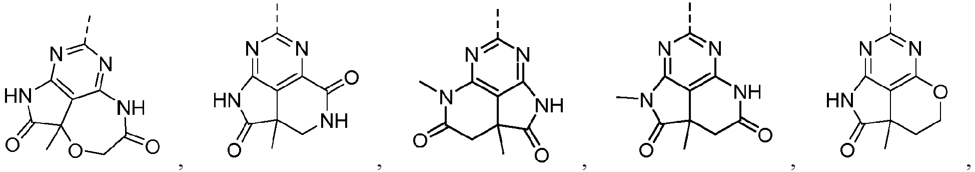 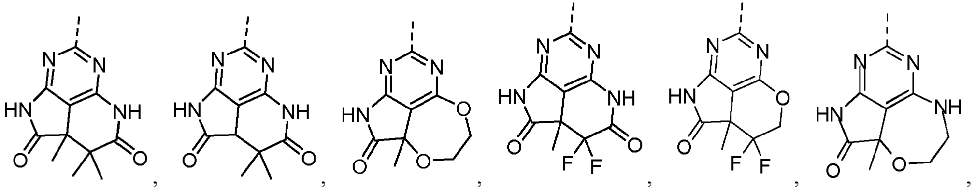 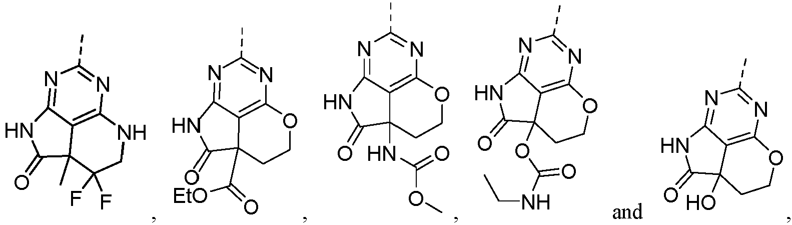 and the other variables are as defined in the present application.

In some embodiments of the present application, the compound, the stereoisomer thereof or the pharmaceutically acceptable salt thereof described above is selected from the group consisting of: 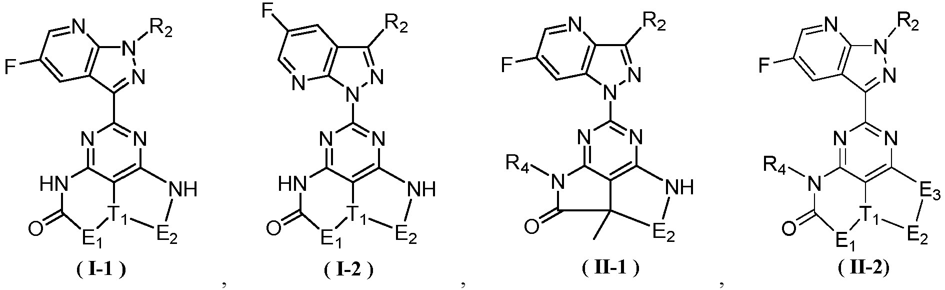 and 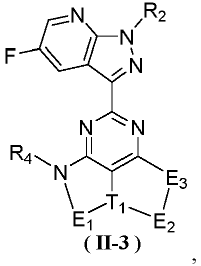 wherein R₂, R₄, T₁, E₁, E₂ and E₃ are as defined in any item of the present application.

Some other embodiments of the present application are derived from any combination of the variables as described above.

The present application also provides a compound, a stereoisomer thereof or a pharmaceutically acceptable salt thereof selected from the group consisting of:

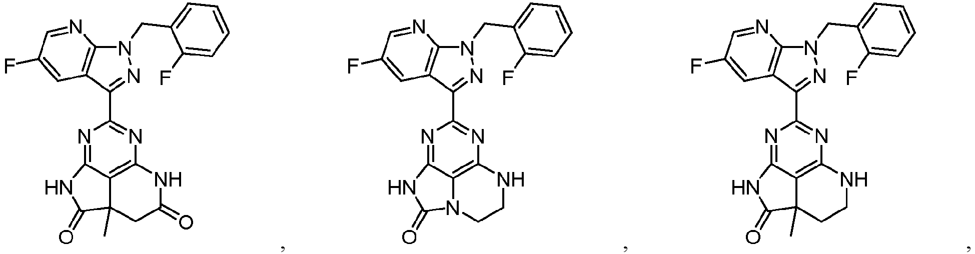

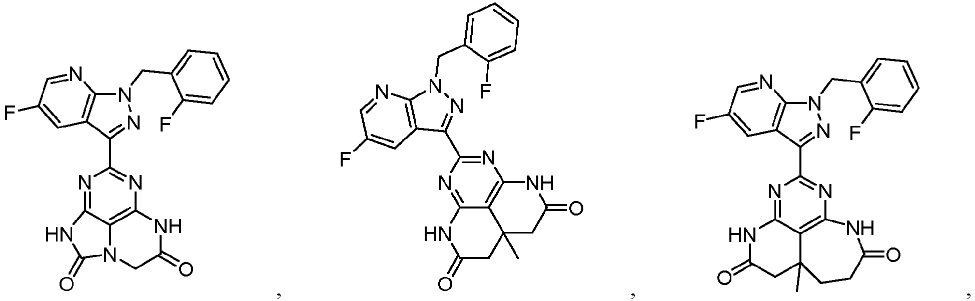

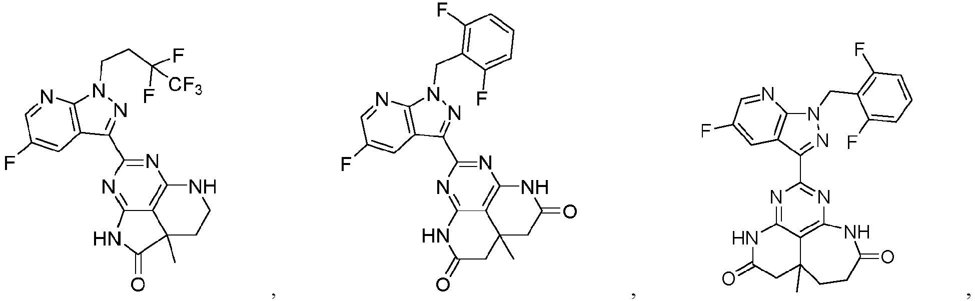

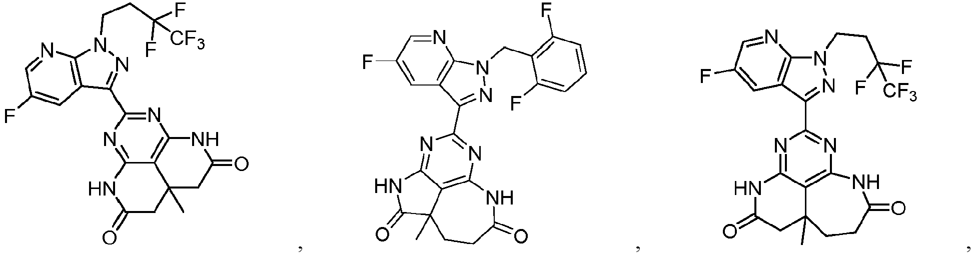

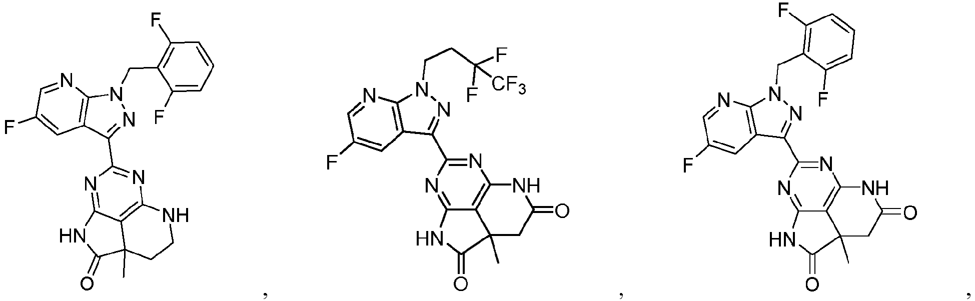

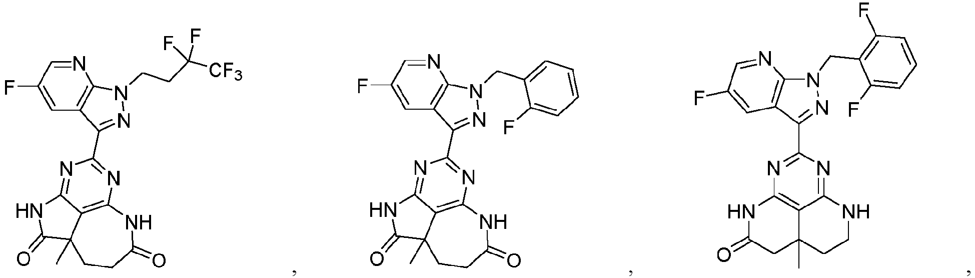

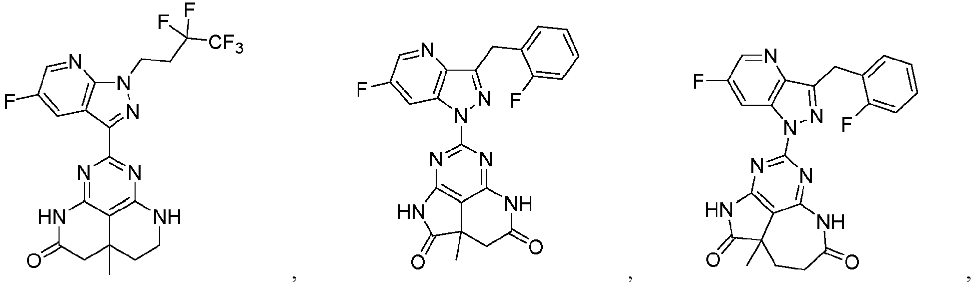

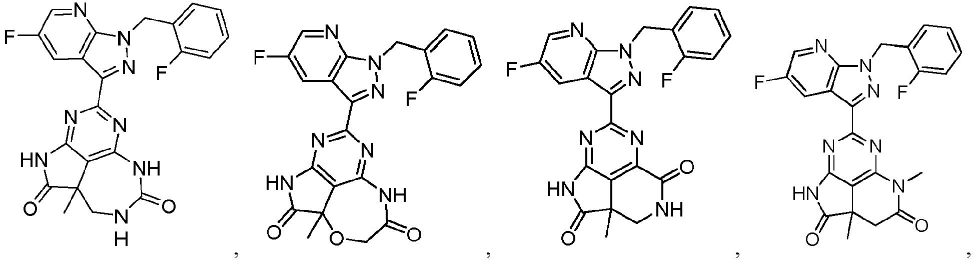

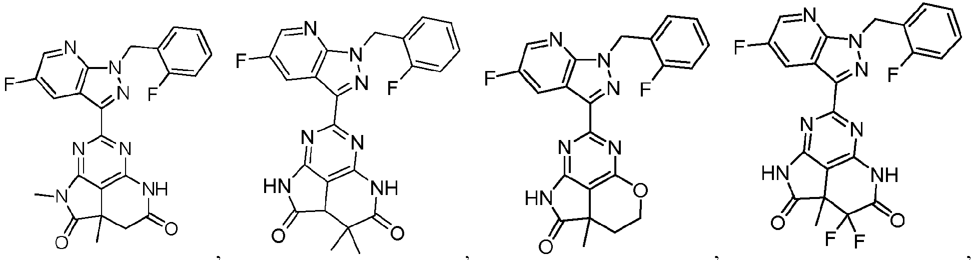

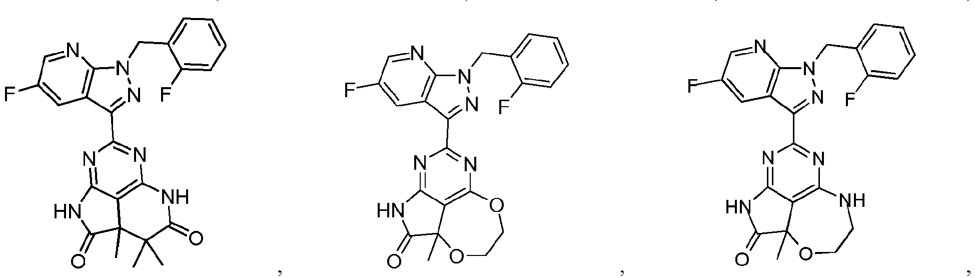

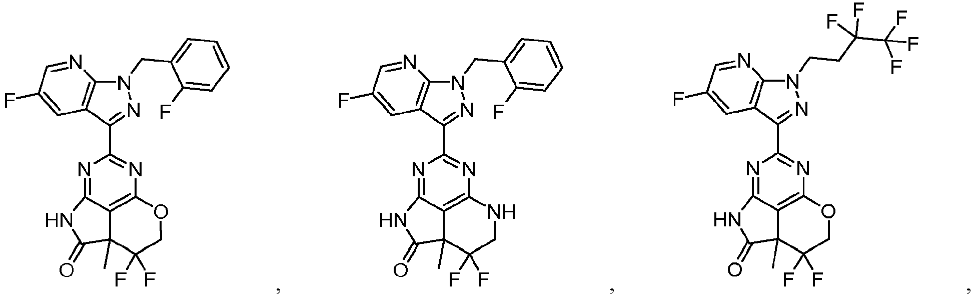

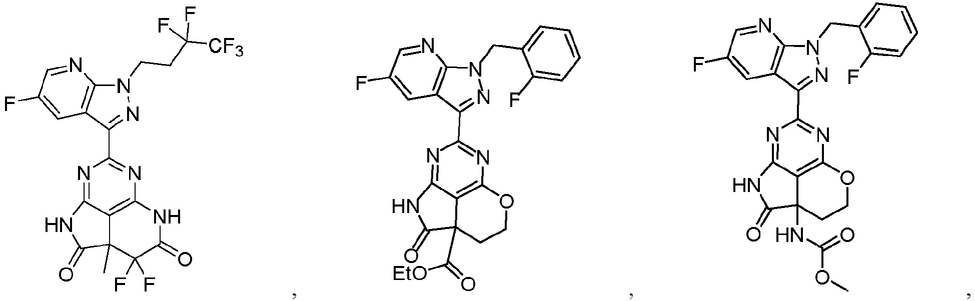

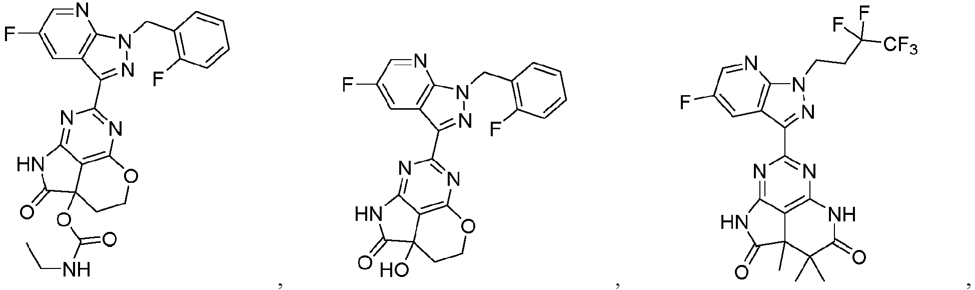

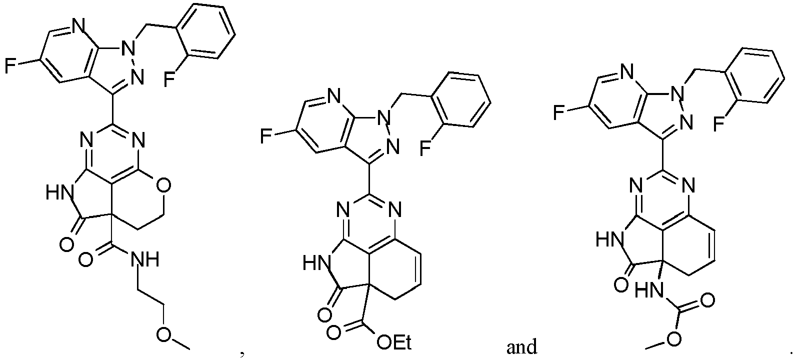

The present application also provides a compound, a stereoisomer thereof or a pharmaceutically acceptable salt thereof selected from the group consisting of:

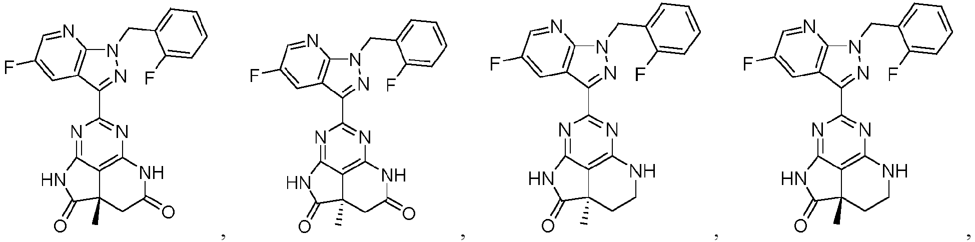

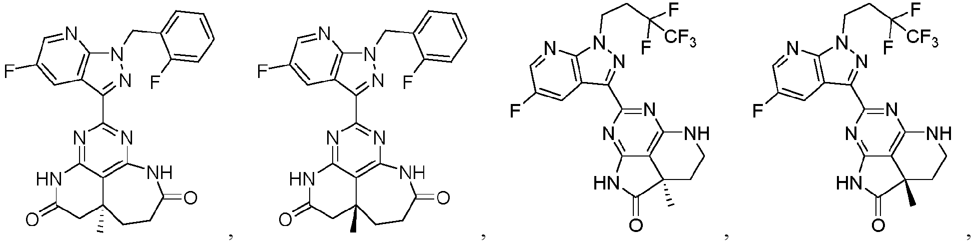

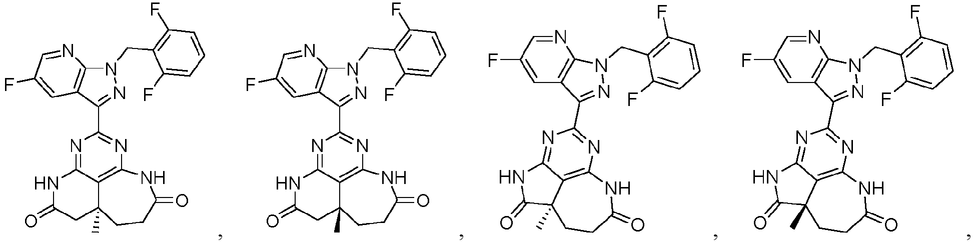

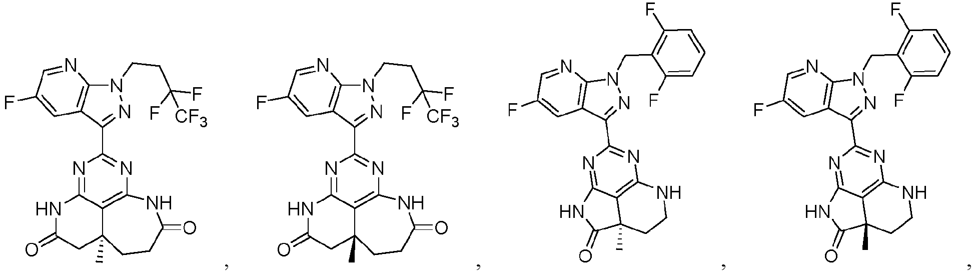

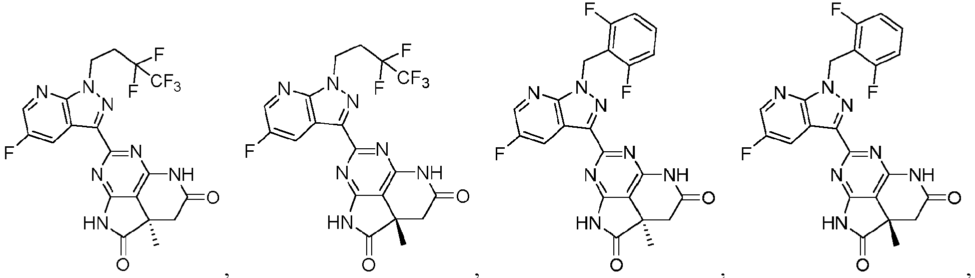

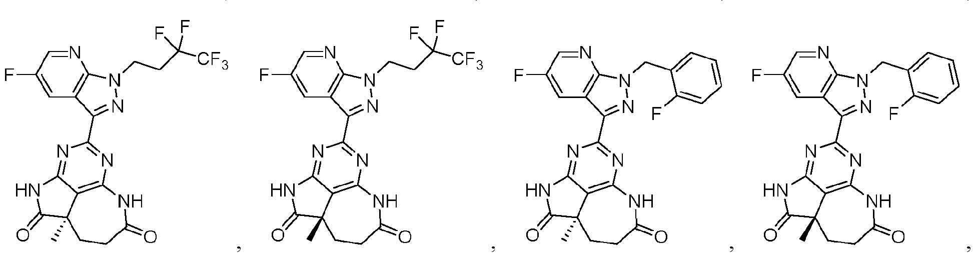

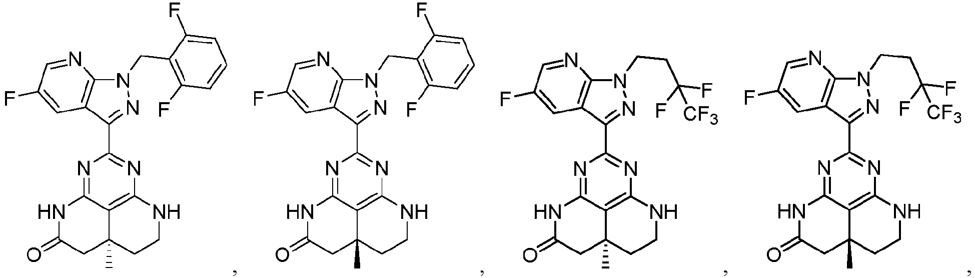

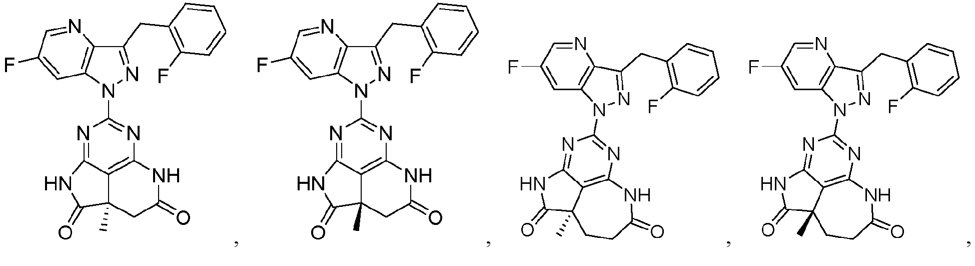

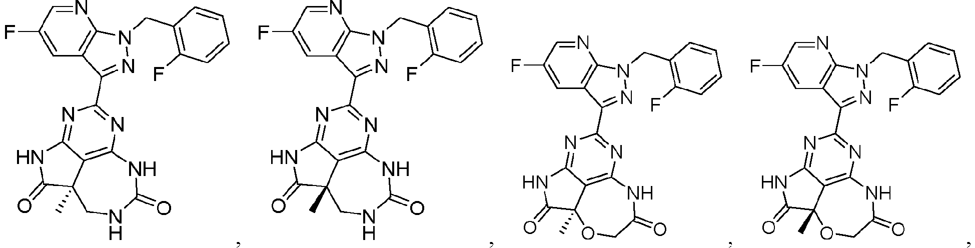

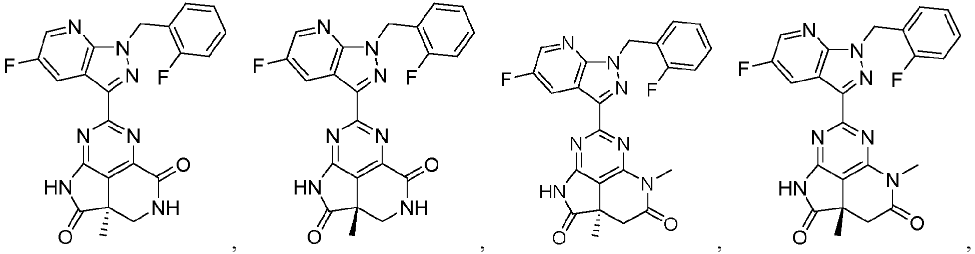

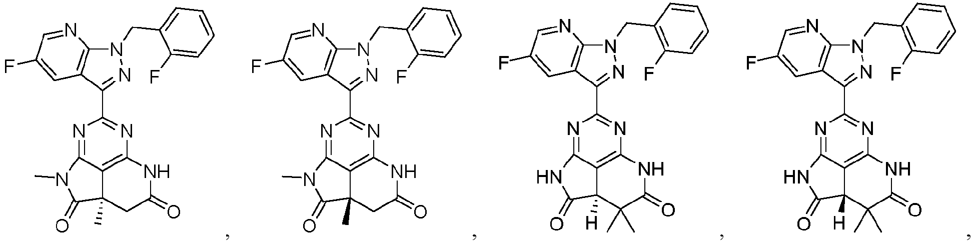

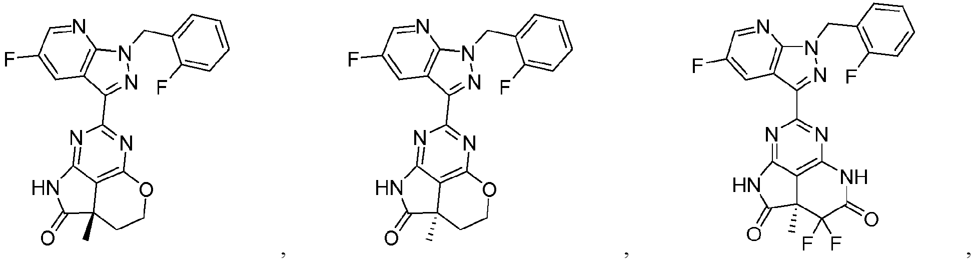

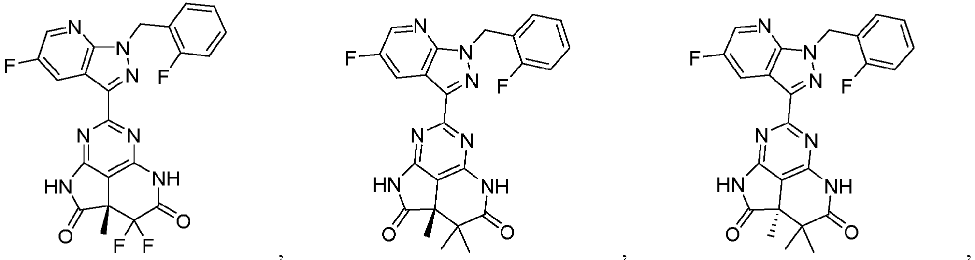

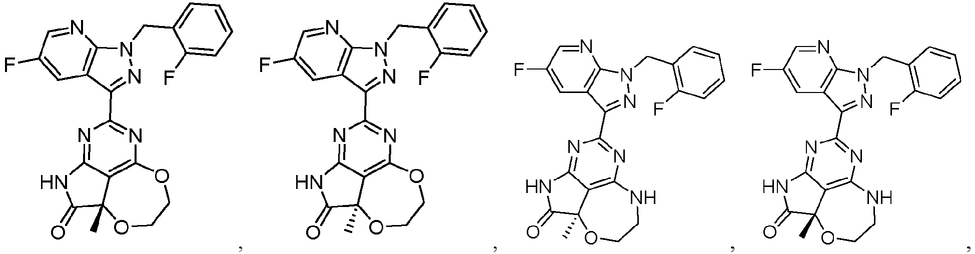

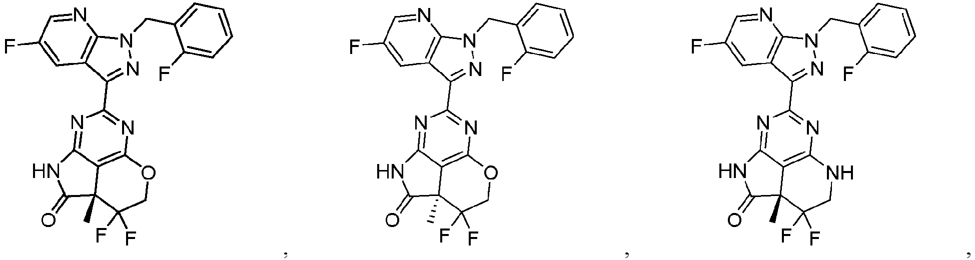

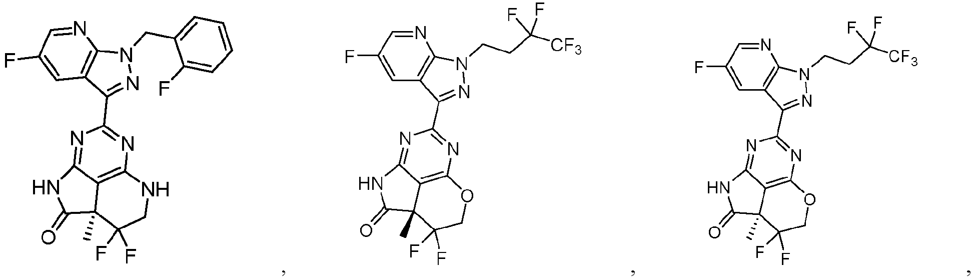

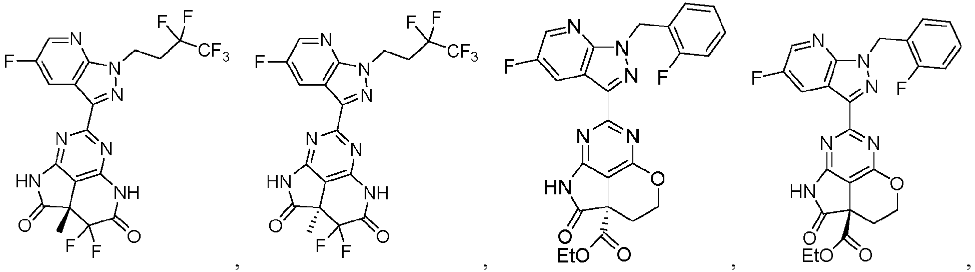

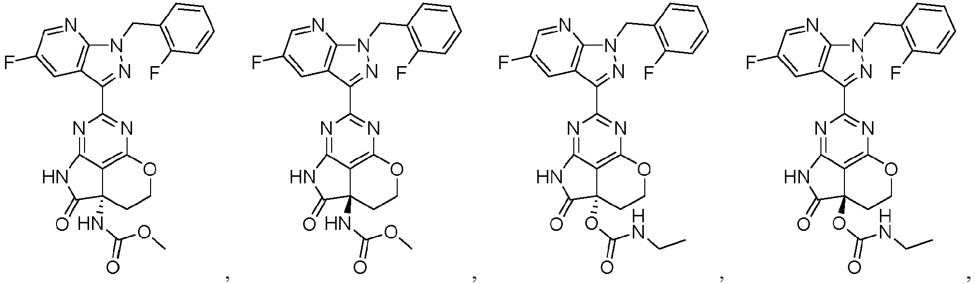

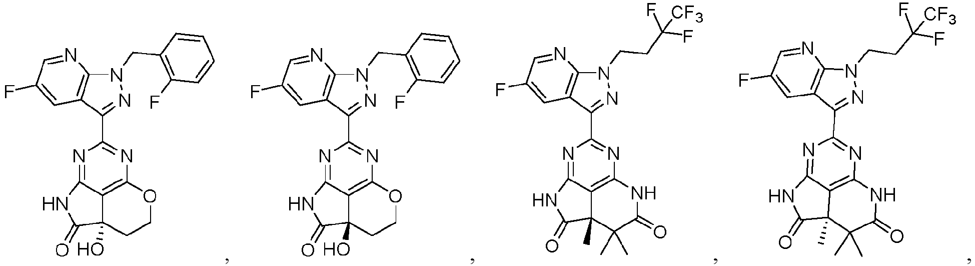

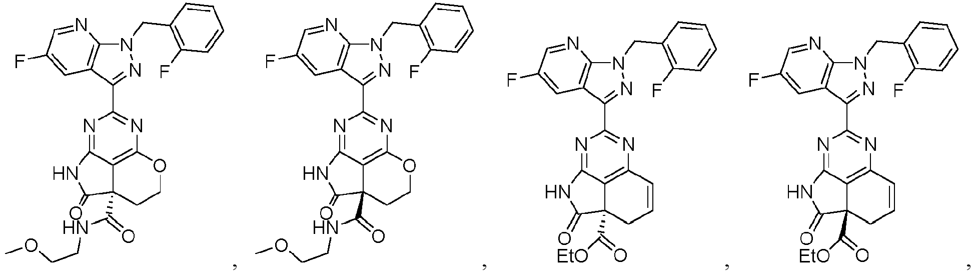

### AMENDMENTS FOR PROSECUTION IN THE EPO REGIONAL PHASE

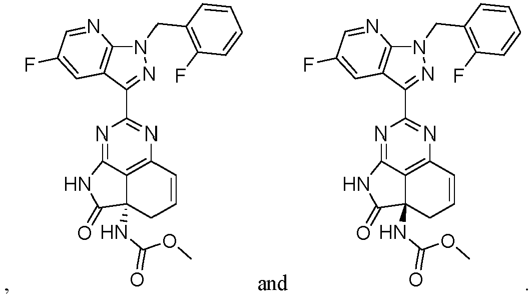

In another aspect, the present application provides a pharmaceutical composition comprising the compound, the stereoisomer thereof or the pharmaceutically acceptable salt thereof disclosed herein. In some embodiments, the pharmaceutical composition of the present application further comprises a pharmaceutically acceptable excipient.

In another aspect, the present application also provides use of the compound, the stereoisomer thereof or the pharmaceutically acceptable salt thereof, or the pharmaceutical composition thereof described above, for preparing a medicament for use in treating an sGC agonist-associated disease.

In another aspect, the present application describes but does not claim a method for treating an sGC agonist-associated disease in a mammal comprising administering to the mammal, preferably a human, in need of the treatment a therapeutically effective amount of the compound, the stereoisomer thereof, the pharmaceutically acceptable salt thereof or the pharmaceutical composition thereof disclosed herein.

In another aspect, the present application describes but does not claim use of the compound, the stereoisomer thereof, the pharmaceutically acceptable salt thereof or the pharmaceutical composition thereof disclosed herein in treating an sGC agonist-associated disease.

In another aspect, the present application provides the compound, the stereoisomer thereof, the pharmaceutically acceptable salt thereof or the pharmaceutical composition thereof disclosed herein for use in treating an sGC agonist-associated disease.

In some embodiments of the present application, the sGC agonist-associated disease is selected from the group consisting of heart failure and hypertension.

### Definitions and description

Unless otherwise stated, the following terms and phrases used herein are intended to have the following meanings. A particular term or phrase, unless otherwise specifically defined, should not be considered as uncertain or unclear but construed according to its common meaning. When referring to a trade name, it is intended to refer to its corresponding commercial product or its active ingredient.

The term "pharmaceutically acceptable" is used herein for those compounds, materials, compositions and/or dosage forms which are, within the sope of sound medical judgment, suitable for use in contact with the tissues of human beings and animals without excessive toxicity, irritation, allergic response, or other problems or complications, and commensurate with reasonable benefit risk ratio.

The term "pharmaceutically acceptable salt" refers to a salt of the compound of the present application, which is prepared from the compound having particular substituents discovered by the present application and a relatively nontoxic acid or base. When the compound disclosed herein contains a relatively acidic functional group, a base addition salt can be obtained by contacting the neutral form of such a compound with a sufficient amount of a base in a pure solution or a suitable inert solvent. When the compound disclosed herein contains a relatively basic functional group, an acid addition salt can be obtained by contacting the neutral form of such a compound with a sufficient amount of an acid in a pure solution or a suitable inert solvent. Certain specific compounds of the present application contain both basic and acidic functional groups that allow the compounds to be converted into either base or acid addition salts.

The pharmaceutically acceptable salts of the present application can be synthesized from a parent compound having an acidic or basic group using conventional chemical methods. In general, such salts are prepared by the following method: reacting the free acid or base form of the compound with a stoichiometric amount of the appropriate base or acid in water or an organic solvent or a mixture thereof.

Unless otherwise stated, the term "isomer" is intended to include geometric isomers, *cis-trans* isomers, stereoisomers, enantiomers, optical isomers, diastereoisomers and tautomers.

The compounds of the present application can occur in the form of a specific geometric isomer or stereoisomer. All such compounds are contemplated herein, including *cis* and *trans* isomers, (-)- and (+)- enantiomers, (R)- and (S)-enantiomers, diastereoisomers, (D)-isomers, (L)-isomers, and racemic mixtures and other mixtures thereof, such as an enantiomer or diastereoisomer enriched mixture, all of which are encompassed within the scope of the present application. Substituents such as alkyl may have an additional asymmetric carbon atom. All these isomers and mixtures thereof are encompassed within the scope of the present application.

Unless otherwise stated, the term "enantiomer" or "optical isomer" refers to stereoisomers that are mirror images of each other.

Unless otherwise stated, the term *"cis-trans* isomer" or "geometric isomer" results from the inability of a single bond of a ring carbon atom or a double bond to rotate freely.

Unless otherwise stated, the term "diastereoisomer" refers to stereoisomers in which molecules each have two or more chiral centers and are not mirror images of each other.

Unless otherwise stated, "(+)" stands for dextrorotation, "(-)" for levorotation and "(±)" for racemization.

Unless otherwise stated, the absolute configuration of a stereogenic center is represented by a wedged solid bond ( 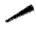 ) and a wedged dashed bond ( 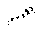 ), and the relative configuration of a stereogenic center is represented by a straight solid bond ( 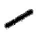 ) and a straight dashed bond ( 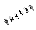 ). A wavy line ( 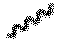 ) represents a wedged solid bond ( 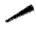 ) or a wedged dashed bond ( 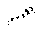 ), or a wavy line ( 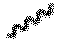 ) represents a straight solid bond ( 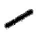 ) and a straight dashed bond ().

The compounds disclosed herein may be present in particular form. Unless otherwise stated, the term "tautomer" or "tautomeric form" means that different functional isomers are in dynamic equilibrium at room temperature and can be rapidly converted into each other. If tautomers are possible (e.g., in solution), the chemical equilibrium of the tautomers can be achieved. For example, a proton tautomer, also known as a prototropic tautomer, includes the interconversion by proton transfer, such as keto-enol isomerization and imine-enamine isomerization. A valence tautomer includes the interconversion by recombination of some bonding electrons. A specific example of the keto-enol tautomerization is the interconversion between tautomers pentane-2,4-dione and 4-hydroxypent-3-en-2-one.

Unless otherwise stated, the term "enriched with one isomer", "isomer enriched", "enriched with one enantiomer", or "enantiomer enriched" means that the content of one of the isomers or enantiomers is less than 100% and more than or equal to 60%, or more than or equal to 70%, or more than or equal to 80%, or more than or equal to 90%, or more than or equal to 95%, or more than or equal to 96%, or more than or equal to 97%, or more than or equal to 98%, or more than or equal to 99%, or more than or equal to 99.5%, or more than or equal to 99.6%, or more than or equal to 99.7%, or more than or equal to 99.8%, or more than or equal to 99.9%.

Unless otherwise stated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee) is 80%.

Optically active (R)- and (S)-isomers and D and L isomers can be prepared by chiral synthesis or chiral reagents or other conventional techniques. An enantiomer of a certain compound of the present application can be prepared by asymmetric synthesis or derivatization using a chiral auxiliary, wherein the resulting diastereoisomeric mixture is separated and the auxiliary group is cleaved so as to provide the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to give the pure enantiomer. Furthermore, the enantiomer and the diastereoisomer are generally isolated through chromatography using a chiral stationary phase, optionally in combination with chemical derivatization (e.g., carbamate generated from amines).

The compound of the present application may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted with deuterium to form a deuterated drug, and the bond formed by deuterium and carbon is firmer than that formed by common hydrogen and carbon. Compared with an un-deuterated drug, the deuterated drug has the advantages of reduced toxic side effects, increased stability, enhanced efficacy, prolonged biological half-life and the like. All isotopic variations of the compound described herein, whether radioactive or not, are encompassed within the scope of the present application.

Unless otherwise specified, the term "C₁₋₃ alkyl" refers to a linear or branched saturated hydrocarbon group consisting of 1 to 3 carbon atoms. The C₁₋₃ alkyl includes, but is not limited to, C₁₋₂ and C₂₋₃ alkyl, etc., and may be monovalent (e.g., methyl), divalent (e.g., methylene) or polyvalent (e.g., methenyl). Examples of C₁₋₃ alkyl include, but are not limited to, methyl (Me), ethyl (Et), propyl (including n-propyl and isopropyl), etc.

Unless otherwise specified, the term "halo" or "halogen", by itself or as part of another substituent, refers to a fluorine, chlorine, bromine or iodine atom.

The term "optional" or "optionally" means that the subsequently described event or circumstance may, but not necessarily, occur, and the description includes instances where the event or circumstance occurs and instances where it does not.

The term "substituted" means that one or more hydrogen atoms on a specific atom are substituted with substituents, wherein the substituents may include deuterium and hydrogen variants, as long as the valence of the specific atom is normal and the substituted compound is stable. When the substituent is an oxygen (i.e., =O), it means that two hydrogen atoms are substituted. Substitution with oxygen does not occur on aromatic groups. The term "optionally substituted" means that an atom can be substituted with a substituent or not. Unless otherwise specified, the type and number of the substituent may be arbitrary as long as being chemically achievable. The "when each of certain groups is optionally substituted" means that any of the groups in the structures described above is optionally substituted; for example, when "E₂ is selected from the group consisting of -(CH₂)ₙ-, -(CH₂)ₚC(O)-, -O(CH₂)_{q}-, - O(CH₂)ᵣC(O)-, -CH₂CH=CH- and -(CH₂)ₛNHC(O)-, wherein each of the CH₂ is optionally substituted with 1 or 2 R_{b}", it means that any CH₂ in the optional groups of E₂ is optionally substituted with 1 or 2 R_{b}.

When any variable (e.g., R) occurs more than once in the constitution or structure of a compound, the variable is independently defined in each case. Thus, for example, if a group is substituted by 0-2 R, the group can be optionally substituted by two R at most, and the definition of R in each case is independent. Furthermore, a combination of a substituent and/or a variant thereof is permissible only if the combination can result in a stable compound.

When the number of a linking group is 0, for example, -(CRR)₀-, it means that the linking group is a single bond.

When one of variables is selected from a single bond, it means that the two groups are linked directly. For example, in A-L-Z, when L represents a single bond, it means that the structure is actually A-Z.

When a substituent is absent, it means that there is no such a substituent in a structure. For example, when X is absent in A-X, it means that the structure is actually A. When the listed linking group does not indicate the direction for linking, the direction for linking is arbitrary. For example, when the linking group L contained in 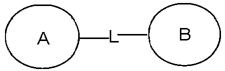 is -M-W-, -M-W- can either link ring A and ring B in a direction same as left-to-right reading order to form 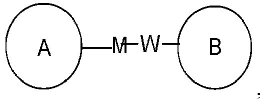 or link ring A and ring B in a direction opposite to the left-to-right reading order to form 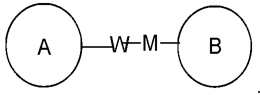 A combination of the linking group, a substituent and/or a variant thereof is permissible only if the combination can result in a stable compound.

Unless otherwise specified, Cₙ₋ₙ₊ₘ or Cₙ-Cₙ₊ₘ includes any one of the specific cases of n to n+m carbon atoms. For example, C₁₋₁₂ includes C₁, C₂, C₃, C₄, C₅, C₆, C₇, C₈, C₉, C₁₀, C₁₁ and C₁₂. Also, any range within n to n+m may be included. For example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.

The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The compound of the present application may be structurally confirmed using conventional methods well known to those skilled in the art; if the present application relates to the absolute configuration of the compound, the absolute configuration may be confirmed by means of conventional techniques in the art. For example, in the single crystal X-ray diffraction (SXRD) method, intensity data of diffraction of the single crystal grown are collected with a Bruker D8 venture diffractometer, the light source is CuKα radiation, and the scanning mode is φ/ω scanning; after related data are collected, the direct method (Shelxs97) is further employed to analyze the crystal structure, and thus the absolute configuration can be confirmed.

The solvents used in the present application are commercially available. The following abbreviations are used in the present application: aq for water; eq for equivalent; min for minute; M for mol/L; DCM for dichloromethane; PE for petroleum ether; DMF for *N*,*N-*dimethylformamide; DMSO for dimethyl sulfoxide; EtOAc for ethyl acetate; EtOH for ethanol; MeOH for methanol; CBz for benzyloxycarbonyl, an amine protecting group; Boc for *tert-*butyloxycarbonyl, an amine protecting group; r.t. for room temperature; O/N for overnight; THF for tetrahydrofuran; Boc₂O for di-tert-butyl dicarbonate; TFA for trifluoroacetic acid; DIPEA for diisopropylethylamine; SOCl₂ for thionyl chloride; IPAm for isopropylamine; and mp for melting point.

### Technical effects

The compound of the present application has significant stimulating activity for guanylate cyclase and has good pharmacokinetic properties including clearance, half-life and intragastric oral bioavailability.

### DETAILED DESCRIPTION

The present application is described in detail below by way of examples. However, this is by no means disadvantageously limiting the scope of the present application. Although the present application has been described in detail herein and specific embodiments have also been disclosed, it will be apparent to those skilled in the art that various changes and modifications can be made to the specific embodiments.

### Example 1

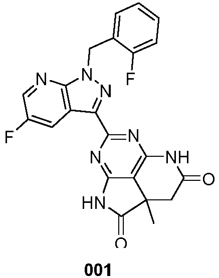

### Synthetic route:

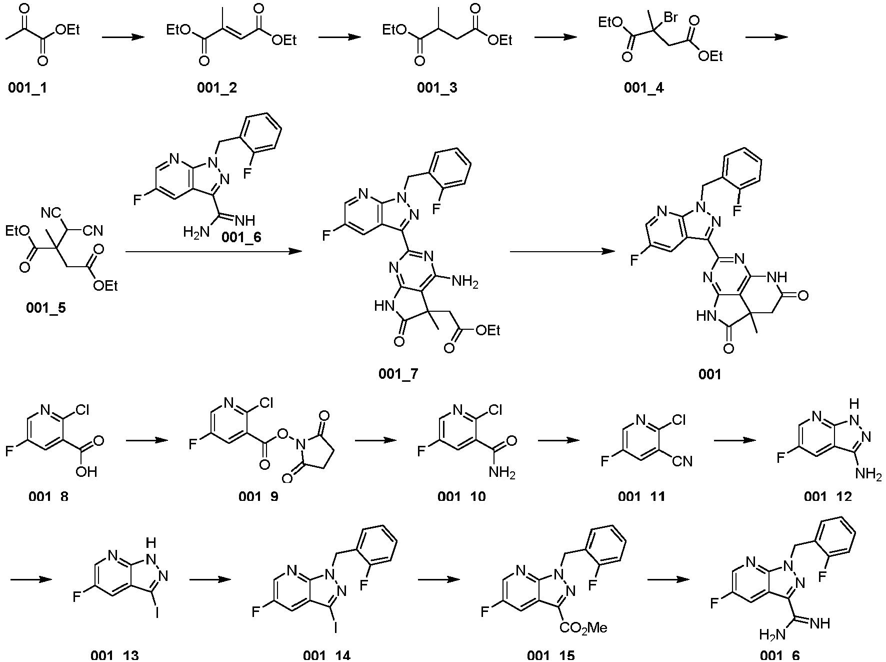

### Step 1: synthesis of compound 001_2

Ethoxyformylmethylene triphenylphosphine (112.51 g, 322.96 mmol) and compound 001_1 (25 g, 215.30 mmol) were dissolved in chloroform (250 mL) at room temperature under nitrogen atmosphere. The reaction mixture was heated to 70 °C and stirred for 12 h. After the reaction was completed, the reaction mixture was cooled to room temperature and concentrated under reduced pressure to remove the solvent, and methyl *tert-butyl* ether (100 mL) was added to the resulting residue. The resulting mixture was stirred for 10 min and filtered, and the filtrate was collected and concentrated under reduced pressure to remove the solvent. The resulting crude product was separated by column chromatography (eluent: petroleum ether/ethyl acetate = 1/0 to 50/1, v/v) to give compound 001_2. ¹H NMR (400 MHz, DMSO-d₆) δ: 6.64 (d, *J=* 1.6 Hz, 1H), 4.22-4.14 (m, 4H), 2.18 (d, *J=* 1.6 Hz, 3H), 1.24 (q, *J=* 7.2 Hz, 6H).

### Step 2: synthesis of compound 001_3

Compound **001_2** (23 g, 123.52 mmol) was dissolved in ethanol (150 mL) at room temperature, and wet palladium/carbon (15 g, 10% purity) was added. The reaction mixture was stirred under hydrogen atmosphere 103,421 kPa, 15 psi) at 15 °C for 12 h. After the reaction was completed, the reaction mixture was filtered. The filtrate was collected and concentrated under reduced pressure to remove the solvent. The resulting crude product was separated by column chromatography (eluent: petroleum ether/ethyl acetate = 1/0 to 20/1, v/v) to give compound **001_3.**

### Step 3: synthesis of compound 001_4

Compound **001_3** (8.6 g, 45.69 mmol) was dissolved in carbon tetrachloride (180 mL) at room temperature, and *N-*bromosuccinimide (8.13 g, 45.69 mmol) and benzoyl peroxide (553.38 mg, 2.28 mmol) were added. The reaction mixture was heated to 75 °C and stirred for 12 h. After the reaction was completed, the reaction mixture was cooled to room temperature and filtered, and the filtrate was collected and concentrated under reduced pressure to remove the solvent. The resulting crude product was separated by column chromatography (eluent: petroleum ether/ethyl acetate = 1/0 to 50/1, v/v) to give compound **001_4.** ¹H NMR (400 MHz, DMSO-d₆) δ: 4.23-4.13 (m, 2H), 4.12-4.00 (m, 2H), 3.31 (*q, J =* 16.8 Hz, 2H), 1.96 (s, 3H), 1.27-1.15 (m, 6H).

### Step 4: synthesis of compound 001_5

At room temperature, malononitrile (2.6 g, 39.31 mmol) was dissolved in tetrahydrofuran (50 mL), and potassium tert-butoxide (44.92 mL, 1 M in tetrahydrofuran) was added. The reaction mixture was stirred at 15 °C for 0.5 h. Compound **001_4** (10 g, 37.44 mmol) was dissolved in tetrahydrofuran (100 mL) at room temperature. The reaction mixture was warmed to 75 °C, and the reaction mixture described above was added dropwise with stirring. The reaction mixture was stirred at 75 °C for 12 h. After the reaction was completed, the reaction mixture was cooled to room temperature, adjusted to pH 3-4 with 2 M diluted aqueous hydrochloric acid solution, and then extracted with ethyl acetate (100 mL × 3). The organic phases were combined, washed with saturated brine (100 mL), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 0/1, v/v) to give compound **001_5**. ¹H NMR (400 MHz, DMSO-d₆) δ: 5.35 (s, 1H), 4.20 (q, *J=* 7.2 Hz, 2H), 4.10 (q, *J=* 7.2 Hz, 2H), 2.96-2.76 (m, 2H), 1.46 (s, 3H), 1.23-1.17 (m, 6H).

### Step 5: synthesis of intermediate 001_9

Compound **001_8** (250 g, 1.42 mol) was dissolved in acetonitrile (2.5 L) at room temperature under nitrogen atmosphere, and 1-hydroxypyrrolidine-2,5-dione (172.10 g, 1.50 mol), *N,N*-dicyclohexylmethane diimine (299.71 g, 1.45 mol) were added. The reaction mixture was stirred at 20 °C for 4 h. After the reaction was completed, the reaction mixture was filtered, and the filter cake was rinsed with acetonitrile (3 L). The filtrate was collected and concentrated under reduced pressure to remove the solvent to give compound **001_9.** ¹H NMR (400 MHz, CDCl₃) δ: 8.53 (d, J = 3.2 Hz, 1 H), 8.16 (dd, J = 3.0, 7.4 Hz, 1 H), 2.93 (s, 4 H).

### Step 6: synthesis of intermediate 001_10

Compound **001_9** (155 g, 568.56 mmol) was dissolved in tetrahydrofuran (300 mL) at room temperature under nitrogen atmosphere, and ammonium hydroxide (564.20 g, 4.02 mol, 25% purity) was added. The reaction mixture was stirred at 20 °C for 5 h. After the reaction was completed, ethyl acetate (300 mL × 3) was added to the reaction mixture for extraction, and the organic phases were combined, dried over anhydrous sodium sulfate, and filtered. The filtrate was concentrated under reduced pressure to remove the solvent to give intermediate compound **001_10.** ¹H NMR (400 MHz, DMSO-d₆) δ: 8.52 (d, *J =* 2.8 Hz, 1 H), 8.10 (s, 1 H), 7.99 (dd, *J =* 2.8, 8.0 Hz, 1 H), 7.88 (s, 1 H).

### Step 7: synthesis of intermediate 001_11

Compound **001_10** (99 g, 567.14 mmol) was dissolved in dichloromethane (200 mL) at room temperature under nitrogen atmosphere. The reaction mixture was cooled to 0 °C, and triethylamine (126.26 g, 1.25 mol) was added, followed by the slow, dropwise addition of trifluoroacetic anhydride (190.59 g, 907.42 mmol). The reaction mixture was stirred at 0 °C for 2 h. After the reaction was completed, saturated aqueous sodium bicarbonate solution was added to the reaction mixture to adjust the pH to 7-8. The aqueous phase was separated and extracted with dichloromethane (1500 mL × 3), and the organic phases were combined, dried over anhydrous sodium sulfate, and filtered. The filtrate was concentrated under reduced pressure, and the resulting crude product was separated by column chromatography (eluent: petroleum ether/ethyl acetate = 50/1 to 10/1, v/v) to give compound **001_11.**

### Step 8: synthesis of intermediate 001_12

Compound **001_11** (104 g, 664.35 mmol) was dissolved in n-butanol (1 L) at room temperature under nitrogen atmosphere, and hydrazine hydrate (339.59 g, 6.65 mol, 98% purity) was added. The reaction mixture was warmed to 120 °C and stirred for 2 h. After the reaction was completed, the reaction mixture was cooled to room temperature, and a solid precipitated. Water (1 L) was added to the reaction mixture. The resulting mixture was stirred for 1 h and filtered, and the filter cake was collected. The filter cake was concentrated under reduced pressure to remove the solvent to give intermediate **001_12.**

### Step 9: synthesis of intermediate 001_13

Compound **001_12** (137 g, 900.55 mmol) was dissolved in tetrahydrofuran (3 L) at room temperature under nitrogen atmosphere. The reaction mixture was cooled to 0 °C, and boron trifluoride diethyl etherate (383.44 g, 2.70 mol, 333.43 mL) was slowly added dropwise. The reaction mixture was cooled to -10 °C, and then a solution of isoamyl nitrite (137.14 g, 1.17 mol) in tetrahydrofuran (358 mL) was added slowly. The reaction mixture was stirred at - 10 °C for 1 h. Then methyl *tert*-butyl ether (3 L) was added to the reaction mixture, and a solid precipitated. The resulting mixture was filtered, and the filter cake was collected. The filter cake was added portionwise to a solution of sodium iodide (175.48 g, 1.17 mol) in acetone (3 L) at 0 °C. The reaction mixture was stirred at 20 °C for 1 h. After the reaction was completed, the reaction mixture was poured into ice water (3 L) and extracted with ethyl acetate (800 mL × 3), and the organic phases were combined, dried over anhydrous sodium sulfate, and filtered. The filtrate was concentrated under reduced pressure to give a crude product. The crude product was separated by column chromatography (eluent: petroleum ether/ethyl acetate = 20/1 to 10/1, v/v) to give intermediate **001_13.** ¹H NMR (400 MHz, DMSO_d₆) δ: 14.24 (s, 1 H), 8.60-8.64 (m, 1 H), 7.86 (dd, *J* = 2.6, 8.4 Hz, 1 H).

### Step 10: synthesis of intermediate 001_14

Compound **001_13** (97 g, 368.81 mmol) was dissolved in N,N-dimethylformamide (1 L) at room temperature, and 2-fluorobenzyl chloride (53.32 g, 368.81 mmol) and cesium carbonate (132.18 g, 405.69 mmol) were added. The reaction mixture was warmed to 80 °C and stirred for 2 h. After the reaction was completed, the reaction mixture was cooled to room temperature, and semi-saturated brine (1 L) was added to the reaction mixture. The resulting mixture was stirred for 10 min, and then ethyl acetate (1.2 L) was added. The organic phase was separated and collected, washed with semi-saturated brine (1 L × 3), dried over anhydrous sodium sulfate, and filtered, and the filtrate was concentrated under reduced pressure to remove the solvent. The resulting crude product was dispersed in methyl *tert*-butyl ether (240 mL). The resulting dispersion was stirred for 30 min and filtered, and the filter cake was collected. The filter cake was concentrated under reduced pressure to remove the solvent to give intermediate **001_14.** ¹H NMR (400 MHz, DMSO_d₆) δ: 8.65-8.73 (m, 1 H), 7.91 (dd, *J* = 2.8, 8.0 Hz, 1 H), 7.32-7.40 (m, 1 H), 7.11-7.25 (m, 3 H), 5.72 (s, 2 H).

### Step 11: synthesis of intermediate 001_15

Compound **001_14** (25 g, 67.36 mmol) was dissolved in N,N-dimethylformamide (750 mL) and methanol (250 mL) at room temperature, and triethylamine (30.72 g, 303.63 mmol) and cyclopenta-2,4-dien-1-yl(diphenyl)phosphino ferrocene dichloropalladium dichloromethane (3.45 g, 4.72 mmol) were added. The reaction mixture was purged 3 times with carbon monoxide and reacted at 80 °C under carbon monoxide atmosphere 103,421 kPa, 15 psi) for 12 h. After the reaction was completed, the reaction mixture was concentrated under reduced pressure to remove the solvent, and the resulting crude product was dissolved in methyl *tert-butyl* ether (200 mL). The resulting solution was stirred for 30 min and filtered, and the filter cake was collected and concentrated under reduced pressure to remove the solvent to give intermediate **001_15.** ¹H NMR (400 MHz, DMSO_d₆) δ: 8.74-8.80 (m, 1 H), 8.25 (dd, *J* = 2.8, 8.4 Hz, 1 H), 7.34-7.41 (m, 1 H), 7.20-7.31 (m, 2 H), 7.13-7.20 (m, 1 H), 5.84 (s, 2 H), 3.92 (s, 3 H).

### Step 12: synthesis of hydrochloride of compound 001_6

Ammonium chloride (33.51 g, 626.52 mmol) was suspended in toluene (580 mL) at room temperature, and a solution of trimethylaluminum (2 M, 300.73 mL) in toluene was added. The reaction mixture was heated to 80 °C, and then compound **001_15** (38 g, 125.30 mmol) was added. The reaction mixture was stirred at 80 °C for 30 min, and then warmed to 110 °C and stirred for 1.5 h. The reaction mixture was cooled to 20-40 °C, and methanol (73.02 mL) was added, followed by the addition of diluted hydrochloric acid (3 M, 801.94 mL) (at below 40 °C). The reaction mixture was warmed to 80 °C and stirred for 30 min, then cooled to 0 °C and stirred for 30 min, and filtered, and the filter cake was collected. The filter cake was rinsed with water (85 mL) and concentrated under reduced pressure to remove the solvent to give the hydrochloride of intermediate **001_6.** ¹H NMR (400 MHz, DMSO_d₆) δ: 9.40-9.78 (d, J = 24.4 Hz, 4 H), 8.85 (m, 1 H), 8.57 (dd, J = 2.4, 8.8 Hz, 1 H), 7.35-7.43 (m, 1 H), 7.28-7.35 (m, 1 H), 7.20-7.28 (m, 1 H), 7.13-7.20 (m, 1 H), 5.89 (s, 2 H).

### Step 13: synthesis of compound 001_7

The hydrochloride of compound **001_6** (450 mg, 1.39 mmol) was dissolved in tert-butanol (13 mL) at room temperature, and compound **001_5** (554.03 mg, 2.20 mmol) and potassium carbonate (768.46 mg, 5.56 mmol) were added. The reaction mixture was heated to 80 °C and stirred for 12 h. After the reaction was completed, the reaction mixture was cooled to room temperature, and water (20 mL) was added to the reaction mixture, followed by the extraction with ethyl acetate (20 mL × 3). The organic phases were combined, washed with saturated brine (20 mL), dried over anhydrous sodium sulfate, and filtered, and the filtrate was concentrated under reduced pressure to remove the solvent. The resulting crude product was separated by column chromatography (eluent: dichloromethane/ethanol = 1/0 to 20/1, v/v) to give compound **001_7.**

### Step 14: synthesis of compound 001

Compound **001_7** (600 mg, 1.22 mmol) was dissolved in toluene (12 mL) at room temperature, and trimethylaluminum (1.82 mL, 2 M in toluene) was added. The reaction mixture was heated to 80 °C and stirred for 12 h. After the reaction was completed, the reaction mixture was cooled to room temperature, and methanol (738.05 µL) was added dropwise at below 40 °C, followed by the dropwise addition of 3 N diluted aqueous hydrochloric acid solution (825.80 µL). The mixture was warmed to 80 °C and stirred for 10 min. Then the reaction mixture was cooled to 0-5 °C and stirred for 30 min, and filtered, and the filter cake was collected, rinsed with water (5 mL), and concentrated under reduced pressure to remove the solvent. The resulting crude product was dissolved in dimethyl sulfoxide (6 mL). The filtrate was separated by preparative HPLC (mobile phase: acetonitrile/water; neutral system: 10 mM NH₄HCO₃), and the resulting crude product was separated by preparative HPLC (mobile phase: acetonitrile/water; acidic system: 0.04% HCl) to give compound **001.** MS-ESI m/z: 448.1 [M+H]⁺. ¹H NMR (400 MHz, DMSO-d₆) δ: 11.36 (s, 1H), 11.12 (s, 1H), 8.77-8.66 (m, 2H), 7.41-7.33 (m, 1H), 7.28-7.20 (m, 2H), 7.18-7.13 (m, 1H), 5.84 (s, 2H), 2.99 (d, *J* = 15.6 Hz, 1H), 2.54 (d, *J =* 2.0 Hz, 1H), 1.38 (s, 3H).

### Example 2

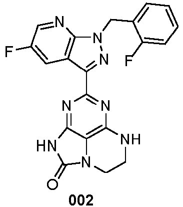

### Synthetic route:

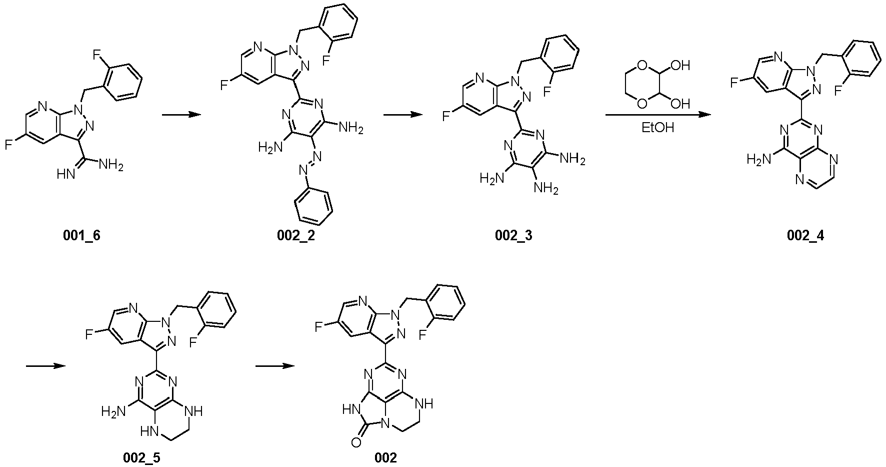

### Step 1: synthesis of compound 002_2

The hydrochloride of compound **001_6** (8.5 g, 26.26 mmol) was dissolved in N,N-dimethylformamide (40 mL) at room temperature, and triethylamine (3.99 g, 39.39 mmol) was added. The reaction mixture was warmed to 85 °C, and a solution of compound E-2-(phenylazo)malononitrile (8.94 g, 52.52 mmol) in N,N-dimethylformamide (40 mL) was added dropwise. The reaction mixture was stirred at 100 °C for 4 h, and then cooled to 25 °C and stirred for 12 h. After the reaction was completed, the reaction mixture was filtered, and the filter cake was rinsed successively with water (30 mL) and methanol (15 mL) and collected to give compound **002_2.**

### Step 2: synthesis of compound 002_3

Wet palladium on carbon (6 g, 10% purity) was placed in a preliminarily dried reaction flask at 25 °C, and then N,N-dimethylformamide (90 mL) was added for wetting, followed by the addition of compound **002_2** (12 g, 26.23 mmol). The reaction mixture was purged three times with hydrogen and stirred under hydrogen atmosphere 103,421 kPa, 15 psi) for 12 h. After the reaction was completed, the reaction mixture was filtered, and the filter cake was rinsed with methanol (150 mL × 3). The organic phases were combined and concentrated under reduced pressure to remove the solvent. Ethanol (30 mL) was added to the resulting crude product. The resulting mixture was stirred for 30 min and filtered, and the filter cake was collected and concentrated under reduced pressure to remove the solvent to give compound **002_3.** ¹H NMR (400 MHz, DMSO-*d*₆) δ: 8.85 (dd, J= 2.8, 8.8 Hz, 1H), 8.62 (s, 1H), 7.95 (s, 1H), 7.39-7.31 (m, 1H), 7.26-7.10 (m, 2H), 5.86 (s, 2H), 5.74 (s, 1H), 4.04 (s, 1H), 2.91-2.87 (m, 1H), 2.89 (s, 1H), 2.73 (s, 2H).

### Step 3: synthesis of compound 002_4

Compound **002_3** (1.8 g, 4.89 mmol) was dissolved in ethanol (40 mL) at room temperature, and 1,4-dioxane-2,3-diol (1.17 g, 9.77 mmol) was added. The reaction mixture was stirred at 15 °C for 12 h, supplemented with 1,4-dioxane-2,3-diol (0.6 g), and stirred at 15 °C for 12 h. After the reaction was completed, the reaction mixture was concentrated under reduced pressure to remove the solvent, and methyl *tert-butyl* ether (20 mL) was added to the resulting residue. The resulting mixture was stirred for 10 min and filtered, and the filtrate was collected and concentrated under reduced pressure to remove the solvent to give compound **002_4.** ¹H NMR (400 MHz, DMSO-d₆) δ: 9.10 *(d, J =* 1.6 Hz, 1H), 8.90 (dd, *J* = 2.8, 8.8 Hz, 1H), 8.81 (*d, J =* 2.0 Hz, 1H), 8.77-8.73 (m, 1H), 8.61 (s, 1H), 8.46 (s, 1H), 7.43-7.33 (m, 1H), 7.30-7.21 (m, 2H), 7.19-7.14 (m, 1H), 5.88 (s, 2H).

### Step 4: synthesis of hydrochloride of compound 002_5

Compound **002_4** (1.3 g, 3.33 mmol) was dissolved in ethanol (60 mL) at room temperature. The reaction mixture was cooled to 0 °C, and sodium borohydride (1.26 g, 3.330 mmol) was added. The reaction mixture was stirred at 15 °C for 0.5 h. Then the reaction mixture was cooled to 0 °C, supplemented with sodium borohydride (377.99 mg, 9.99 mmol), and stirred at 15 °C for 2 h. After the reaction was completed, the reaction mixture was cooled to 0 °C, then adjusted to pH 3-4 by adding dropwise concentrated hydrochloric acid, and concentrated under reduced pressure to remove the solvent. The resulting residue was separated by preparative HPLC (mobile phase: acetonitrile/water; acidic system: 0.05% HCl) to give the hydrochloride of compound **002_5.**

### Step 5: synthesis of compound 002

The hydrochloride of compound **002_5** (58 mg, 134.62 µmol, HCl) was dissolved in tetrahydrofuran (2 mL) at room temperature under nitrogen atmosphere. The reaction mixture was cooled to 0 °C, and a solution of methyl *p-*nitrophenyl chloroformate (27.13 mg, 134.62 µmol) in tetrahydrofuran (1 mL) was added dropwise at 0 °C. The reaction mixture was stirred at 0 °C for 2 h. Then N,N-diisopropylethylamine (34.80 mg, 269.24 µmol) was added, and the reaction mixture was stirred at 15 °C for 2 h. The reaction system was concentrated under reduced pressure to give an oil. *N,N-dimethylformamide* (1 mL) was added, and the reaction system was purged three times with nitrogen, warmed to 120 °C and stirred for 12 h, and then warmed to 140 °C and stirred for 16 h. After the reaction was completed, the reaction mixture was cooled to room temperature and dissolved in dimethyl sulfoxide. Then the resulting mixture was separated by preparative HPLC (mobile phase: acetonitrile/water; acidic system: 0.05% HCl) to give compound **002**. MS-ESI m/z: 421.1 [M+H]⁺. ¹H NMR (400 MHz, DMSO-d₆) δ: 11.39 (s, 1H), 8.69 (d, *J =* 1.6 Hz, 1H), 8.64 (dd, *J* = 2.6, 8.8 Hz, 1H), 7.67 (s, 1H), 7.40-7.31 (m, 1H), 7.27-7.20 (m, 2H), 7.18-7.11 (m, 1H), 5.79 (s, 2H), 3.84 (t, *J =* 5.0 Hz, 2H), 3.60 (s, 2H).

### Example 3

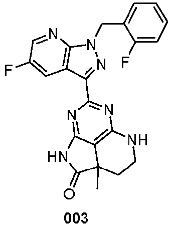

### Synthetic route:

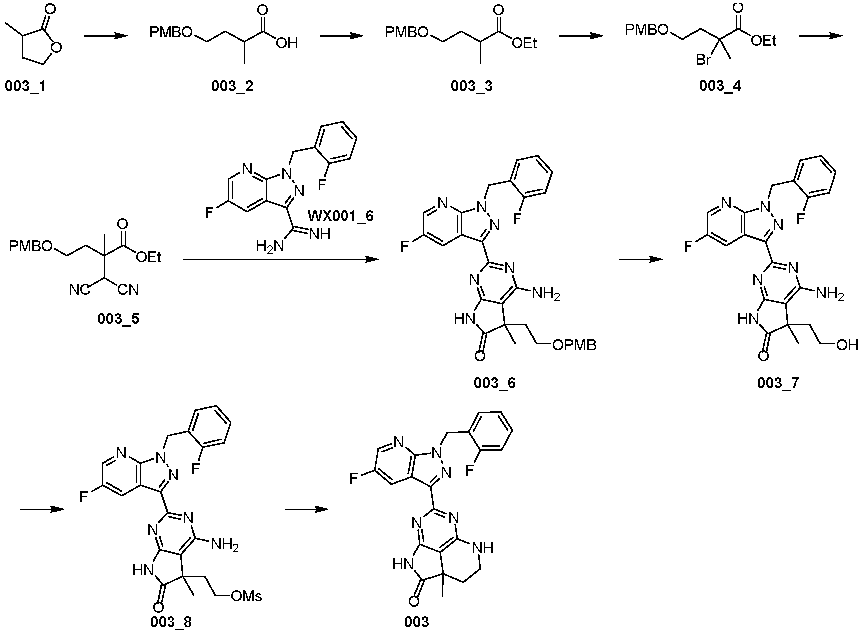

### Step 1: synthesis of compound 003_2

Compound **003_1** (50 g, 499.42 mmol), *p*-methoxybenzyl chloride (312.86 g, 2.00 mol, 272.05 mL) and potassium hydroxide (140.11 g, 2.50 mol) were dissolved in toluene (900 mL) at room temperature under nitrogen atmosphere. The reaction mixture was warmed to 110 °C and stirred for 12 h, and then concentrated under reduced pressure to remove toluene, and the resulting residue was dissolved in methanol (1 L), followed by the addition of a solution of potassium hydroxide (49.88 g, 888.97 mmol) in water (500 mL). Then the reaction mixture was warmed to 85 °C and stirred for 12 h. After the reaction was completed, the reaction mixture was cooled to room temperature and concentrated under reduced pressure to remove the solvent. Water (800 mL) was added to the residue, and the resulting mixture was washed with methyl *tert-butyl* ether (500 mL × 3). The organic phase was discarded. The aqueous phase was adjusted to pH 2 with 12 N concentrated hydrochloric acid and then extracted with dimethyltetrahydrofuran (800 mL × 2). The organic phases were combined, washed with saturated brine (800 mL × 2), dried over anhydrous sodium sulfate, and filtered, and the filtrate was concentrated under reduced pressure to remove the solvent to give compound **003_2**. ¹H NMR (400 MHz, CDCl₃) δ: 7.29 (s, 2H), 6.90 (d, *J* = 8.8 Hz, 2H), 4.41-4.52 (m, 2H), 3.83 (s, 3H), 3.55 (t, *J =* 6.0 Hz, 2H), 2.65-2.76 (m, 1H), 2.01-2.12 (m, 1H), 1.69-1.79 (m, 1H), 1.23 (d, *J=* 7.2 Hz, 3H).

### Step 2: synthesis of compound 003_3

Compound **003_2** (117 g, 491.02 mmol) was dissolved in dichloromethane (1 L) at room temperature under nitrogen atmosphere, and then ethanol (226.21 g, 4.91 mol), 1-(3-dimethylaminopropyl)-3-acetaldehyde hydrochloride (112.95 g, 589.23 mmol) and 4-dimethylaminopyridine (7.20 g, 58.92 mmol) were successively added. The reaction mixture was stirred at 20 °C for 12 h and then concentrated under reduced pressure to remove the solvent. The resulting residue was diluted with ethyl acetate (100 mL) and then washed with water (100 mL) followed by 10% aqueous citric acid solution (150 mL × 3) and saturated brine (100 mL × 2). The organic phase was dried over anhydrous sodium sulfate and filtered, and the filtrate was concentrated under reduced pressure to remove the solvent. The resulting crude product was separated by column chromatography (eluent: petroleum ether/ethyl acetate = 1/0 to 2/3, v/v) to give compound **003_3**. ¹H NMR (400 MHz, CDCl₃) δ: 7.26 (d, *J =* 8.8 Hz, 2H), 6.88 (d, *J=* 8.8 Hz, 2H), 4.42 (s, 2H), 4.11 (q, *J =* 7.2 Hz, 2H), 3.81 (s, 3H), 3.519-3.432 (m, 2H), 2.68-2.57 (m, 1H), 2.07-1.95 (m, 1H), 1.69 (dd, *J* = 6.4, 14.0 Hz, 1H), 1.24 (t, *J* = 7.2 Hz, 3H), 1.17 (d, *J=* 7.2 Hz, 3H).

### Step 3: synthesis of compound 003_4

Diisopropylamine (16.93 g, 167.27 mmol) was added to anhydrous tetrahydrofuran (450 mL) at 20 °C under nitrogen atmosphere. The reaction mixture was cooled to -78 °C, and then a solution of n-butyllithium in tetrahydrofuran (72.99 mL, 2.5 M in tetrahydrofuran) was slowly added dropwise. The reaction mixture was warmed to 0 °C and stirred for 0.5 h, and then cooled to -78 °C. Then compound **003_3** (40.5 g, 152.07 mmol) was dissolved in anhydrous tetrahydrofuran (160 mL), and the resulting solution was added dropwise to the reaction mixture described above. The reaction mixture was stirred at -78 °C for 1 h. Then carbon tetrabromide (75.64 g, 228.10 mmol) was dissolved in anhydrous tetrahydrofuran (220 mL), and the resulting solution was added dropwise to the reaction system. The reaction mixture was warmed to 20 °C and stirred for 10.5 h. After the reaction was completed, the reaction mixture was poured into an aqueous solution of ammonium chloride (1.5 L) and extracted with ethyl acetate (600 mL × 2). The organic phases were combined, washed with saturated brine (800 mL), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 8/1, v/v) to give compound **003_4**.

### Step 4: synthesis of compound 003_5

Potassium tert-butoxide (191.18 mL, 1 M in tetrahydrofuran) was added to a solution of malononitrile (11.48 g, 173.80 mmol) in tetrahydrofuran (100 mL) at 20 °C under nitrogen atmosphere. Then the reaction mixture was warmed to 75 °C. A solution of compound **003_4** (30 g, 86.90 mmol) in tetrahydrofuran (250 mL) was added dropwise to the reaction mixture described above. The reaction mixture was stirred at 75 °C for 12 h. After the reaction was completed, the reaction mixture was concentrated under reduced pressure to remove the solvent, and the resulting residue was diluted with water (100 mL), adjusted to pH 6 with 1 N diluted hydrochloric acid, and then extracted with 2-methyltetrahydrofuran (200 mL × 3). The organic phases were combined, washed with saturated brine (200 mL), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 6/1, v/v) to give compound **003_5**.

### Step 5: synthesis of compound 003_6

The hydrochloride of compound **001_6** (3.76 g, 13.09 mmol) was dissolved in *tert*-butanol (40 mL) at room temperature under nitrogen atmosphere, and compound **003_5** (6.83 g, 20.68 mmol) and potassium carbonate (5.95 g, 43.06 mmol) were added. The reaction mixture was warmed to 80 °C and stirred for 12 h. After the reaction was completed, the reaction mixture was cooled to room temperature and concentrated under reduced pressure to remove the solvent. The residue was diluted with water (100 mL) and ethyl acetate (100 mL). The organic phase was separated and collected, and the aqueous phase was extracted with ethyl acetate (100 mL × 3). The organic phases were combined, washed with saturated brine (100 mL × 2), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 1/1, v/v) to give compound **003_6.**

### Step 6: synthesis of compound 003_7

Compound **003_6** (3 g, 5.25 mmol) was dissolved in dichloromethane (30 mL) and water (3 mL) at room temperature under nitrogen atmosphere. The reaction mixture was cooled to 0 °C, and 2,3-dichloro-5,6-dicyan-p-benzoquinone (2.38 g, 10.50 mmol) was added. The reaction mixture was stirred at 20 °C for 12 h. After the reaction was completed, the reaction mixture was poured into water (50 mL) and extracted with ethyl acetate (40 mL × 3). The organic phases were combined, washed with semi-saturated brine (50 mL), dried over anhydrous sodium sulfate, and filtered, and the filtrate was concentrated under reduced pressure to remove the solvent. The resulting crude product was separated by reversed-phase MPLC (mobile phase: methanol/aqueous hydrochloric acid solution = 2:1 to 1:2, v/v) to give compound **003_7**. ¹H NMR (400 MHz, DMSO-d₆) δ: 11.02 (s, 1H), 8.91-8.69 (m, 2H), 7.42-7.32 (m, 1H), 7.29-7.10 (m, 4H), 5.83 (s, 2H), 3.23-3.01 (m, 2H), 2.37-2.25 (m, 1H), 2.01-1.87 (m, 1H), 1.32 (s, 3H).

### Step 7: synthesis of compound 003_8

Compound **003_7** (330 mg, 676.38 µmol) was dissolved in anhydrous tetrahydrofuran (3 mL) at room temperature under nitrogen atmosphere, and diisopropylethylamine (349.66 mg, 2.71 mmol) and 4-dimethylaminopyridine (8.26 mg, 67.64 µmol) were added. Methanesulfonyl chloride (116.22 mg, 1.01 mmol) was added dropwise to the reaction mixture at 0 °C. The reaction mixture was stirred at 0 °C for 2 h. After the reaction was completed, the reaction mixture was poured into saturated aqueous sodium bicarbonate solution (20 mL) at 0 °C and extracted with ethyl acetate (30 mL × 3). The organic phases were combined, washed with semi-saturated brine (30 mL × 2), dried over anhydrous sodium sulfate, and filtered, and the filtrate was concentrated under reduced pressure to remove the solvent. The resulting crude product was separated by column chromatography (eluent: 2-methyltetrahydrofuran/ethyl acetate = 1/0 to 4/1, v/v) to give compound **003_8**.

### Step 8: synthesis of compound 003

Compound **003_8** (240 mg, 453.24 mmol) was dissolved in tetrahydrofuran (6 mL) at room temperature. The reaction mixture was cooled to -65 °C, and lithium bis(trimethylsilyl)amide (1.36 mL, 1 M in tetrahydrofuran) was added. The reaction mixture was stirred at -65 °C to 15 °C for 12 h. After the reaction was completed, 3 M diluted aqueous hydrochloric acid solution was added dropwise to the reaction mixture until the pH = 5-6. Then water (5 mL) was added, followed by the extraction with ethyl acetate (25 mL × 3). The organic phases were combined, washed with saturated brine (3 mL), dried over anhydrous sodium sulfate, and filtered, and the filtrate was concentrated under reduced pressure to remove the solvent. The resulting crude product was separated by preparative HPLC (mobile phase: acetonitrile/water; neutral system: 10 mM NH₄HCO₃) to give compound 003. MS-ESI m/z: 434.2 [M+H]⁺. ¹H NMR (400 MHz, DMSO-d₆) δ: 10.82 (s, 1H), 8.81-8.58 (m, 2H), 7.64 (s, 1H), 7.32-7.41 (m, 1H), 7.29-7.09 (m, 3H), 5.81 (s, 2H), 3.53-3.43 (m, 1H), 1.84 (d, *J =* 12.0 Hz, 1H), 1.39-1.19 (m, 4H).

### Example 4

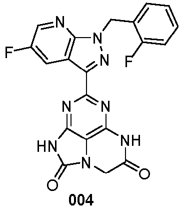

### Synthetic route:

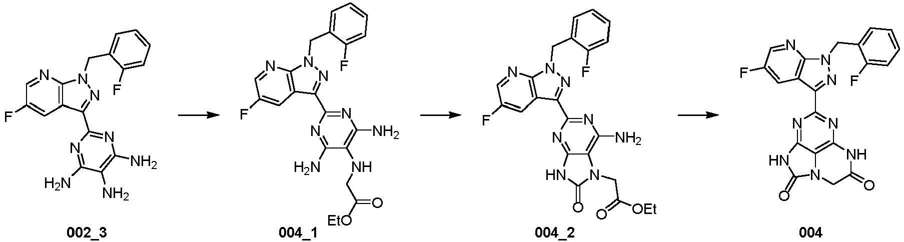

### Step 1: synthesis of compound 004_1

Compound **002_3** (1 g, 2.71 mmol) was dissolved in N,N-dimethylformamide (30 mL) at room temperature under nitrogen atmosphere, and ethyl bromoacetate (476.05 mg, 2.85 mmol) and *N,N*-diisopropylethylamine (350.87 mg, 2.71 mmol) were added. The reaction mixture was stirred at 15 °C for 8 h, and then warmed to 50 °C and stirred for 4 h. After the reaction was completed, the reaction mixture was cooled to room temperature. Semi-saturated brine (30 mL) was added to the reaction system, followed by the extraction with ethyl acetate (50 mL × 3). The organic phases were combined, washed with semi-saturated brine (30 mL × 3), dried over anhydrous sodium sulfate, and filtered, and the filtrate was concentrated under reduced pressure to remove the solvent. The resulting crude product was separated by column chromatography (eluent: dichloromethane/methanol = 1/0 to 10/1, v/v) to give compound **004_1.**

### Step 2: synthesis of compound 004_2

Compound **004_1** (0.6 g, 1.32 mmol) was dissolved in THF (10 mL) at room temperature under nitrogen atmosphere. The reaction mixture was cooled to 0 °C, and triphosgene (129.30 mg, 435.71 µmol) and triethylamine (400.81 mg, 3.96 mmol) were added. The reaction mixture was stirred at 0 °C for 0.5 h, and then warmed to 25 °C and reacted for another 2 h. After the reaction was completed, the reaction mixture was quenched with saturated aqueous sodium bicarbonate solution (15 mL) and diluted with ethyl acetate (20 mL). The organic phase was separated and collected, and the aqueous phase was extracted with ethyl acetate (30 mL × 3). The organic phases were combined, washed with saturated brine (50 mL × 2), dried over anhydrous sodium sulfate, and filtered, and the filtrate was concentrated under reduced pressure to remove the solvent. The resulting crude product was separated by column chromatography (eluent: dichloromethane/methanol = 1/0 to 10/1, v/v) to give compound **004_2.**

### Step 3: synthesis of compound 004

Compound **004_2** (0.58 g, 1.21 mmol) was dissolved in acetic acid (8 mL) and xylene (8 mL) at room temperature under nitrogen atmosphere. The reaction mixture was warmed to 160 °C, and the reaction system was stirred at 160 °C for 24 h. After the reaction was completed, the reaction mixture was cooled to room temperature and concentrated under reduced pressure to remove the solvent. The resulting crude product was separated by preparative HPLC (mobile phase: acetonitrile/water; acidic system: 0.04% HCl) to give compound **004.** MS-ESI m/z: 435.1 [M+H]⁺. ¹H NMR (400 MHz, DMSO-d₆) δ: 11.90 (s, 1H), 11.70 (s, 1H), 8.78-8.59 (m, 2H), 7.37 (q, *J* = 7.2 Hz, 1H), 7.32-7.12 (m, 3H), 5.81 (s, 2H), 4.59 (s, 2H).

### Examples 5 and 6

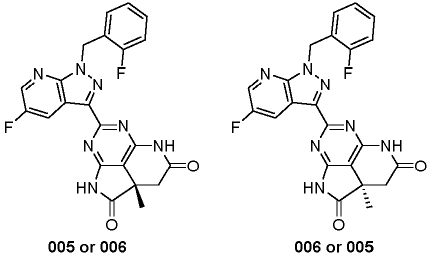

### Synthetic route:

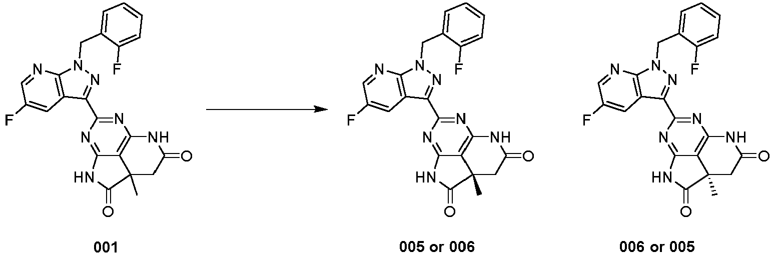

### Synthesis of compounds 005 and 006

Compound **001** (200 mg, 447.03 µmol) was purified by chiral column chromatography (column model: DAICEL CHIRALCEL OJ (250 mm × 30 mm, 10 µm); mobile phase: [Neu-ACN]; B(ACN)%: 50%-50%, 8 min) to give compounds **005** and **006.**

**005** (retention time: 1.80 min): MS-ESI m/z: 448.1 [M+H]⁺. ¹H NMR (400 MHz, DMSO-d₆) δ: 11.37 (s, 1H), 11.13 (s, 1H), 8.87-8.61 (m, 2H), 7.41-7.34 (m, 1H), 7.29-7.19 (m, 2H), 7.19-7.14 (m, 1H), 5.84 (s, 2H), 3.00 (d, *J* = 15.6 Hz, 1H), 2.53 (s, 1H), 1.37 (s, 3H).

**006** (retention time: 1.93 min): MS-ESI m/z: 448.0 [M+H]⁺. ¹H NMR (400 MHz, DMSO-d₆) δ: 11.37 (s, 1H), 11.13 (s, 1H), 8.87-8.61 (m, 2H), 7.41-7.34 (m, 1H), 7.29-7.19 (m, 2H), 7.19-7.13 (m, 1H), 5.84 (s, 2H), 3.00 (d, *J* = 15.6 Hz, 1H), 2.53 (s, 1H), 1.37 (s, 3H).

### Example 7

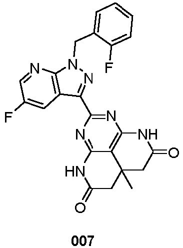

### Synthetic route:

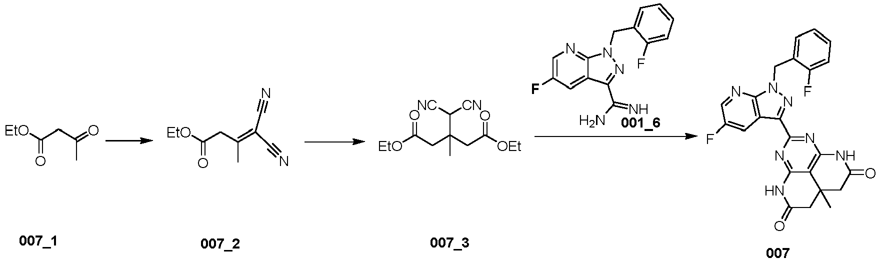

### Step 1: synthesis of compound 007_2

Compound **007_1** (1 g, 7.68 mmol, 970.87 µL) was dissolved in toluene (20 mL) at room temperature under nitrogen atmosphere, and malononitrile (507.61 mg, 7.68 mmol), glacial acetic acid (461.44 mg, 7.68 mmol) and ammonium acetate (592.30 mg, 7.68 mmol) were added. The reaction mixture was stirred at 20 °C for 12 h, supplemented with malononitrile (101.52 mg, 1.54 mmol), and warmed to 50 °C and stirred for 12 h. After the reaction was completed, the reaction mixture was poured into water (50 mL), and ethyl acetate (50 mL) was added for dilution. The organic phase was separated and collected, and the aqueous phase was extracted with ethyl acetate (50 mL × 3). The organic phases were combined, washed with saturated brine (50 mL × 2), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 10/1, v/v) to give compound 007_2. ¹H NMR (400 MHz, DMSO-d₆) δ: 4.14 (q, *J =* 7.2 Hz, 2H), 3.74 (s, 2H), 2.34 (s, 3H), 1.21 (t, *J =* 7.2 Hz, 3H).

### Step 2: synthesis of compound 007_3

Ethyl bromoacetate (1.87 g, 11.22 mmol) was dissolved in anhydrous tetrahydrofuran (12 mL) at room temperature under nitrogen atmosphere, and compound **007_2** (500 mg, 2.81 mmol), titanocene dichloride (72.69 mg, 280.60 µmol) and activated zinc powder (366.97 mg, 5.61 mmol) were added. The reaction mixture was stirred at 25 °C for 12 h. After the reaction was completed, the reaction system was quenched with 1 N diluted hydrochloric acid (15 mL) and diluted with ethyl acetate (25 mL). The organic phase was separated and collected, and the aqueous phase was extracted with ethyl acetate (25 mL × 3). The organic phases were combined, washed with saturated brine (30 mL × 3), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 6/1, v/v) to give compound **007_3.** ¹H NMR (400 MHz, CDCl₃) δ: 5.00 (s, 1H), 4.17 (d, *J =* 7.2 Hz, 4H), 2.75 (s, 4H), 1.43 (s, 3H), 1.29 (t, *J =* 7.2 Hz, 6H).

### Step 3: synthesis of compound 007

Compound **007_3** (24 mg, 90.13 µmol) was dissolved in tert-butanol (1 mL) at room temperature under nitrogen atmosphere, and the hydrochloride of **001_6** (29.18 mg, 90.13 µmol) and potassium carbonate (24.91 mg, 180.25 µmol) were added. The reaction mixture was stirred at 85 °C for 12 h. After the reaction was completed, the reaction mixture was cooled to room temperature and concentrated under reduced pressure to remove the solvent. The resulting crude product was separated by preparative HPLC (mobile phase: acetonitrile/water; acidic system: 0.04% HCl) to give compound **007.** MS-ESI m/z: 462.1 [M+H]⁺. ¹H NMR (400 MHz, DMSO-d₆) δ: 11.25 (s, 2H), 9.00 (dd, *J* = 2.8, 8.8 Hz, 1H), 8.77-8.63 (m, 1H), 7.41-7.32 (m, 1H), 7.28-7.09 (m, 3H), 5.82 (s, 2H), 2.80 *(d, J =* 16.0 Hz, 2H), 2.54 (d, *J =* 15.6 Hz, 2H), 1.12 (s, 3H).

### Example 8

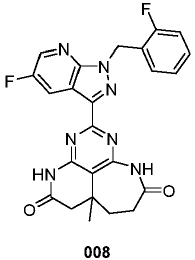

### Synthetic route

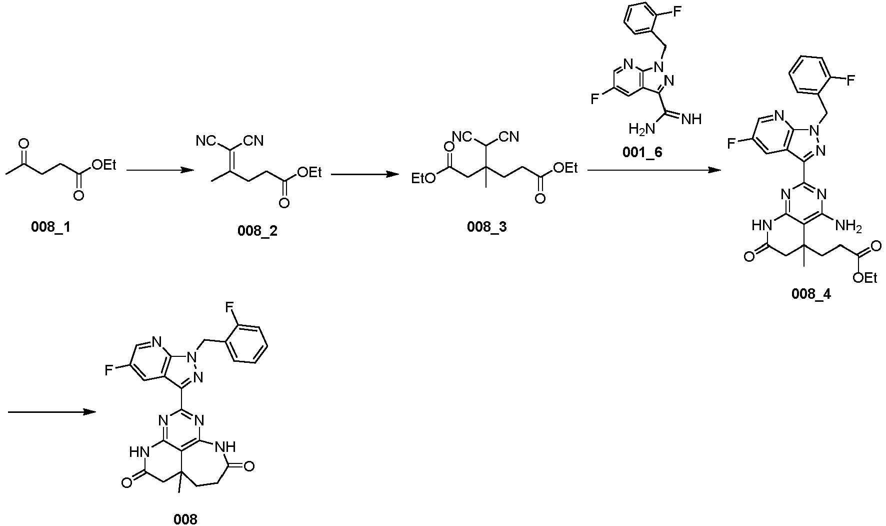

### Step 1: synthesis of compound 008_2

Compound **008_1** (10 g, 69.36 mmol) was dissolved in anhydrous toluene (200 mL) at room temperature, and malononitrile (5.04 g, 76.30 mmol), glacial acetic acid (4.17 g, 69.36 mmol) and ammonium acetate (5.35 g, 69.36 mmol) were added. The reaction mixture was stirred at 15 °C for 12 h. After the reaction was completed, water (200 mL) was added to the reaction mixture. Then the reaction mixture was adjusted to pH 3-4 with 3 M diluted hydrochloric acid and then extracted with ethyl acetate (200 mL × 3). The organic phases were combined, washed with saturated brine (100 mL), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 3/1, v/v) to give compound **008_2**. ¹H NMR (400 MHz, DMSO-d₆) δ: 4.08 (q, *J =* 7.2 Hz, 2H), 2.84-2.76 (m, 2H), 2.72-2.64 (m, 2H), 2.26 (s, 3H), 1.19 (t, *J =* 6.8 Hz, 3H).

### Step 2: synthesis of compound 008_3

Ethyl bromoacetate (1.66 g, 9.97 mmol) was dissolved in anhydrous tetrahydrofuran (25 mL) at room temperature under nitrogen atmosphere, and compound **008_2** (958 mg, 4.98 mmol), titanocene dichloride (129.11 mg, 498.40 µmol) and activated zinc powder (651.81 mg, 9.97 mmol) were added. The reaction mixture was stirred at 25 °C for 12 h. After the reaction was completed, the reaction mixture was quenched with 1 N diluted hydrochloric acid (20 mL), and water (50 mL) and ethyl acetate (50 mL) were added. The organic phase was separated and collected, and the aqueous phase was extracted with ethyl acetate (50 mL × 3). The organic phases were combined, washed with saturated brine (50 mL × 2), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 3/1, v/v) to give compound **008_3**.

### Step 3: synthesis of compound 008_4

Compound **008_3** (100 mg, 356.74 µmol) and the hydrochloride of compound **001_6** (57.74 mg, 178.37 µmol) were dissolved in tert-butanol (3.5 mL) at room temperature under nitrogen atmosphere, and 1,8-diazabicycloundec-7-ene (67.89 mg, 445.92 µmol) was added. The reaction mixture was heated to 85 °C and stirred for 12 h. After the reaction was completed, the reaction mixture was cooled to room temperature and concentrated under reduced pressure to give a crude product. Two batches of the crude product were combined, diluted with ethyl acetate (25 mL) and water (15 mL), and extracted with ethyl acetate (25 mL × 3). The organic phases were combined, washed with saturated brine (35 mL × 2), dried over anhydrous sodium sulfate, and filtered, and the filtrate was concentrated under reduced pressure to remove the solvent. The resulting crude was separated by preparative thin-layer chromatography (developing solvent: DCM:MeOH = 15:1, v/v) to give compound **008_4**.

### Step 4: synthesis of compound 008

Intermediate **008_4** (42 mg, 80.53 µmol) was dissolved in anhydrous toluene (1 mL) at room temperature under nitrogen atmosphere, and a solution of trimethylaluminum (2.5 M, 96.64 µL) in toluene was added. The reaction mixture was heated to 110 °C and stirred for 12 h. After the reaction was completed, the reaction mixture was cooled to room temperature, diluted with 1 N diluted hydrochloric acid (1 mL) and methanol (5 mL), and stirred at 25 °C for 0.5 h. The solvent was removed by concentration under reduced pressure, and the resulting crude product was separated by preparative HPLC (mobile phase: acetonitrile/water; acidic system: 0.04% HCl) to give crude compound **008.** The crude product was separated again by preparative HPLC (mobile phase: acetonitrile/water; acidic system: 0.04% HCl) to give compound **008.** MS-ESI m/z: 476.2 [M+H]⁺. ¹H NMR (400 MHz, DMSO-d₆) δ: 11.21 (s, 1H), 10.57 (s, 1H), 9.32 (dd, *J =* 2.8, 9.2 Hz, 1H), 8.70 (s, 1H), 7.35 (d, *J =* 3.2 Hz, 1H), 7.28-7.19 (m, 1H), 7.17-7.08 (m, 2H), 5.84 (s, 2H), 2.77-2.65 (m, 2H), 2.54 (s, 2H), 2.01 (*d, J =* 10.8 Hz, 1H), 1.89 (s, 1H), 1.19 (s, 3H).

### Example 9

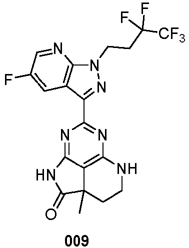

### Synthetic route:

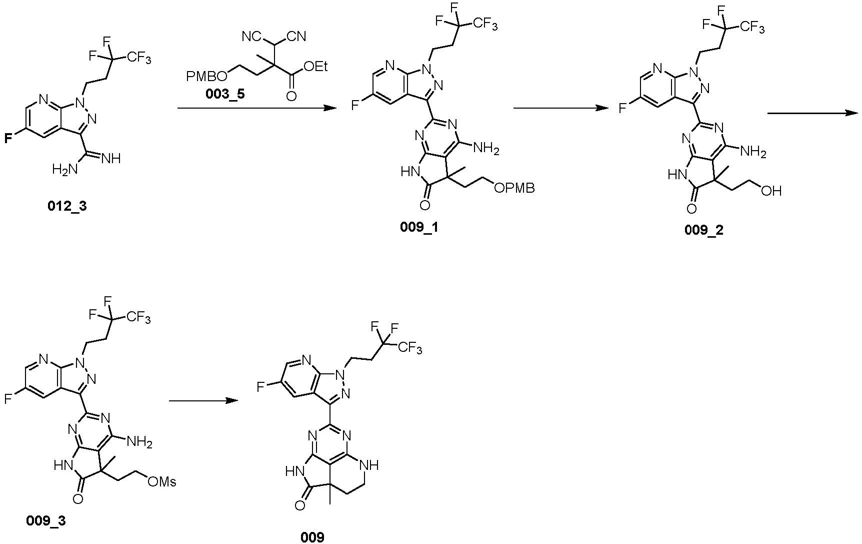

### Step 1: synthesis of intermediate 009_1

Compound **012_3** (4.2 g, 11.61 mmol, hydrochloride) was dissolved in tert-butanol (84 mL) at room temperature, and then potassium carbonate (6.42 g, 46.45 mmol) and **003_5** (6.14 g, 18.58 mmol) were added. The reaction mixture was warmed to 80 °C and stirred for 12 h. After the reaction was completed, the reaction mixture was cooled to room temperature, quenched with water (200 mL), and extracted with ethyl acetate (200 mL). The organic phases were combined, dried over anhydrous sodium sulfate, and filtered, and the filtrate was concentrated under reduced pressure to remove the solvent. The resulting residue was separated by column chromatography (eluent: petroleum ether/ethyl acetate = 1/0 to 3/2, v/v) to give compound **009_1.**

### Step 2: synthesis of intermediate 009_2

Compound **009_1** (2.4 g, 3.94 mmol) was dissolved in dichloromethane (12 mL) and water (1.2 mL) at room temperature. The reaction mixture was cooled to 0 °C, and then dichlorodicyanobenzoquinone (1.07 g, 4.73 mmol) was added slowly. The reaction mixture was stirred at room temperature for 12 h. After the reaction was completed, a saturated aqueous sodium bicarbonate solution (30 mL) was added, followed by the extraction with dichloromethane (50 mL). The organic phases were combined, dried over anhydrous sodium sulfate, and filtered, and the filtrate was concentrated under reduced pressure to remove the solvent. The resulting residue was separated by column chromatography (eluent: petroleum ether/ethyl acetate = 1/0 to 1/4, v/v) to give compound **009_2.**

### Step 3: synthesis of intermediate 009_3

**009_2** (1.08 g, 2.04 mmol, 93% purity) was dissolved in tetrahydrofuran (20 mL) and dichloromethane (20 mL) at 0 °C, and then methanesulfonyl chloride (234.08 mg, 2.04 mmol), N,N-diethylacetamide (792.30 mg, 6.13 mmol) and 4-dimethylaminopyridine (24.96 mg, 204.34 µmol) were added. The reaction mixture was warmed to room temperature and stirred for 1 h. After the reaction was completed, the reaction mixture was quenched with saturated aqueous sodium bicarbonate solution (30 mL) and extracted with ethyl acetate (40 mL). The organic phases were combined, dried over anhydrous sodium sulfate, and filtered, and the filtrate was concentrated under reduced pressure to remove the solvent. The resulting residue was separated by column chromatography (eluent: petroleum ether/ethyl acetate = 1/0 to 1/4, v/v) to give compound **009_3**.

### Step 4: synthesis of intermediate 009

Compound **009_3** (500 mg, 2.73 mmol) was dissolved in tetrahydrofuran (5 mL) at room temperature. Then the resulting solution was cooled to -78 °C, and a solution of lithium bis(trimethylsilyl)amide (2.73 mL, 1 M, 2.73 mmol) in tetrahydrofuran was added. The reaction mixture was warmed to 25 °C, and then hexamethylphosphoric triamide (489.98 mg, 2.73 mmol) was added. The reaction mixture was stirred at 25 °C for 12 h. After the reaction was completed, the reaction mixture was quenched with saturated aqueous ammonium chloride solution (20 mL) and extracted with ethyl acetate (40 mL). The organic phases were combined, dried over anhydrous sodium sulfate, and filtered, and the filtrate was concentrated under reduced pressure to remove the solvent. The resulting residue was separated by preparative HPLC (mobile phase: acetonitrile/water; neutral system: 10 mM NH₄HCO₃) to give compound **009.** MS-ESI m/z: 472.0 [M+H]⁺. ¹H NMR (400 MHz, DMSO_*d₆*) δ: 8.74-8.68 (s, 1H), 8.68-8.63 (m, 1H), 7.67-7.59 (m, 1H), 4.91-4.80 (m, 2H), 3.58-3.42 (m, 2H), 3.05-2.89 (m, 2H), 1.89-1.82 (m, 1H), 1.37-1.33 (m, 3H), 1.32-1.23 (m, 1H).

### Example 10

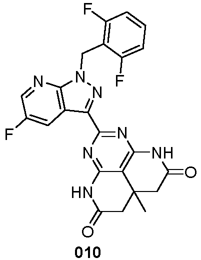

### Synthetic route:

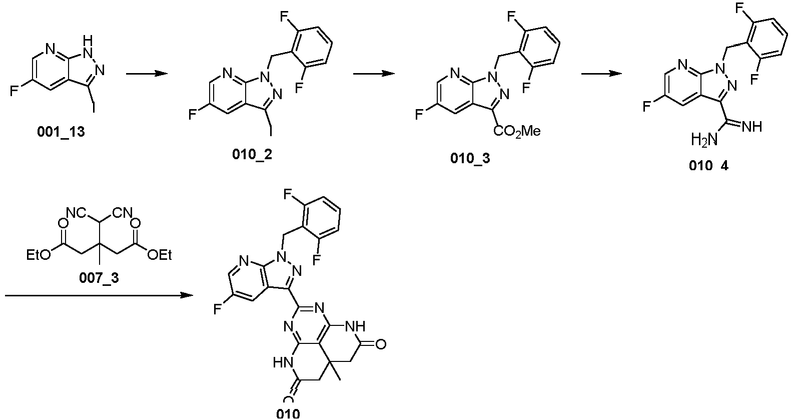

### Step 1: synthesis of compound 010_2

Compound **001_13** (10 g, 38.02 mmol) was dissolved in N,N-dimethylformamide (20 mL) at room temperature, and then cesium carbonate (37.16 g, 114.06 mmol) and 2,6-difluorobenzyl bromide (7.87 g, 38.02 mmol) were added. The reaction mixture was warmed to 80 °C and stirred for 2 h. After the reaction was completed, the reaction mixture was cooled to room temperature, quenched with water (200 mL), and extracted with ethyl acetate (200 mL). The organic phase was dried over anhydrous sodium sulfate, and filtered, and the filtrate was concentrated under reduced pressure to give a crude product. The crude product was separated by column chromatography (eluent: petroleum ether/ethyl acetate = 1/0 to 3/2, v/v) to give compound **010_2.**

### Step 2: synthesis of compound 010_3

Compound **010_2** (8.5 g, 21.84 mmol) was dissolved in N,N-dimethylformamide (100 mL) and methanol (40 mL) at room temperature, and then 1,1-bis(diphenylphosphino)ferrocene palladium dichloride (1.12 g, 1.53 mmol) was added. The reaction mixture was stirred under carbon monoxide atmosphere 103,421 kPa, 15 psi) at 80 °C for 12 h. After the reaction was completed, the reaction mixture was cooled to room temperature and filtered, and the filtrate was concentrated under reduced pressure to remove the solvent. The resulting crude product was separated by column chromatography (eluent: petroleum ether/ethyl acetate = 1/0 to 7/3, v/v) to give compound **010_3**.

### Step 3: synthesis of hydrochloride of compound 010_4

Ammonium chloride (298.04 mg, 5.57 mmol) was suspended in toluene (4 mL) at room temperature under nitrogen atmosphere, and a solution of trimethylaluminum (2.67 mL, 2 M, 5.35 mmol) in toluene was added. The reaction mixture was warmed to 80 °C, and then compound **010_3** (358 mg, 1.11 mmol) was added. The reaction mixture was stirred at 80 °C for 0.5 h, and then warmed to 110 °C and stirred for 1.5 h. After the reaction was completed, the reaction mixture was cooled to 30 °C. Methanol (639.34 µL, 16.05 mmol) and 3 N diluted aqueous hydrochloric acid solution (7.13 mL, 21.34 mmol) was added. Then the reaction mixture was warmed to 80 °C and stirred for 0.5 h, and then cooled to 0 °C and stirred for 0.5 h. The reaction mixture was filtered, and the filter cake was rinsed with toluene (10 mL) and concentrated under reduced pressure to remove the solvent to give the hydrochloride of

### compound 010_4.

### Step 4: synthesis of compound 010

Compound **007_3** (76.00 mg, 285.40 µmol) was dissolved in *tert*-butanol (2.5 mL) at room temperature under nitrogen atmosphere, and compound **010_4** (97.53 mg, 285.40 µmol, hydrochloride) and potassium carbonate (78.89 mg, 570.80 µmol) were added successively. The reaction mixture was warmed to 85 °C and stirred at 85 °C for 16 h. After the reaction was completed, the reaction mixture was cooled to room temperature and concentrated under reduced pressure. The resulting crude product was separated by preparative HPLC (mobile phase: acetonitrile/water; acidic system: 0.04% HCl) to give compound **010.** MS-ESI m/z: 480.1 [M+H]⁺. ¹H NMR (400 MHz, DMSO-d₆) δ: 11.25 (s, 2H), 9.00 (dd, *J =* 2.8, 9.0 Hz, 1H), 8.72 (dd, *J =* 1.6, 2.8 Hz, 1H), 7.53-7.41 (m, 1H), 7.23-7.03 (m, 2H), 5.84 (s, 2H), 2.80 (d, *J =* 16.0 Hz, 2H), 2.58-2.52 (m, 2H), 1.12 (s, 3H).

### Example 11

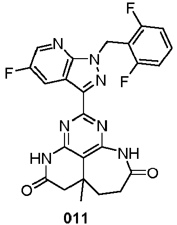

### Synthetic route:

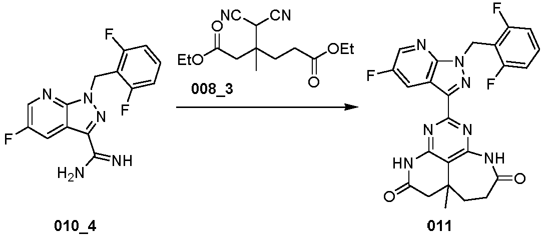

### Step 1: synthesis of compound 011

Compound **010_4** (250 mg, 731.60 µmol, hydrochloride) and compound **008_3** (328.13 mg, 1.17 mmol) were dissolved in tert-butanol (5 mL) at room temperature under nitrogen atmosphere, and 1,8-diazabicycloundec-7-ene (278.44 mg, 1.83 mmol) was added. The reaction mixture was warmed to 95 °C and stirred for 2 h. After the reaction was completed, the reaction mixture was cooled to room temperature and concentrated under reduced pressure to remove the solvent. The resulting crude product was separated by preparative HPLC (mobile phase: acetonitrile/water; acidic system: 0.04% HCl) to give compound 011. MS-ESI m/z: 494.2 [M+H]⁺. ¹H NMR (400 MHz, DMSO-d₆) δ: 11.20 (s, 1H), 10.56 (s, 1H), 9.41-9.17 (m, 1H), 8.70 (s, 1H), 7.55-7.36 (m, 1H), 7.29-6.92 (m, 2H), 5.95-5.70 (m, 2H), 2.82-2.61 (m, 2H), 2.58-2.53 (m, 1H), 2.44-2.32 (m, 1H), 2.08-1.97 (m, 1H), 1.90-1.79 (m, 1H), 1.25-1.15 (m, 3H).

### Example 12

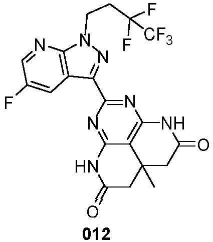

### Synthetic route:

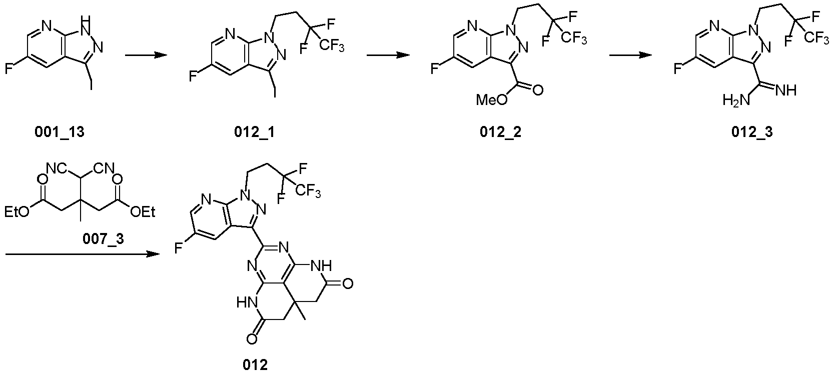

### Step 1: synthesis of compound 012_1

Compound **001_13** (10 g, 38.02 mmol) was dissolved in N,N-dimethylformamide (50 mL) at room temperature, and then potassium carbonate (15.76 g, 114.06 mmol) and 1,1,1,2,2-pentafluoro-4-iodobutane (52.08 g, 190.11 mmol) were added. The reaction mixture was warmed to 40 °C and stirred for 2 h. After the reaction was completed, the reaction mixture was cooled to room temperature, quenched with water (200 mL), and extracted with ethyl acetate (200 mL). The organic phases were combined, dried over anhydrous sodium sulfate, and filtered, and the filtrate was concentrated under reduced pressure to remove the solvent to give a crude product. The crude product was separated by column chromatography (eluent: petroleum ether/ethyl acetate = 1/0 to 3/1, v/v) to give compound **012_1**.

### Step 2: synthesis of compound 012_2

Compound **012_1** (7.5 g, 18.29 mmol) was dissolved in *N,N*-dimethylformamide (60 mL) and methanol (20 mL) at room temperature, and then triethylamine (7.4 g, 73.16 mmol) followed by 1,1-bis(diphenylphosphino)ferrocene palladium dichloride (936.76 mg, 1.28 mmol) was added. The reaction mixture was stirred under carbon monoxide atmosphere 103,421 kPa, 15 psi) at 80 °C for 12 h. After the reaction was completed, the reaction mixture was filtered, and the filtrate was concentrated under reduced pressure to give a crude product. The crude product was separated by column chromatography (eluent: petroleum ether/ethyl acetate = 1/0 to 7/3, v/v) to give compound **012_2.**

### Step 3: synthesis of hydrochloride of compound 012_3

Ammonium chloride (4.31 g, 80.60 mmol) was suspended in toluene (55 mL) at room temperature under nitrogen atmosphere, and then a solution of trimethylaluminum (38.69 mL, 2 M, 77.37 mmol) in toluene was added. The reaction mixture was warmed to 80 °C, and then compound **012_2** (5.5 g, 16.12 mmol) was added. The reaction mixture was stirred for 0.5 h, then warmed to 110 °C and stirred for 1.5 h, and then cooled to 30 °C. Methanol (9.39 mL, 232.12 mmol) and 3 M diluted aqueous hydrochloric acid solution (103.16 mL, 309.49 mmol) were added successively. Then the reaction mixture was warmed to 80 °C and stirred for 0.5 h, and then cooled to 0 °C and stirred for 0.5 h. After the reaction was completed, the reaction mixture was filtered, and the filter cake was rinsed with toluene (20 mL) and then concentrated under reduced pressure to give compound **012_3**.

### Step 4: synthesis of compound 012

Compound **012_3** (100 mg, 276.49 µmol, hydrochloride) and compound **007_3** (117.80 mg, 442.39 µmol) were dissolved in tert-butanol (2 mL) at room temperature under nitrogen atmosphere, and potassium carbonate (95.53 mg, 691.23 µmol) was added. The reaction mixture was heated to 85 °C and stirred for 12 h. After the reaction was completed, the reaction mixture was cooled to room temperature and concentrated under reduced pressure to remove the solvent. The resulting crude product was separated by preparative HPLC (mobile phase: acetonitrile/water; acidic system: 0.04% HCl) to give compound 012. MS-ESI m/z: 500.1 [M+H]⁺. ¹H NMR (400 MHz, DMSO-d₆) δ: 11.27 (s, 2H), 9.01 (dd, *J =* 2.8, 9.2 Hz, 1H), 8.73 (s, 1H), 4.90 (t, *J =* 6.4 Hz, 2H), 3.06-2.90 (m, 2H), 2.82 (d, *J* = 16.0 Hz, 2H), 2.57 (d, *J* = 16.0 Hz, 2H), 1.14 (s, 3H).

### Example 13

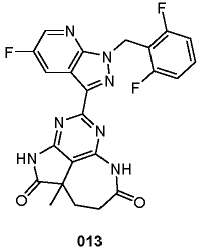

### Synthetic route:

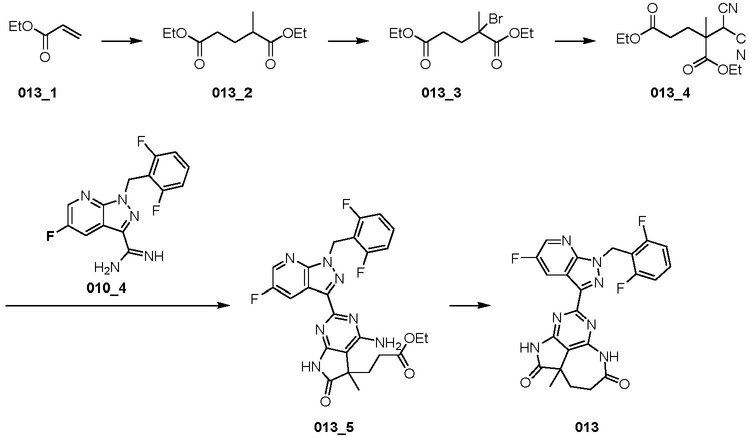

### Step 1: synthesis of intermediate 013_2

*N,N-diisopropylethylamine* (32.09 g, 317.13 mmol) was dissolved in tetrahydrofuran (400 mL) at room temperature. The reaction mixture was cooled to -78 °C, and a solution of n-butyllithium (129.85 mL, 2.5 M) in n-hexane was added. The reaction mixture was warmed to 0 °C and stirred for 0.5 h. Then the reaction mixture was cooled to - 78 °C, and ethyl propionate (32.09 g, 317.13 mmol) and a solution of compound **013_1** (25 g, 249.71 mmol) in tetrahydrofuran (30 mL) were added successively. The reaction mixture was stirred at -78 °C for 1 h. After the reaction was completed, the reaction mixture was poured into saturated aqueous ammonium chloride solution (800 mL). The aqueous phase was separated and extracted with ethyl acetate (500 mL × 3). The organic phases were combined, washed with saturated brine (500 mL × 2), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 10/1, v/v) to give intermediate **013_2.** ¹H NMR (400 MHz, CDCl₃) δ: 4.14 (qd, *J =* 7.2, 2.0 Hz, 4H), 2.48 (dd, *J* = 14.0, 7.2 Hz, 1H), 2.30-2.37 (m, 2H), 1.92-2.04 (m, 1H), 1.73-1.84 (m, 1H), 1.24-1.29 (m, 6H), 1.18 (d, *J* = 6.8 Hz, 3H).

### Step 2: synthesis of intermediate 013_3

Intermediate **013_2** (10.2 g, 50.43 mmol) was dissolved in chlorobenzene (200 mL) at room temperature, and *N-*bromosuccinimide (8.98 g, 50.43 mmol) and azobisisobutyronitrile (828.16 mg, 5.04 mmol) were added. The reaction mixture was heated to 75 °C and stirred for 12 h. After the reaction was completed, the reaction mixture was cooled to room temperature and filtered. The filtrate was collected and concentrated under reduced pressure to remove the solvent. The resulting crude product was separated by column chromatography (eluent: petroleum ether/ethyl acetate = 1/0 to 24/1, v/v) to give intermediate **013_3**. ¹H NMR (400 MHz, CDCl₃) δ: 4.25 (q, *J =* 7.2 Hz, 2H), 4.15-4.19 (m, 2H), 2.45-2.55 (m, 3H), 2.35-2.41 (m, 1H), 1.91 (s, 3H), 1.28-1.36 (m, 6H).

### Step 3: synthesis of intermediate 013_4

Compound malononitrile (3.45 g, 52.29 mmol) was dissolved in tetrahydrofuran (260 mL) at 20 °C under nitrogen atmosphere, and a solution of potassium tert-butoxide (59.76 mL, 1 M) in tetrahydrofuran was added. The reaction mixture was stirred at 20 °C for 0.5 h and then warmed to 75 °C, followed by the dropwise addition of a solution of intermediate **013_3** (14 g, 49.80 mmol) in tetrahydrofuran (20 mL). The reaction mixture was stirred at 75 °C for 12 h. After the reaction was completed, the reaction mixture was cooled to room temperature and poured into water (400 mL), and ethyl acetate (200 mL) was added for dilution. The organic phase was separated and collected, and the aqueous phase was adjusted to pH 3-5 with 1 N diluted aqueous hydrochloric acid solution and then extracted with ethyl acetate (300 mL × 2). The organic phases were combined, washed with saturated brine (400 mL × 2), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 50/1, v/v) to give intermediate **013_4.** ¹H NMR (400 MHz, CDCl₃) δ: 4.28 (q, *J =* 6.8 Hz, 2H), 4.22 (s, 1H), 4.16 (q, *J =* 7.2 Hz, 2H), 2.34-2.40 (m, 2H), 2.15-2.21 (m, 2H), 1.56 (s, 3H), 1.34 (t, *J=* 7.2 Hz, 3H), 1.28 (t, *J =* 7.2 Hz, 3H).

### Step 4: synthesis of intermediate 013_5

Compound **010_4** (1.0 g, 2.93 mmol, hydrochloride) and intermediate **013_4** (1.25 g, 4.68 mmol) were dissolved in tert-butanol (10 mL) at room temperature under nitrogen atmosphere, and potassium carbonate (2.02 g, 14.63 mmol) was added. The reaction mixture was warmed to 80 °C and stirred for 12 h. After the reaction was completed, the reaction mixture was cooled to room temperature and concentrated under reduced pressure to remove the solvent, and the resulting residue was diluted with water (40 mL) and ethyl acetate (40 mL). The organic phase was separated and then collected, and the aqueous phase was extracted with ethyl acetate (40 mL × 3). The organic phases were combined, washed with saturated brine (50 mL × 2), dried over anhydrous sodium sulfate, and filtered, and the filtrate was concentrated under reduced pressure to remove the solvent. The resulting crude product was separated by column chromatography (eluent: petroleum ether/2-methyltetrahydrofuran = 1/0 to 1/1, v/v) to give intermediate **013_5**. ¹H NMR (400 MHz, DMSO_d₆) δ: 11.09 (s, 1H), 8.85 (dd, *J* = 2.8, 8.8 Hz, 1H), 8.71 (s, 1H), 7.51-7.42 (m, 1H), 7.29-7.22 (m, 1H), 7.19-7.16 (m, 1H), 6.90 (s, 2H), 5.80 (s, 2H), 3.91 (q, *J* = 7.2 Hz, 2H), 2.44-2.35 (m, 1H), 1.98-1.81 (m, 3H), 1.34 (s, 3H), 1.06 (t, *J =* 7.2 Hz, 3H).

### Step 5: synthesis of compound 013

Intermediate **013_5** (180 mg, 342.54 µmol) was dissolved in toluene (4 mL) at room temperature, and a solution of trimethylaluminum (513.81 µL, 2 M) in toluene was added. The reaction mixture was heated to 110 °C and stirred for 12 h. After the reaction was completed, the reaction mixture was cooled to room temperature, and diluted aqueous hydrochloric acid solution (2.19 mL, 3 M) was added dropwise at below 40 °C. The mixture was stirred at room temperature for 1 h, then poured into water (20 mL), and extracted with ethyl acetate (20 mL), and the aqueous phase was extracted with 2-methyltetrahydrofuran (20 mL × 3). The organic phases were combined, washed with saturated brine (20 mL × 2), dried over anhydrous sodium sulfate, and filtered, and the filtrate was concentrated under reduced pressure to remove the solvent. The resulting crude product was separated by preparative HPLC (mobile phase: acetonitrile/water; acidic system: 0.04% HCl) to give compound 013. MS-ESI m/z: 480.1 [M+H]⁺. ¹H NMR (400 MHz, DMSO_d₆) δ: 11.48 (s, 1H), 10.90 (s, 1H), 9.09 (dd, *J =* 2.8, 9.2 Hz, 1H), 8.74-8.72 (m, 1H), 7.53-7.43 (m, 1H), 7.15 (t, *J =* 8.0 Hz, 2H), 5.83 (d, *J* = 3.2 Hz, 2H), 2.80 (t, *J =* 6.4 Hz, 2H), 2.16-2.08 (m, 1H), 2.02-1.92 (m, 1H), 1.32 (s, 3H).

### Example 14

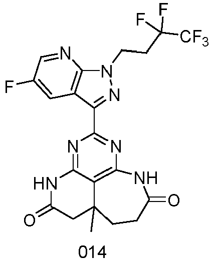

### Synthetic route:

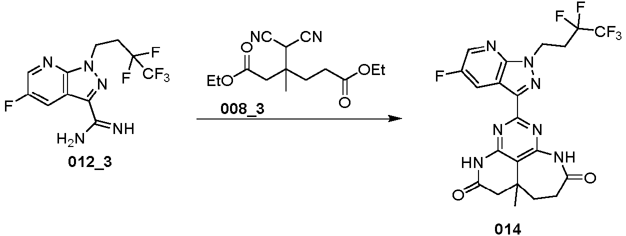

### Synthesis of compound 014

Intermediate **008_3** (223.22 mg, 796.30 µmol) and intermediate **012_3** (180 mg, 497.69 µmol, hydrochloride) were dissolved in tert-butanol (3.6 mL) at room temperature under nitrogen atmosphere, and 1,8-diazabicycloundec-7-ene (189.42 mg, 1.24 mmol) was added. The reaction mixture was heated to 95 °C and stirred for 36 h. After the reaction was completed, the reaction mixture was cooled to room temperature and concentrated under reduced pressure to remove the solvent. The resulting crude product was separated by preparative HPLC (mobile phase: acetonitrile/water; acidic system: 0.04% HCl) to give compound 014. MS-ESI m/z: 514.1 [M+H]⁺. ¹H NMR (400 MHz, DMSO_d₆) δ: 11.20 (s, 1H), 10.55 (s, 1H), 9.29 (dd, *J =* 2.8, 9.2 Hz, 1H), 8.69 (s, 1H), 4.87 (t, *J =* 6.8 Hz, 2H), 3.02-2.86 (m, 3H), 2.80-2.64 (m, 2H), 2.26 (*d, J=* 15.6 Hz, 1H), 2.08-1.96 (m, 1H), 1.90-1.80 (m, 1H), 1.18 (s, 3H).

### Example 15

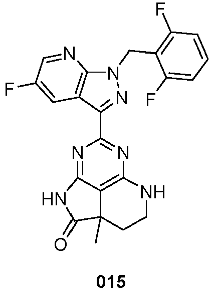

### Synthetic route:

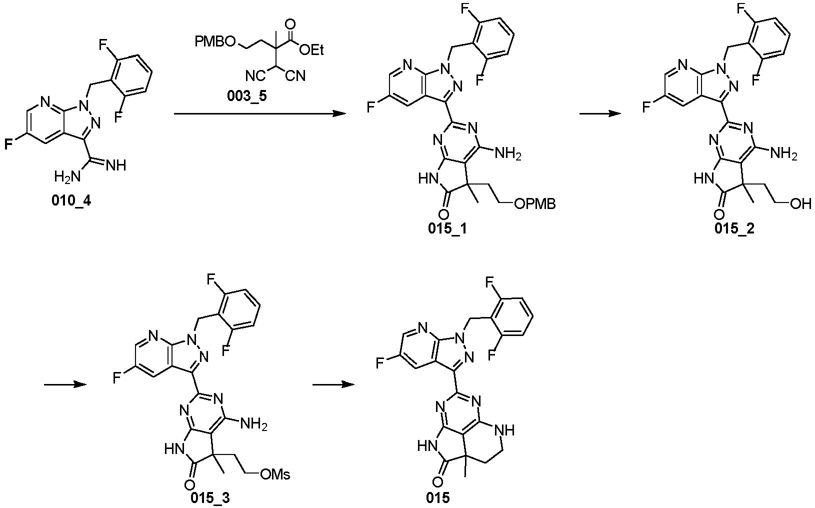

### Step 1: synthesis of intermediate 015_1

**010_4** (2.5 g, 7.32 mmol, hydrochloride) and **003_5** (3.87 g, 11.71 mmol) were dissolved in *tert-butanol* (50 mL) at room temperature under nitrogen atmosphere, and potassium carbonate (4.04 g, 29.28 mmol) was added. The reaction mixture was warmed to 80 °C and stirred for 12 h. After the reaction was completed, the reaction mixture was cooled to room temperature and concentrated under reduced pressure to remove the solvent, and water (80 mL) and ethyl acetate (80 mL) were added to the resulting residue. The aqueous phase was separated and extracted with ethyl acetate (80 mL × 3). The organic phases were combined, washed with saturated brine (100 mL × 2), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 1/1, v/v) to give compound **015_1.** ¹H NMR (400 MHz, DMSO_d₆) δ: 10.92 (s, 1H), 8.85 (dd,*J* = 2.8, 8.8 Hz, 1H), 8.72 (s, 1H), 7.51-7.42 (m, 1H), 7.14 *(t, J =* 8.0 Hz, 2H), 7.01 (*d, J =* 8.8 Hz, 2H), 6.79 (s, 2H), 6.67 *(d, J =* 8.4 Hz, 2H), 5.81 (s, 2H), 4.20 *(d, J =* 11.6 Hz, 1H), 4.06 (*d, J =* 11.6 Hz, 1H), 3.51 (s, 3H), 3.16-3.09 (m, 1H), 3.07-2.99 (m, 1H), 2.43-2.35 (m, 1H), 2.13-2.02 (m, 1H), 1.31 (s, 3H).

### Step 2: synthesis of intermediate 015_2

Intermediate **015_1** (1.8 g, 3.05 mmol) was dissolved in dichloromethane (36 mL) and water (3.6 mL) at room temperature. The reaction mixture was cooled to 0 °C, and dichlorodicyanobenzoquinone (831.68 mg, 3.66 mmol) was added portionwise. The reaction mixture was stirred at 15 °C for 12 h. After the reaction was completed, the reaction mixture was concentrated under reduced pressure to remove the solvent. Saturated aqueous sodium bicarbonate solution (100 mL) was added to the residue, and 2-methyltetrahydrofuran (100 mL) was added for dilution. The organic phase was separated and then collected, and the aqueous phase was extracted with 2-methyltetrahydrofuran (100 mL × 2). The organic phases were combined, washed successively with saturated aqueous sodium bicarbonate solution (100 mL × 2) and saturated brine (100 mL × 2), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 3/2, v/v) to give intermediate **015_2.** ¹H NMR (400 MHz, DMSO_d₆) δ: 10.88 (s, 1H), 8.85 (dd, *J =* 2.8, 8.8 Hz, 1H), 8.71 (dd, *J =* 1.6, 2.8 Hz, 1H), 7.52-7.42 (m, 1H), 7.19-7.11 (m, 2H), 6.81 (s, 2H), 5.80 (s, 1H), 3.11-3.16 (m, 1H), 3.07-2.99 (m, 1H), 2.35-2.22 (m, 1H), 1.85-1.75 (m, 1H), 1.30 (s, 3H).

### Step 3: synthesis of intermediate 015_3

Intermediate **015_2** (1 g, 2.13 mmol) was dissolved in dichloromethane (10 mL) and tetrahydrofuran (10 mL) at room temperature. The reaction mixture was cooled to 0 °C, and methanesulfonyl chloride (248.91 mg, 2.17 mmol) followed by *N,N*-diisopropylethylamine (825.98 mg, 6.39 mmol) and 4-dimethylaminopyridine (26.03 mg, 213.03 µmol) was added. The reaction mixture was stirred at 20 °C for 1 h. After the reaction was completed, the reaction mixture was poured into a 0 °C saturated aqueous sodium bicarbonate solution (100 mL) and extracted with ethyl acetate (100 mL). The organic phases were combined, washed with saturated brine (400 mL × 2), dried over anhydrous sodium sulfate, and filtered, and the filtrate was concentrated under reduced pressure to remove the solvent. The resulting crude product was separated by column chromatography (eluent: petroleum ether/ethyl acetate = 1/0 to 3/2, v/v) to give intermediate **015_3.** ¹H NMR (400 MHz, DMSO_d₆) δ: 11.08 (s, 1H), 8.85 (dd, *J* = 2.8, 8.8 Hz, 1H), 8.71 (dd, *J* = 1.2, 2.8 Hz, 1H), 7.52-7.42 (m, 1H), 7.14 *(t, J=* 8.0 Hz, 2H), 6.93 (s, 2H), 5.81 (s, 2H), 4.03-3.95 (m, 1H), 3.84-3.75 (m, 1H), 2.98 (s, 3H), 2.60-2.53 (m, 1H), 2.24-2.15 (m, 1H), 1.34 (s, 3H).

### Step 4: synthesis of compound 015

Intermediate **015_3** (500 mg, 913.23 µmol) was dissolved in tetrahydrofuran (10 mL) at room temperature, and hexamethylphosphoric triamide (507.32 mg, 2.83 mmol) was added. The reaction mixture was cooled to -78 °C, and a solution of lithium bis(trimethylsilyl)amide (2.83 mL, 1 M) in tetrahydrofuran was added. The reaction mixture was warmed to 20 °C and stirred for 5 h. After the reaction was completed, the reaction mixture was poured into ice water (30 mL), and ethyl acetate (30 mL) was added for dilution. The organic phase was separated and then collected, and the aqueous phase was extracted with ethyl acetate (30 mL × 3). The organic phases were combined, washed with saturated brine (50 mL × 2), dried over anhydrous sodium sulfate, and filtered, and the filtrate was concentrated under reduced pressure to remove the solvent. The resulting crude product was separated by preparative HPLC (mobile phase: acetonitrile/water; neutral system: 10 mM NH₄HCO₃) to give compound 015. MS-ESI m/z: 452.1 [M+H]⁺. ¹H NMR (400 MHz, DMSO_d₆) δ: 8.72 (dd, *J =* 1.6, 2.8 Hz, 1H), 8.65 (dd, *J =* 2.8, 8.8 Hz, 1H), 7.65 (d, *J =* 4.4 Hz, 1H), 7.52-7.42 (m, 1H), 7.15 (t, *J =* 8.0 Hz, 2H), 5.80 (s, 2H), 3.57- 3.39 (m, 2H), 1.83 (d, *J =* 12.0 Hz, 1H), 1.32 (s, 3H), 1.29-1.19 (m, 1H).

### Example 16

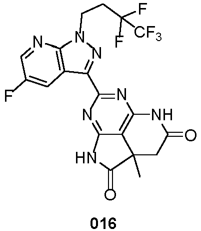

### Synthetic route:

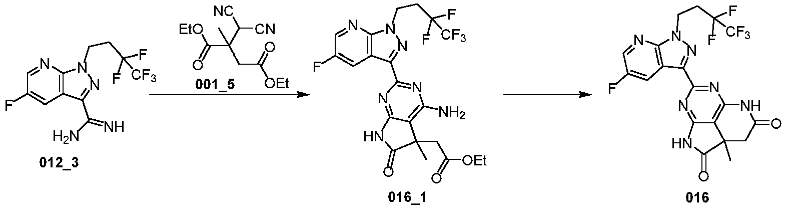

### Step 1: synthesis of intermediate 016_1

Compound **012_3** (750 mg, 2.07 mmol, hydrochloride) was dissolved in tert-butanol (15 mL) at room temperature, and compound **001_5** (784.68 mg, 3.11 mmol) and potassium carbonate (1.15 g, 8.29 mmol) were added. The reaction mixture was heated to 80 °C and stirred for 12 h. After the reaction was completed, the reaction mixture was cooled to room temperature, and water (30 mL) was added to the reaction mixture, followed by the extraction with ethyl acetate (30 mL × 3). The organic phases were combined, washed with saturated brine (30 mL), dried over anhydrous sodium sulfate, and filtered. The filtrate was concentrated under reduced pressure to remove the solvent, and the resulting residue was separated by column chromatography (eluent: dichloromethane/methanol = 1/0 to 50/1, v/v) to give compound **016_1.** ¹H NMR (400 MHz, DMSO_d6) δ: 11.04 (s, 1H), 8.84 (dd, *J =* 2.8, 8.8 Hz, 1H), 8.72-8.69 (m, 1H), 6.88 (s, 2H), 4.87 (t, *J =* 6.4 Hz, 2H), 3.92-3.82 (m, 2H), 3.37 (d, *J* = 16.4 Hz, 1H), 3.06-2.90 (m, 2H), 2.80 (d, *J* = 16.4 Hz, 1H), 1.33 (s, 3H), 0.99 (t, *J =* 7.1 Hz, 3H).

### Step 2: synthesis of compound 0016

Compound **016_1** (50 mg, 94.09 µmol) was dissolved in toluene (1 mL) at room temperature, and a solution of trimethylaluminum (141.13 µL, 2 M) in toluene was added. The reaction mixture was heated to 80 °C and stirred for 12 h. After the reaction was completed, the reaction mixture was cooled to room temperature, and diluted aqueous hydrochloric acid solution (602.17 µL, 3 M) was added dropwise at below 40 °C. The mixture was stirred at 15 °C for 10 min. Then water (3 mL) was added to the mixture, followed by the extraction with 2-methyltetrahydrofuran (3 mL × 3). The organic phases were combined, washed with saturated brine (3 mL), dried over anhydrous sodium sulfate, and filtered, and the filtrate was concentrated under reduced pressure to remove the solvent. The resulting residue was separated by preparative HPLC (mobile phase: acetonitrile/water; acidic system: 0.04%M HCl) to give compound **016.** MS-ESI m/z: 486.1 [M+H]⁺. ¹H NMR (400 MHz, DMSO_d₆) δ: 11.39 (s, 1H), 11.13 (s, 1H), 8.76-8.74 (m, 1H), 8.73-8.69 (m, 1H), 4.91 (t, *J =* 6.8 Hz, 2H), 3.08-2.82 (m, 1H), 2.54 (s, 1H), 1.39 (s, 3H).

### Example 17

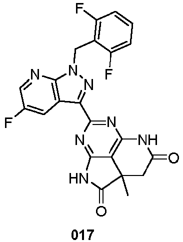

### Synthetic route:

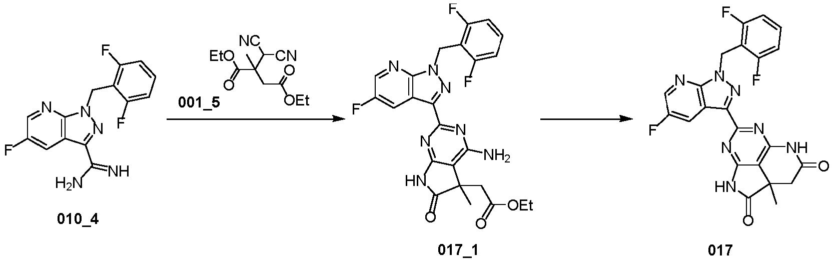

### Step 1: synthesis of intermediate 017_1

Compound **010_4** (1.1 g, 3.22 mmol, hydrochloride) was dissolved in tert-butanol (20 mL) at room temperature, and compound **001_5** (1.22 g, 4.83 mmol) and potassium carbonate (1.78 g, 12.88 mmol) were added. The reaction mixture was heated to 80 °C and stirred for 12 h. After the reaction was completed, the reaction mixture was cooled to room temperature, and water (30 mL) was added to the reaction mixture, followed by the extraction with ethyl acetate (30 mL × 3). The organic phases were combined, washed with saturated brine (30 mL), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 2/1, v/v) to give compound **017_1.** ¹H NMR (400 MHz, DMSO_d₆) δ: 11.02 (s, 1H), 8.83 (dd, *J* = 2.8, 8.8 Hz, 1H), 8.72-8.70 (m, 1H), 7.53-7.41 (m, 1H), 7.14 (t, *J =* 8.0 Hz, 2H), 6.86 (s, 2H), 5.80 (s, 2H), 3.85 (t, *J =* 6.8 Hz, 2H), 3.37 (s, 1H), 2.77 (d, *J =* 16.8 Hz, 1H), 1.30 (s, 3H), 0.97 (t, *J =* 7.2 Hz, 3H).

### Step 2: synthesis of intermediate 017

Compound **017_1** (300 mg, 568.37 µmol) was dissolved in toluene (6 mL) at room temperature, and a solution of trimethylaluminum (852.55 µL, 2 M) in toluene was added. The reaction mixture was heated to 80 °C and stirred for 12 h. After the reaction was completed, the reaction mixture was cooled to room temperature, and diluted aqueous hydrochloric acid solution (3.64 mL, 3 M) was added dropwise at below 40 °C. The mixture was stirred at 15 °C for 10 min. Then water (10 mL) was added to the mixture, followed by the extraction with 2-methyltetrahydrofuran (20 mL × 3). The organic phases were combined, washed with saturated brine (10 mL), dried over anhydrous sodium sulfate, and filtered, and the filtrate was concentrated under reduced pressure to remove the solvent. The resulting residue was separated by preparative HPLC (mobile phase: acetonitrile/water; neutral system: 10 mM NH₄HCO₃) to give compound **017.** MS-ESI m/z: 466.0 [M+H]⁺.¹H NMR (400 MHz, DMSO_d6) δ: 11.36 (s, 1H), 11.12 (s, 1H), 8.76 (s, 1H), 8.70 (dd, *J* = 3.2, 9.6 Hz, 1H), 7.52-7.43 (m, 1H), 7.15 (*t, J =* 8.0 Hz, 2H), 5.84 (s, 2H), 2.98 (d, *J=* 16.0 Hz, 1H), 2.52 (s, 1H), 1.36 (s, 3H).

### Example 18

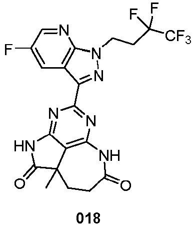

### Synthetic route:

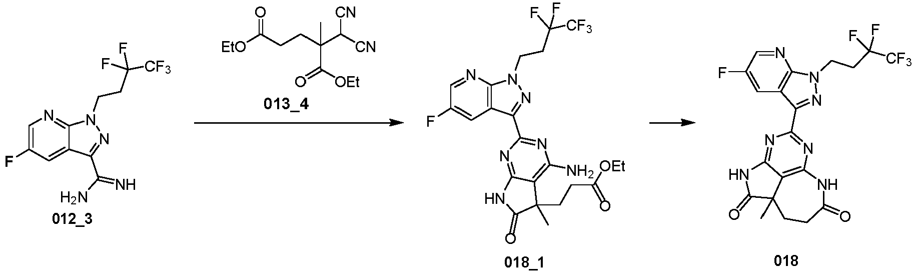

### Step 1: synthesis of intermediate 018_1

Intermediate **012_3** (750 mg, 2.07 mmol, hydrochloride) and intermediate **013_4** (883.53 mg, 3.32 mmol) were dissolved in tert-butanol (20 mL) at room temperature under nitrogen atmosphere, and potassium carbonate (1.15 g, 8.29 mmol) was added. The reaction mixture was warmed to 80 °C and stirred for 12 h. After the reaction was completed, the reaction mixture was cooled to room temperature and concentrated under reduced pressure to remove the solvent, and the resulting residue was diluted with water (100 mL) and ethyl acetate (100 mL). The organic phase was separated and then collected, and the aqueous phase was extracted with ethyl acetate (100 mL × 3). The organic phases were combined, washed with saturated brine (100 mL × 2), dried over anhydrous sodium sulfate, and filtered, and the filtrate was concentrated under reduced pressure to remove the solvent. The resulting crude product was separated by column chromatography (eluent: dichloromethane/ethanol = 1/0 to 19/1, v/v) to give intermediate **018_1.** ¹H NMR (400 MHz, DMSO_d₆) δ: 11.11 (s, 1H), 8.85 (dd, *J=* 2.8, 8.8 Hz, 1H), 8.71-8.69 (m, 1H), 6.92 (s, 2H), 4.87 (t, *J =* 6.8 Hz, 2H), 3.95-3.87 (m, 2H), 3.06-2.87 (m, 2H), 2.46-2.39 (m, 1H), 1.99-1.86 (m, 3H), 1.37 (s, 3H), 1.07 (t, *J =* 6.8 Hz, 3H).

### Step 2: synthesis of compound 018

Intermediate **018_1** (300 mg, 513.17 µmol, 93.3% purity) was dissolved in toluene (8 mL) at room temperature, and a solution of trimethylaluminum (769.75 µL, 2 M) in toluene was added. The reaction mixture was heated to 110 °C and stirred for 12 h. After the reaction was completed, the reaction mixture was cooled to 0 °C, and diluted aqueous hydrochloric acid solution (3.28 mL, 3 M) was added dropwise. The reaction mixture was stirred at 0 °C for 0.5 h and then poured into water (30 mL), and 2-methyltetrahydrofuran (30 mL) was added for dilution. The organic phase was separated and then collected, and the aqueous phase was extracted with 2-methyltetrahydrofuran (30 mL × 3). The organic phases were combined, washed with saturated brine (40 mL × 2), dried over anhydrous sodium sulfate, and filtered, and the filtrate was concentrated under reduced pressure to remove the solvent. The resulting crude product was separated by preparative HPLC (mobile phase: acetonitrile/water; neutral system: 10 mM NH₄HCO₃) to give compound **018.** MS-ESI m/z: 500.1 [M+H]⁺. ¹H NMR (400 MHz, DMSO_d₆) δ: 11.49 (s, 1H), 10.90 (s, 1H), 9.09 (dd, *J =* 2.8, 9.2 Hz, 1H), 8.73-8.71 (m, 1H), 4.90 (t, *J =* 6.8 Hz, 2H), 3.07-2.91 (m, 2H), 2.85-2.79 (m, 2H), 2.19-2.10 (m, 1H), 2.05-1.93 (m, 1H), 1.35 (s, 3H).

### Example 19

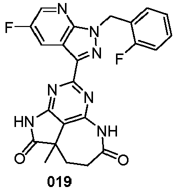

### Synthetic route:

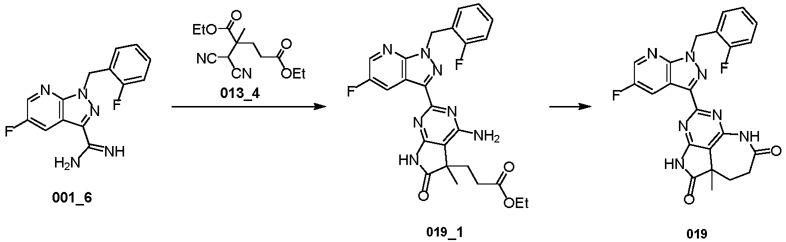

### Step 1: synthesis of intermediate 019_1

The hydrochloride of intermediate **001_6** (1 g, 3.09 mmol) was dissolved in tert-butanol (20 mL) at room temperature under nitrogen atmosphere, and intermediate **013_4** (1.23 g, 4.63 mmol) and potassium carbonate (1.07 g, 7.72 mmol) were added. The reaction mixture was heated to 80 °C and stirred for 12 h. After the reaction was completed, the reaction mixture was cooled to room temperature. Saturated brine (30 mL) was added to the reaction mixture, followed by the extraction with ethyl acetate (50 mL × 3). The organic phases were combined, washed with saturated brine (30 mL × 3), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 1/1, ethyl acetate/2-methyltetrahydrofuran = 20/1, v/v) to give intermediate **019_1.** ¹H NMR (400 MHz, DMSO_d₆) δ: 11.08 (s, 1H), 8.85 (dd, *J =* 2.6, 8.8 Hz, 1H), 8.70 (dd, *J =* 1.6, 2.6 Hz, 1H), 7.42-7.31 (m, 1H), 7.28-7.09 (m, 3H), 6.90 ( s, 2H), 5.87-5.77 (m, 2H), 3.99-3.86 (m, 2H), 2.46-2.35 (m, 1H), 2.04-1.90 (m, 3H),1.36 (s, 3H), 1.07 (t, *J =* 7.2 Hz, 3H).

### Step 2: synthesis of compound 019

Intermediate **019_1** (260 mg, 512.32 µmol) was dissolved in toluene (6 mL) at room temperature, and a solution of trimethylaluminum (768.49 µL, 2 M) in toluene was added. The reaction mixture was heated to 110 °C and stirred for 12 h. After the reaction was completed, the reaction mixture was cooled to room temperature, diluted with 2-methyltetrahydrofuran (10 mL), and adjusted to pH 5-6 with 1 M diluted hydrochloric acid. Water (5 mL) was added, followed by the extraction with 2-methyltetrahydrofuran (30 mL × 3). The organic phases were combined, washed with saturated brine (30 mL × 3), dried over anhydrous sodium sulfate, and filtered, and the filtrate was concentrated under reduced pressure to remove the solvent. The resulting crude product was separated by preparative HPLC (mobile phase: acetonitrile/water; acidic system: 0.04% HCl) to give compound 019. MS-ESI m/z: 462.1 [M+H]^{+ 1}H NMR (400 MHz, DMSO_d₆) δ: 11.45 (s, 1H), 10.87 (s, 1H), 9.06 (dd, *J =* 2.8, 9.0 Hz, 1H), 8.68 (dd, *J* = 1.6, 2.8 Hz, 1H), 7.41-7.29 (m, 1H), 7.24-7.07 (m, 3H), 5.84-5.76 (m, 2H), 2.85-2.71 (m, 2H), 2.15-2.03 (m, 1H), 2.02-1.90 (m, 1H), 1.30 (s, 3H).

### Examples 20 and 21

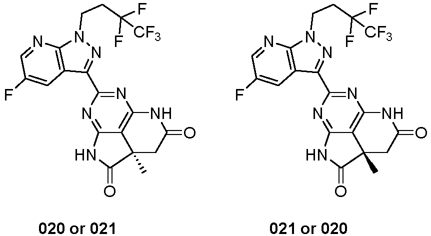

### Synthetic route:

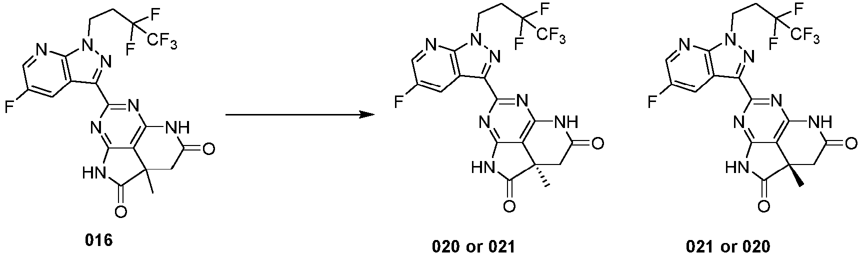

### Synthesis of compounds 020 and 021

Compound **016** (65 mg, 133.93 µmol) was separated by chiral column chromatography (column model: DAICEL CHIRALCEL OJ (250 mm × 30 mm, 10 µm); mobile phase: [Neu-ACN]; B(ACN)%: 30%-30%, 10 min) to give compounds **020** and **021.**

**020** (retention time: 1.087 min): MS-ESI m/z: 486.1 [M+H]⁺. ¹H NMR (400 MHz, DMSO_d6) δ: 11.14 (s, 2H), 8.76-8.71 (m, 1H), 8.71 (dd, *J =* 2.0, 8.8 Hz, 1H), 4.90 (t, *J =* 7.2 Hz, 2H), 3.09-2.89 (m, 3H), 2.55-2.52 (m, 1H), 1.38 (s, 3H).

**021** (retention time: 1.204 min): MS-ESI m/z: 486.0 [M+H]⁺. ¹H NMR (400 MHz, DMSO_d6) δ: 11.15 (s, 2H), 8.78-8.65 (m, 2H), 4.91 (t, *J =* 6.4 Hz, 2H), 3.08-2.91 (m, 3H), 2.56-2.52 (m, 1H), 1.38 (s, 3H).

### Example 22

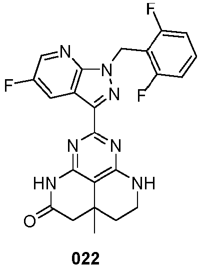

### Synthetic route:

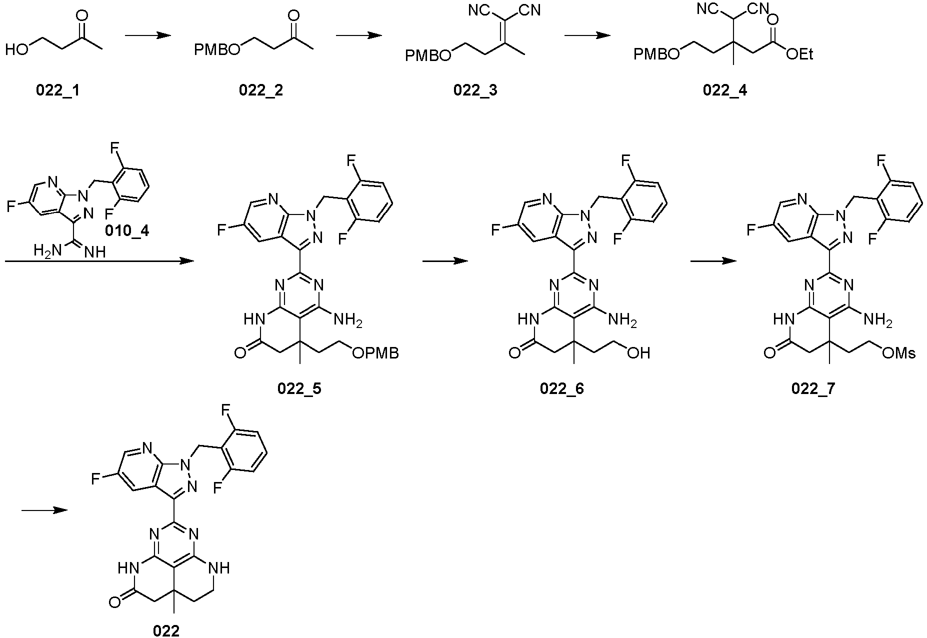

### Step 1: synthesis of intermediate 022_2

**022_1** (5 g, 56.75 mmol, 4.90 mL) was placed in a dry reaction flask at room temperature under nitrogen atmosphere, and *p*-methoxybenzyl chloride (9.78 g, 62.43 mmol, 8.50 mL) and N,N-diisopropylethylamine (14.67 g, 113.50 mmol, 19.77 mL) were added. The reaction system was warmed to 150 °C and stirred for 2 h. After the reaction was completed, the reaction system was cooled to room temperature and let stand, and the solution separated into layers. Ethyl acetate (50 mL) and 10% sodium bisulfate solution (50 mL) were added for dilution. The organic phase was separated and collected, and the aqueous phase was extracted with ethyl acetate (50 mL × 3). The organic phases were combined and then washed with saturated brine (75 mL × 3), dried over anhydrous sodium sulfate, and filtered, and the filtrate was concentrated under reduced pressure to give a crude product. The resulting residue was separated by column chromatography (eluent: petroleum ether/ethyl acetate = 1/0 to 8/1, v/v) to give the target intermediate **022_2.** ¹H NMR (400 MHz, CDCl₃) δ: 7.24 (d, *J* = 8.6 Hz, 2H), 6.87 (d, *J =* 8.6 Hz, 2H), 4.44 (s, 2H), 3.79 (s, 3H), 3.70 (t, *J =* 6.4 Hz, 2H), 2.69 (t, *J =* 6.2 Hz, 2H), 2.16 (s, 3H).

### Step 2: synthesis of intermediate 022_3

Intermediate **022_2** (5 g, 24.01 mmol) and malononitrile (1.59 g, 24.01 mmol) were dissolved in toluene (50 mL) at room temperature under nitrogen atmosphere, and acetic acid (1.44 g, 24.01 mmol, 1.37 mL) and ammonium acetate (1.85 g, 24.01 mmol) were added. The reaction system was warmed to 50 °C and stirred for 12 h. The reaction system was cooled to room temperature, and ethyl acetate (50 mL) and saturated brine (50 mL) were added. The organic phase was separated and collected, and the aqueous phase was extracted with ethyl acetate (50 mL × 3). The organic phases were combined, then washed with saturated brine (65 mL × 3), dried over anhydrous sodium sulfate, and filtered, and the filtrate was concentrated under reduced pressure to give a crude product. The resulting residue was separated by column chromatography (eluent: petroleum ether/ethyl acetate = 1/0 to 8/1, v/v) to give intermediate **022_3.** ¹H NMR (400 MHz, CDCl₃) δ: 7.23 (d, *J =* 8.8 Hz, 2H), 6.93-6.87 (m, 2H), 4.45 (s, 2H), 3.82 (s, 3H), 3.67 (t, *J* = 6.0 Hz, 2H), 2.84 (t, *J =* 5.8 Hz, 2H), 2.29 (s, 3H).

### Step 3: synthesis of intermediate 022_4

Intermediate **022_3** (3.40 g, 13.27 mmol), ethyl bromoacetate (4.43 g, 26.53 mmol, 2.93 mL) were dissolved in tetrahydrofuran (30 mL) at room temperature under nitrogen atmosphere, and titanocene dichloride (343.63 mg, 1.33 mmol, 214.77 µL) and activated zinc powder (2.60 g, 39.80 mmol) were added. The reaction mixture was stirred at room temperature for 12 h. After the reaction was completed, the reaction mixture was quenched with 40 mL of 1 M diluted hydrochloric acid and diluted with ethyl acetate (80 mL). The aqueous phase was separated and extracted with ethyl acetate (100 mL × 3). The organic phases were combined, washed with saturated brine (80 mL × 2), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 10/3, v/v) to give intermediate **022_4.** ¹H NMR (400 MHz, CDCl₃) δ: 7.26 (d, *J =* 8.4 Hz, 2H), 6.90 (d, *J =* 8.4 Hz, 2H), 4.83 (s, 1H), 4.38 (s, 2H), 4.19-4.08 (m, 2H), 3.82 (s, 3H), 3.58 (t, *J =* 5.6 Hz, 2H), 2.69-2.54 (m, 2H), 2.13-1.91 (m, 2H), 1.33 (s, 3H), 1.25 (t, *J=* 7.2 Hz, 3H).

### Step 4: synthesis of intermediate 022_5

Intermediate **022_4** (1.40 g, 4.06 mmol) was dissolved in tert-butanol (25 mL) at room temperature under nitrogen atmosphere, and **010_4** (868.18 mg, 2.54 mmol, hydrochloride) and potassium carbonate (877.85 mg, 6.35 mmol) were added. The reaction mixture was warmed to 85 °C and stirred for 12 h. After the reaction was completed, the reaction system was cooled to room temperature, quenched with 50 mL of saturated brine, and diluted with ethyl acetate (80 mL). The organic phase was separated and collected, and the aqueous phase was extracted with ethyl acetate (80 mL × 3). The organic phases were combined, washed with saturated brine (50 mL × 3), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 2/1, ethyl acetate/2-methyltetrahydrofuran = 10/1, v/v) to give intermediate **022_5.** ¹H NMR (400 MHz, DMSO_d₆) δ: 10.62 (s, 1H), 9.06 (dd, *J* = 2.8, 9.2 Hz, 1H), 8.69 (dd, *J* = 1.6, 2.8 Hz, 1H), 7.51-7.43 (m, 1H), 7.17-7.11 (m, 4H), 6.80-6.77 (m, 2H), 6.65 (s, 2H), 5.81 (s, 2H), 4.28 (s, 2H), 3.63 (s, 3H), 3.45-3.39 (m, 2H), 2.70-2.65 (m, 1H), 2.45-2.41 (m, 1H), 1.78-1.71 (m, 1H), 2.23-2.17 (m, 1H), 1.35 (s, 3H).

### Step 5: synthesis of intermediate 022_6

Intermediate **022_5** (620.00 mg, 1.03 mmol) was dissolved in dichloromethane (6 mL) and water (0.6 mL) at room temperature under nitrogen atmosphere, and dichlorodicyanobenzoquinone (349.76 mg, 1.54 mmol) was added at 0 °C. The reaction mixture was stirred at room temperature for 12 h. After the reaction was completed, the reaction mixture was poured into saturated aqueous sodium bicarbonate solution (20 mL), followed by the extraction with dichloromethane (30 mL × 3). The organic phase was collected, dried over anhydrous sodium sulfate, and filtered, and the filtrate was concentrated under reduced pressure to remove the solvent. The resulting residue was separated by column chromatography (eluent: petroleum ether/ethyl acetate = 1/0 to 5/4, ethyl acetate/2-methyltetrahydrofuran = 10/1 to 5/1, v/v) to give intermediate **022_6.** ¹H NMR (400 MHz, DMSO_*d₆*) δ: 10.60 (s, 1H), 9.05 (dd, *J* = 2.8, 9.2 Hz, 1H), 8.68 (dd, *J* = 1.6, 2.8 Hz, 1H), 7.53-7.39 (m, 1H), 7.16-7.10 (m, 2H), 6.65 (d, *J* = 2.8 Hz, 2H), 5.80 (s, 2H), 4.51 (t, *J =* 4.8 Hz, 1H), 3.43-3.37 (m, 2H), 2.62 (d, *J* = 16.0 Hz, 1H), 2.42 (d, *J =* 16.0 Hz, 1H), 2.05-1.97 (m, 1H), 1.68-1.60 (m, 1H), 1.35 (s, 3H).

### Step 6: synthesis of intermediate 022_7

Intermediate **022_6** (0.3 g, 620.55 µmol) was dissolved in dichloromethane (3 mL) and tetrahydrofuran (3 mL) at room temperature under nitrogen atmosphere, and methanesulfonyl chloride (106.63 mg, 930.82 pmol, 72.04 µL), N,N-diisopropylethylamine (240.60 mg, 1.86 mmol, 324.26 µL) and 4-dimethylaminopyridine (7.58 mg, 62.05 µmol) were added at 0 °C. The reaction mixture was stirred at room temperature for 1 h. After the reaction was completed, the reaction mixture was poured into saturated aqueous sodium bicarbonate solution (30 mL) at 0 °C and extracted with ethyl acetate (50 mL × 3). The organic phases were combined, washed with saturated brine (30 mL × 3), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 2/1, ethyl acetate/2-methyltetrahydrofuran = 10/1, v/v) to give intermediate **022_7.** ¹H NMR (400 MHz, DMSO_*d₆*) δ: 10.73 (s, 1H), 9.05 (dd, *J* = 2.8, 9.0 Hz, 1H), 8.69 (dd, *J* = 1.4, 2.8 Hz, 1H), 7.50-7.42 (m, 1H), 7.15-7.10 (m, 1H), 6.86 (s, 2H), 6.77 (s, 2H), 5.80 (s, 1H), 4.20-4.15 (m, 2H), 3.34-3.32 (m, 2H), 2.75-2.66 (m, 1H), 2.43 *(d, J* = 7.2 Hz, 2H), 2.08-2.07 (m, 3H), 1.62-1.55 (m, 2H).

### Step 7: synthesis of compound 022

Intermediate **022_7** (0.28 g, 498.63 µmol) was dissolved in tetrahydrofuran (6 mL) at room temperature under nitrogen atmosphere, and hexamethylphosphoric triamide (277.00 mg, 1.55 mmol, 271.57 µL) was added. The reaction system was cooled to -78 °C, and lithium bis(trimethylsilyl)amide (1 M in tetrahydrofuran, 1.55 mL) was added. The reaction mixture was warmed to room temperature and stirred for 1 h. After the reaction was completed, the reaction mixture was poured into ice water (30 mL) and extracted with ethyl acetate (30 mL), and the aqueous phase was extracted with ethyl acetate (30 mL × 3). The organic phases were combined, washed with saturated brine (50 mL × 2), dried over anhydrous sodium sulfate, and filtered, and the filtrate was concentrated under reduced pressure to remove the solvent. The resulting residue was purified by preparative HPLC (mobile phase: acetonitrile/water; neutral system: 0.01% NH₄HCO₃) to give compound **022.** MS-ESI *m*/*z:* 466.1 [M+H]⁺. ¹H NMR (400 MHz, DMSO_*d₆*) δ: 10.65 (s, 1H), 8.91 (dd, *J* = 2.8, 9.0 Hz, 1H), 8.68 (dd, *J* = 1.2, 2.4 Hz, 1H), 7.80 (s, 1H), 7.50-7.42 (m, 1H), 7.17-7.10 (m, 2H), 5.79 (s, 2H), 3.38 (d, *J* = 9.0 Hz, 2H), 2.43-2.32 (m, 1H), 1.81 (d, *J=* 12.8 Hz, 1H), 1.63-1.55 (m, 1H), 1.10 (s, 3H).

### Example 23

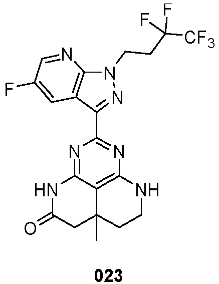

### Synthetic route:

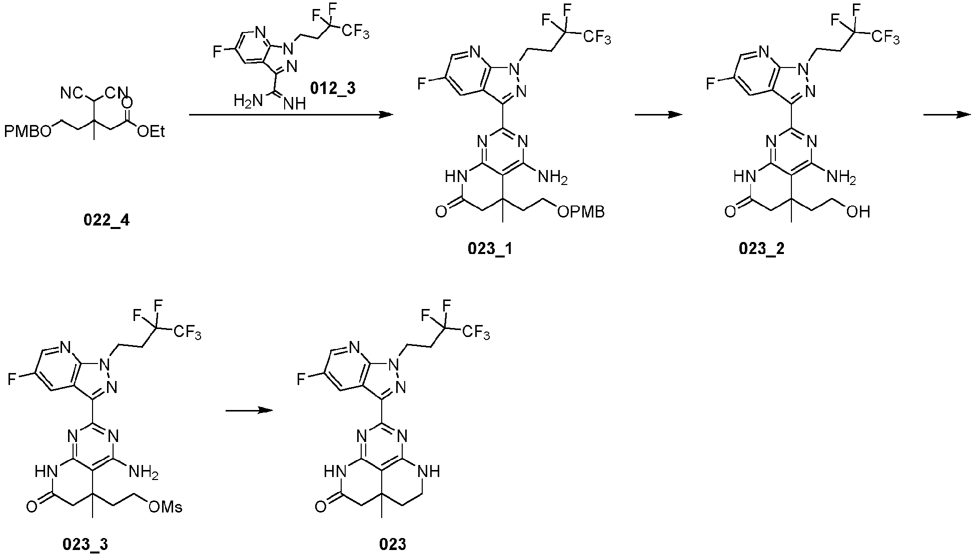

### Step 1: synthesis of intermediate 023_1

Intermediate **022_4** (1.52 g, 4.42 mmol) was dissolved in tert-butanol (20 mL) at room temperature under nitrogen atmosphere, and intermediate 012_3 (1.00 g, 2.76 mmol, hydrochloride) and 1,8-diazabicycloundec-7-ene (1.05 g, 6.91 mmol, 1.04 mL) were added. The reaction mixture was warmed to 85 °C and stirred for 12 h. After the reaction was completed, the reaction mixture was cooled to room temperature, and saturated brine (50 mL) and ethyl acetate (80 mL) were added to the reaction system. The organic phase was separated and collected, and the aqueous phase was extracted with ethyl acetate (80 mL × 3). The organic phases were combined, washed with saturated brine (50 mL × 3), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 2/1, ethyl acetate/2-methyltetrahydrofuran = 10/1, v/v) to give intermediate **023_1.** MS-ESI *m*/*z:* 624.5 [M+H]⁺. ¹H NMR (400 MHz, CDCl₃) δ: 8.54-8.51 (m, 2H), 7.21 (d, *J =* 8.6 Hz, 2H), 6.86 (d, *J* = 8.8 Hz, 2H), 5.49 (s, 2H), 5.00-4.91 (m, 2H), 4.45-4.34 (m, 2H), 3.79 (s, 3 H), 2.87-2.80 (m, 2H), 2.68-2.61 (m, 2H), 1.60 (s, 3H).

### Step 2: synthesis of intermediate 023_2

Intermediate **023_1** (0.67 g, 1.07 mmol) was dissolved in dichloromethane (6 mL) and water (0.6 mL) at room temperature under nitrogen atmosphere, and dichlorodicyanobenzoquinone (365.87 mg, 1.61 mmol) was added at 0 °C. The reaction mixture was stirred at 25 °C for 12 h. After the reaction was completed, the reaction mixture was poured into saturated aqueous sodium bicarbonate solution (20 mL) and extracted with dichloromethane (30 mL × 3). The organic phase was dried over anhydrous sodium sulfate and filtered, and the filtrate was concentrated under reduced pressure to remove the solvent. The resulting residue was separated by column chromatography (eluent: petroleum ether/ethyl acetate = 1/0 to 5/4, ethyl acetate/2-methyltetrahydrofuran = 10/1 to 5/1, v/v) to give intermediate **023_2.** MS-ESI *m*/*z:* 504.4 [M+H]⁺. ¹H NMR (400 MHz, DMSO_*d₆*) δ: 10.61 (s, 1H), 9.06 (dd, *J =* 2.8, 9.0 Hz, 1H), 8.68 (s, 1H), 6.69-6.65 (m, 2H), 4.94-4.84 (m, 2H), 4.53 (s, 1H), 3.42 (s, 2H), 3.00-2.90 (m, 2H), 2.64 (d, *J* = 16.4 Hz, 1H), 2.43 (s, 1H), 1.81-1.68 (m, 2H), 1.38 (s, 3H).

### Step 3: synthesis of intermediate 023_3

Intermediate **023_2** (0.46 g, 913.79 µmol) was dissolved in dichloromethane (5 mL) and tetrahydrofuran (5 mL) at room temperature under nitrogen atmosphere, and 4-dimethylaminopyridine (11.16 mg, 91.38 µmol), methanesulfonyl chloride (157.01 mg, 1.37 mmol, 106.09 µL) and N,N-diisopropylethylamine (354.29 mg, 2.74 mmol, 477.48 µL) were added at 0 °C. The reaction mixture was reacted at room temperature for 1 h. After the reaction was completed, the reaction mixture was poured into saturated aqueous sodium bicarbonate solution (30 mL) at 0 °C and extracted with ethyl acetate (50 mL × 3). The organic phases were combined, washed with saturated brine (30 mL × 3), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 2/1, ethyl acetate/2-methyltetrahydrofuran = 10/1, v/v) to give intermediate **023_3.** ¹H NMR (400 MHz, DMSO_*d₆*) δ: 10.74 (s, 1H), 9.08-9.04 (m, 2H), 8.69 (s, 2H), 6.80 (s, 2H), 4.87 (t, *J* = 6.6 Hz, 2H), 4.21 (br s, 2H), 3.14 (s, 3H), 2.78-2.66 (m, 2H), 2.45-2.42 (m, 2H), 1.37 (s, 2H).

### Step 4: synthesis of compound 023

Intermediate **023_3** (0.4 g, 687.89 µmol) was dissolved in tetrahydrofuran (8 mL) at room temperature under nitrogen atmosphere, and hexamethylphosphoric triamide (382.14 mg, 2.13 mmol, 374.64 µL) was added. The reaction system was cooled to -78 °C, and lithium bis(trimethylsilyl)amide (1 M in tetrahydrofuran, 2.13 mL) was added. The reaction mixture was brought back to room temperature and stirred for 1 h. After the reaction was completed, the reaction mixture was poured into ice water (30 mL) to quench the reaction and extracted with ethyl acetate (30 mL), and the aqueous phase was also extracted with ethyl acetate (30 mL × 3). The organic phases were combined, washed with saturated brine (50 mL × 2), dried over anhydrous sodium sulfate, and filtered, and the filtrate was concentrated under reduced pressure to remove the solvent. The resulting residue was purified twice by preparative HPLC (mobile phase: acetonitrile/water; acidic system: 0.04% HCl) to give the target compound **023.** MS-ESI *m*/*z:* 486.2 [M+H]⁺. ¹H NMR (400 MHz, DMSO_*d₆*) δ: 10.84 (s, 1H), 8.89 (dd, *J* = 2.6, 8.8 Hz, 1H), 8.73-8.71 (m, 1H), 7.99 (s, 1H), 4.88 (t, *J =* 6.8 Hz, 2H), 3.05-2.93 (m, 2H), 2.67-2.58 (m, 2H), 2.45 (s, 1H), 1.87 (d, *J* = 12.8 Hz, 1H), 1.67-1.57 (m, 1H), 1.14 (s, 3H).

### Example 24

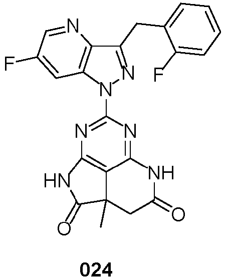

### Synthetic route:

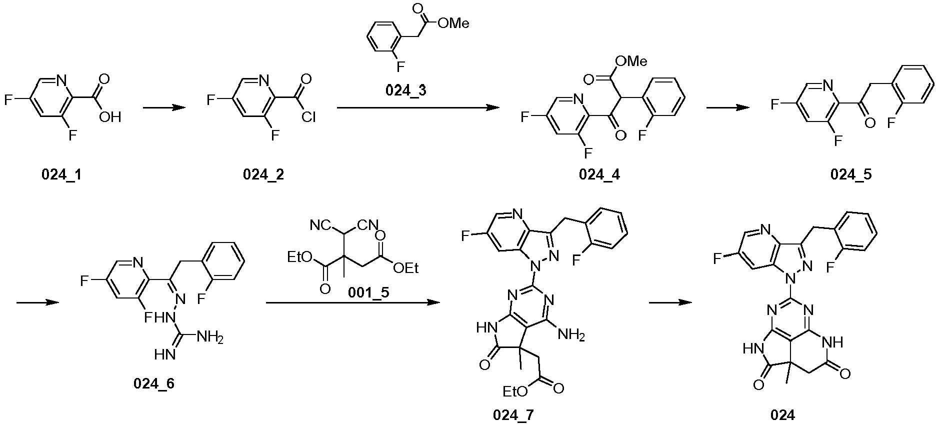

### Step 1: synthesis of intermediate 024_2

Compound 024_1 (50 g, 314.29 mmol) was dissolved in dichloromethane (500 mL) at room temperature under nitrogen atmosphere, and then thionyl chloride (186.95 g, 1.57 mol) and *N,N*-dimethylformamide (2.3 g, 31.43 mmol) were added to the reaction system. The reaction mixture was heated to 40 °C and stirred for 12 h. After the reaction was completed, the reaction mixture was concentrated under reduced pressure. Then toluene (200 mL) was added, and the reaction mixture was concentrated again to give intermediate 024_2.

### Step 2: synthesis of intermediate 024_4

Lithium bis(trimethylsilyl)amide (1 M, 11.27 mL) was dissolved in tetrahydrofuran (18 mL) at room temperature under nitrogen atmosphere. The resulting solution was cooled to -78 °C, and then a solution of **024_3** (26.05 g, 154.90 mmol) in tetrahydrofuran (150 mL) was added dropwise. The reaction mixture was stirred at -78 °C for 1 h, and then a solution of **024_2** (33 g, 185.88 mmol) in tetrahydrofuran (150 mL) was added dropwise. The reaction mixture was stirred at -78 °C for 1 h, and then brought back to room temperature and stirred for 3 h. The reaction mixture was poured into saturated aqueous ammonium chloride solution (600 mL) to quench the reaction and extracted with ethyl acetate (500 mL × 3). The organic phases were combined, dried over anhydrous sodium sulfate, and filtered, and then the filtrate was concentrated under reduced pressure to remove the solvent. The resulting residue was separated by column chromatography (eluent: petroleum ether/ethyl acetate = 1/0 to 3/1, v/v) to give intermediate **024_4.**

### Step 3: synthesis of intermediate 024_5

Intermediate **024_4** (17.5 g, 56.56 mmol) was dissolved in dimethyl sulfoxide (52.5 mL) in a microwave tube at room temperature under nitrogen atmosphere, and sodium chloride (3.64 g, 62.23 mmol) and water (11.2 mL) were added. The resulting mixture was reacted at 150 °C at 12 bar for 30 min and concentrated under reduced pressure to remove the solvent. The resulting residue was separated by column chromatography (eluent: petroleum ether/ethyl acetate = 1/0 to 1/1, v/v) to give intermediate **024_5.**

### Step 4: synthesis of intermediate 024_6

Intermediate **024_5** (11.5 g, 45.78 mmol) and aminoguanidine hydrochloride (10.12 g, 91.56 mmol, hydrochloride) were dissolved in ethylene glycol (120 mL) at room temperature under nitrogen atmosphere, and then boron trifluoride diethyl etherate (14.62 g, 103.00 mmol) was added to the reaction system. The reaction mixture was heated to 120 °C and stirred for 2 h. After the reaction was completed, the reaction mixture was cooled to room temperature. Water (100 mL) was added to the reaction mixture, and the pH was adjusted to 11 with 1 N aqueous sodium hydroxide solution, followed by the extraction with ethyl acetate (20 mL). The organic phase was dried over anhydrous sodium sulfate and filtered, and the filtrate was concentrated under reduced pressure to remove the solvent to give intermediate **024_6.**

### Step 5: synthesis of intermediate 024_7

Intermediate **024_6** (3 g, 9.76 mmol) was dissolved in isopropanol (30 mL) at room temperature, and intermediate **001_5** (4.93 g, 19.53 mmol) and potassium hydroxide (821.66 mg, 14.64 mmol) were added. The reaction mixture was stirred at 80 °C for 12 h. After the reaction was completed, the reaction mixture was concentrated under reduced pressure. The resulting residue was separated by preparative HPLC (mobile phase: acetonitrile/water; hydrochloric acid system: 0.05% HCl) to give intermediate **024_7.**

### Step 6: synthesis of compound 024

Intermediate **024_7** (430 mg, 0.871 mmol) was dissolved in toluene (8 mL) at room temperature, and a solution of trimethylaluminum (2 M, 1.31 mL) in toluene was added. The reaction mixture was heated to 120 °C and reacted for 12 h. After the reaction was completed, the reaction mixture was quenched with 1 N hydrochloric acid (8 mL) and extracted with ethyl acetate (30 mL). The organic phase was dried over anhydrous sodium sulfate and filtered, and the filtrate was concentrated under reduced pressure to remove the solvent. The resulting residue was separated by preparative HPLC (mobile phase: acetonitrile/water; hydrochloric acid system: 0.05% HCl) to give compound **024.** MS-ESI m/z: 448.4 [M+H]⁺. ¹H NMR (400 MHz, CD₃CN) δ: 9.10-8.97 (m, 2H), 8.83 (dd, *J* = 2.4, 10.0 Hz, 1H), 8.61-8.53 (m, 1H), 7.38 (t, *J =* 8.0 Hz, 1H), 7.31-7.25 (m, 1H), 7.14-7.09 (m, 2H), 4.48 (s, 2H), 2.81-2.66 (m, 2H), 1.47 (s, 3H).

### Example 25

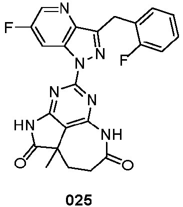

### Synthetic route:

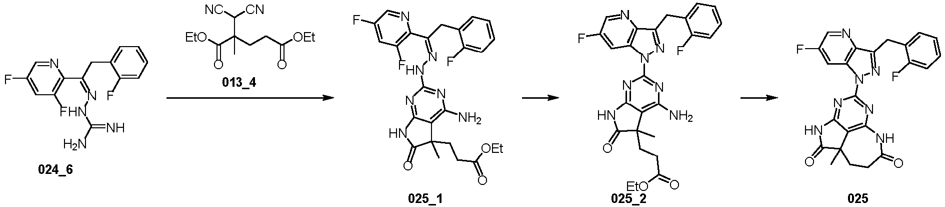

### Step 1: synthesis of intermediate 025_1

Intermediate **024_6** (2 g, 6.51 mmol) was dissolved in tetrahydrofuran (25 mL) and ethanol (25 mL) at room temperature under nitrogen atmosphere, and then compound 013_4 (2.6 g, 9.76 mmol) and sodium ethoxide (1.11 g, 16.27 mmol) were added to the reaction system. The reaction mixture was heated to 80 °C and stirred for 12 h. After the reaction was completed, the reaction mixture was concentrated. The resulting residue was purified by column chromatography (eluent: dichloromethane/methanol = 1/0 to 9/1, v/v) to give intermediate **025_1.**

### Step 2: synthesis of intermediate 025_2

Intermediate **025_1** (0.5 g, 0.948 mmol) was dissolved in pyridine (10 mL) at room temperature. The reaction mixture was stirred at 90 °C for 36 h. After the reaction was completed, the reaction mixture was concentrated. The resulting residue was purified by preparative HPLC (mobile phase: acetonitrile/water; hydrochloric acid system: 0.05% HCl) to give intermediate **025_2.** ¹H NMR (400 MHz, DMSO_*d₆*) *δ:* 11.23 (s, 1H), 9.03-9.00 (m, 1H), 8.67 (s, 1H), 7.37-7.28 (m, 1H), 7.20-7.18 (m, 1H), 7.16-7.10 (m, 3H), 4.42 (s, 2H), 3.95-3.89 (m, 2H), 2.50-2.40 (m, 2H), 1.96-1.90 (m, 3H), 1.36 (s, 3H), 1.09-1.05 (m, 3H).

### Step 3: synthesis of compound 025

Intermediate **025_2** (39 mg, 76.85 µmol) was dissolved in a mixture of xylene (0.8 mL) and acetic acid (2.4 mL) at room temperature under nitrogen atmosphere. The reaction mixture was warmed to 140 °C and stirred for 48 h. After the reaction was completed, the reaction mixture was cooled to room temperature and then concentrated under reduced pressure to remove the solvent. The resulting residue was purified by preparative HPLC (mobile phase: acetonitrile/water; acidic system: 0.05% HCl) to give compound **025.** MS-ESI *m*/*z:* 462.4 [M+H]⁺. ¹H NMR (400 MHz, DMSO_*d₆*) δ: 11.63 (s, 1H), 11.03 (s, 1H), 9.24 (dd, *J =* 10.2, 2.6 Hz, 1H), 8.70 (d, *J =* 2.0 Hz, 1H), 7.40-7.36 (m, 1H), 7.32-7.26 (m, 1H), 7.21-7.11 (m, 2H), 4.44 (s, 2H), 2.83 (t, *J* = 6.8 Hz, 2H), 2.16-2.09 (m, 1H), 2.00-1.93 (m, 1H), 1.33 (s, 3H).

### Example 26

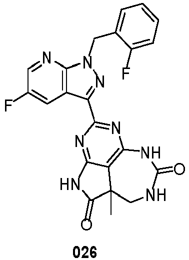

### Synthetic route:

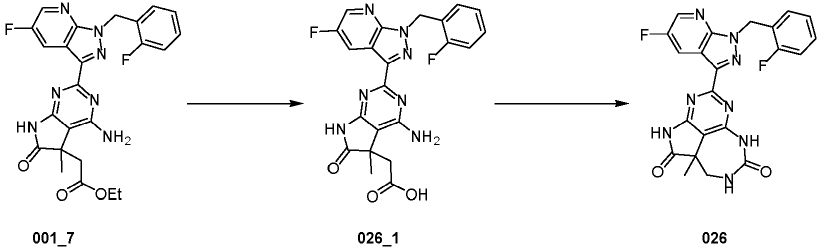

### Step 1: synthesis of intermediate 026_1

Intermediate **001_7** (50 mg, 101.32 µmol) was dissolved in a mixture of tetrahydrofuran (1 mL) and water (1 mL) under nitrogen atmosphere, and then lithium hydroxide monohydrate (8.50 mg, 202.65 µmol) was added. The reaction mixture was reacted at room temperature for 12 h. After the reaction was completed, the reaction mixture was concentrated under reduced pressure to remove the solvent, then adjusted to pH 4-5 with 3 M diluted aqueous hydrochloric acid solution, diluted with acetonitrile, and then purified by preparative HPLC (mobile phase: acetonitrile/water; acidic system: 0.4% HCl) to give intermediate **026_1.** MS-ESI *m*/*z:* 466.1 [M+H]⁺. ¹H NMR (400 MHz, DMSO_*d₆*) δ: 10.96 (s, 1H), 8.83 (dd, *J =* 8.8, 2.8 Hz, 1H), 8.70 (dd, *J =* 2.8, 1.6 Hz, 1H), 7.41-7.32 (m, 1H), 7.26-7.11 (m, 3H), 6.98-6.61 (m, 2H), 5.81 (s, 2H), 3.29 (d, *J* = 16.8 Hz, 1H), 2.72 (d, *J* = 16.8 Hz, 1H), 1.30 (s, 3H).

### Step 2: synthesis of compound 026

Intermediate **026_1** (500 mg, 1.07 mmol) was dissolved in dioxane (8 mL) at room temperature under nitrogen atmosphere, and triethylamine (166.33 mg, 1.64 mmol) and diphenylphosphoryl azide (452.35 mg, 1.64 mmol) were added. The reaction mixture was stirred at 80 °C for 12 h. After the reaction was completed, the reaction mixture was concentrated under reduced pressure to remove the solvent. The resulting residue was separated by preparative HPLC (mobile phase: acetonitrile/water; neutral system: 10 mM NH₄HCO₃) followed by preparative HPLC (mobile phase: acetonitrile/water; acidic system: 0.05% HCl) to give compound **026.** MS-ESI m/z: 463.1 [M+H]⁺. ¹H NMR (400 MHz, DMSO_*d₆*) δ: 11.47 (s, 1H), 10.36 (s, 1H), 9.21 (dd, *J* = 9.2, 2.8 Hz, 1H), 8.68 (d, *J* = 1.6 Hz, 1H), 7.63 (d, *J* = 7.2 Hz, 1H), 7.39-7.33 (m, 1H), 7.25-7.18 (m, 2H), 7.17-7.12 (m, 1H), 5.82 (d, *J* = 2.4 Hz, 2H), 3.23 (d, *J* = 12.8 Hz, 1H), 3.12-3.03 (m, 1H), 1.33 (s, 3H).

### Example 27

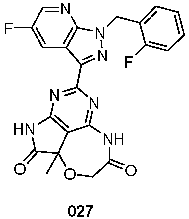

### Synthetic route:

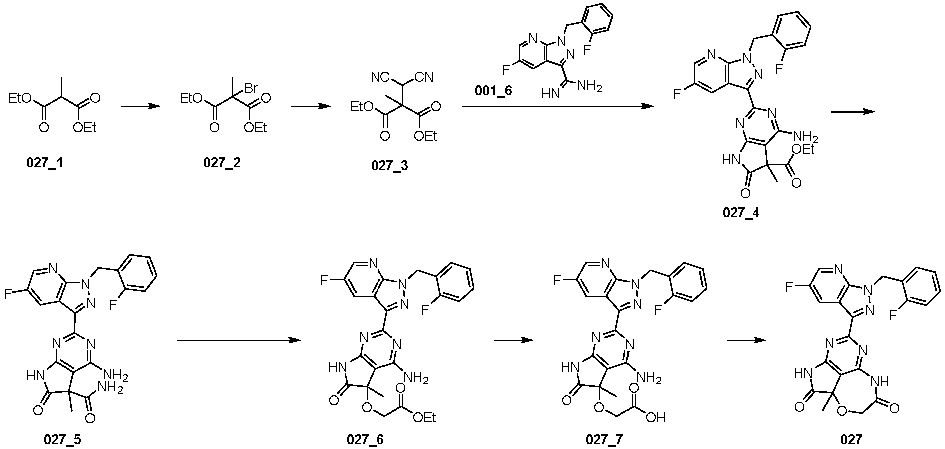

### Step 1: synthesis of intermediate 027_2

Compound **027_1** (25 g, 143.52 mmol) was dissolved in ethyl acetate (100 mL) and chloroform (100 mL) at room temperature under nitrogen atmosphere, and copper bromide (64.11 g, 287.04 mmol) was added. The reaction mixture was warmed to 100 °C and stirred for 36 h. The reaction mixture was cooled to room temperature and filtered, and the filtrate was concentrated under reduced pressure to remove the solvent. The resulting residue was separated by column chromatography (eluent: petroleum ether/ethyl acetate = 1/0 to 50/1, v/v) to give intermediate **027_2.** ¹H NMR (400 MHz, CDCl₃) δ: 4.29 (q, *J* = 7.2 Hz, 4H), 2.09 (s, 3H), 1.31 (t, *J* = 7.2 Hz, 6H).

### Step 2: synthesis of intermediate 027_3

A solution of potassium *tert*-butoxide (1 M, 47.41 mL) in tetrahydrofuran was added dropwise to a solution of malononitrile (2.74 g, 41.49 mmol) in tetrahydrofuran (50 mL) at room temperature under nitrogen atmosphere. The reaction mixture was stirred at room temperature for 0.5 h. Then the reaction mixture was added dropwise to intermediate **027_2** (10 g, 39.51 mmol) in tetrahydrofuran (100 mL) at 75 °C. The reaction mixture was stirred at 75 °C for 12 h. After the reaction was completed, the reaction mixture was cooled to room temperature. Water (100 mL) was added to the reaction mixture, and the pH was adjusted to 3-4 with 3 M diluted hydrochloric acid, followed by the extraction with ethyl acetate (100 mL × 3). The organic phases were combined, washed with saturated brine (100 mL), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 10/1, v/v) to give intermediate **027_3.** ¹H NMR (400 MHz, DMSO_*d₆*) δ: 5.72 (s, 1H), 4.30-4.34 (m, 4H), 1.61 (s, 3H), 1.22 (t, *J=* 7.2 Hz, 6H).

### Step 3: synthesis of intermediate 027_4

Intermediate **001_6** (2 g, 6.18 mmol, hydrochloride) was dissolved in tert-butanol (20 mL) at room temperature under nitrogen atmosphere, and then intermediate **027_3** (2.33 g, 9.76 mmol) and potassium carbonate (3.42 g, 24.71 mmol) were added to the reaction system. The reaction mixture was heated to 80 °C and stirred for 12 h. After the reaction was completed, the reaction mixture was concentrated under reduced pressure to remove the solvent. The resulting residue was separated by column chromatography (eluent: dichloromethane/ethanol = 1/0 to 2/1, v/v) to give intermediate **027_4.**

### Step 4: synthesis of intermediate 027_5

Intermediate **027_4** (1.25 g, 2.61 mmol) was dissolved in methanol (5 mL) and 25% ammonium hydroxide (5 mL) at room temperature under nitrogen atmosphere. The reaction mixture was stirred in a sealed autoclave at 50 °C for 12 h. After the reaction was completed, the reaction mixture was concentrated under reduced pressure to remove the solvent. Then anhydrous toluene (20 mL) was added, and the reaction mixture was concentrated again to give intermediate **027_5.**

### Step 5: synthesis of intermediate 027_6

Intermediate **027_5** (0.5 g, 1.11 mmol) was dissolved in ethyl glycolate (5.46 mL, 56.62 mmol) at room temperature under nitrogen atmosphere, and N-bromosuccinimide (434.69 mg, 2.44 mmol) and 1,8-diazabicyclo[5,4,0]undec-7-ene (0.67 mL, 4.44 mmol) were added. The reaction mixture was stirred at 80 °C for 0.5 h. After the reaction was completed, the reaction mixture was concentrated under reduced pressure. The resulting residue was separated by preparative HPLC (mobile phase: acetonitrile/water; acidic system: 0.04% HCl) to give intermediate **027_6.** MS-ESI m/z: 510.5 [M+H]⁺. ¹H NMR (400 MHz, DMSO_*d₆*) δ: 11.24 (s, 1H), 8.87-8.84 (m, 1H), 8.71-8.70 (m, 1H), 7.38-7.36 (m, 1H), 7.25-7.15 (m, 3H), 7.13 (br.s, 1H), 5.82 (s, 2H), 4.04 (q, *J =* 7.2 Hz, 2H), 3.84 (s, 2H), 1.60 (s, 3H), 1.13 (t, *J* = 7.2 Hz, 3H).

### Step 6: synthesis of intermediate 027_7

Intermediate **027_6** (300 mg, 0.589 mmol) was dissolved in methanol (0.75 mL) and water (2.25 mL) at room temperature under nitrogen atmosphere, and lithium hydroxide monohydrate (123.54 mg, 2.94 mmol) was added. The reaction mixture was stirred at room temperature for 2 h. After the reaction was completed, the reaction mixture was concentrated under reduced pressure to remove methanol. Then water (20 mL) was added, followed by the extraction with ethyl acetate (40 mL). The organic phase was discarded, and the aqueous phase was adjusted to about pH 4 with 1 M diluted hydrochloric acid and then extracted with 2-methyltetrahydrofuran (60 mL). The organic phase was concentrated under reduced pressure to give intermediate **027_7.**

### Step 7: synthesis of compound 027

Intermediate **027_7** (0.15 g, 0.312 mmol) was dissolved in dioxane (5 mL) at room temperature under nitrogen atmosphere, and thionyl chloride (3 mL, 41.35 mmol) was added. The reaction mixture was stirred at 80 °C for 2 h. After the reaction was completed, the reaction mixture was concentrated. The resulting residue was separated by preparative HPLC (mobile phase: acetonitrile/water; hydrochloric acid system: 0.05% HCl) to give compound 027. MS-ESI m/z: 464.4 [M+H]⁺. ¹H NMR (400 MHz, DMSO_ *d*₆) δ: 11.62 (s, 1H), 11.45 (s, 1H), 9.00 (dd, *J=* 2.8, 9.2 Hz, 1H), 8.74 (s, 1H), 7.39-7.36 (m, 1H), 7.25-7.21 (m, 2H), 7.18-7.14 (m, 1H), 5.85 (s, 2H), 4.61 (d, *J =* 16 Hz, 1H), 4.44 (d, *J =* 16 Hz, 1H),1.57 (s, 3H).

### Example 28

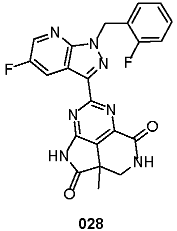

### Synthetic route:

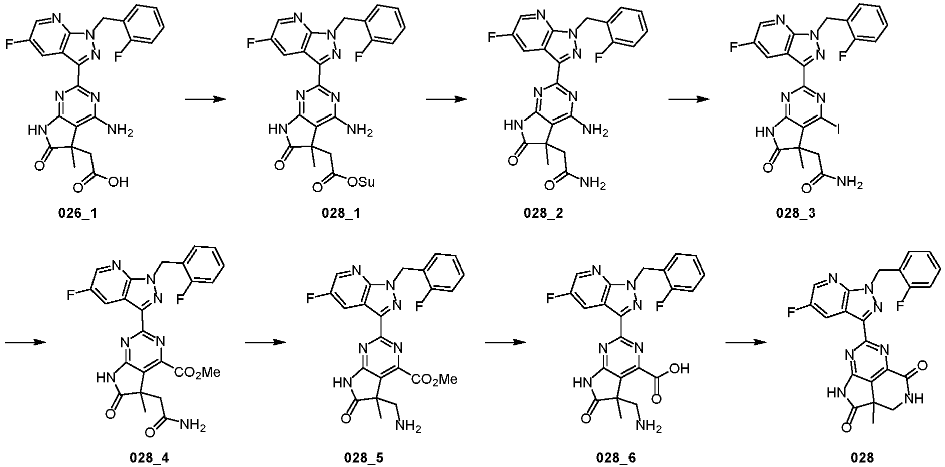

### Step 1: synthesis of intermediate 028_1

Intermediate **026_1** (4.6 g, 9.88 mmol) was dissolved in acetonitrile (50 mL) at room temperature under nitrogen atmosphere, and *N*-hydroxysuccinimide (1.19 g, 10.38 mmol) and *N*,*N*-dicyclohexylcarbodiimide (2.08 g,10.08 mmol) were added. The reaction mixture was stirred at room temperature for 4 h, supplemented with *N-*hydroxysuccinimide (227.50 mg, 1.98 mmol) and *N,N*-dicyclohexylcarbodiimide (407.86 mg, 1.98 mmol), and stirred at room temperature for another 12 h. After the reaction was completed, the reaction mixture was filtered, and the filter cake was rinsed with ice-cold acetonitrile (20 mL). The filtrate was collected and concentrated under reduced pressure to remove the solvent to give intermediate **028_1.**

### Step 2: synthesis of intermediate 028_2

Intermediate **028_1** (5.5 g, 9.78 mmol) was dissolved in tetrahydrofuran (80 mL) under nitrogen atmosphere, and ammonium hydroxide (6.85 g, 48.89 mmol) was slowly added dropwise. The reaction mixture was stirred at room temperature for 12 h. After the reaction was completed, the reaction mixture was concentrated under reduced pressure to remove the solvent, diluted with water (100 mL), and extracted with 2-methyltetrahydrofuran (200 mL × 3). The organic phases were combined and concentrated under reduced pressure to remove the solvent. The resulting solid was suspended in anhydrous toluene (20 mL × 2), and the organic solvent was removed by concentration under reduced pressure. The resulting residue was slurried with methyl *tert-butyl* ether (50 mL) for 10 min. The mixture was filtered, and the filter cake was collected and concentrated under reduced pressure to remove organic solvent to give intermediate **028_2.**

### Step 3: synthesis of intermediate 028_3

Intermediate **028_2** (3.3 g, 7.11 mmol) was dissolved in dioxane (60 mL) at room temperature under nitrogen atmosphere, and isoamyl nitrite (4.16 g, 35.53 mmol) and diiodomethane (5.71 g, 21.32 mmol) were added. The reaction mixture was warmed to 85 °C and stirred for 4 h. After the reaction was completed, the reaction mixture was brought back to room temperature and concentrated under reduced pressure to remove the solvent. A mixture (5 mL) of ethyl acetate and dichloromethane (1:1) was added to the resulting residue, and the resulting mixture was stirred for 10 min and filtered. The filter cake was collected and concentrated under reduced pressure to remove the solvent to give intermediate **028_3.**

### Step 4: synthesis of intermediate 028_4

Intermediate **028_3** (2.1 g, 3.65 mmol) was dissolved in *N*,*N*-methylformamide (12 mL) and methanol (5 mL) at room temperature under nitrogen atmosphere, and 1,1-bis(diphenylphosphino)ferrocene palladium dichloride (186.96 mg, 255.52 µmol) and *N*,*N*-diisopropylethylamine (10.4 mL) were added. The reaction mixture was purged three times with carbon monoxide, warmed to 80 °C and stirred under carbon monoxide 103,421 kPa, 15 psi) for 12 h, supplemented with 1,1-bis(diphenylphosphino)ferrocene palladium dichloride (186.96 mg, 255.52 µmol), and stirred at 80 °C for another 12 h. After the reaction was completed, the reaction mixture was cooled to room temperature and concentrated under reduced pressure to remove the solvent. The resulting residue was separated by column chromatography (eluent: petroleum ether/ethyl acetate =2/1, dichloromethane:methanol = 1/0 to 10/1, v/v) to give intermediate **028_4.**

### Step 5: synthesis of intermediate 028_5

Intermediate **028_4** (300 mg, 591.19 µmol) was dissolved in acetonitrile (4 mL) and water (0.8 mL) at room temperature under nitrogen atmosphere, and bis(trifluoroacetoxy)iodobenzene (1.27 g, 2.96 mmol) was added slowly at 0 °C. The reaction mixture was stirred at room temperature for 12 h, supplemented with bis(trifluoroacetoxy)iodobenzene (1.27 g, 2.96 mmol) at 0 °C, and warmed to 35 °C and stirred for 12 h. After the reaction was completed, the reaction mixture was cooled to room temperature, adjusted to pH 3-4 with 1 M diluted aqueous hydrochloric acid solution, and extracted with 2-methyltetrahydrofuran (20 mL × 3). The organic phases were combined, washed with saturated brine (20 mL), dried over anhydrous sodium sulfate, and filtered, and the filtrate was concentrated under reduced pressure to remove the solvent. The resulting residue was separated by column chromatography (eluent: dichloromethane/methanol = 1/0 to 10/1, v/v) to give intermediate **028_5.**

### Step 6: synthesis of intermediate 028_6

Intermediate **028_5** (45 mg, 93.86 µmol) was dissolved in tetrahydrofuran (1 mL) and water (0.2 mL) at room temperature under nitrogen atmosphere, and then lithium hydroxide monohydrate (7.88 mg, 187.72 µmol) was added. The reaction mixture was stirred at room temperature for 12 h. After the reaction was completed, the reaction mixture was adjusted to pH 5-7 with 3 M diluted hydrochloric acid and then extracted with 2-methyltetrahydrofuran (10 mL × 4). The organic phases were combined, washed with saturated brine (5 mL), dried over anhydrous sodium sulfate, and filtered, and the filtrate was concentrated under reduced pressure to remove the solvent to give intermediate **028_6.**

### Step 7: synthesis of compound 028

Intermediate **028_6** (160 mg, 343.78 µmol) was dissolved in thionyl chloride (2 mL) and dioxane (2 mL) at room temperature under nitrogen atmosphere. The reaction mixture was warmed to 75 °C and stirred for 2 h. After the reaction was completed, the reaction mixture was cooled to room temperature and concentrated under reduced pressure to remove the solvent. The resulting residue was purified by preparative HPLC (mobile phase: acetonitrile/water; acidic system: 0.04% HCl) to give compound **028.** MS-ESI *m*/*z:* 448.1 [M+H]⁺. ¹H NMR (400 MHz, DMSO_*d₆*) δ: 11.73 (s, 1H), 8.77 (s, 1H), 8.61 (d, *J* = 7.6 Hz, 1H), 8.35 (d, *J* = 4.0 Hz, 1H), 7.37 (d, *J* = 6.4 Hz, 1H), 7.10-7.30 (m, 3H), 5.87 (s, 2H), 3.68 (d, *J =* 12.4 Hz, 1H), 3.28-3.29 (m, 1H), 1.54 (s, 3H).

### Examples 29 and 30

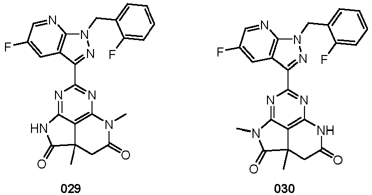

### Synthetic route:

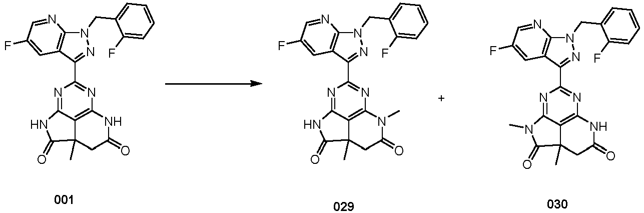

Compound **001** (300 mg, 670.55 µmol) was dissolved in tetrahydrofuran (5 mL) at room temperature under nitrogen atmosphere. The resulting solution was cooled to -65 °C, and a solution of lithium bis(trimethylsilyl)amide (1 M, 1.41 mL) in *n*-hexane was added. The reaction mixture was stirred at -65 °C for 0.5 h, and then iodomethane (99.94 mg, 704.07 µmol) was added dropwise. The reaction mixture was stirred at room temperature for 12 h, supplemented with iodomethane (95.18 mg, 670.55 µmol), and stirred at room temperature for 3 h. After the reaction was completed, 1 M diluted aqueous hydrochloric acid solution (0.2 mL) was added to the reaction mixture, and the solvent was removed by concentration under reduced pressure. The resulting residue was separated by preparative HPLC (mobile phase: acetonitrile/water; neutral system: 10 mM NH₄HCO₃) to give compounds **029** and **030.**

029: MS-ESI *m*/*z:* 462.1 [M+H]⁺. ¹H NMR (400 MHz, DMSO_*d₆*) δ: 11.46 (s, 1H), 8.75 (d, *J =* 0.8 Hz, 1H), 8.56 (dd, *J* = 8.4, 2.8 Hz, 1H), 7.31-7.41 (m, 1H), 7.23 (t, *J =* 8.8 Hz, 2H), 7.15 (t, *J =* 7.6 Hz, 1H), 5.87 (s, 2H), 3.39 (s, 3H), 3.07 (d, *J =* 15.2 Hz, 1H), 2.68 (d, *J =* 15.2 Hz, 1H), 1.38 (s, 3H).

030: MS-ESI *m*/*z:* 462.1 [M+H]⁺. ¹H NMR (400 MHz, DMSO_*d₆*) δ: 11.18 (s, 1H), 8.70-8.80 (m, 2H), 7.33-7.40 (m, 1H), 7.19-7.27 (m, 1H), 7.11-7.19 (m, 2H), 5.88 (s, 2H), 3.21 (s, 3H), 2.96 (d, *J =* 15.2 Hz, 1H), 2.55-2.58 (m, 1H), 1.39 (s, 3H).

### Example 31

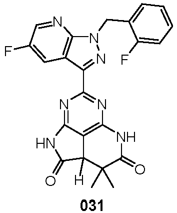

### Synthetic route:

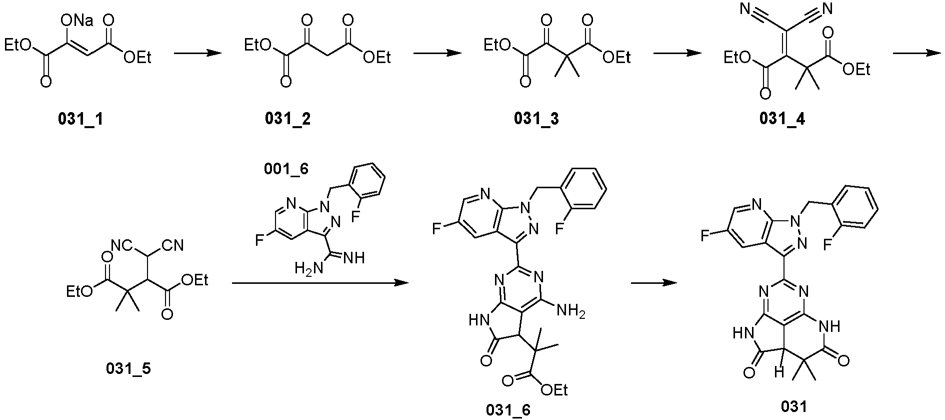

### Step 1: synthesis of intermediate 031_2

**031_1** (20 g, 95.17 mmol) was dissolved in water (150 mL) at room temperature under nitrogen atmosphere, and diluted hydrochloric acid (1 M, 70 mL) was added. The reaction mixture was stirred at room temperature for 10 min. After the reaction was completed, the reaction mixture was extracted with ethyl acetate (50 mL × 3). The organic phases were combined, washed with saturated brine (150 mL × 2), dried over anhydrous sodium sulfate, and filtered, and the filtrate was concentrated under reduced pressure to remove the solvent to give intermediate **031_2.**

### Step 2: synthesis of intermediate 031_3

Intermediate **031_2** (13 g, 69.08 mmol) was dissolved in acetone (400 mL) at room temperature under nitrogen atmosphere, and potassium carbonate (19.10 g, 138.17 mmol) was added. The reaction mixture was stirred at room temperature for 20 min, and then iodomethane (58.83 g, 414.50 mmol) was added. The reaction mixture was stirred at 60 °C for 12 h. After the reaction was completed, the reaction mixture was concentrated under reduced pressure to remove the solvent, and the resulting residue was separated by column chromatography (eluent: petroleum ether/ethyl acetate = 1/0 to 10/1, v/v) to give intermediate **031_3.** ¹H NMR (400 MHz, CDCl₃) δ: 4.32 (q, *J =* 7.2 Hz, 2H), 4.19 (q, *J* = 7*.*2 Hz, 2H), 1.56 (s, 6H), 1.37 (t, *J* = 7.2 Hz, 3H), 1.24 (t, *J =* 7.2 Hz, 3H).

### Step 3: synthesis of intermediate 031_4

Intermediate **031_3** (2.46 g, 11.38 mmol) and malononitrile (3.01 g, 45.51 mmol) were dissolved in ethanol (25 mL) at room temperature under nitrogen atmosphere, and pyridine (4.50 g, 56.88 mmol, 4.59 mL) was added. The reaction system was warmed to 70 °C and stirred for 12 h. After the reaction was completed, the reaction system was cooled to room temperature, adjusted to pH 3-4 with 1 M hydrochloric acid solution, and diluted with ethyl acetate (50 mL) and water (30 mL). The organic phase was separated and collected, and the aqueous phase was extracted with ethyl acetate (50 mL × 3). The organic phases were combined, washed successively with 1 N hydrochloric acid solution (50 mL × 3) and saturated brine (50 mL × 2), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 1/1, v/v) to give intermediate **031_4.**

### Step 4: synthesis of intermediate 031_5

Intermediate **031_4** (3 g, 11.35 mmol) was dissolved in chloroform (15 mL) and ethanol (15 mL), and then diethyl 2,6-dimethyl-1,4-dihydro-3,5-pyridinedicarboxylate (3.16 g, 12.49 mmol) was added. The reaction system was warmed to 50 °C and stirred for 12 h, supplemented with diethyl 2,6-dimethyl-1,4-dihydro-3,5-pyridinedicarboxylate (862.60 mg, 3.41 mmol), warmed to 50 °C and stirred for 12 h, supplemented with diethyl 2,6-dimethyl-1,4-dihydro-3,5-pyridinedicarboxylate (2.88 g, 11.35 mmol), and warmed to 50 °C and stirred for 12 h. After the reaction was completed, the reaction mixture was cooled to room temperature and concentrated under reduced pressure to remove the solvent. The resulting residue was separated by column chromatography (eluent: petroleum ether/ethyl acetate = 1/0 to 7/1, v/v) to give intermediate **031_5.** ¹H NMR (400 MHz, CDCl₃) δ: 4.34-4.19 (m, 5H), 3.66 (d, *J=* 7.0 Hz, 1H), 1.38-1.29 (m, 12H).

### Step 5: synthesis of intermediate 031_6

Intermediate **031_5** (1.73 g, 6.50 mmol) and **001_6** (1.31 g, 4.06 mmol, hydrochloride) were dissolved in *tert-*butanol (20 mL) at room temperature under nitrogen atmosphere, and then potassium carbonate (1.40 g, 10.15 mmol) was added. The reaction system was warmed to 85 °C and stirred at 85 °C for 12 h. After the reaction was completed, the reaction system was cooled to room temperature, quenched with water (35 mL), and diluted with ethyl acetate (50 mL). The organic phase was separated and collected, and the aqueous phase was extracted with ethyl acetate (40 mL × 3). The organic phases were combined, washed with saturated brine (40 mL × 3), dried over anhydrous sodium sulfate, and filtered, and the filtrate was concentrated under reduced pressure to give a crude product. The resulting crude product was separated by column chromatography (eluent: petroleum ether/ethyl acetate = 1/0 to 0/1, v/v) to give intermediate **031_6.**

### Step 6: synthesis of compound 031

Intermediate **031_6** (0.9 g, 1.77 mmol) was dissolved in toluene (18 mL) at room temperature under nitrogen atmosphere, and then a solution of trimethylaluminum (2 M, 2.84 mL) in toluene was added dropwise to the reaction system. The reaction mixture was warmed to 80 °C and stirred for 12 h. After the reaction was completed, the reaction mixture was slowly poured into diluted hydrochloric acid (1 M, 30 mL) to quench the reaction and extracted with ethyl acetate (30 mL × 3). The organic phases were combined, dried over anhydrous sodium sulfate, and filtered, and the filtrate was concentrated under reduced pressure. The residue was separated by preparative HPLC (mobile phase: acetonitrile/water; acidic system: 0.04% HCl) to give compound **031.** MS-ESI m/z: 462.2[M+H]⁺. ¹H NMR (400 MHz, DMSO_d₆) δ: 11.48 (s, 1H), 11.11 (s, 1H), 8.75-8.74 (m, 1H), 8.72-8.69 (m, 1H), 7.40-7.35 (m, 1H), 7.27-7.21 (m, 2H), 7.18-7.15 (m, 1H), 5.84 (s, 2H), 3.97 (s, 1H), 1.46 (s, 3H), 0.77 (s, 3H).

### Examples 32 and 33

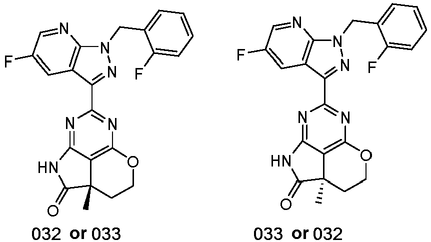

### Synthetic route:

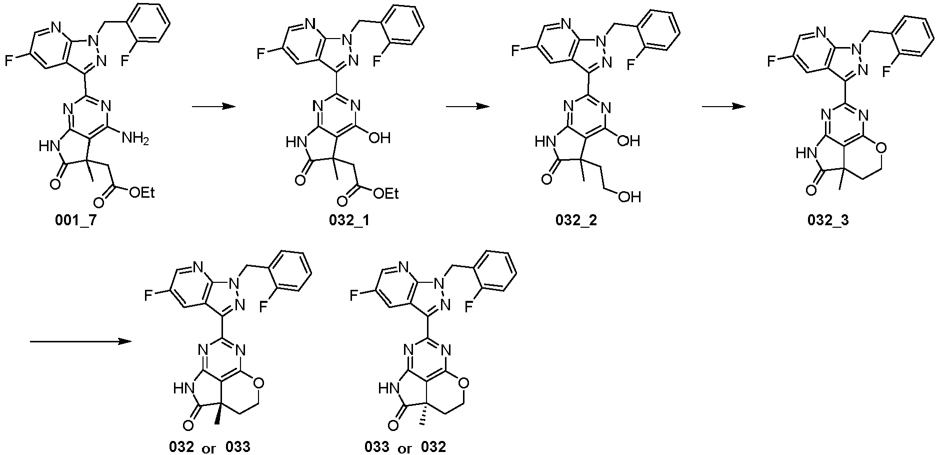

### Step 1: synthesis of intermediate 032_1

Intermediate **001_7** (3 g, 6.08 mmol) was dissolved in glacial acetic acid (20 mL) at room temperature under nitrogen atmosphere, and then a solution of sodium nitrite (1.26 g, 18.24 mmol) in water (10 mL) was added to the reaction system. The reaction mixture was heated to 90 °C and stirred for 2 h. After the reaction was completed, the reaction mixture was cooled to room temperature, diluted with ethyl acetate (100 mL), and then washed with saturated aqueous sodium bicarbonate (40 mL). The organic phase was dried over anhydrous sodium sulfate and filtered, and the filtrate was concentrated under reduced pressure to remove the solvent. The resulting residue was separated by column chromatography (eluent: petroleum ether/ethyl acetate = 1/0 to 1/3, v/v) to give intermediate **032_1.**

### Step 2: synthesis of intermediate 032_2

Intermediate **032_1** (0.3 g, 0.607 mmol) was dissolved in tetrahydrofuran (9 mL) at room temperature, and Red-Al (1.93 g, 6.67 mmol, 70% purity) was added at 5-10 °C. The reaction mixture was stirred at room temperature for 16 h. After the reaction was completed, the reaction mixture was quenched with 1 M hydrochloric acid (20 mL) and then extracted with ethyl acetate (50 mL). The organic phase was dried over anhydrous sodium sulfate and filtered, and the filtrate was concentrated under reduced pressure to remove the solvent. The resulting residue was separated by column chromatography (eluent: dichloromethane/methanol = 1/0 to 9/1, v/v) to give intermediate **032_2.**

### Step 3: synthesis of intermediate 032_3

Intermediate **032_2** (120 mg, 0.265 mmol) was dissolved in tetrahydrofuran (2 mL) at room temperature, and a solution of *N,N,N',N'*-tetramethylazodicarbonamide (205.52 mg, 1.19 mmol) in tetrahydrofuran (0.5 mL) followed by a solution of tri-tert-butylphosphine (241.49 mg, 1.19 mmol) in tetrahydrofuran (0.5 mL) was added at 0 °C. The reaction mixture was heated to 70 °C and stirred for 3 h. After the reaction was completed, the reaction mixture was concentrated under reduced pressure to remove the solvent. The resulting crude product was separated by preparative HPLC (mobile phase: acetonitrile/water; neutral system: 10 mM NH₄HCO₃) to give intermediate **032_3.** MS-ESI m/z: 435.4 [M+H]⁺. ¹H NMR (400 MHz, DMSO_*d₆*) δ: 11.24 (s, 1H), 8.74 (d, *J =* 1.0 Hz, 1H), 8.52-8.49 (m, 1H), 7.40-7.34 (m, 1H), 7.26-7.14 (m, 3H), 5.84 (s, 2H), 4.72-4.59 (m, 2H), 2.00-1.97 (m, 1H), 1.82-1.74 (m, 1H), 1.46 (s, 3H)

### Step 3: synthesis of compounds 032 and 033

Intermediate **032_3** was separated by chiral column chromatography (column model: DAICEL CHIRALPAK AS (250 mm × 30 mm, 10 µm); mobile phase: [Neu-ACN]%: 45%-45%, 12 min) to give **032** and **033.**

**032** (retention time: 1.269 min): MS-ESI m/z: 435.1 [M+H]⁺. ¹H NMR (400 MHz, DMSO_d₆) *δ:* 11.23 (s, 1H), 8.73 (s, 1H), 8.50 (dd, *J* = 8.4, 2.8 Hz, 1H), 7.39-7.34 (m, 1H), 7.29-7.20 (m, 2H), 7.17-7.14 (m, 1H), 5.84 (s, 2H), 4.70-4.59 (m, 2H), 1.99 (d, *J =* 13.6 Hz, 1H), 1.81-1.73 (m, 1H), 1.46 (s, 3H).

**033** (retention time: 1.554 min): MS-ESI m/z: 435.1 [M+H]⁺. ¹H NMR (400 MHz, DMSO_*d₆*) δ: 11.24 (s, 1H), 8.73 (s, 1H), 8.50 (dd, *J* = 8.4, 2.8 Hz, 1H), 7.39-7.34 (m, 1H), 7.29-7.20 (m, 2H), 7.17-7.14 (m, 1H), 5.84 (s, 2H), 4.70-4.59 (m, 2H), 1.99 (d, *J =* 13.6 Hz, 1H), 1.81-1.73 (m, 1H), 1.46 (s, 3H).

### Example 34

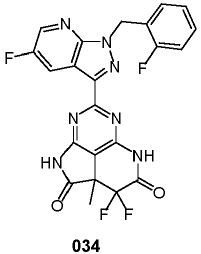

### Synthetic route:

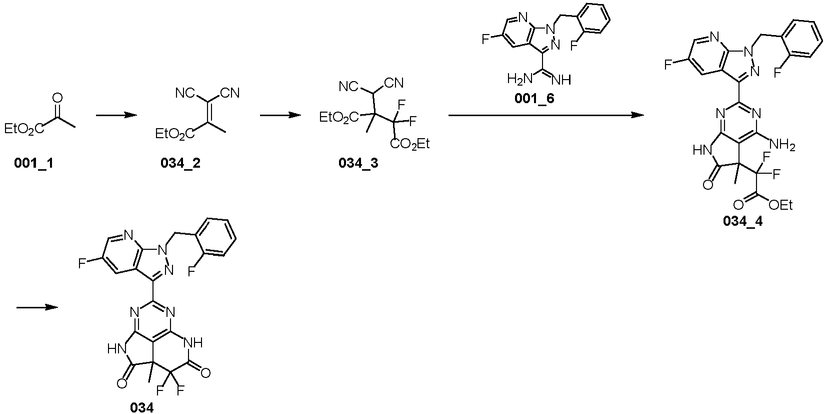

### Step 1: synthesis of intermediate 034_2

Compound **001_1** (28.67 g, 246.87 mmol, 27.3 mL) and malononitrile (17.12 g, 259.21 mmol, 16.31 mL) were dissolved in tetrahydrofuran (300 mL) at room temperature under nitrogen atmosphere. The reaction mixture was stirred for 1 h. After the reaction was completed, a solution of intermediate **034_2** in tetrahydrofuran was obtained.

### Step 2: synthesis of intermediate 034_3

Zinc powder (48.32 g, 738.98 mmol) activated by diluted hydrochloric acid and titanocene dichloride (6.38 g, 24.63 mmol, 3.99 mL) were placed in a dry reaction flask at room temperature under nitrogen atmosphere, and tetrahydrofuran (340 mL) was added. A tenth of a solution of ethyl difluorobromoacetate (100 g, 492.65 mmol, 63.29 mL) in tetrahydrofuran (60 mL) was added to the reaction system. The reaction system was stirred at room temperature, and the remaining solution of ethyl difluorobromoacetate in tetrahydrofuran was slowly added dropwise after the reaction was initiated. The reaction system was stirred at room temperature for 0.5 h and then filtered, and the filter cake was rinsed with tetrahydrofuran (60 mL). The filtrates were combined and added to a solution of intermediate **034_2** (40.44 g, 246.33 mmol) in tetrahydrofuran at room temperature. The reaction system was stirred at room temperature for 12 h. After the reaction was completed, the pH was adjusted to about 3 with 1 N hydrochloric acid solution, and ethyl acetate (250 mL) and water (200 mL) were added for dilution. The organic phase was separated and collected, and the aqueous phase was extracted with ethyl acetate (150 mL × 3). The organic phases were combined, washed with saturated brine (250 mL × 3), dried over anhydrous sodium sulfate, and filtered, and the filtrate was concentrated under reduced pressure to give a crude product. The resulting crude product was separated by column chromatography (eluent: petroleum ether/ethyl acetate = 1/0 to 9/1, v/v) to give intermediate **034_3.** ¹H NMR (400 MHz, CDCl₃) δ: 4.69 (s, 1H), 4.46-4.31 (m, 4H), 1.81 (s, 3H), 1.46-1.31 (m, 6H).

### Step 3: synthesis of intermediate 034_4

Intermediate **034_3** (7.6 g, 26.37 mmol) was dissolved in *tert*-butanol (110 mL) at room temperature under nitrogen atmosphere, and then potassium carbonate (5.69 g, 41.19 mmol) and **001_6** (5.33 g, 16.48 mmol, hydrochloride) were added. The reaction mixture was warmed to 85 °C and stirred for 40 h. After the reaction was completed, the reaction mixture was cooled to room temperature and diluted with water (100 mL) and ethyl acetate (100 mL). The organic phase was separated and collected, and the aqueous phase was extracted with ethyl acetate (100 mL × 3). The organic phases were combined, washed with saturated brine (50 mL), dried over anhydrous sodium sulfate, and filtered, and then the filtrate was concentrated under reduced pressure to remove the solvent. The resulting residue was separated by column chromatography (eluent: petroleum ether/2-methyltetrahydrofuran = 1/0 to 1/1, v/v) to give intermediate **034_4.** ¹H NMR (400 MHz, DMSO_*d₆*) δ: 11.80 (d, *J =* 2.8 Hz, 1H), 9.01 (dd, *J=* 2.4, 8.8 Hz, 1H), 8.69 (dd, *J* = 1.6, 2.4 Hz, 1H), 7.38-7.33 (m, 1H), 7.24-7.11 (m, 3H), 6.94 (s, 1H), 5.81 (s, 2H), 4.23-4.09 (m, 2H), 1.81 (d, *J =* 2.8 Hz, 3H), 1.17 (t, *J =* 7.2 Hz, 3H).

### Step 4: synthesis of compound 034

Intermediate **034_4** (300 mg, 566.63 µmol) was dissolved in toluene (6 mL) at room temperature under nitrogen atmosphere, and a solution of trimethylaluminum (2 M, 849.95 µL, 1.70 mmol) in toluene was added. The reaction mixture was warmed to 80 °C and stirred for 4 h. After the reaction was completed, the reaction mixture was cooled to room temperature, quenched by slowly adding diluted hydrochloric acid (3 M, 3.68 mL), and diluted with 2-methyltetrahydrofuran (10 mL). Then the aqueous phase was separated and extracted with 2-methyltetrahydrofuran (10 mL × 2). The organic phases were combined, dried over anhydrous sodium sulfate, and filtered, and then the filtrate was concentrated under reduced pressure to remove the solvent. The crude product was separated by preparative HPLC (mobile phase: acetonitrile/water; acidic system: 0.04% HCl) to give compound 034. MS-ESI m/z: 484.1 [M+H]⁺. ¹H NMR (400 MHz, DMSO_*d₆*) δ: 12.32 (d, *J* = 2.0 Hz, 1H), 12.02 (s, 1H), 8.77 (dd, *J* = 1.6, 2.8 Hz, 1H), 8.65 (dd, *J* = 2.8, 8.8 Hz, 1H), 7.41-7.35 (m, 1H), 7.30-7.15 (m, 3H), 5.86 (s, 2H), 1.55 (s, 3H).

### Examples 35 and 36

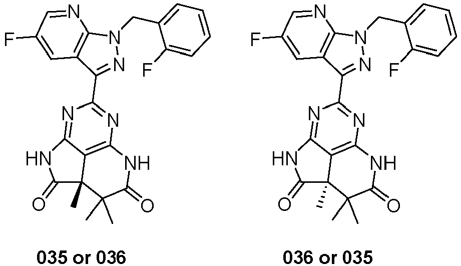

### Synthetic route:

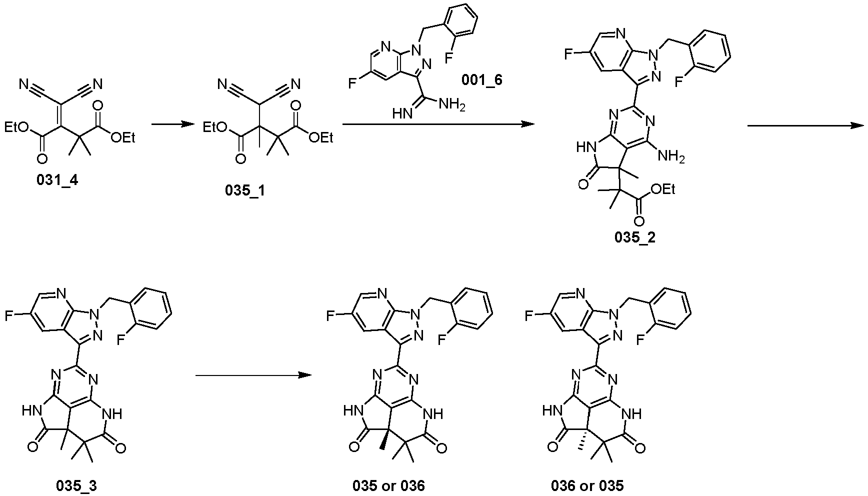

### Step 1: synthesis of intermediate 035_1

Intermediate **031_4** (2.1 g, 7.95 mmol) was dissolved in anhydrous tetrahydrofuran (20 mL) at room temperature under nitrogen atmosphere. The reaction system was cooled to 0 °C, and a methylmagnesium bromide solution (3 M, 3.97 mL) was added dropwise. The reaction system was stirred at 0 °C for 15 min. After the reaction was completed, the reaction mixture was quenched with 1 N diluted hydrochloric acid (50 mL), diluted with water (30 mL), and then extracted with ethyl acetate (50 mL), and the aqueous phase was also extracted with ethyl acetate (10 mL × 3). The organic phases were combined, washed with saturated brine (40 mL × 3), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 9/1, v/v) to give intermediate **035_1.** ¹H NMR (400 MHz, CDCl₃) δ: 4.88 (s, 1H), 4.35-4.12 (m, 4H), 1.63 (s, 3H), 1.42 (s, 3H), 1.33-1.31 (m, 9H).

### Step 2: synthesis of intermediate 035_2

Intermediate **001_6** (608.22 mg, 1.88 mmol, hydrochloride) and intermediate **035_1** (790 mg, 2.82 mmol) were dissolved in *tert*-butanol (12 mL) at room temperature under nitrogen atmosphere, and potassium carbonate (778.99 mg, 5.64 mmol) was added. The reaction system was warmed to 85 °C and stirred for 12 h. After the reaction was completed, the reaction mixture was cooled to room temperature, diluted with water (30 mL), and extracted with ethyl acetate (40 mL), and the aqueous phase was extracted with ethyl acetate (30 mL × 3). The organic phases were combined, washed with saturated brine (30 mL × 3), dried over anhydrous sodium sulfate, and filtered, and the filtrate was concentrated under reduced pressure to remove the solvent. The resulting residue was separated by column chromatography (eluent: petroleum ether/ethyl acetate = 1/0 to 1/4, v/v) to give intermediate **035_2.**

### Step 3: synthesis of intermediate 035_3

Intermediate **035_2** (465 mg, 891.63 µmol) was dissolved in toluene (10 mL) at room temperature under nitrogen atmosphere, and then a solution of trimethylaluminum (2 M, 1.43 mL) in toluene was added. The reaction system was warmed to 80 °C and stirred for 12 h. After the reaction was completed, the reaction mixture was cooled to room temperature, quenched with 3 M hydrochloric acid solution (6 mL), and then diluted with ethyl acetate (40 mL) and water (20 mL). The organic phase was separated and collected, and the aqueous phase was extracted with ethyl acetate (40 mL × 3). The organic phases were combined, washed with saturated brine (50 mL × 2), dried over anhydrous sodium sulfate, and filtered, and the filtrate was concentrated under reduced pressure to remove the solvent. The resulting residue was separated by preparative HPLC (mobile phase: acetonitrile/water; acidic system: 0.04% HCl) to give intermediate **035_3.** MS-ESI m/z: 476.2[M+H]⁺. ¹H NMR (400 MHz, DMSO_*d₆*) δ: 11.53 (s, 1H), 11.18 (s, 1H), 8.75-8.69 (m, 2H), 7.40-7.35 (m, 1H), 7.28-7.14 (m, 3H), 5.84 (s, 2H), 1.32 (d, *J* = 5.8 Hz, 6H), 0.76 (s, 3H).

### Step 4: synthesis of compounds 035 and 036

Intermediate 035_3 was separated by chiral column chromatography (column model: DAICEL CHIRALCEL OD (250 mm × 30 mm, 10 µm); mobile phase: [Neu-ACN]%: 50%-50%, 12 min) to give compounds 035 and 036.

035 (retention time: 1.696 min): MS-ESI m/z: 476.2 [M+H]⁺. ¹H NMR (400 MHz, DMSO_*d₆*) δ: 11.53 (s, 1H), 11.19 (s, 1H), 8.74 (dd, *J* = 1.6, 2.6 Hz, 1H), 8.70 (dd, *J* = 2.8, 8.8 Hz, 1H), 7.41-7.33 (m, 1H), 7.29-7.13 (m, 3H), 5.84 (s, 2H), 1.32 (d, *J =* 5.6 Hz, 6H), 0.76 (s, 3H).

036 (retention time: 2.403 min): MS-ESI m/z: 476.2 [M+H]⁺. ¹H NMR (400 MHz, DMSO_*d₆*) δ: 11.53 (s, 1H), 11.18 (s, 1H), 8.74 (dd, *J =* 1.6, 2.6 Hz, 1H), 8.70 (dd, *J* = 2.8, 8.8 Hz, 1H), 7.42-7.32 (m, 1H), 7.31-7.12 (m, 3H), 5.84 (s, 2H), 1.32 (d, *J =* 5.6 Hz, 6H), 0.76 (s, 3H).

### Examples 37 and 38

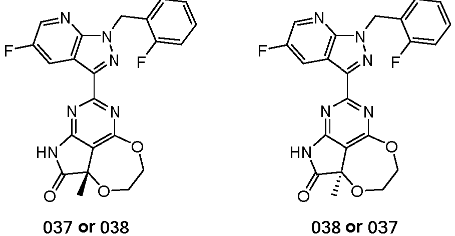

### Synthetic route:

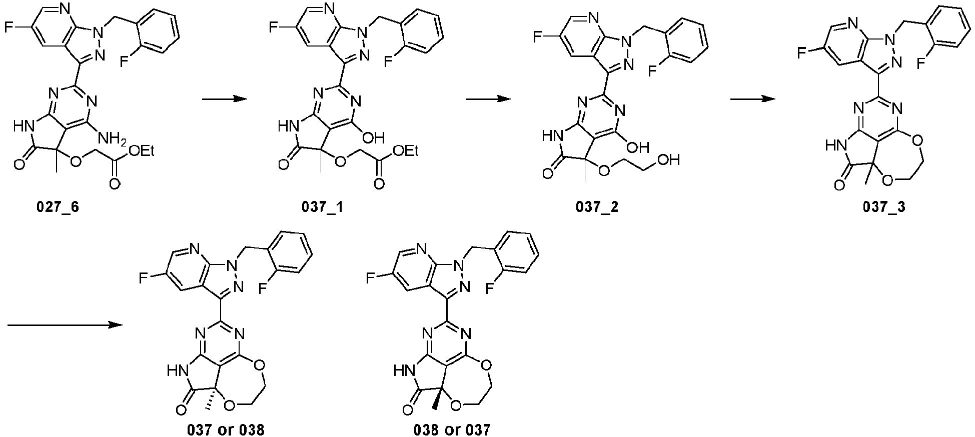

### Step 1: synthesis of intermediate 037_1

Intermediate **027_6** (690 mg, 1.35 mmol) was dissolved in trifluoroacetic acid (9 mL) at room temperature under nitrogen atmosphere. The reaction mixture was cooled to 0 °C, and a solution of sodium nitrite (280.33 mg, 4.06 mmol) in water (0.9 mL) was added. The reaction mixture was stirred at 0 °C for 1 h. After the reaction was completed, the reaction mixture was diluted with water (30 mL) and filtered to give a yellow solid. Water was azeotropically removed with toluene (20 mL), and the solvent was removed by concentration under reduced pressure to give intermediate **037_1.**

### Step 2: synthesis of intermediate 037_2

Intermediate **037_1** (470 mg, 920.76 µmol) was dissolved in tetrahydrofuran (1.5 mL) at room temperature under nitrogen atmosphere. The resulting solution was cooled to 10 °C, and a solution of Red-Al (1.06 g, 3.68 mmol, 70% purity) in toluene was added. The reaction mixture was stirred at room temperature for 12 h. After the reaction was completed, the reaction mixture was made neutral by adding 1 M diluted hydrochloric acid and diluted with water (20 mL) and 2-methyltetrahydrofuran (50 mL), and then the aqueous phase was separated and extracted with 2-methyltetrahydrofuran (50 mL × 3). The organic phases were combined, dried over anhydrous sodium sulfate, and filtered, and then the filtrate was concentrated under reduced pressure to remove the solvent. The resulting residue was separated by column chromatography (eluent: petroleum ether/2-methyltetrahydrofuran = 1/0 to 0/1, v/v) to give intermediate **037_2.** ¹H NMR (400 MHz, DMSO_*d₆*) δ: 12.75 (s, 1H), 11.27 (s, 1H), 8.79 (dd, *J* = 1.2, 2.4 Hz, 1H), 8.55 (s, 1H), 7.41-7.35 (m, 2H), 7.26-7.14 (m, 2H), 5.86 (s, 2H), 4.52 (t, *J=* 5.6 Hz, 1H), 3.43 (q, *J=* 5.6 Hz, 2H), 3.27-3.22 (m, 1H), 3.18-3.13 (m, 1H), 1.50 (s, 3H).

### Step 3: synthesis of intermediate 037_3

Intermediate **037_2** (250 mg, 533.72 µmol) was dissolved in tetrahydrofuran (5 mL) at room temperature under nitrogen atmosphere, and the resulting solution was cooled to 0 °C. A solution of *N,N,N',N'-*tetramethylazodicarbonamide (413.54 mg, 2.40 mmol) in tetrahydrofuran (1.25 mL) was added, and finally a solution of triphenylphosphine (629.94 mg, 2.4 mmol) in tetrahydrofuran (1.25 mL) was added at 0 °C. The reaction mixture was stirred at 70 °C for 5 h. After the reaction was completed, the reaction mixture was cooled to room temperature and filtered, and the filtrate was concentrated under reduced pressure to remove the solvent. The crude product was separated by preparative HPLC (mobile phase: acetonitrile/water; acidic system: 0.04% HCl) to give intermediate **037_3.** MS-ESI *m*/*z:* 451.1 [M+H]⁺. ¹H NMR (400 MHz, DMSO_*d₆*) δ: 11.58 (s, 1H), 8.76 (dd, *J =* 1.6, 2.8 Hz, 1H), 8.51 (dd, *J* = 2.8, 8.4 Hz, 1H), 7.40-7.35 (m, 1H), 7.28-7.14 (m, 3H), 5.90-5.81 (m, 2H), 4.80 (s, 1H), 4.35-4.18 (m, 3H), 1.64 (s, 3H).

### Step 4: synthesis of compounds 037 and 038

Intermediate **037_3** was separated by chiral column chromatography (column model: DAICEL CHIRALPAK AS (250 mm × 30 mm, 10 µm); mobile phase: [Neu-ACN]%: 45%-45%, 12 min) to give **037** and **038.**

**037** (retention time: 1.215 min): MS-ESI *m*/*z:* 451.1 [M+H]⁺. ¹H NMR (400 MHz, DMSO_ *d*₆) δ: 11.59 (s, 1H), 8.76 (s, 1H), 8.51 (dd, *J* = 2.8, 8.4 Hz, 1H), 7.38-7.35 (m, 1H), 7.28-7.14 (m, 3H), 5.90-5.81 (m, 2H), 4.81 (s, 1H), 4.36 (s, 1H), 4.29-4.17 (m, 2H), 1.64 (s, 3H).

**038** (retention time: 1.574 min): MS-ESI *m*/*z:* 451.1 [M+H]⁺. ¹H NMR (400 MHz, DMSO_ *d*₆) δ: 11.58 (s, 1H), 8.76 (dd, *J=* 1.6, 2.4 Hz, 1H), 8.51 (dd, *J* = 2.8, 8.8 Hz, 1H), 7.40-7.35 (m, 1H), 7.27-7.14 (m, 3H), 5.90-5.81 (m, 2H), 4.82 *(d, J=* 12 Hz, 1H), 4.35-4.29 (m, 1H), 4.25-4.18 (m, 2H), 1.64 (s, 3H).

### Example 39

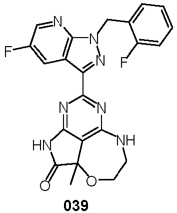

### Synthetic route:

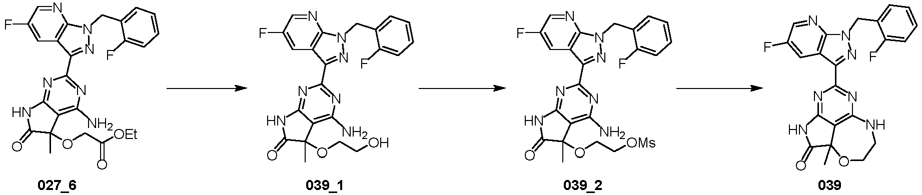

### Step 1: synthesis of intermediate 039_1

Intermediate **027_6** (200 mg, 392.57 mmol) was dissolved in tetrahydrofuran (4 mL) at room temperature under nitrogen atmosphere. The reaction mixture was cooled to 10 °C, and Red-Al (453.50 mg, 1.57 mmol, 70% purity) was added. The reaction mixture was brought back to room temperature and stirred for 12 h. After the reaction was completed, the reaction mixture was poured into diluted hydrochloric acid (1 M, 10 mL) to quench the reaction, and 2-methyltetrahydrofuran (10 mL) was added for dilution. Then the aqueous phase was separated and extracted with 2-methyltetrahydrofuran (10 mL × 2). The organic phases were combined, dried over anhydrous sodium sulfate, and filtered, and then the filtrate was concentrated under reduced pressure to remove the solvent. The resulting residue was separated by column chromatography (eluent: petroleum ether/2-methyltetrahydrofuran = 1/0 to 5/4, v/v) to give intermediate **039_1.**

### Step 2: synthesis of intermediate 039_2

Intermediate **039_1** (130 mg, 278.12 µmol) was dissolved in tetrahydrofuran (1.1 mL) and dichloromethane (1.1 mL) at room temperature under nitrogen atmosphere, and N,N diisopropylethylamine (71.89 mg, 556.24 µmol) and 4-dimethylaminopyridine (3.40 mg, 27.81 µmol) were added successively. The resulting mixture was cooled to 0 °C, and then methanesulfonyl chloride (47.79 mg, 417.18 µmol) was added. The reaction mixture was stirred at room temperature for 1 h. After the reaction was completed, the reaction mixture was poured into saturated aqueous sodium bicarbonate solution (10 mL) to quench the reaction, and 2-methyltetrahydrofuran (10 mL) was added for dilution. Then the aqueous phase was separated and extracted with 2-methyltetrahydrofuran (10 mL × 2). The organic phases were combined, dried over anhydrous sodium sulfate, and filtered, and then the filtrate was concentrated under reduced pressure to remove the solvent. The resulting residue was separated by column chromatography (eluent: petroleum ether/2-methyltetrahydrofuran = 1/0 to 5/4, v/v) to give intermediate **039_2.** ¹H NMR (400 MHz, DMSO_*d₆*) δ: 11.26 (s, 1H), 8.87 (dd, *J* = 2.8, 8.8 Hz, 1H), 8.71 (s, 1H), 7.39- 7.34 (m, 1H), 7.25-7.20 (m, 2H), 7.17-7.13 (m, 1H), 6.64 (s, 2H), 5.82 (s, 2H), 4.29-4.27 (m, 2H), 3.20 (s, 3H), 1.57 (s, 3H).

### Step 3: synthesis of compound 039

Intermediate **039_2** (60 mg, 109.99 µmol) was dissolved in tetrahydrofuran (1 mL) at room temperature under nitrogen atmosphere. The resulting solution was cooled to -78 °C, and a solution of lithium bis(trimethylsilyl)amide (1 M, 274.97 µL) in n-hexane and hexamethylphosphoric triamide (49.27 mg, 274.97 mmol) were added successively. The reaction mixture was stirred at room temperature for 2 h, cooled to -78 °C, supplemented with the solution of lithium bis(trimethylsilyl)amide (1 M, 274.97 µL) in n-hexane and hexamethylphosphoric triamide (49.27 mg, 274.97 mmol), and stirred at room temperature for 12 h. After the reaction was completed, the reaction mixture was poured into saturated aqueous ammonium chloride solution (10 mL) to quench the reaction and diluted with 2-methyltetrahydrofuran (10 mL). Then the aqueous phase was separated and extracted with 2-methyltetrahydrofuran (10 mL × 2). The organic phases were combined, dried over anhydrous sodium sulfate, and filtered, and then the filtrate was concentrated under reduced pressure to remove the solvent. The crude product was separated by preparative HPLC (mobile phase: acetonitrile/water; acidic system: 0.04% HCl) to give compound **039.** MS-ESI m/z: 450.2 [M+H]⁺. ¹H NMR (400 MHz, DMSO_*d₆*) δ: 11.07 (s, 1H), 8.76 (dd, *J* = 2.8, 8.8 Hz, 1H), 8.71 (s, 1H), 7.68 (s, 1H), 7.37-7.35 (m, 1H), 7.24-7.21 (m, 2H), 7.17-7.13 (m, 1H), 5.82 *(d, J*= 3.6 Hz, 2H), 4.12-3.98 (m, 4H), 1.55 (s, 3H).

### Examples 40 and 41

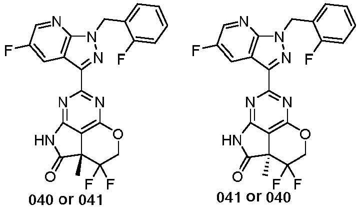

### Synthetic route:

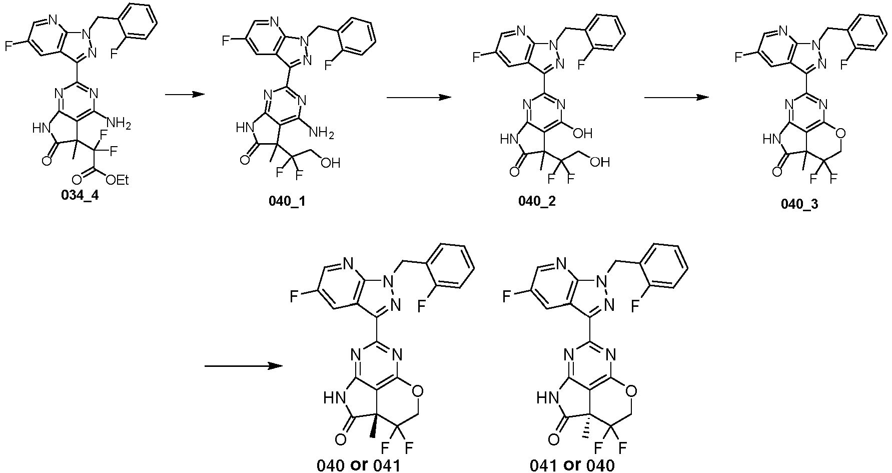

### Step 1: synthesis of intermediate 040_1

Intermediate **034_4** (2 g, 3.78 mmol) was dissolved in tetrahydrofuran (40 mL) at room temperature under nitrogen atmosphere. The reaction mixture was cooled to 0 °C, and lithium aluminum hydride (286.75 mg, 7.56 mmol) was added. The reaction mixture was stirred at 0 °C for 2 h. After the reaction was completed, the reaction mixture was quenched to neutrality with 1 M diluted hydrochloric acid and diluted with 2-methyltetrahydrofuran (50 mL). Then the aqueous phase was separated and extracted with 2-methyltetrahydrofuran (50 mL × 3). The organic phases were combined and concentrated under reduced pressure to remove the solvent. The resulting residue was separated by column chromatography (eluent: petroleum ether/2-methyltetrahydrofuran = 1/0 to 1/1, v/v) to give intermediate **040_1.**

### Step 2: synthesis of intermediate 040_2

Intermediate **040_1** (720 mg, 1.48 mmol) was dissolved in trifluoroacetic acid (15 mL) at room temperature under nitrogen atmosphere. The resulting solution was cooled to 0 °C, and then a solution of sodium nitrite (305.76 mg, 4.43 mmol) in water (1.5 mL) was added. The reaction mixture was stirred at 0 °C for 1 h. After the reaction was completed, the reaction mixture was diluted with water (50 mL) and filtered, and the filter cake was collected, rinsed with water (2 mL), and concentrated under reduced pressure to remove the solvent. The resulting residue was separated by column chromatography (eluent: petroleum ether/2-methyltetrahydrofuran = 1/0 to 5/4, v/v) to give intermediate **040_2.**

### Step 3: synthesis of intermediate 040_3

Intermediate **040_2** (260 mg, 532.36 µmol) was dissolved in tetrahydrofuran (4 mL) at room temperature under nitrogen atmosphere, and the resulting solution was cooled to 0 °C. A solution of *N,N,N',N'-*tetramethylazodicarbonamide (412.49 mg, 2.40 mmol) in tetrahydrofuran (4 mL) was added, and finally a solution of triphenylphosphine (628.33 mg, 2.40 mmol) in tetrahydrofuran (2 mL) was added at 0 °C. The reaction mixture was stirred at 75 °C for 12 h. After the reaction was completed, the reaction mixture was cooled to room temperature and filtered, and the filtrate was concentrated under reduced pressure to remove the solvent. The resulting residue was separated by column chromatography (eluent: petroleum ether/2-methyltetrahydrofuran = 1/0 to 0/1, v/v) to give a crude product. The crude product was separated by preparative HPLC (mobile phase: acetonitrile/water; acidic system: 0.04% HCl) to give intermediate **040_3.** MS-ESI m/z: 471.4 [M+H]⁺. ¹H NMR (400 MHz, DMSO_*d₆*) δ: 12.32 (*d, J =* 2.4 Hz, 1H), 8.76 (dd, *J* = 1.6, 2.8 Hz, 1H), 8.62 (dd, *J* = 2.8, 8.4 Hz, 1H), 7.40-7.34 (m, 1H), 7.25-7.20 (m, 2H), 7.17-7.14 (m, 1H), 5.86 (s, 2H), 5.06-5.03 (m, 1H), 4.97-4.94 (m, 1H), 1.48 (s, 3H).

### Step 4: synthesis of compounds 040 and 041

Compound **040_3** was separated by chiral column chromatography (column model: REGIS (R,R)WHELK-0O1 (250 mm × 25 mm, 10 µm); mobile phase: [Neu-ACN]; B%: 50%-50%, 10 min) to give compounds **040** and **041.**

**040** (retention time: 1.284 min): MS-ESI m/z: 471.4 [M+H]⁺. ¹H NMR (400 MHz, DMSO_*d₆*) δ: 12.33 (s, 1H), 8.75 (s, 1H), 8.61 (dd, *J* = 2.8, 8.8 Hz, 1H), 7.37 (m, 1H), 7.25-7.20 (m, 2H), 7.17-7.13 (m, 1H), 5.86 (s, 2H), 5.06-4.94 (m, 2H), 1.48 (s, 3H).

**041** (retention time: 1.400 min): MS-ESI m/z: 471.4 [M+H]⁺. ¹H NMR (400 MHz, DMSO_ *d*₆) δ: 12.33 (s, 1H), 8.76 (s, 1H), 8.63-8.61 (m, 1H), 7.37 (d, *J =* 5.2 Hz, 1H), 7.25-7.23 (m, 2H), 7.16 (t, *J =* 7.2 Hz, 1H), 5.86 (s, 2H), 5.06-4.94 (m, 2H), 1.48 (s, 3H).

### Examples 42 and 43

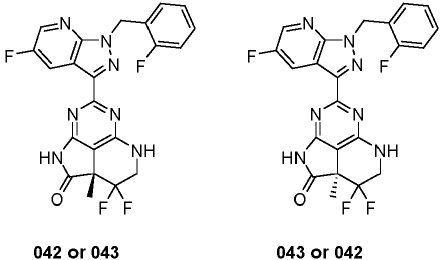

### Synthetic route:

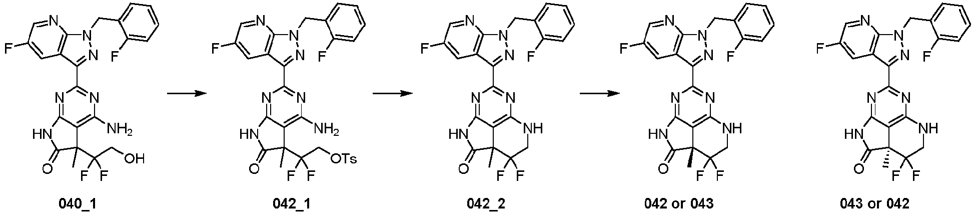

### Step 1: synthesis of intermediate 042_1

Intermediate **040_1** (1.4 g, 2.87 mmol) was dissolved in tetrahydrofuran (15 mL) and dichloromethane (15 mL) at room temperature under nitrogen atmosphere, and then N,N-diisopropylethylamine (1.3 g, 10.05 mmol, 1.75 mL), p-toluenesulfonyl chloride (1.37 g, 7.18 mmol) and 4-dimethylaminopyridine (35.09 mg, 287.23 µmol) were added successively. The reaction mixture was warmed to 50 °C and stirred for 12 h. After the reaction was completed, the reaction mixture was cooled to room temperature, and saturated aqueous citric acid solution (50 mL) and 2-methyltetrahydrofuran (30 mL) were added. The aqueous phase was separated and extracted with 2-methyltetrahydrofuran (50 mL × 3). The organic phases were combined, dried over anhydrous sodium sulfate, and filtered, and then the filtrate was concentrated under reduced pressure to remove the solvent. The resulting residue was separated by column chromatography (eluent: petroleum ether/2-methyltetrahydrofuran = 1/0 to 1/1, v/v) and preparative HPLC (mobile phase: acetonitrile/water; acidic system: 0.04% HCl) to give intermediate **042_1.** ¹H NMR (400 MHz, DMSO_d₆) δ: 11.58 (d, *J =* 4.0 Hz, 1H), 9.02 (dd, *J* = 2.8, 8.8 Hz, 1H), 8.72 (dd, *J =* 1.6, 2.8 Hz, 1H), 7.50 (d, *J =* 8.4 Hz, 2H), 7.40-7.34 (m, 1H), 7.25-7.20 (m, 2H), 7.17-7.15 (m, 3H), 5.88-5.79 (m, 2H), 4.45 (t, *J =* 5.2 Hz, 2H), 1.97 (s, 3H), 1.51-1.47 (m, 3H).

### Step 2: synthesis of intermediate 042_2

Intermediate **042_1** (70 mg, 109.10 µmol) was dissolved in tetrahydrofuran (2 mL) at room temperature under nitrogen atmosphere. The resulting solution was cooled to -78 °C, and hexamethylphosphoric triamide (48.88 mg, 272.76 mmol, 47.92 µL) and a solution of lithium bis(trimethylsilyl)amide (1 M, 272.76 µL) in n-hexane were added successively. The reaction mixture was brought back to room temperature and stirred for 12 h. After the reaction was completed, the reaction mixture was slowly poured into diluted hydrochloric acid (1 M, 5 mL), and semi-saturated brine (5 mL) and 2-methyltetrahydrofuran (5 mL) were added for dilution. Then the aqueous phase was separated and extracted with 2-methyltetrahydrofuran (5 mL × 3). The organic phases were combined, washed with saturated brine (10 mL), dried over anhydrous sodium sulfate, and filtered, and then the filtrate was concentrated under reduced pressure to remove the solvent. The resulting residue was separated by preparative HPLC (mobile phase: acetonitrile/water; acidic system: 0.04% HCl) to give intermediate **042_2.** MS-ESI m/z: 470.1 [M+H]⁺. ¹H NMR (400 MHz, DMSO_d₆) δ: 11.76 (s, 1H), 8.72 (s, 1H), 8.67-8.66 (m, 1H), 7.83 (s, 1H), 7.37 (d, *J =* 7.6 Hz, 1H), 7.25-7.23 (m, 2H), 7.17-7.13 (m, 1H), 5.82 (s, 2H), 4.03 *(d, J=* 10.0 Hz, 1H), 3.61 *(d, J=* 11.2 Hz, 1H), 1.34 (s, 3H).

### Step 3: synthesis of compounds 042 and 043

Compound **042_2** was separated by chiral column chromatography (column model: DAICEL CHIRALPAKAS (250 mm × 30 mm, 10 µm); mobile phase: [0.1% NH₃H₂O MeOH]%: 35%-35%, 15 min) to give compounds **042** and **043.**

**042** (retention time: 1.169 min): MS-ESI m/z: 470.1 [M+H]⁺. ¹H NMR (400 MHz, DMSO_*d₆*) δ: 11.71 (s, 1H), 8.72 (s, 1H), 8.67 (d, *J* = 8.0 Hz, 1H), 7.83 (s, 1H), 7.36 (d, *J =* 6.0 Hz, 1H), 7.2-7.23 (m, 2H), 7.17-7.13 (m, 1H), 5.82 (s, 2H), 4.03 (d, *J* = 9.2 Hz, 1H), 3.62 (d,*J* = 10.8 Hz, 1H), 1.34 (s, 3H).

**043** (retention time: 1.190 min): MS-ESI m/z: 470.1 [M+H]⁺. ¹H NMR (400 MHz, DMSO_*d₆*) δ: 11.71 (s, 1H), 8.72 (s, 1H), 8.66 (dd, *J* = 2.8, 8.8 Hz, 1H), 7.82 (s, 1H), 7.39-7.34 (m, 1H), 7.25-7.21 (m, 2H), 7.17-7.13 (m, 1H), 5.82 (s, 2H), 4.03 (d, *J* = 10.0 Hz, 1H), 3.62 (d, *J* = 12.0 Hz, 1H), 1.34 (s, 3H).

### Examples 44 and 45

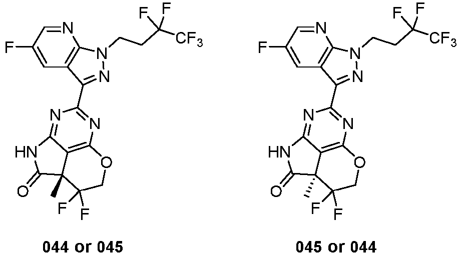

### Synthetic route:

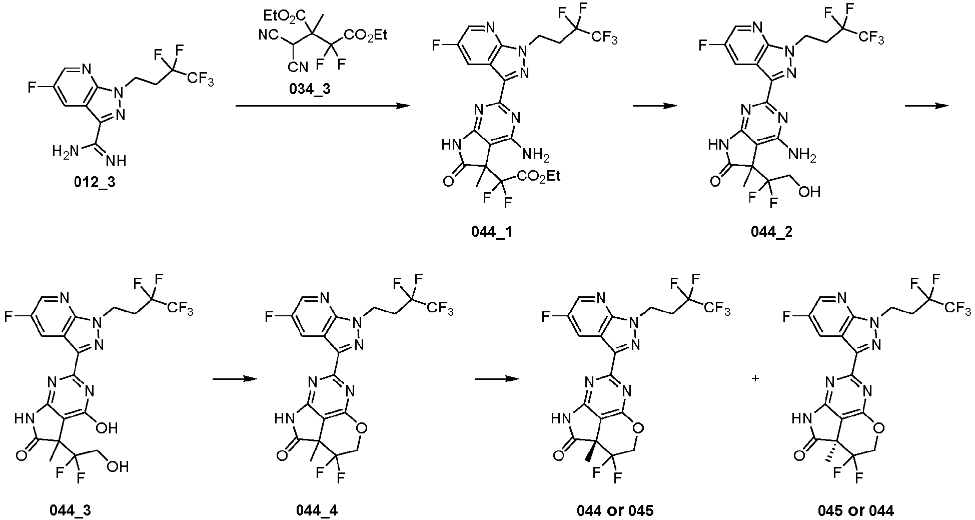

### Step 1: synthesis of intermediate 044_1

Intermediate **034_3** (9.37 g, 32.52 mmol) was dissolved in tert-butanol (40 mL) at room temperature under nitrogen atmosphere, and potassium carbonate (5.24 g, 37.93 mmol) and intermediate **012_3** (3.92 g, 10.84 mmol, hydrochloride) were added. The reaction mixture was warmed to 85 °C and stirred for 12 h. The reaction mixture was cooled to room temperature and filtered, and the filtrate was concentrated under reduced pressure to remove the solvent. The resulting residue was separated by column chromatography (eluent: dichloromethane/methanol = 1/0 to 95/5, v/v) to give intermediate **044_1.**

### Step 2: synthesis of intermediate 044_2

Intermediate **044_1** (1 g, 1.76 mmol) was dissolved in tetrahydrofuran (20 mL) at room temperature under nitrogen atmosphere. The resulting solution was cooled to 0 °C, and lithium aluminum hydride (133.79 mg, 3.52 mmol) was added slowly. The reaction mixture was stirred at 0 °C for 1 h. The reaction mixture was slowly poured into diluted hydrochloric acid (1 N, 20 mL) to quench the reaction, and 2-methyltetrahydrofuran (10 mL) was added for extraction. The organic phase was dried over anhydrous sodium sulfate and filtered, and the filtrate was concentrated under reduced pressure to remove the solvent. The resulting residue was separated by preparative HPLC (mobile phase: acetonitrile/water; acidic system: 0.1% TFA) to give intermediate **044_2.**

### Step 3: synthesis of intermediate 044_3

Intermediate **044_2** (98 mg, 186.54 µmol) was dissolved in trifluoroacetic acid (2 mL) at room temperature under nitrogen atmosphere. The reaction mixture was cooled to 0 °C, and a solution of sodium nitrite (38.61 mg, 559.62 µmol) in water (0.2 mL) was added. The reaction mixture was stirred at 0 °C for 1 h. After the reaction was completed, water (8 mL) was added to the reaction mixture, and a solid precipitated. The resulting mixture was filtered, and the resulting filter cake was concentrated with anhydrous toluene (10 mL) to give intermediate **044_3.**

### Step 4: synthesis of intermediate 044_4

Triphenylphosphine (73.25 mg, 279.29 µmol) was added to a reaction flask at room temperature under nitrogen atmosphere. Then a solution of intermediate 044_3 (98 mg, crude) in tetrahydrofuran (1.7 mL) was added to the reaction system, and finally a solution of N,N,N',N'-tetramethylazodicarbonamide (56.47 mg, 279.29 µmol) in tetrahydrofuran (0.3 mL) was added to the reaction system. The reaction mixture was stirred at 70 °C for 12 h. After the reaction was completed, the reaction mixture was concentrated, and the resulting residue was separated by preparative HPLC (mobile phase: acetonitrile/water; acidic system: 0.04% HCl) to give intermediate **044_4.** MS-ESI m/z: 509.2 [M+H]⁺. ¹H NMR (400 MHz, DMSO_*d₆*) δ: 12.33 (s, 1H), 8.77 (s, 1H), 8.63 (dd, *J =* 2.8, 8.4 Hz, 1H), 5.07-5.05 (m, 1H), 4.98-4.90 (m, 3H), 3.06-2.94 (m, 2H), 1.49 (s, 3H).

### Step 5: synthesis of compounds 044 and 045

Intermediate **044_4** was subjected to chiral resolution (column model: REGIS(S,S)WHELK-O1 (250 mm × 25 mm, 10 µm); mobile phase: [Neu-ACN]%: 30%-30%, 5 min) to give compounds **044** and **045.** The chiral analysis method: OD_MeOH_IPAm; gradient: 5-50%; flow rate: 3.4 mL/min; column temperature: 35 °C_; time: 3 min.

**044** (retention time: 0.789 min): MS-ESI m/z: 509.2 [M+H]⁺. ¹H NMR (400 MHz, DMSO_*d₆*) δ: 12.34 (s, 1H), 8.78 (q, *J=* 1.6 Hz, 1H), 8.63 (dd, *J =* 2.8, 8.8 Hz, 1H), 5.07-5.05 (m, 1H), 4.98-4.90 (m, 3H), 3.05-2.95 (m, 2H), 1.49 (s, 3H).

**045** (retention time: 0.768 min): MS-ESI m/z: 509.1 [M+H]⁺. ¹H NMR (400 MHz, DMSO_*d₆*) δ: 12.34 (s, 1H), 8.77 (q, *J=* 1.2 Hz, 1H), 8.62 (dd, *J=* 2.8, 8.8 Hz, 1H), 5.07-5.05 (m, 1H), 4.98-4.90 (m, 3H), 3.06-2.95 (m, 2H), 1.49 (s, 3H).

### Examples 46 and 47

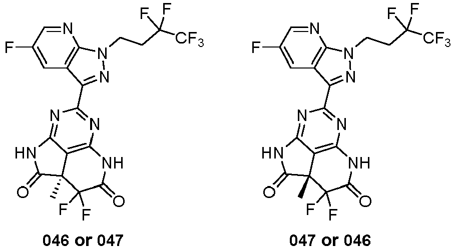

### Synthetic route:

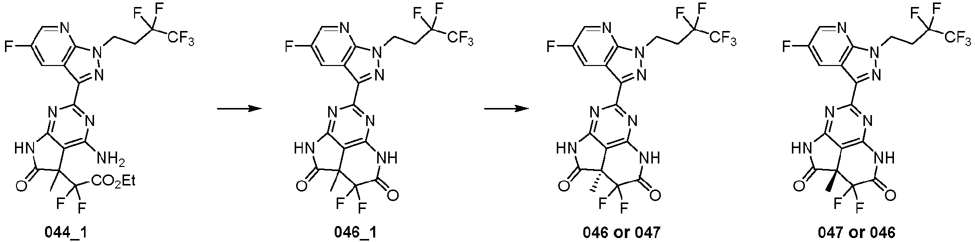

### Step 1: synthesis of intermediate 046_1

Intermediate **044_1** (150 mg, 264.37 µmol) was dissolved in anhydrous toluene (2 mL) at room temperature under nitrogen atmosphere, and a solution of trimethylaluminum (2 M, 396.55 µL) in toluene was slowly added. The reaction mixture was stirred at 80 °C for 4 h. After the reaction was completed, the reaction mixture was cooled to room temperature, adjusted to pH 6 with 1 M diluted hydrochloric acid, and then extracted with ethyl acetate (20 mL). The organic phase was dried over anhydrous sodium sulfate and filtered, and the filtrate was concentrated under reduced pressure to remove the solvent. The residue was separated by preparative HPLC (mobile phase: acetonitrile/water; acidic system: 0.04% HCl) to give intermediate **046_1.** ¹H NMR (400 MHz, DMSO_*d₆*) *δ:* 12.34 (s, 1H), 12.05 (s, 1H), 8.79-8.78 (m, 1H), 8.66 (dd, *J =* 2.8, 8.8 Hz, 1H), 4.93 (t, *J =* 6.8 Hz, 2H), 3.05-2.95 (m, 2H), 1.57 (s, 3H).

### Step 2: synthesis of compounds 046 and 047

Intermediate **046_1** was separated by chiral column chromatography (column model: REGIS(S,S)WHELK-O1 (250 mm × 25 mm, 10 µm); mobile phase: [Neu-MeOH]%: 20%-20%, 4 min) to give compounds **046** and **047.** The chiral analysis method: OD_MeOH_IPAm; gradient: 5-50%; flow rate: 3.4 mL/min; column temperature: 35 °C; time: 3 min.

**046** (retention time: 0.850 min): MS-ESI m/z: 522.0 [M+H]⁺. ¹H NMR (400 MHz, DMSO_*d₆*) δ: 12.40-12.25 (m, 1H), 12.12-11.98 (m, 1H), 8.78 (q, *J =* 1.2 Hz, 1H), 8.66 (dd, *J =* 3.2, 8.4 Hz, 1H), 4.93 (t, *J* = 6.8 Hz, 2H), 3.07-2.94 (m, 2H),1.57 (s, 3H).

**047** (retention time: 0.847 min): MS-ESI m/z: 522.0 [M+H]⁺. ¹H NMR (400 MHz, DMSO_*d₆*) *δ:* 12.38-12.10 (m, 2H), 8.79 (q, *J* = 1.6 Hz, 1H), 8.67 (dd, *J =* 3.2, 8.8 Hz, 1H), 4.94 (t, *J =* 7.2 Hz, 2H), 3.06-2.95 (m, 2H), 1.57 (s, 3H).

### Example 48

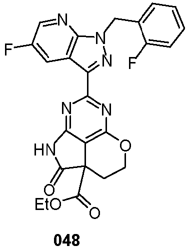

### Synthetic route:

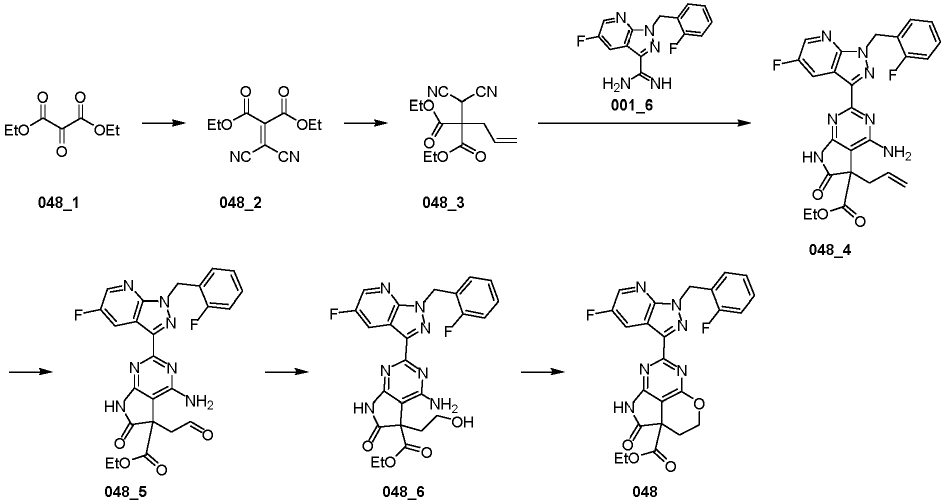

### Step 1: synthesis of intermediate 048_2

Compound 048_1 (50 g, 287.11 mmol) was dissolved in anhydrous toluene (1000 mL) at room temperature under nitrogen atmosphere, and malononitrile (18.02 g, 272.75 mmol), β-aminopropionic acid (766.67 mg, 8.61 mmol) and acetic acid (3.17 g, 52.73 mmol) were added thereto. The reaction system was warmed to 125 °C and stirred for 12 h. After the reaction was completed, the system was cooled to room temperature and concentrated under reduced pressure to remove the solvent. The resulting residue was separated by column chromatography (eluent: petroleum ether/ethyl acetate = 1/0 to 3/1, v/v) to give intermediate **048_2.** ¹H NMR (400 MHz, DMSO_d₆) δ: 4.39 (q, *J* = 7.2 Hz, 4H), 1.28 (t, *J =* 7.2 Hz, 6H).

### Step 2: synthesis of intermediate 048_3

Intermediate **048_2** (30 g, 135.02 mmol) was dissolved in tetrahydrofuran (300 mL) at room temperature under nitrogen atmosphere. The resulting solution was cooled to -78 °C, and a solution of allylmagnesium bromide (1 M, 202.52 mL) in tetrahydrofuran was slowly added dropwise. The reaction system was stirred at -78 °C for 15 min. After the reaction was completed, the reaction system was quenched with a saturated ammonium chloride solution (300 mL) and diluted with ethyl acetate (200 mL) and water (100 mL). The aqueous phase was separated and extracted with ethyl acetate (300 mL × 3). The organic phases were combined, washed with saturated brine (250 mL × 3), dried over anhydrous sodium sulfate, and filtered, and the filtrate was concentrated under reduced pressure to give a crude product. The resulting crude product was separated by column chromatography (eluent: petroleum ether/ethyl acetate = 1/0 to 3/1, v/v) to give intermediate 048_3. ¹H NMR (400 MHz, DMSO_*d₆*) *δ:* 5.81-5.67 (m, 1H), 5.64-5.58 (m, 1H), 5.34-5.23 (m, 2H), 4.36-4.22 (m, 4H), 2.81 (d, *J=* 7.2 Hz, 2H), 1.23 (t, *J =* 7.2 Hz, 6H).

### Step 3: synthesis of intermediate 048_4

Intermediate **048_3** (5.5 g, 20.81 mmol) was dissolved in tert-butanol (55 mL) at room temperature under nitrogen atmosphere, and intermediate **001_6** (4.21 g, 13.01 mmol, hydrochloride) and potassium bicarbonate (3.26 g, 32.52 mmol) were added thereto. The reaction system was warmed to 85 °C and stirred for 12 h. After the reaction was completed, the reaction mixture was cooled to room temperature, diluted with water (50 mL), and extracted with ethyl acetate (60 mL × 3). The organic phases were combined, washed with saturated brine (50 mL × 2), dried over anhydrous sodium sulfate, and filtered, and the filtrate was concentrated under reduced pressure to remove the solvent. The resulting residue was stirred with methyl *tert-butyl* ether (30 mL) at room temperature for 30 min, and a pale yellow solid precipitated. The resulting mixture was filtered, and the solid was collected and concentrated under reduced pressure to give a crude product. The crude product was stirred with acetonitrile (30 mL) at room temperature for 1 h, and a white solid precipitated. The resulting mixture was filtered, and the solid was collected and concentrated under reduced pressure to give intermediate **048_4.** The filtrate was collected and concentrated under reduced pressure to remove the solvent, and the resulting crude product was separated by column chromatography (eluent: petroleum ether/ethyl acetate = 1/0 to 7/3, v/v) to give intermediate **048_4.** ¹H NMR (400 MHz, DMSO_d₆) δ: 11.37 (s, 1H), 8.87 (dd, *J* = 2.8, 8.8 Hz, 1H), 8.70 (*dd, J* = 1.6, 2.4 Hz, 1H), 7.41-7.33 (m, 1H), 7.27-7.19 (m, 2H), 7.18-7.12 (m, 1H), 6.92 (br s, 2H), 5.87-5.76 (m, 2H), 5.39-5.26 (m, 1H), 5.08 (dd, *J =* 1.6, 17.0 Hz, 1H), 4.95 (dd, *J =* 2.0, 10 Hz, 1H), 4.18-4.08 (m, 2H), 3.26 (dd, *J* = 7.2, 14.0 Hz, 1H), 2.80 (dd, *J =* 7.2, 14.0 Hz, 1H), 1.14-1.09 (m, 3H).

### Step 4: synthesis of intermediate 048_5

Intermediate **048_4** (1 g, 1.98 mmol) was dissolved in dichloromethane (20 mL) and tetrahydrofuran (20 mL) at room temperature under nitrogen atmosphere, and the reaction system was cooled to -40 °C. Ozone was bubbled into the reaction system until the reaction system turned blue, and then oxygen was bubbled in for another 15 min. Thiourea (451.78 mg, 5.94 mmol) was added to the reaction system, and the resulting mixture was stirred at room temperature for 12 h. After the reaction was completed, no peroxide remained as detected using potassium iodide starch paper. The reaction mixture was filtered, and the filtrate was collected and concentrated under reduced pressure to remove the solvent to give intermediate **048_5.**

### Step 5: synthesis of intermediate 048_6

Intermediate **048_5** (1 g, 1.97 mmol) was dissolved in tetrahydrofuran (10 mL) at room temperature under nitrogen atmosphere. The reaction system was cooled to 0 °C, and sodium cyanoborohydride (123.84 mg, 1.97 mmol) was added. The reaction system was stirred at 0 °C for 0.5 h, and warmed to 25 °C and stirred for 11.5 h. After the reaction was completed, the reaction mixture was diluted with water (50 mL) and extracted with 2-methyltetrahydrofuran (80 mL × 3). The organic phases were combined, washed with saturated brine (50 mL × 2), dried over anhydrous sodium sulfate, and filtered, and the filtrate was concentrated under reduced pressure to remove the solvent. The resulting crude product was separated by column chromatography (eluent: dichloromethane/methanol = 30/1 to 10/1, v/v) to give a crude product. The crude product was separated by preparative HPLC (mobile phase: acetonitrile/water; acidic system: 0.1% TFA) to give intermediate **048_6.** ¹H NMR (400 MHz, DMSO_d₆) δ: 11.04 (s, 1H), 8.90-8.78 (m, 1H), 8.71 (br s, 1H), 7.40-7.33 (m, 1H), 7.25-7.16 (m, 3H), 7.13-6.90 (m, 2H), 5.81 (br s, 2H), 4.17-4.06 (m, 2H), 3.99 (q, *J* = 7.2 Hz, 2H), 3.56 (dd, *J* = 3.6, 7.2 Hz, 1H), 2.33 (br s, 2H), 1.11 (t, *J =* 7.2 Hz, 3H).

### Step 6: synthesis of compound 048

Intermediate **048_6** (250 mg, 490.71 µmol) was dissolved in N,N-dimethylformamide (5 mL) in a preliminarily dried reaction flask at room temperature under nitrogen atmosphere, and *tert-butyl* nitrite (759.03 mg, 7.36 mmol) and water (1.00 g, 55.51 mmol) were added thereto. The reaction system was stirred at room temperature for 12 h. After the reaction was completed, the reaction mixture was diluted with water (10 mL) and extracted with 2-methyltetrahydrofuran (10 mL × 3). The organic phases were combined, washed with saturated brine (10 mL × 2), dried over anhydrous sodium sulfate, and filtered, and the filtrate was concentrated under reduced pressure to remove the solvent. The resulting residue was separated by column chromatography (eluent: dichloromethane/methanol = 1/0 to 9/1, v/v) and preparative HPLC (mobile phase: acetonitrile/water; acidic system: 0.04% HCl) to give compound **048.** ¹H NMR (400 MHz, DMSO_d₆) δ: 11.67 (s, 1H), 8.78-8.74 (m, 1H), 8.51 (dd, *J* = 2.8, 8.4 Hz, 1H), 7.42-7.33 (m, 1H), 7.28-7.15 (m, 3H), 5.86 (s, 2H), 4.78 (br dd, *J =* 2.8, 12.0 Hz, 1H), 4.35 (br t, *J* = 11.6 Hz, 1H), 4.24-4.10 (m, 2H), 2.70-2.62 (m, 1H), 2.33 (s, 1H), 1.14 (t, *J* = 7.0 Hz, 3H).

### Example 49

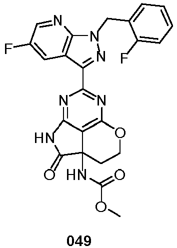

### Synthetic route:

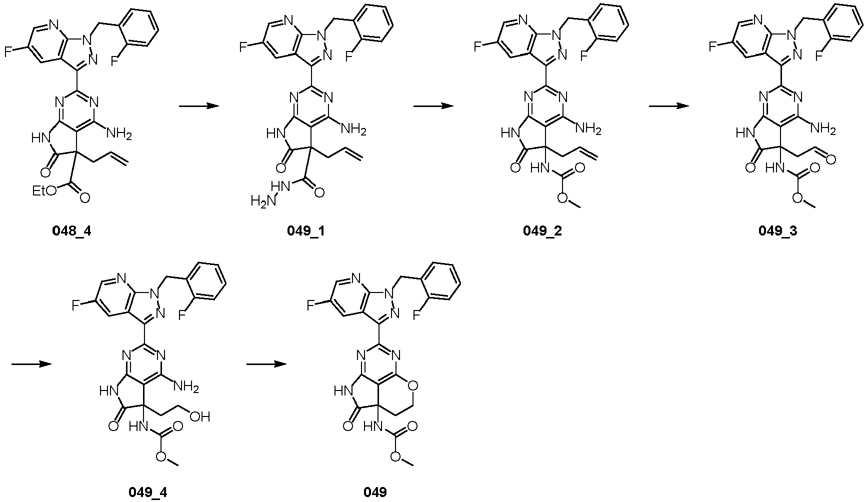

### Step 1: synthesis of intermediate 049_1

Intermediate **048_4** (8.7 g, 17.21 mmol) was dissolved in tetrahydrofuran (90 mL) at room temperature under nitrogen atmosphere, and hydrazine hydrate (91.44 g, 1.79 mol, 88.78 mL, 98% purity) was added. The reaction system was warmed to 85 °C and stirred at 85 °C for 2 h. After the reaction was completed, the reaction system was cooled to room temperature, and a solid precipitated. The resulting mixture was filtered, and the solid was collected and concentrated under reduced pressure to remove the solvent to give intermediate **049_1.** Meanwhile, the filtrate was collected, diluted with water (80 mL) and 2-methyltetrahydrofuran (100 mL). The organic phase was separated and collected, and the aqueous phase was extracted with 2-methyltetrahydrofuran (200 mL × 3). The organic phases were combined, washed with saturated brine (100 mL × 2), dried over anhydrous sodium sulfate, and filtered, and the filtrate was concentrated under reduced pressure to give intermediate **049_1.**

### Step 2: synthesis of intermediate 049_2

Intermediate **049_1** (4.74 g, 9.64 mmol) was dissolved in tetrahydrofuran (50 mL) and methanol (50 mL) at room temperature under nitrogen atmosphere, and trifluoroacetic acid (1.65 g, 14.47 mmol, 1.07 mL) was added. The reaction system was cooled to 0 °C, and *tert-*butyl nitrite (2.98 g, 28.93 mmol, 3.44 mL) was added dropwise. The reaction system was warmed to 85 °C and stirred for 1.5 h. After the reaction was completed, the reaction system was cooled to room temperature, and a solid precipitated. The resulting mixture was filtered, and the filtrate was collected and concentrated under reduced pressure to remove the solvent to give a residue. The residue was dissolved in methyl *tert*-butyl ether (20 mL). The resulting solution was stirred for 30 min, and a yellow solid precipitated. The resulting mixture was filtered, and the solid was collected to give intermediate **049_2.** The filtrate was concentrated under reduced pressure and then separated by column chromatography (eluent: petroleum ether/ethyl acetate = 1/0 to 0/1, v/v) to give intermediate **049_2.**

### Step 3: synthesis of intermediate 049_3

Intermediate **049_2** was dissolved in *N*-methylpyrrolidone (20 mL), tetrahydrofuran (5 mL) and dichloromethane (5 mL) at room temperature under nitrogen atmosphere. The reaction system was cooled to -60 °C. Ozone was bubbled into the reaction system for 30 min, and oxygen for 15 min. After the bubbling, the reaction system was warmed to 20 °C, and thiourea (1.35 g, 17.77 mmol) was added. The reaction mixture was stirred at 20 °C for 12 h and poured into water (20 mL), followed by the extraction with 2-methyltetrahydrofuran (10 mL × 3). The organic phase was washed with saturated brine (5 mL), dried over anhydrous sodium sulfate, and filtered, and the filtrate was concentrated to give intermediate **049_3.**

### Step 4: synthesis of intermediate 049_4

Intermediate **049_3** (4 g, 7.87 mmol) was dissolved in tetrahydrofuran (40 mL) at room temperature under nitrogen atmosphere. The reaction system was cooled to 0 °C, and sodium cyanoborohydride (494.39 mg, 7.87 mmol) was added. The reaction system was stirred at 0 °C for 0.5 h, brought back to room temperature and stirred for 11.5 h, and supplemented with sodium cyanoborohydride (247.20 mg, 3.93 mmol). The reaction mixture was heated to 50 °C and stirred for 3 h. After the reaction was completed, the reaction system was cooled to room temperature, and the reaction mixture was directly concentrated to remove the solvent. The resulting residue was separated by column chromatography (eluent: dichloromethane/methanol = 100/1 to 40/1, v/v) to give intermediate **049_4.** ¹H NMR (400 MHz, DMSO_*d*₆) δ: 10.93 (s, 1H), 8.81 (dd, *J* = 2.8, 8.8 Hz, 1H), 8.70-8.69 (m, 1H), 7.44-7.33 (m, 2H), 7.24-7.12 (m, 4H), 6.74 (br s, 1H), 5.81 (s, 2H), 4.62 (t, *J* = 5.0 Hz, 1H), 4.37-4.33 (m, 1H), 3.46 (s, 3H), 3.38-3.36 (m, 1H), 2.25-2.16 (m, 1H), 2.06-1.97 (m, 1H).

### Step 5: synthesis of compound 049

Intermediate **049_4** (200 mg, 391.81 µmol) was dissolved in *N*,*N-*dimethylformamide (4 mL) and water (0.4 mL) in a preliminarily dried reaction flask at room temperature under nitrogen atmosphere, and *tert-butyl* nitrite (606.05 mg, 5.88 mmol, 699.02 µL) was added thereto at 20 °C. The reaction system was stirred at 20 °C for 2 h, and heated to 60 °C and stirred for 0.5 h. After the reaction was completed, the reaction mixture was directly filtered through a filter head, and the filtrate was collected and subjected to two rounds of preparative separation, (mobile phase: acetonitrile/water; acidic system: 0.04% HCl) and (mobile phase: acetonitrile/water; neutral system: 10 mM NH₄HCO₃), to give compound **049.** MS-ESI m/z: 494.0 [M+H]⁺. ¹H NMR (400 MHz, DMSO_*d*₆) δ: 11.46 (br s, 1H), 8.75 (d, *J* = 0.8 Hz, 1H), 8.51 (dd, *J* = 2.8, 8.4 Hz, 1H), 8.37 (br s, 1H), 7.39-7.34 (m, 1H), 7.23 (t, *J* = 9.0 Hz, 2H), 7.17-7.13 (m, 1H), 5.85 (s, 2H), 4.70 (br dd, *J =* 4.0, 12.0 Hz, 1H), 4.56-4.51 (m, 1H), 3.49 (s, 3H), 2.25 (br d, *J* = 13.6 Hz, 1H), 1.77 (dt, *J* = 4.4, 13.4 Hz, 1H).

### Examples 50 and 51

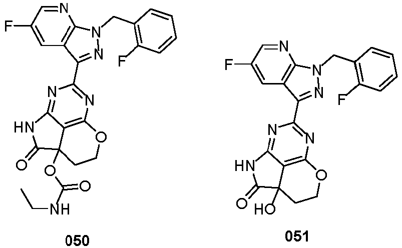

### Synthetic route:

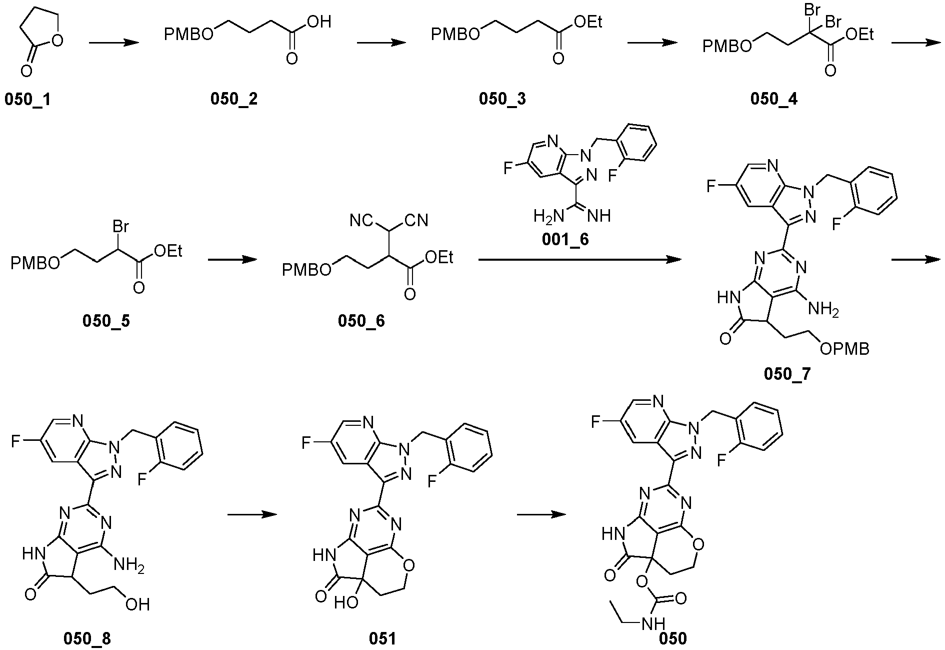

### Step 1: synthesis of intermediate 050_2

Intermediate **050_1** (50 g, 580.79 mmol) was dissolved in toluene (900 mL) at room temperature under nitrogen atmosphere, and p-methoxybenzyl chloride (363.83 g, 2.32 mol) and potassium hydroxide (162.93 g, 2.90 mol) were added. The reaction mixture was warmed stepwise to 110 °C (first 60 °C, then 85 °C, and finally 110 °C) and stirred at 110 °C for 12 h. Then the reaction mixture was concentrated under reduced pressure to remove toluene. The residue was dissolved in methanol (1000 mL), and then a solution of potassium hydroxide (58.00 g, 1.03 mol) in water (500 mL) was added. The resulting mixture was stirred at 85 °C for 12 h. After the reaction was completed, the reaction mixture was concentrated under reduced pressure to remove the solvent. The resulting residue was diluted with water (800 mL) and extracted with methyl *tert-butyl* ether (600 mL × 3). The organic phase was discarded, and the aqueous phase was adjusted to pH 1-2 with concentrated hydrochloric acid and extracted with 2-methyltetrahydrofuran (800 mL × 3). The organic phases were combined, washed with saturated brine (800 mL × 2), dried over anhydrous sodium sulfate, and filtered, and then the filtrate was concentrated under reduced pressure to remove the solvent to give intermediate **050_2.** ¹H NMR (400 MHz, CDCl₃) δ: 9.92 (s, 1H), 7.28 (d, *J* = 8.4 Hz, 2H), 6.91 (d, *J* = 8.4 Hz, 2H), 4.47 (s, 2H), 3.83 (s, 3H), 3.53 (t, *J =* 6.0 Hz, 2H), 2.50 (t, *J =* 7.2 Hz, 2H), 1.96 (m, 2H).

### Step 2: synthesis of intermediate 050_3

Intermediate **050_2** (108.6 g, 484.28 mmol) was dissolved in dichloromethane (1000 mL) at room temperature under nitrogen atmosphere, and ethanol (223.10 g, 4.84 mmol), 1-(3-dimethylaminopropyl)-3-ethylcarbodiimide hydrochloride (111.40 g, 581.13 mmol) and 4-dimethylaminopyridine (7.10 g, 58.11 mmol) were added. The reaction mixture was stirred at room temperature for 12 h and concentrated under reduced pressure to remove the solvent. The resulting residue was diluted with water (1500 mL) and ethyl acetate (1000 mL). Then the aqueous phase was separated and also extracted with ethyl acetate (800 mL × 2). The organic phases were combined, washed successively with saturated aqueous citric acid solution (1000 mL × 3) and saturated brine (1000 mL × 2), dried over anhydrous sodium sulfate, and filtered, and then the filtrate was concentrated under reduced pressure to remove the solvent. The resulting residue was separated by column chromatography (eluent: petroleum ether/ethyl acetate = 1/0 to 1/1, v/v) to give intermediate **050_3.** ¹H NMR (400 MHz, CDCl₃) δ: 7.28 (d, *J =* 8.4 Hz, 2H), 6.90 (d, *J =* 8.4 Hz, 2H), 4.45 (s, 2H), 4.14 (q, *J =* 7.2 Hz, 2H), 3.83 (s, 3H), 3.51 (t, *J* = 6.0 Hz, 2H), 2.43 (t, *J* = 7.6 Hz, 2H), 1.99-1.92 (m, 2H), 1.27 (t, *J =* 7.2 Hz, 3H).

### Step 3: synthesis of intermediate 050_4

A solution of lithium diisopropylamine (2 M, 80.94 mL) in tetrahydrofuran was dissolved in tetrahydrofuran (450 mL) at room temperature under nitrogen atmosphere. The resulting solution was cooled to -78 °C, and then a solution of intermediate **050_3** (37.13 g, 147.16 mmol) in tetrahydrofuran (150 mL) was added slowly. The resulting mixture was stirred at -78 °C for 1 h, and a solution of carbon tetrabromide (73.20 g, 220.74 mmol) in tetrahydrofuran (200 mL) was added. The reaction mixture was brought back to room temperature and stirred for 12 h. The reaction mixture was slowly poured into saturated aqueous ammonium chloride solution (1000 mL), followed by the extraction with ethyl acetate (600 mL × 3). The organic phases were combined, washed with saturated brine (600 mL × 2), dried over anhydrous sodium sulfate, and filtered, and the filtrate was concentrated under reduced pressure to give a residue. The resulting residue was separated by column chromatography (eluent: petroleum ether/ethyl acetate = 1/0 to 7/3, v/v) to give intermediate **050_4.** ¹H NMR (400 MHz, CDCl₃) δ: 7.27-7.24 (m, 2H), 6.90-6.87 (m, 2H), 4.44-4.42 (m, 2H), 4.22 (q, *J* = 6.8 Hz, 2H), 3.81 (s, 3H), 3.74 (t, *J =* 6.4 Hz, 2H), 2.96 (t, *J i=* 6.4 Hz, 2H), 1.28 (t, *J i=* 7.2 Hz, 3H).

### Step 4: synthesis of intermediate 050_5

Intermediate **050_4** (14.7 g, 35.85 mmol) was dissolved in acetonitrile (150 mL) at room temperature under nitrogen atmosphere. The resulting solution was cooled to 0 °C, and then triethylamine (3.63 g, 35.85 mmol) and diethyl phosphite (4.95 g, 35.85 mmol) were added successively. The reaction mixture was brought back to room temperature and stirred for 1 h. The reaction mixture was concentrated under reduced pressure to remove the solvent, and the resulting residue was separated by column chromatography (eluent: petroleum ether/ethyl acetate = 1/0 to 17/3, v/v) to give intermediate **050_5.** ¹H NMR (400 MHz, CDCl₃) δ: 7.25 (d, *J* = 8.8 Hz, 2H), 6.90-6.88 (m, 2H),4.49 (m, 1H), 4.44 (s, 2H), 4.25-4.18 (m, 2H), 3.82 (s, 3H), 3.61-3.57 (m, 2H), 2.44-2.35 (m, 1H), 2.24-2.16 (m, 1H), 1.28 (t, *J* = 7.2 Hz, 3H).

### Step 5: synthesis of intermediate 050_6

A solution of potassium *tert*-butoxide (1 M, 40.94 mL) in tetrahydrofuran was added dropwise to a solution of malononitrile (11.3 g, 34.12 mmol) in tetrahydrofuran (80 mL) at room temperature under nitrogen atmosphere. The reaction mixture was stirred at room temperature for 30 min. The reaction mixture was added to a solution of intermediate **050_5** (11.3 g, 34.12 mmol) in tetrahydrofuran (80 mL) at 75 °C. The resulting mixture was stirred for 12 h. After the reaction was completed, the reaction mixture was cooled to room temperature, poured into diluted hydrochloric acid (1 M, 100 mL), and extracted with ethyl acetate (100 mL), and the aqueous phase was also extracted with ethyl acetate (100 mL × 2). The organic phases were combined, dried over anhydrous sodium sulfate, and filtered, and the filtrate was concentrated under reduced pressure to give a residue. The residue was separated by column chromatography (eluent: petroleum ether/ethyl acetate = 1/0 to 7/3, v/v) to give intermediate **050_6.** ¹H NMR (400 MHz, CDCl₃) δ: 7.26-7.24 (d, *J =* 8.8 Hz, 2H), 6.91 (d, *J =* 8.4 Hz, 2H), 4.44 (s, 2H), 4.35 (d, *J* = 6.4 Hz, 1H), 4.29-4.23 (m, 2H), 3.83 (s, 3H), 3.62-3.54 (m, 2H), 3.24-3.19 (m, 1H), 2.30-2.23 (m, 1H), 2.17-2.10 (m, 1H), 1.30 (t, *J* = 7.2 Hz, 3H).

### Step 6: synthesis of intermediate 050_7

Intermediate **001_6** (1.02 g, 3.16 mmol, hydrochloride) was dissolved in ethanol (20 mL) at room temperature under nitrogen atmosphere, and sodium ethoxide (537.78 mg, 7.90 mmol) and intermediate **050_6** (1 g, 3.16 mmol) were added successively. The reaction mixture was stirred at 80 °C for 12 h. After the reaction was completed, the reaction mixture was cooled to room temperature and concentrated under reduced pressure to remove the solvent, and the residue was diluted with water (50 mL) and extracted with 2-methyltetrahydrofuran (50 mL). The aqueous phase was separated and extracted with 2-methyltetrahydrofuran (50 mL × 2). The organic phases were combined, dried over anhydrous sodium sulfate, and filtered, and then the filtrate was concentrated under reduced pressure to remove the solvent. The resulting residue was separated by column chromatography (eluent: petroleum ether/2-methyltetrahydrofuran = 1/0 to 3/7, v/v) to give intermediate **050_7.** ¹H NMR (400 MHz, DMSO_*d₆*) δ: 10.93 (s, 1H), 8.84 (dd, *J =* 2.8, 8.8 Hz, 1H), 8.70 (dd, *J =* 1.6, 2.8 Hz, 1H), 7.39-7.33 (m, 1H), 7.27-7.22 (m, 2H), 7.17-7.13 (m, 1H), 7.06 (d, *J =* 8.4 Hz, 2H), 6.81 (s, 2H), 6.71 (d, *J =* 8.4 Hz, 2H), 5.81 (s, 2H), 4.28 (d, *J =* 11.6 Hz, 1H), 4.14 (d, *J* = 11.6 Hz, 1H), 3.85-3.67 (m, 2H), 3.54 (s, 3H), 3.52-3.49 (m, 1H), 2.44 (t, *J* = 7.2 Hz, 2H).

### Step 7: synthesis of intermediate 050_8

Compound intermediate **050_7** (5.5 g, 9.86 mmol) was dissolved in 1,2-dichloroethane (110 mL) at room temperature under nitrogen atmosphere. The resulting solution was cooled to 0 °C, and a solution of aluminum trichloride (3.95 g, 29.59 mmol) in toluene was added. The reaction mixture was stirred at room temperature for 12 h. After the reaction was completed, the reaction mixture was poured into diluted hydrochloric acid (1 M, 200 mL) and extracted with 2-methyltetrahydrofuran (200 mL), and the aqueous phase was also extracted with 2-methyltetrahydrofuran (200 mL × 2). The organic phases were combined, dried over anhydrous sodium sulfate, and filtered, and the filtrate was concentrated under reduced pressure to remove the solvent. The resulting residue was separated by column chromatography (eluent: dichloromethane/methanol = 1/0 to 20/1, v/v) to give intermediate **050_8.**

### Step 8: synthesis of compound 051

Intermediate **050_8** (800 mg, 1.83 mmol) was dissolved in *N*,*N*-dimethylformamide (16 mL) and water (1.6 mL) at room temperature under nitrogen atmosphere, and *tert*-butyl nitrite (2.83 g, 27.43 mmol) was added. The reaction mixture was stirred at room temperature for 12 h. After the reaction was completed, the reaction mixture was diluted with 2-methyltetrahydrofuran (80 mL) and washed with semi-saturated brine (50 mL × 3). The organic phase was dried over anhydrous sodium sulfate and filtered, and then the filtrate was concentrated under reduced pressure to remove the solvent. The resulting residue was separated by preparative HPLC (mobile phase: acetonitrile/water; acidic system: 0.04% HCl) to give compound **051.** MS-ESI m/z: 437.0 [M+H]⁺. ¹H NMR (400 MHz, DMSO_*d₆*) δ: 11.23 (s, 1H), 8.75 (dd, *J* = 1.6, 2.8 Hz, 1H), 8.51 (dd, *J* = 2.8, 8.4 Hz, 1H), 7.40-7.35 (m, 1H), 7.28-7.21 (m, 2H), 7.18-7.14 (m, 1H), 6.65 (s, 1H), 5.85 (s, 2H), 4.73 (dd, *J* = 3.2, 11.6 Hz, 1H), 4.64-4.55 (m, 1H), 2.05 *(d, J=* 14.0 Hz, 1H), 1.76 (dt, *J* = 4.8, 13.6 Hz, 1H).

### Step 9: synthesis of compound 050

Compound **051** (60 mg, 137.50 µmol) was dissolved in tetrahydrofuran (2 mL) at room temperature under nitrogen atmosphere. The resulting solution was cooled to 0 °C, and sodium hydride (16.5 mg, 412.49 µmol, 60% purity) was added. The resulting mixture was stirred at 0 °C for 30 min, and then ethyl isocyanate (4.89 mg, 68.75 µmol) was added. The reaction mixture was stirred at room temperature for 2 h. After the reaction was completed, saturated aqueous ammonium chloride solution (5 mL) was added, followed by the extraction with 2-methyltetrahydrofuran (5 mL). The aqueous phase was also extracted with 2-methyltetrahydrofuran (5 mL × 2). The organic phases were combined, dried over anhydrous sodium sulfate, and filtered, and then the filtrate was concentrated under reduced pressure to remove the solvent. The resulting residue was separated by preparative HPLC (mobile phase: acetonitrile/water; acidic system: 0.04% HCl) to give compound 050. MS-ESI m/z: 508.0 [M+H]⁺. ¹H NMR (400 MHz, DMSO_*d₆*) δ: 11.55 (s, 1H), 8.75 (s, 1H), 8.73-8.70 (m, 1H), 8.49 (t, *J* = 6.0 Hz, 1H), 7.37 (d, *J* = 7.2 Hz, 1H), 7.23 (t, *J =* 9.2 Hz, 2H), 7.17-7.13 (m, 1H), 5.86 (s, 2H), 4.72 (d, *J =* 12.8 Hz, 1H), 4.31 (t, *J =* 10.8 Hz, 1H), 3.18-3.08 (m, 2H), 2.43 (s, 2H), 1.01 (t, *J* = 7.2 Hz, 3H).

### Examples 52 and 53

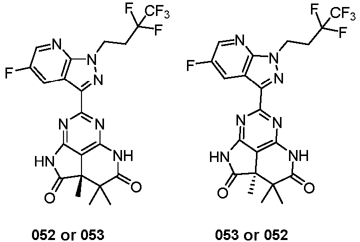

### Synthetic route:

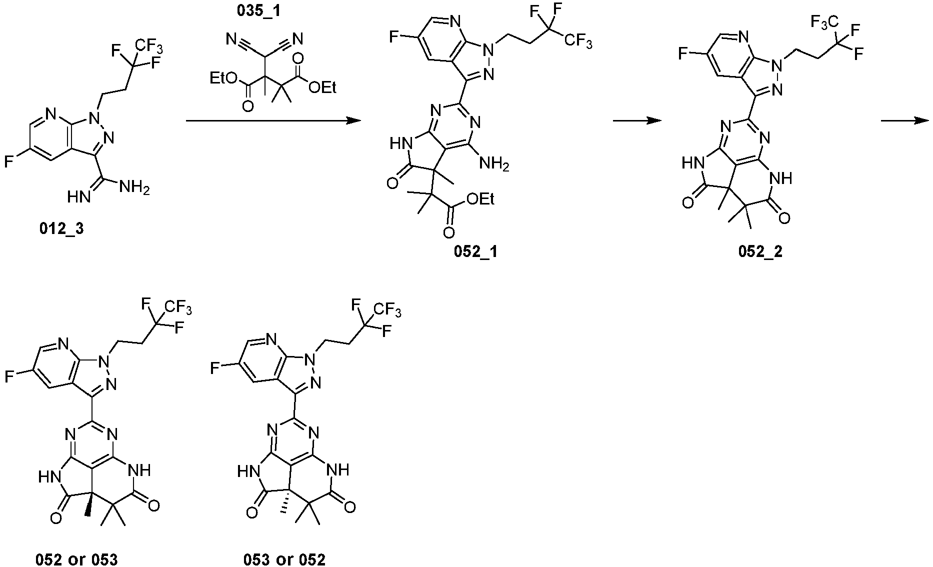

### Step 1: synthesis of intermediate 052_1

Intermediate **012_3** (300 mg, 0.829 mmol, hydrochloride) and intermediate **035_1** (348.78 mg, 1.24 mmol) were dissolved in *tert*-butanol (6 mL) at room temperature under nitrogen atmosphere, and potassium carbonate (343.92 mg, 2.49 mmol) was added. The reaction system was warmed to 85 °C and stirred for 12 h. The reaction system was cooled to room temperature, and water (40 mL) and 2-methyltetrahydrofuran (30 mL) were added. The organic phase was separated and collected, washed with saturated brine (20 mL), dried over anhydrous sodium sulfate, and filtered, and the filtrate was concentrated under reduced pressure to remove the solvent. The resulting residue was separated by column chromatography (eluent: dichloromethane/methanol = 1/0 to 98/2, v/v) to give intermediate **052_1.**

### Step 2: synthesis of intermediate 052_2

Intermediate **052_1** (250 mg, 446.86 µmol) was dissolved in toluene (4 mL) at room temperature under nitrogen atmosphere, and a solution of trimethylaluminum (2 M, 0.715 mL) in toluene was added under nitrogen atmosphere. The reaction system was warmed to 80 °C and stirred for 12 h. After the reaction was completed, 6 mL of a 3 M hydrochloric acid solution was added, and ethyl acetate (40 mL) and water (20 mL) were added for dilution. The organic phase was separated and collected, and the aqueous phase was extracted with ethyl acetate (40 mL × 3). The organic phases were combined, washed with saturated brine (50 mL × 2), dried over anhydrous sodium sulfate, and filtered, and the filtrate was concentrated under reduced pressure to remove the solvent. The resulting residue was separated by preparative HPLC (mobile phase: acetonitrile/water; acidic system: 0.04% HCl) to give the target intermediate **052_2.** MS-ESI m/z: 514.2[M+H]⁺. ¹H NMR (400 MHz, DMSO_*d₆*) δ: 11.57 (s, 1H), 11.21 (s, 1H), 8.76-8.75 (m, 1H), 8.71 (dd, *J* = 2.8, 8.4 Hz, 1H), 4.91 (t, *J =* 6.8 Hz, 2H), 3.07-2.93 (m, 2H), 1.34 (d, *J* = 4 Hz, 6H), 0.78 (s, 3H).

### Step 3: synthesis of compounds 052 and 053

Intermediate **052_2** (185 mg, 360.35 µmol) was separated by chiral column chromatography (column model: DAICEL CHIRALCEL OD (250 mm × 30 mm, 10 µm); mobile phase: [Neu-IPA]%: 25%-25%, 4 min) to give the target compounds **052** and **053.**

**052** (retention time: 2.411 min): MS-ESI m/z: 514.2 [M+H]⁺. ¹H NMR (400 MHz, DMSO_ *d*₆) *δ:* 11.55 (s, 1H), 11.18 (s, 1H), 8.76-8.75 (m, 1H), 8.71 (dd, *J* = 2.8, 8.4 Hz, 1H), 4.91 (t, *J =* 6.4 Hz, 2H), 3.06-2.93 (m, 2H), 1.33 (d, *J* = 6.4 Hz, 6H), 0.77 (s, 3H).

**053** (retention time: 2.642 min): MS-ESI m/z: 514.2 [M+H]⁺. ¹H NMR (400 MHz, DMSO_ *d*₆) δ: 11.55 (s, 1H), 11.17 (s, 1H), 8.76-8.75 (m, 1H), 8.71 (dd, *J* = 2.8, 8.4 Hz, 1H), 4.91 (t, *J* = 6.4 Hz, 2H), 3.11-2.90 (m, 2H), 1.33 (d, *J* = 6.4 Hz, 6H), 0.77 (s, 3H).

### Example 54

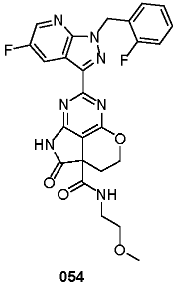

### Synthetic route:

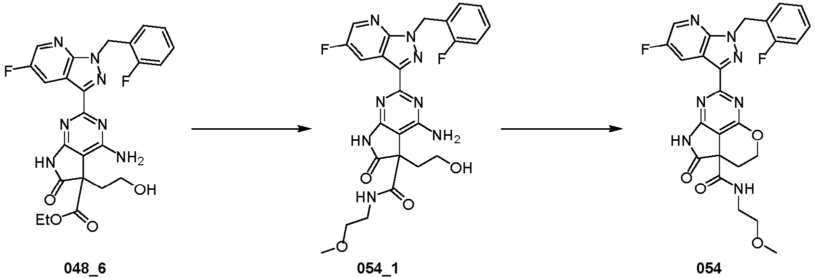

### Step 1: synthesis of intermediate 054_1

**048_6** (1 g, 1.96 mmol) was dissolved in tetrahydrofuran (10 mL) at room temperature under nitrogen atmosphere, and then 2-methoxyethylamine (4.42 g, 58.89 mmol) was added. The reaction system was stirred at 65 °C for 12 h. After the reaction was completed, the reaction mixture was cooled to room temperature, diluted with water (10 mL), adjusted to pH 5-6 with 3 M diluted hydrochloric acid, and extracted with 2-methyltetrahydrofuran (20 mL × 3). The organic phases were combined, washed with saturated brine (15 mL × 2), dried over anhydrous sodium sulfate, and filtered, and the filtrate was concentrated under reduced pressure to remove the solvent. The resulting residue was separated by column chromatography (eluent: dichloromethane/methanol = 1/0 to 97/3, v/v) to give compound **054_1.**

### Step 2: synthesis of compound 054

**054_1** (188 mg, 349.11 µmol) was dissolved in N,N-dimethylformamide (2 mL) in a preliminarily dried reaction flask at room temperature under nitrogen atmosphere, and then *tert-butyl* nitrite (540 mg, 5.24 mmol) and water (200 mg, 11.10 mmol) were added. The reaction system was stirred at room temperature for 12 h. After the reaction was completed, the reaction mixture was separated by preparative HPLC (mobile phase: acetonitrile/water; acidic system: 0.04% HCl) to give compound **054.** MS-ESI m/z: 522.2 [M+H]⁺.¹H NMR (400 MHz, DMSO_*d₆*) δ: 11.44 (s, 1H), 8.78-8.75 (m, 1H), 8.53 (dd, *J* = 2.8, 8.4 Hz, 1H), 8.01 (t, *J =* 5.6 Hz, 1H), 7.41-7.33 (m, 1H), 7.27-7.16 (m, 3H), 5.91-5.81 (m, 2H), 4.72 (d, *J =* 12.4 Hz, 1H), 4.13-4.05 (m, 1H), 3.29-3.16 (m, 7H), 2.72 (d, *J* = 13.4 Hz, 1H), 1.88-1.78 (m, 1H).

### Example 55

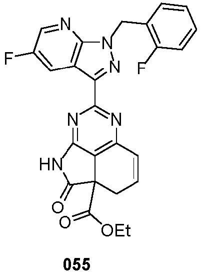

### Synthetic route:

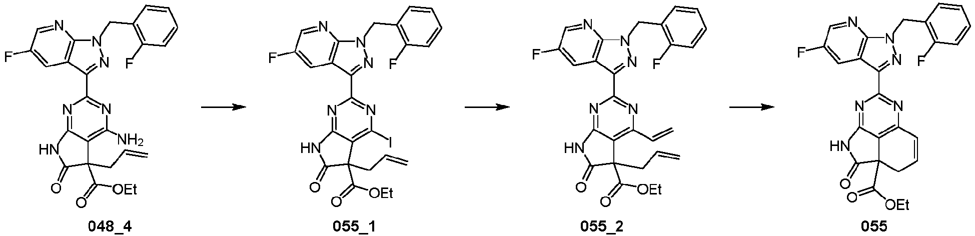

### Step 1: synthesis of intermediate 055_1

Intermediate **048_4** (3.0 g, 5.94 mmol) was dissolved in 1,4-dioxane (30 mL) at room temperature under nitrogen atmosphere, and isoamyl nitrite (3.48 g, 29.68 mmol, 4.00 mL) and diiodomethane (4.77 g, 17.81 mmol, 1.44 mL) were added to the reaction system. The reaction system was warmed to 100 °C and stirred for 2 h. The reaction system changed from an emulsion to a clear liquid. The reaction mixture was stirred at 100 °C for another 4 h. After the reaction was completed, the reaction mixture was directly concentrated to remove the solvent to give a crude product. The crude product was separated and purified by column chromatography (eluent: petroleum ether/ethyl acetate = 1/0 to 0/1, v/v) to give intermediate **055_1.**

### Step 2: synthesis of intermediate 055_2

Intermediate **055_1** (800 mg, 1.30 mmol), vinyltri-n-butyltin (617.36 mg, 1.95 mmol, 566.39 µL) and [1,1-bis(diphenylphosphino)ferrocene]dichloropalladium dichloromethane (106.0 mg, 129.79 µmol) were dispersed in 1,4-dioxane (10 mL) at room temperature under nitrogen atmosphere. The reaction vessel was placed in an oil bath at 80 °C and the dispersion was stirred for 12 h. After the reaction was completed, the reaction mixture was poured into water (100 mL) and extracted with ethyl acetate (40 mL × 3). The organic phases were combined, washed with saturated brine (50 mL), dried over anhydrous sodium sulfate, and then filtered, and the filtrate was concentrated. The crude product was separated and purified by column chromatography (eluent: petroleum ether/ethyl acetate = 1/0 to 1/1, v/v) to give intermediate **055_2.**

### Step 3: synthesis of compound 055

Intermediate **055_2** (300 mg, 580.83 µmol) was dissolved in dichloromethane (5 mL) at room temperature under nitrogen atmosphere, and the GRUBB'S second-generation catalyst (49.31 mg, 58.08 µmol) was added. Then the reaction mixture was stirred at room temperature for 15 h. After the reaction was completed, the reaction mixture was directly concentrated to remove the solvent to give a residue. The residue was separated and purified by column chromatography (eluent: petroleum ether/ethyl acetate = 1/0 to 1/1, v/v) to give a crude product. The crude product was separated by preparative HPLC (mobile phase: acetonitrile/water; hydrochloric acid system: 0.04% HCl) to give compound 055. MS-ESI m/z: 489.1 [M+H]⁺. ¹H NMR (400 MHz, DMSO_*d₆*) δ: 11.76 (s, 1H), 8.75-8.74 (m, 1H), 8.63 (dd, *J =* 3.2, 5.6 Hz, 1H), 7.41-7.35 (m, 1H), 7.29-7.21 (m, 2H), 7.19-7.15 (m, 1H), 6.74 (dd, *J =* 2.4, 9.6 Hz, 1H), 6.62-6.58 (m, 1H), 5.86 (s, 2H), 4.20-4.05 (m, 2H), 3.26 (dd, *J =* 5.6, 17.6 Hz, 1H), 2.75 (td, *J* = 2.4, 17.6 Hz, 1H), 1.08 (t, *J =* 7.2 Hz, 3H).

### Example 56

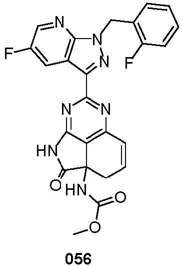

### Synthetic route:

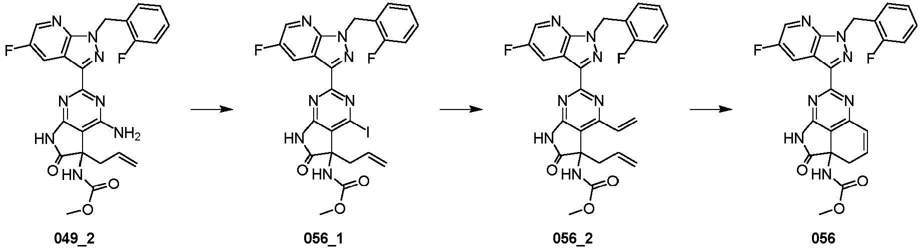

### Step 1: synthesis of intermediate 056_1

Intermediate **049_2** (0.5 g, 987.24 µmol) was dissolved in 1,4-dioxane (5 mL) at room temperature under nitrogen atmosphere, and isoamyl nitrite (578.27 mg, 4.94 mmol) and diiodomethane (793.24 mg, 2.96 mmol) were added to the reaction system. The reaction system was warmed to 85 °C and stirred for 2 h. The reaction system changed from an emulsion to a clear liquid. After the reaction was completed, the reaction mixture was poured into water (50 mL) and extracted with ethyl acetate (20 mL × 3). The organic phases were combined, washed with saturated brine (50 mL), dried over anhydrous sodium sulfate, and then filtered, and the filtrate was concentrated. The resulting residue was separated and purified by column chromatography (eluent: petroleum ether/ethyl acetate = 1/0 to 0/1, v/v) to give intermediate **056_1.**

### Step 2: synthesis of intermediate 056_2

Intermediate **056_1** (500 mg, 809.92 µmol), vinyltri-n-butyltin (385.23 mg, 1.21 mmol) and [1,1-bis(diphenylphosphino)ferrocene]dichloropalladium dichloromethane (66.14 mg, 80.99 µmol) were dissolved in 1,4-dioxane (10 mL) at room temperature under nitrogen atmosphere. The reaction vessel was placed in an oil bath at 80 °C and the dispersion was stirred for 12 h. After the reaction was completed, the reaction mixture was poured into water (50 mL) and extracted with ethyl acetate (20 mL × 3). The organic phases were combined, washed with saturated brine (50 mL), dried over anhydrous sodium sulfate, and then filtered, and the filtrate was concentrated. The resulting residue was separated and purified by column chromatography (eluent: petroleum ether/ethyl acetate = 1/0 to 1/1, v/v) to give intermediate **056_2.**

### Step 3: synthesis of compound 056

Intermediate **056_2** (150 mg, 289.86 µmol) was dissolved in dichloromethane (3 mL) at room temperature under nitrogen atmosphere, and the GRUBB'S second-generation catalyst (24.61 mg, 28.99 µmol) was added. The reaction system was stirred at room temperature for 12 h. After the reaction was completed, the reaction mixture was directly concentrated to remove the solvent to give a residue. The residue was separated and purified by column chromatography (eluent: petroleum ether/ethyl acetate = 1/0 to 1/2, v/v) to give a crude product. The crude product was separated by preparative HPLC (mobile phase: acetonitrile/water; hydrochloric acid system: 0.04% HCl) to give compound 056. MS-ESI m/z: 490.1 [M+H]⁺. ¹H NMR (400 MHz, MeOD_*d₄*) δ: 8.65-8.60 (m, 2H), 7.33 (q, *J* = 7.2 Hz, 1H), 7.24 (br t, *J* = 7.6 Hz, 1H), 7.15-7.07 (m, 2H), 6.84 (dd, *J* = 2.8, 9.6 Hz, 1H), 6.71 (ddd, *J =* 2.0, 5.6, 9.6 Hz, 1H), 5.95 (s, 2H), 3.56 (s, 3H), 2.89 (dd, *J* = 5.6, 18.0 Hz, 1H), 2.64 (td, *J* = 2.4, 18.0 Hz, 1H).

### Bioassays

### Experimental Example 1: In Vitro Activity Assay

1. **InCap cell-based cGMP expression assay**
1. **Procedure**
1) **Preparation of solutions**
• **10% BSA (bovine serum albumin)**
10 g of BSA was dissolved in 100 mL of double distilled water (ddH₂O) to give 10% BSA.
• **5 mM DETA (diethylenetriamine)-NO**
10 mg of DETA-NO was weighed out and dissolved in 12.2 mL of double distilled water (ddH₂O) to give 5 mM DETA-NO, which was aliquoted and cryopreserved in a freezer at -20 °C.
• **Washing buffer (50 mL)**

| **Volume** | **Final concentration** |
|---|---|
| 49 mL of Earls balanced salt solution (EBSS) | 1× |
| 500 µL of hydroxyethylpiperazineethanesulfonic acid (HEPES) 1 M | 10 mM |
| 250 µL of 10% BSA stabilizer | 0.05% |
| 250 µL of MgCl₂ 1 M | 5 mM |

• **Assay buffer (50 mL)**

| **Volume** | **Final concentration** |
|---|---|
| 48.95 mL of Earls balanced salt solution (EBSS) | 1× |
| 500 µL of hydroxyethylpiperazineethanesulfonic acid (HEPES) 1 M | 10 mM |
| 250 µL of 10% BSA | 0.05% |
| 50 µL of isobutylmethylxanthine (IBMX) 500 mmol/L | 0.5 mM |
| 250 µL of MgCl₂ 1 M | 5 mM |

• **Detection buffer**
a) To 1 mL of a lysis buffer was added 50 µL of cGMP-D2 (D2-labeled cyclic guanosine monophosphate), and the mixture was well mixed.
b) To 1 mL of a lysis buffer was added 50 µL of anti-cGMP cryptate (Eu³⁺ cryptate-labeled anti-cyclic guanosine monophosphate antibody), and the mixture was well mixed.

1) **Dilution of compounds**
(1) The compounds were diluted to 5 mM with DMSO. 10 µL of each of the compounds was transferred to a shallow well plate for Echo.
(2) The compounds were gradiently diluted with Echo. Each of the compounds was diluted to obtain 10 concentration gradients, and 50 nL of each of the concentration gradients was added to a 384 microwell plate.

2) **Preparation of LNCap cells**
(1) LNCap medium: RPMI1640 + 10% fetal bovine serum + 1% bispecific antibody.
(2) Phosphate buffered saline, pancreatin and the medium used in the process of cell passaging were pre-heated in a water bath at 37 °C.
(3) The cells (the 14th generation) were taken out of the 37 °C, 5% CO₂ incubator, and the old medium was pipetted off the culture flask.
(4) 5 mL of phosphate buffered saline was pipetted into the culture flask to rinse the cells, and then the liquid was discarded.
(5) 3 mL of pancreatin was pipetted into the culture flask. After the culture flask was shaken, the liquid was discarded, and the culture flask was placed in the incubator.
(6) After about 2 min the culture flask was taken out. After all the cells were observed to separate, 9 mL of the medium was added to the culture flask and the mixture was pipetted several times. The cell suspension was transferred to a 50 mL centrifuge tube.
(7) 0.7 mL of the cell suspension was pipetted into a counter cup and the cells were counted on a ViCell XR. The remaining cells were centrifuged at 1000 rpm for 5 min, and the supernatant was removed.
(8) The cells were washed by adding 10 mL of the washing buffer and centrifuged at 1000 rpm for 5 min, and the supernatant was removed.
(9) The assay buffer was added to adjust the cell concentration to 1.25 × 10⁶/mL. The cells were added to the microwell plate at 8 µL/well.

4) **Formulation and addition of DETA-NO**
(1) 10 µL of 5 mM DETA-NO was added to 1240 µL of the assay buffer and to 1657 µL of the assay buffer to give 40 µM DETA-NO and 30 µM DETA-NO, respectively.
(2) DETA-NO was transferred to the 384 microwell plate at 2 µL/well using Bravo.
(3) The mixtures were centrifuged at 1500 rpm for 5 min. The microwell plate was incubated at 37 °C for 30 min.

5) **Preparation of cGMP standard curve**
(1) 1 mM of the cGMP stock solution was diluted to 10 µM with the assay buffer. Then 4-fold gradient dilution was performed to obtain 11 concentration gradients.
(2) The cGMP dilutions were added to the microwell plate at 10 µL/well.

6) **Addition of detection reagents and plate reading**
(1) cGMP-D2 was transferred to the 384 microwell plate at 5 µL/well using Bravo. The mixtures were centrifuged at 1500 rpm for 1 min.
(2) The anti-cGMP cryptate was transferred to the 384 microwell plate at 5 µL/well using Bravo. The mixtures were centrifuged at 1500 rpm for 1 min.
(3) The plate was incubated at room temperature for 1 h.
(4) 665/615 was read using envision.

7) **Data analysis**
(1) cGMP standard curve: a standard curve was generated using Graphpad prism based on the cGMP concentrations and 665/615 ratios.
(2) Conversion of HTRF (homogeneous time-resolved fluorescence) ratios (665/615) to cGMP concentrations: in Graphpad prism, the HTRF ratios (665/615) were copied into the ratio column of the cGMP standard curve, and the "Log inhibitor vs response-variable slope" analysis was run with "interpolate" selected to convert the HTRF ratios (665/615) to cGMP concentrations.
(3) Compound activation curve: a curve was generated using the "Log agonist vs response-variable slope" analysis method in Graphpad prism based on the cGMP concentrations obtained by the conversion and the compound concentrations.

**Table 1. MEC values of the stimulating activity of the compounds of the present application for sGC**

| Compound No. | MEC (nM) | Compound No. | MEC (nM) | Compound No. | MEC (nM) |
|---|---|---|---|---|---|
| 001 | 6.38 | 016 | 17.51 | 034 | 31.53 |
| 002 | 81.22 | 017 | 3.46 | 035 | 10.08 |
| 003 | 7.97 | 018 | 13.12 | 036 | <5.08 |
| 004 | 142.79 | 019 | 2.4 | 037 | 33.86 |
| 005 | 5.21 | 020 | 28.72 | 038 | 19.13 |
| 006 | 2.32 | 021 | 18.99 | 039 | 28.15 |
| 007 | 55.54 | 022 | 24.9 | 040 | 931.1 |
| 008 | 88.37 | 023 | 26.12 | 041 | 160.5 |
| 009 | 5.8 | 024 | 7.59 | 042 | 24.32 |
| 010 | 51.76 | 028 | 21.26 | 043 | 29.99 |
| 011 | 178.6 | 029 | 37.34 | 048 | 33.55 |
| 012 | 118.6 | 030 | 84.34 | 049 | 126.5 |
| 013 | 3.12 | 031 | 19.78 | 050 | 231.7 |
| 014 | 402.7 | 032 | 23.75 | 052 | 68.64 |
| 015 | 4.66 | 033 | 20.71 | 053 | 25.81 |

| | | | | | |
|---|---|---|---|---|---|
| MEC: minimum effective concentration to stimulate cGMP production (three times greater than the basal value) in lnCap cells. Conclusion: the compounds of the present application can effectively stimulate sGC, significantly increasing the cGMP level. | | | | | |

### Experimental Example 2: In Vivo Pharmacokinetic Property Study

Objective: this study was intended to determine the pharmacokinetic parameters of the compounds in male SD rats.
Materials:
   Sprague Dawley rats (Male, 200-300 g, 7-9 weeks old, Shanghai SLAC)

### Method:

The project used 4 male SD rats: one group of 2 SD rats were dosed by intravenous injection at 0.3 mg/kg, 0.15 mg/mL, and another group of 2 SD rats were orally dosed at 1 mg/kg, 0.2 mg/mL. Plasma samples were collected at 0.083 h (the intravenous injection group only), 0.25 h, 0.5 h, 1 h, 2 h, 4 h, 6 h, 8 h, 12 h and 24 h after the dosing and then subjected to LC-MS/MS analysis, and data were recorded. Relevant pharmacokinetic parameters were calculated using the Phoenix WinNonlin 6.3 software based on the recorded data.

The results are shown in Table 2.

**Table 2. In vivo pharmacokinetic results**

| Test sample | | 053 |
|---|---|---|
| iv (0.3 mg/kg) | **Cl (mL/min/kg)** | 6.88 |
| | **V_{dss} (L/kg)** | 4.65 |
| | **T_{1/2} (h)** | 8.45 |
| po (1 mg/kg) | **T_{1/2} (h)** | 7.63 |
| | %F | 75.9% |

| | | |
|---|---|---|
| Conclusion: the compounds of the present application have good clearance, half-lives and intragastric oral bioavailability. | | |

## Claims

1. A compound of formula (II), a stereoisomer thereof or a pharmaceutically acceptable salt thereof, 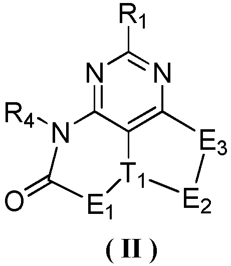 wherein,
R₁ is selected from the group consisting of 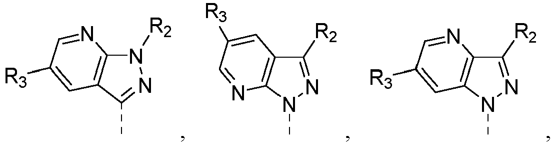 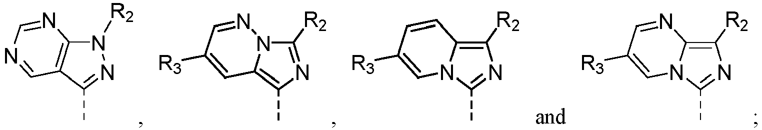
each R₂ is independently selected from the group consisting of 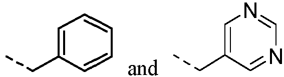 substituted with 1 or 2 R_{d} and C₁₋₃ alkyl substituted with 1, 2 or 3 R_{d};
each R₃ is independently selected from the group consisting of H and halogen;
R₄ is selected from the group consisting of H and C₁₋₃ alkyl;
E₁ is selected from -(CH₂)ₘ-;
m is selected from the group consisting of 0, 1 and 2;
E₂ is selected from the group consisting of -(CH₂)ₙ-, -(CH₂)ₚC(O)-, -O(CH₂)_{q}-, -O(CH₂)ᵣC(O)-, -CH₂CH=CH- and -(CH₂)ₛNHC(O)-, wherein each of the CH₂ optionally substituted with 1 or 2 R_{b};
E₃ is selected from the group consisting of a single bond, NR_{c} and O;
n is selected from the group consisting of 1, 2 and 3;
p is selected from the group consisting of 0, 1 and 2;
q is selected from the group consisting of 1 and 2;
r is selected from the group consisting of 1 and 2;
s is selected from the group consisting of 1 and 2;
T₁ is selected from the group consisting of N and CRₐ;
each Rₐ is independently selected from the group consisting of H, OH, -OC(=O)NHEt, -CO₂Et, -NHCO₂CH₃, -C(=O)NH(CH₂)₂OCH₃ and C₁₋₃ alkyl;
each R_{b} is independently selected from the group consisting of F and CH₃;
each R_{c} is independently selected from the group consisting of H and CH₃;
each R_{d} is independently selected from the group consisting of halogen and CF₃.

2. The compound, the stereoisomer thereof or the pharmaceutically acceptable salt thereof according to claim 1, wherein each Rₐ is independently selected from the group consisting of H, OH, -OC(=O)NHEt, -CO₂Et, -C(=O)NH(CH₂)₂OCH₃, -NHCO₂CH₃ and CH₃.

3. The compound, the stereoisomer thereof or the pharmaceutically acceptable salt thereof according to claim 1, wherein each R_{d} is independently selected from the group consisting of F and CF₃.

4. The compound, the stereoisomer thereof or the pharmaceutically acceptable salt thereof according to claim 1, wherein each R₂ is independently selected from the group consisting of 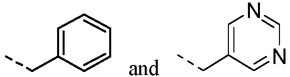 substituted with 1 or 2 R_{d} and C₁₋₃ alkyl substituted with 1, 2 or 3 R_{d}; optionally, R₂ is independently selected from the group consisting of 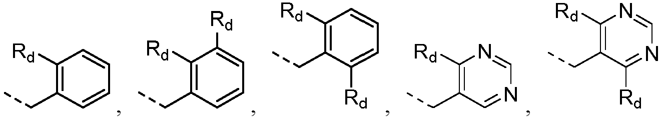 and 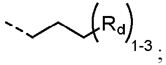 optionally, each R₂ is independently selected from the group consisting of

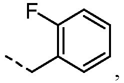

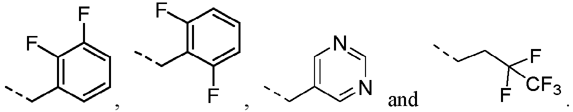

5. The compound, the stereoisomer thereof or the pharmaceutically acceptable salt thereof according to claim 1, wherein each R₃ is independently selected from the group consisting of H and F.

6. The compound, the stereoisomer thereof or the pharmaceutically acceptable salt thereof according to claim 1, wherein R₁ is selected from the group consisting of 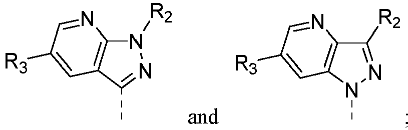 ; optionally, R₁ is selected from the group consisting of 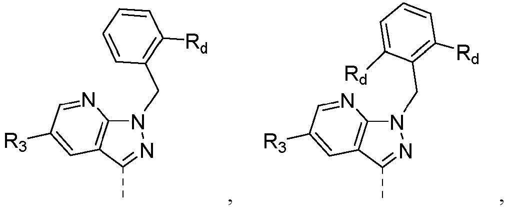 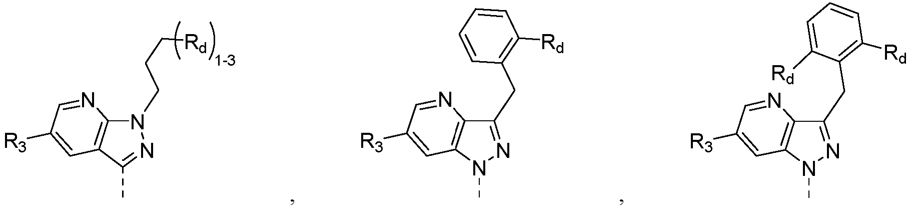 and 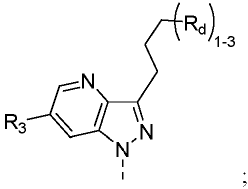 optionally, R₁ is selected from the group consisting of

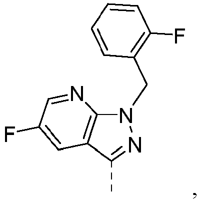

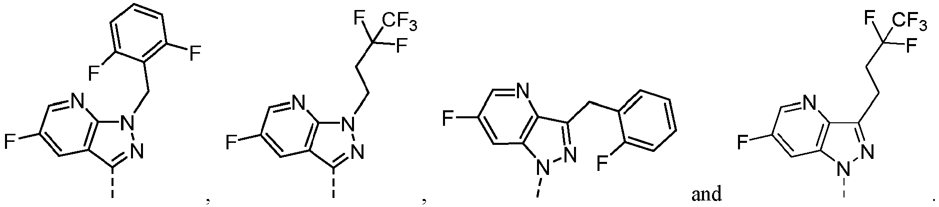

7. The compound, the stereoisomer thereof or the pharmaceutically acceptable salt thereof according to claim 1, wherein E₁ is selected from the group consisting of a single bond, -CH₂- and -(CH₂)₂-.

8. The compound, the stereoisomer thereof or the pharmaceutically acceptable salt thereof according to claim 1, wherein E₂ is selected from the group consisting of -CH₂-, -(CH₂)₂-, -(CH₂)₃-, -CH₂CO-, -(CH₂)₂CO-, -O(CH₂)₂-, -OCH₂C(O)- and -CH₂NHC(O)-, wherein each of the CH₂ is optionally substituted with 1 or 2 R_{b}; optionally, E₂ is selected from the group consisting of -CH₂-, -(CH₂)₂-, -CF₂CH₂-, -(CH₂)₃-, -CH₂CH=CH-, -CH₂CO-, -CO-, -C(CH₃)₂CO-, -CF₂CO-, -(CH₂)₂CO-, -O(CH₂)₂-, -OCH₂C(O)- and -CH₂NHC(O)-.

9. The compound, the stereoisomer thereof or the pharmaceutically acceptable salt thereof according to claim 1, wherein E₃ is selected from the group consisting of a single bond, NH, N(CH₃) and O.

10. The compound, the stereoisomer thereof or the pharmaceutically acceptable salt thereof according to claim 1, wherein T₁ is selected from the group consisting of N, CH, C(OH), C(OC(=O)NHEt), C(CO₂Et), C(NHCO₂CH₃), C[C(=O)NH(CH₂)₂OCH₃] and C(CH₃).

11. The compound, the stereoisomer thereof or the pharmaceutically acceptable salt thereof according to claim 1, wherein the structural unit 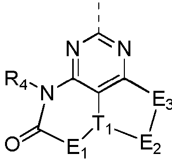 is selected from the group consisting of 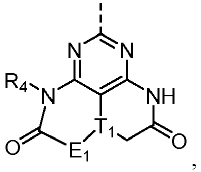 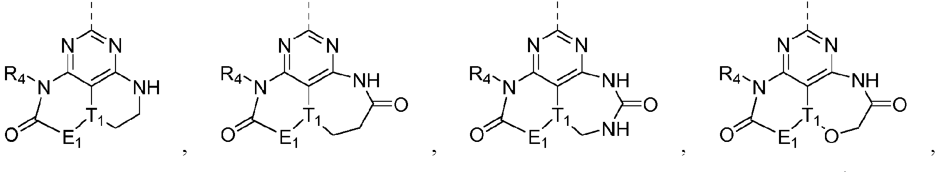 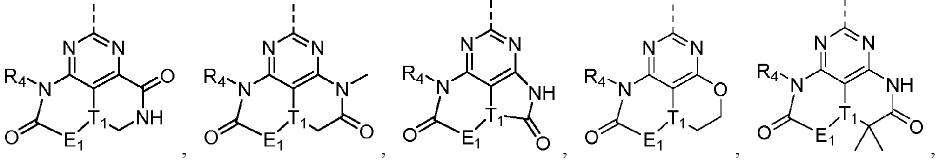 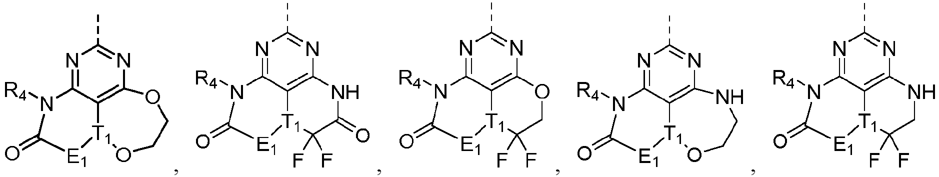 and 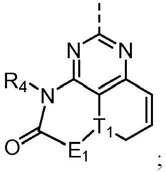 optionally, the structural unit 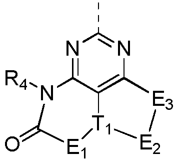 is selected from the group consisting of 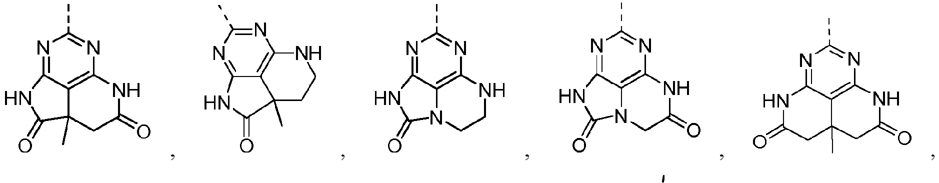 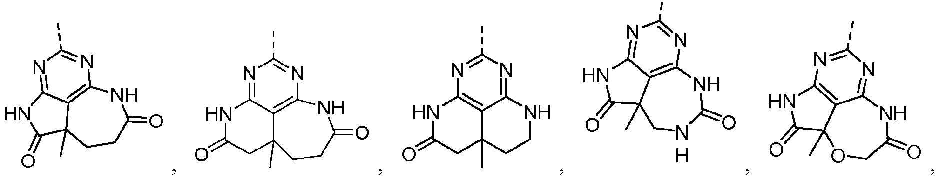 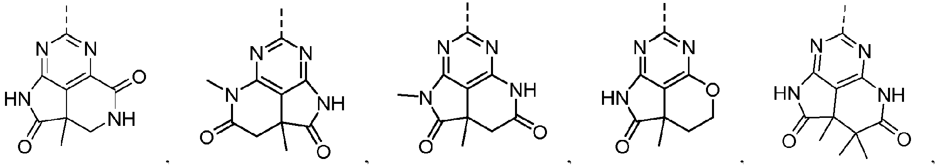 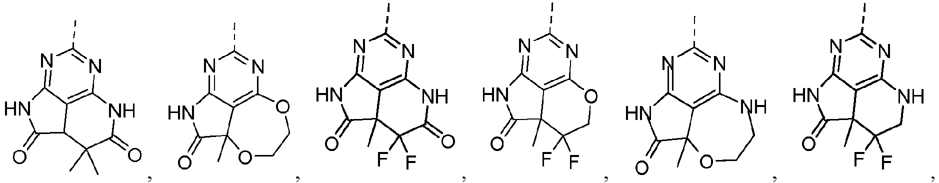 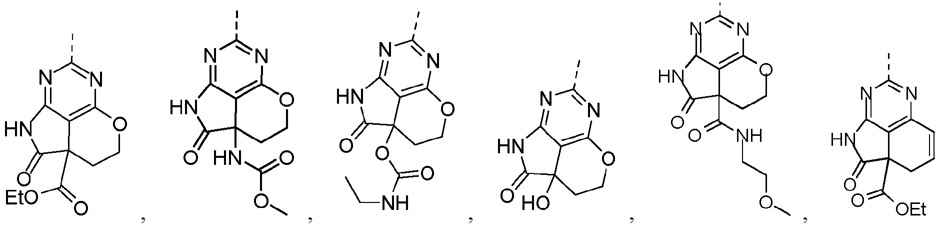 and

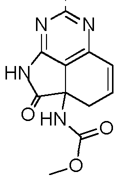

12. The compounds, the stereoisomers thereof or the pharmaceutically acceptable salts thereof according to claims 1 to 11, wherein the compounds are selected from the group consisting of 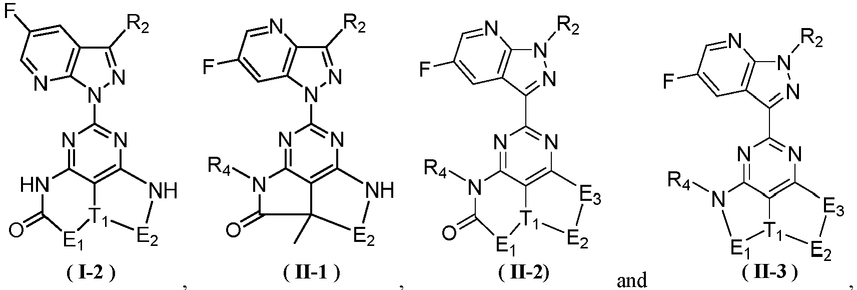 wherein R₂, R₄, T₁, E₁, E₂ and E₃ are as defined in any one of claims 1 to 11.

13. Compounds according to claim 1 of the following formulas, stereoisomers thereof or pharmaceutically acceptable salts thereof: 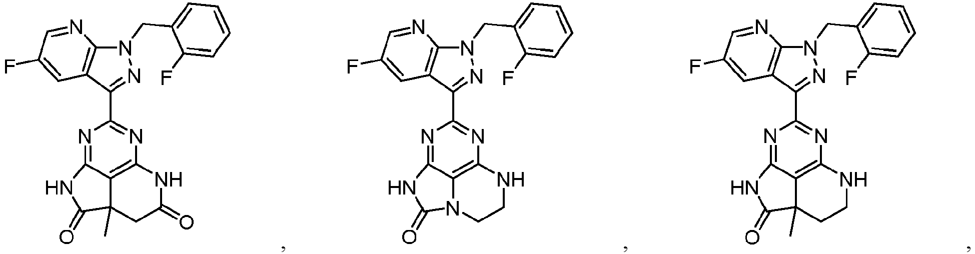 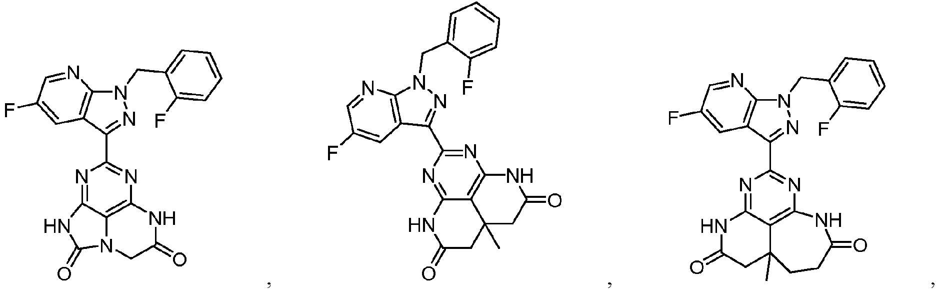 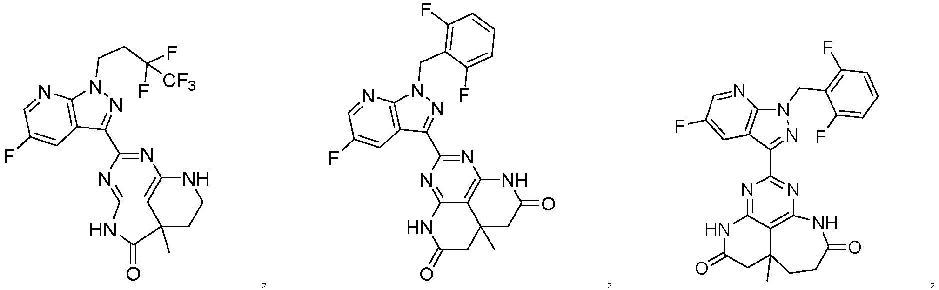 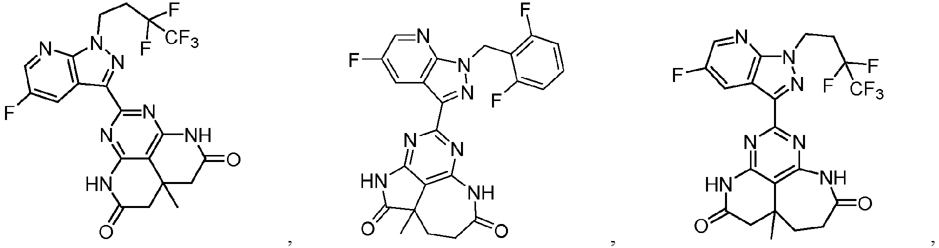 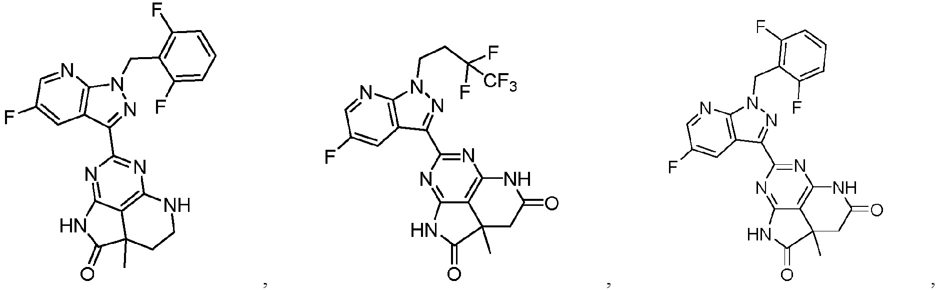 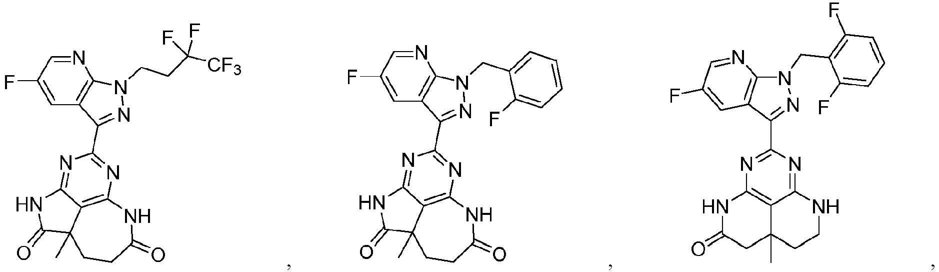 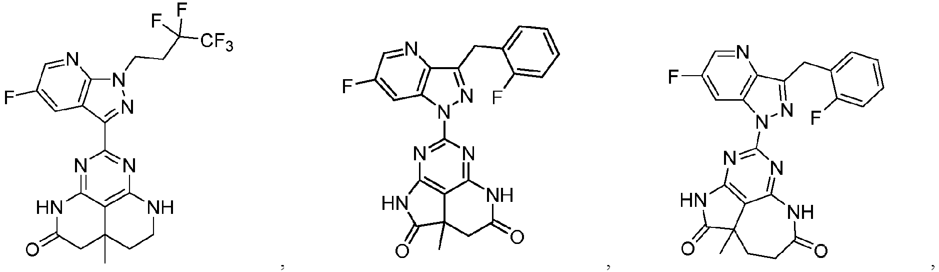 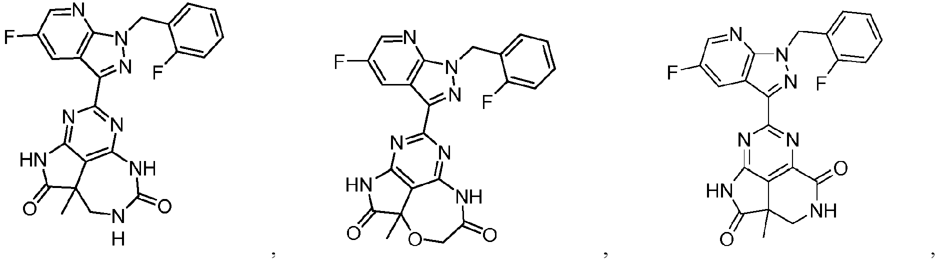 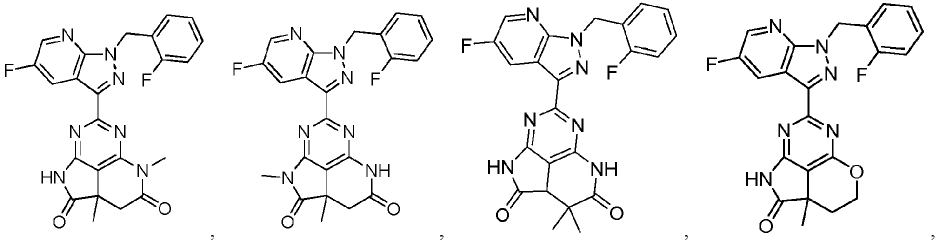 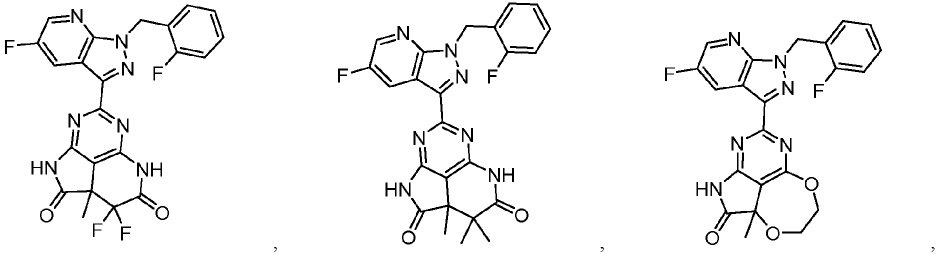 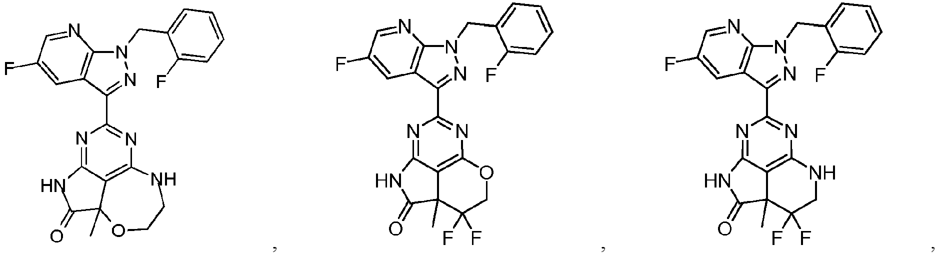 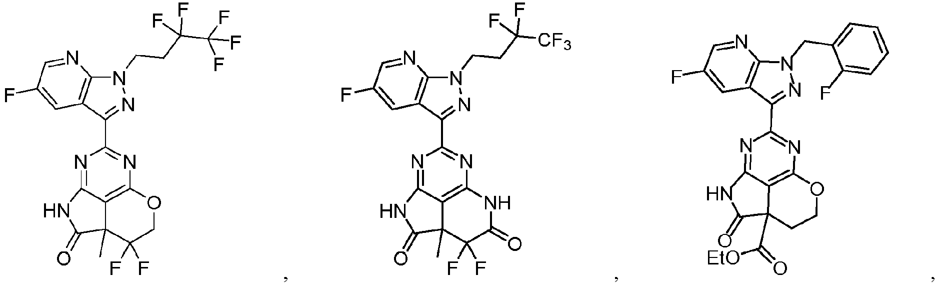 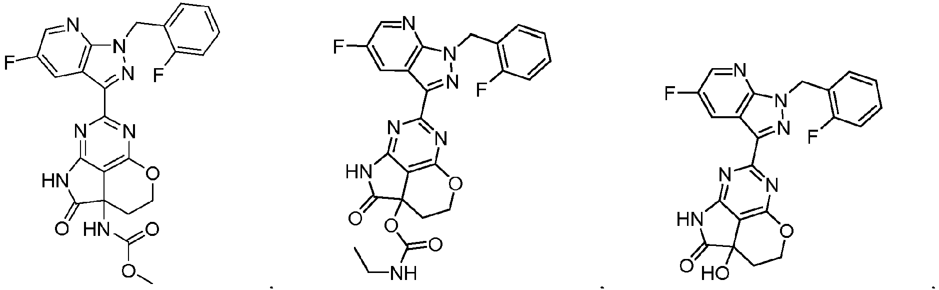 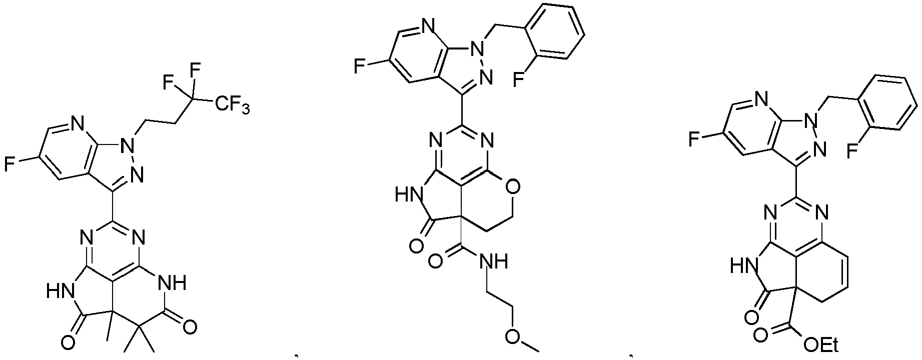 and

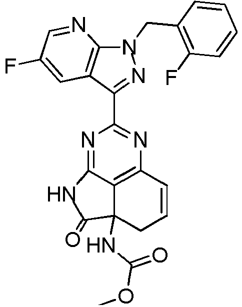

14. Compounds according to claim 1 of the following formulas or pharmaceutically acceptable salts or stereoisomers thereof

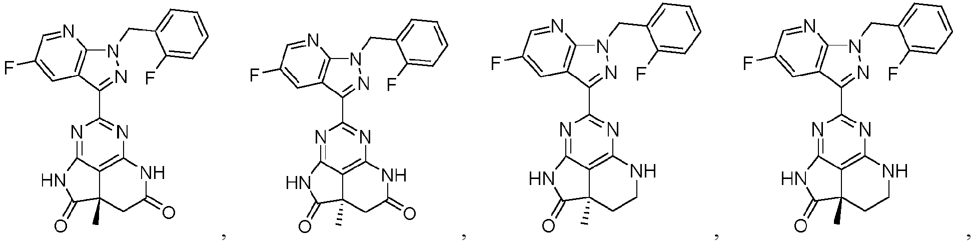

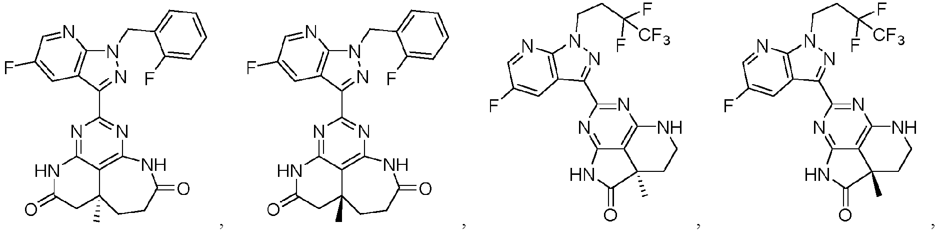

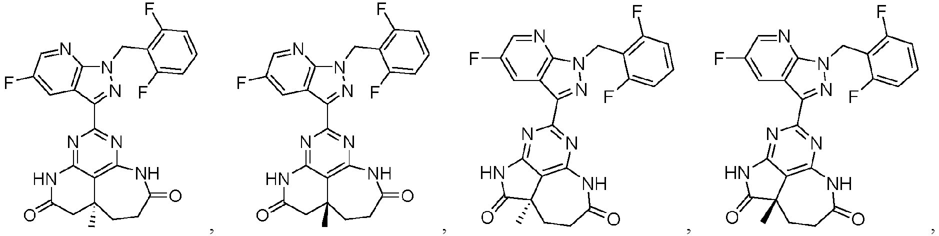

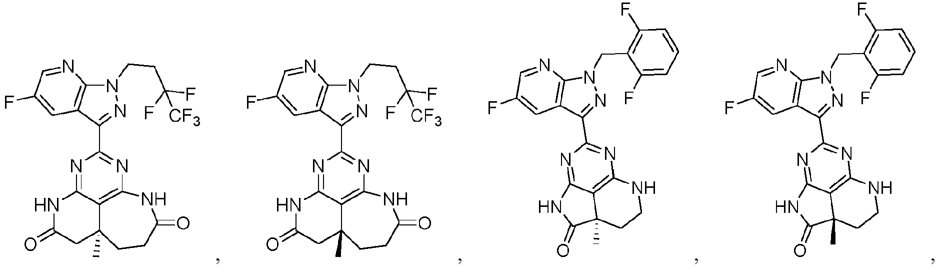

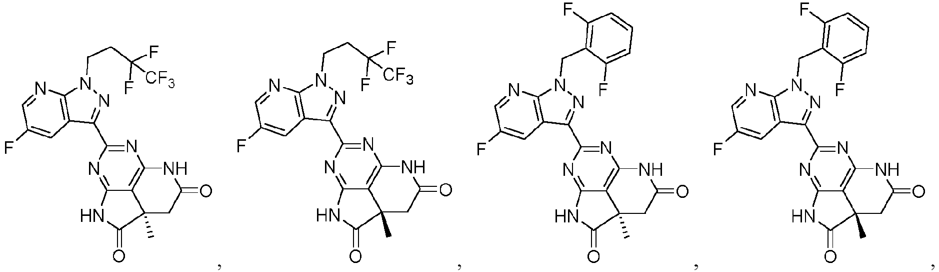

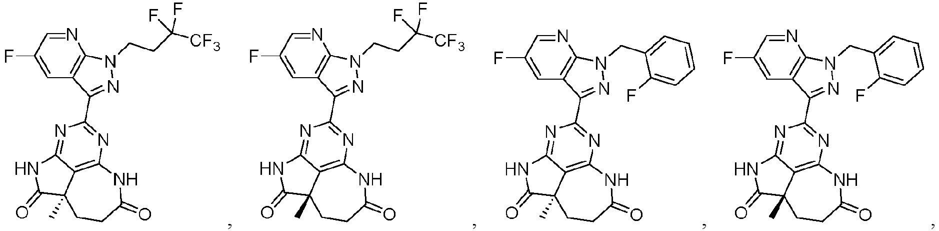

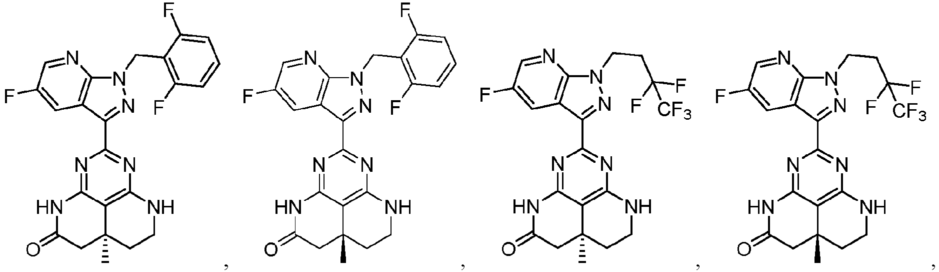

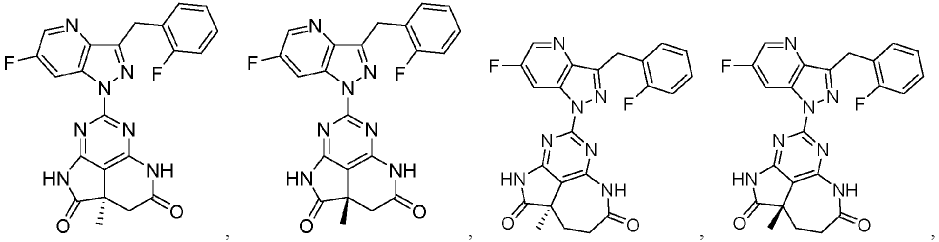

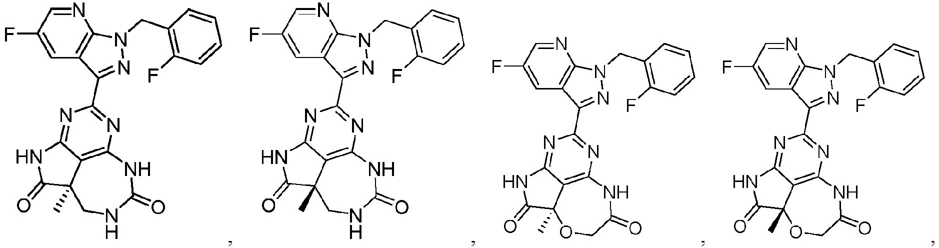

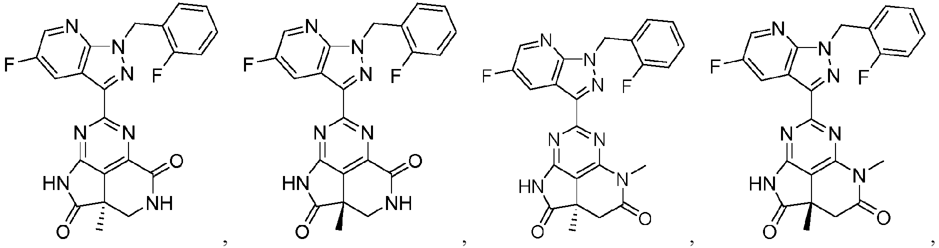

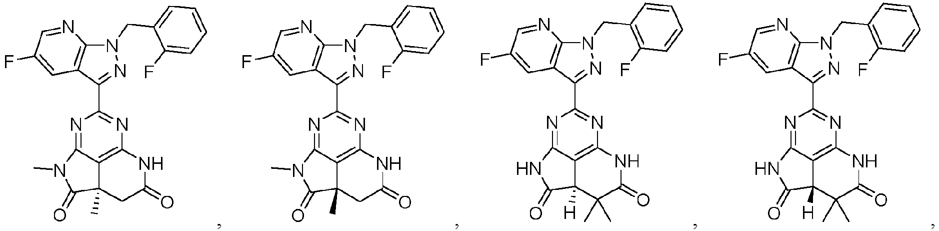

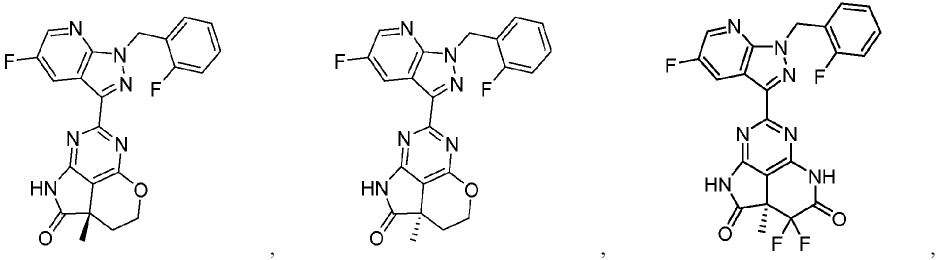

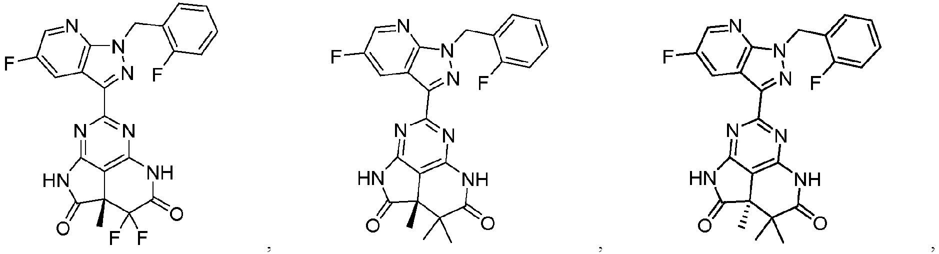

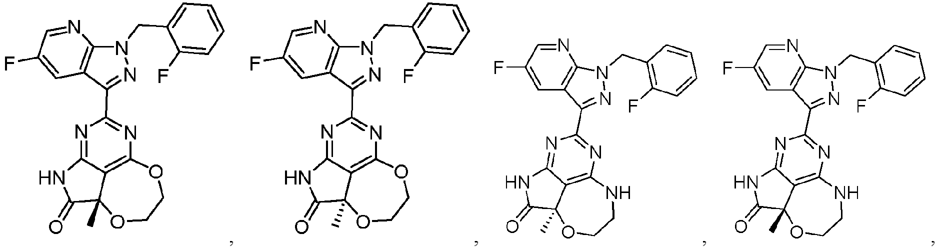

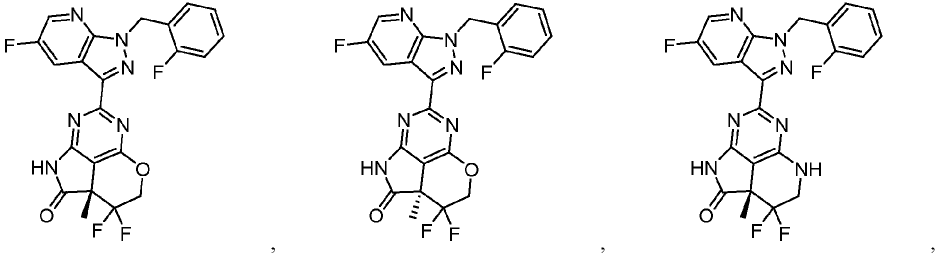

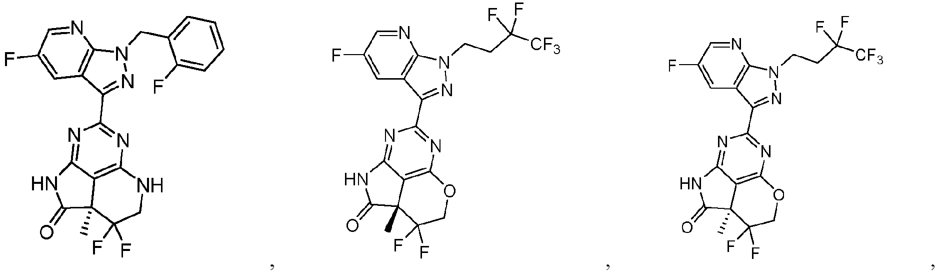

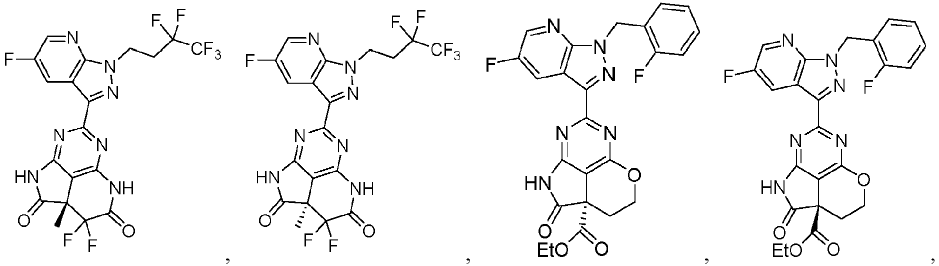

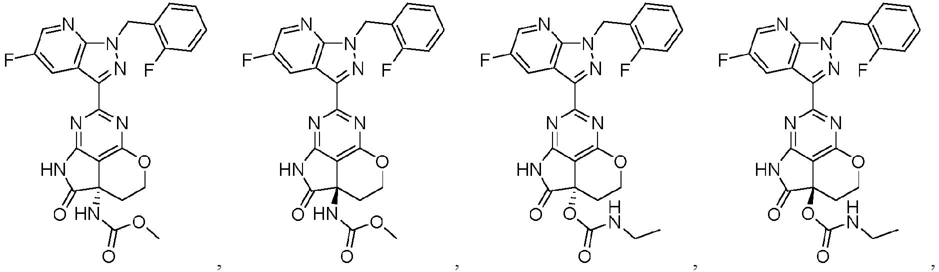

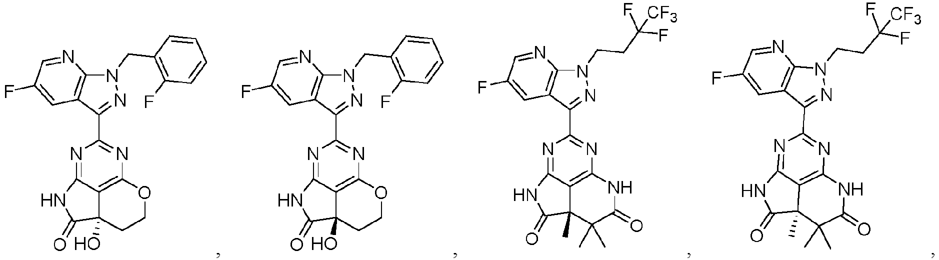

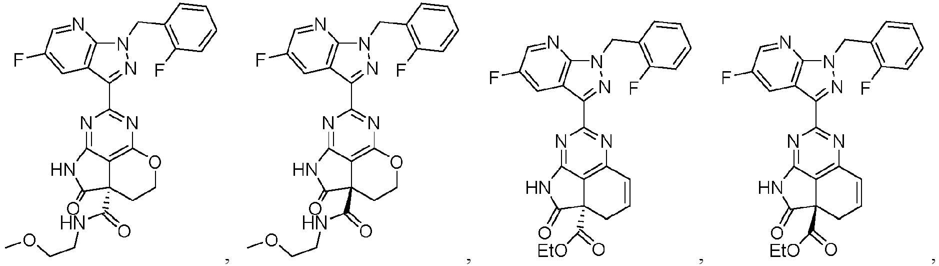

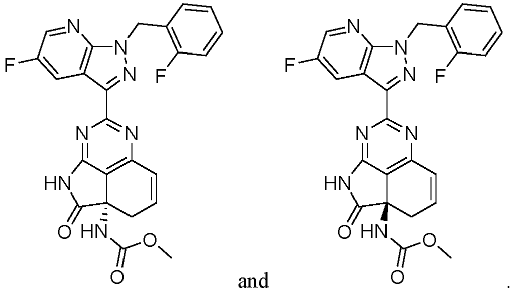

15. A pharmaceutical composition comprising the compound, the stereoisomer thereof or the pharmaceutically acceptable salt thereof according to any one of claims 1 to 14, and optionally further comprising a pharmaceutically acceptable excipient.

16. The compound, the stereoisomer thereof or the pharmaceutically acceptable salt thereof according to any one of claims 1 to 14 or the pharmaceutical composition according to claim 15 for use in treating an sGC agonist-associated disease; optionally, wherein the sGC agonist-associated disease is heart failure or hypertension.

## Patentansprüche

1. Verbindung der Formel (II), ein Stereoisomer davon oder ein pharmazeutisch akzeptables Salz davon, 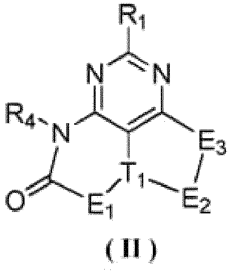 wobei
R₁ aus der Gruppe ausgewählt ist, bestehend aus 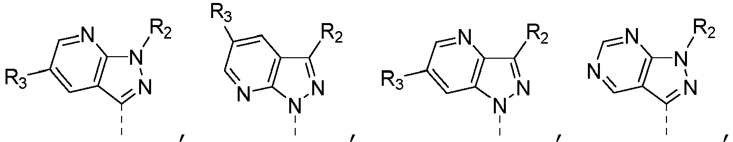 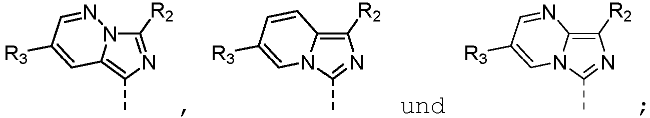
jeder R₂ unabhängig aus der Gruppe ausgewählt ist,
bestehend aus 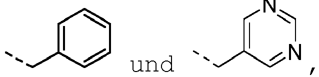 substituiert mit 1 oder 2 R_{d}, und C₁₋₃-Alkyl, substituiert mit 1, 2 oder 3 R_{d};
jeder R₃ unabhängig aus der Gruppe ausgewählt ist, bestehend aus H und Halogen;
R₄ aus der Gruppe ausgewählt ist, bestehend aus H und C₁₋₃-Alkyl;
E₁ ausgewählt ist aus -(CH₂)ₘ-;
m aus der Gruppe ausgewählt ist, bestehend aus 0, 1 und 2;
E₂ aus der Gruppe ausgewählt ist, bestehend aus -(CH₂)ₙ-, -(CH₂)ₚC(O)-, -O(CH₂)_{q}-, -O(CH₂)ᵣC(O)-, -CH₂CH=CH- und -(CH₂)ₛNHC(O)-, wobei jeder der CH₂ optional mit 1 oder 2 R_{b} substituiert ist;
E₃ aus der Gruppe ausgewählt ist, bestehend aus einer Einfachbindung, NR_{c} und O;
n aus der Gruppe ausgewählt ist, bestehend aus 1, 2 und 3;
p aus der Gruppe ausgewählt ist, bestehend aus 0, 1 und 2;
q aus der Gruppe ausgewählt ist, bestehend aus 1 und 2;
r aus der Gruppe ausgewählt ist, bestehend aus 1 und 2;
s aus der Gruppe ausgewählt ist, bestehend aus 1 und 2;
T₁ aus der Gruppe ausgewählt ist, bestehend aus N und CRₐ;
jeder Rₐ unabhängig aus der Gruppe ausgewählt ist, bestehend aus H, OH, -OC(=O)NHEt, -CO₂Et, -NHCO₂CH₃, -C(=O)NH(CH₂)₂OCH₃ und C₁₋₃-Alkyl;
jeder R_{b} unabhängig aus der Gruppe ausgewählt ist, bestehend aus F und CH₃;
jeder R_{c} unabhängig aus der Gruppe ausgewählt ist, bestehend aus H und CH₃;
jeder R_{d} unabhängig aus der Gruppe ausgewählt ist, bestehend aus Halogen und CF₃.

2. Verbindung, Stereoisomer davon oder pharmazeutisch akzeptables Salz davon gemäß Anspruch 1, wobei jeder Rₐ unabhängig aus der Gruppe ausgewählt ist, bestehend aus H, OH, -OC(=O)NHEt, -CO₂Et, -C(=O)NH(CH₂)₂OCH₃, -NHCO₂CH₃ und CH₃.

3. Verbindung, Stereoisomer davon oder pharmazeutisch akzeptables Salz davon gemäß Anspruch 1, wobei jeder R_{d} unabhängig aus der Gruppe ausgewählt ist, bestehend aus F und CF₃.

4. Verbindung, Stereoisomer davon oder pharmazeutisch akzeptables Salz davon gemäß Anspruch 1, wobei jeder R₂ unabhängig aus der Gruppe ausgewählt ist, bestehend aus 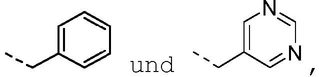 substituiert mit 1 oder 2 R_{d}, und C₁₋₃-Alkyl, substituiert mit 1, 2 or 3 R_{d}; optional R₂ unabhängig aus der Gruppe ausgewählt ist, bestehend aus 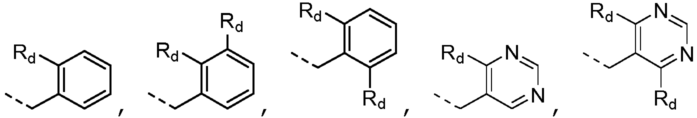 und 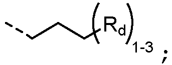 optional jeder R₂ unabhängig aus der Gruppe ausgewählt ist, bestehend aus

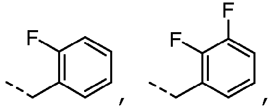

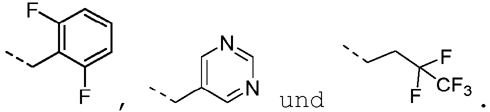

5. Verbindung, Stereoisomer davon oder pharmazeutisch akzeptables Salz davon gemäß Anspruch 1, wobei jeder R₃ unabhängig aus der Gruppe ausgewählt ist, bestehend aus H und F.

6. Verbindung, Stereoisomer davon oder pharmazeutisch akzeptables Salz davon gemäß Anspruch 1, wobei R₁ aus der Gruppe ausgewählt ist, bestehend aus 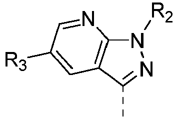 und 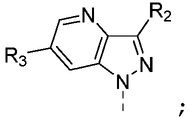 optional R₁ aus der Gruppe ausgewählt ist, bestehend aus 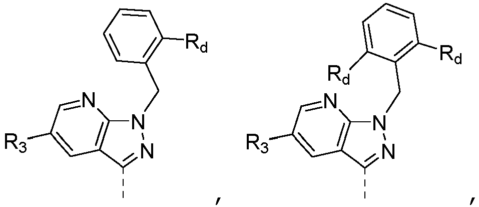 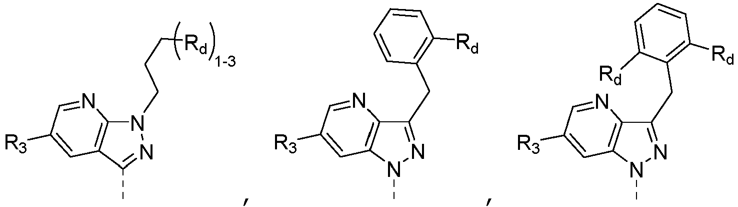 und 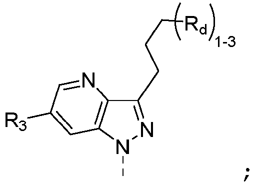 optional R₁ aus der Gruppe ausgewählt ist, bestehend aus

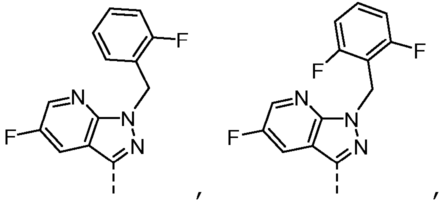

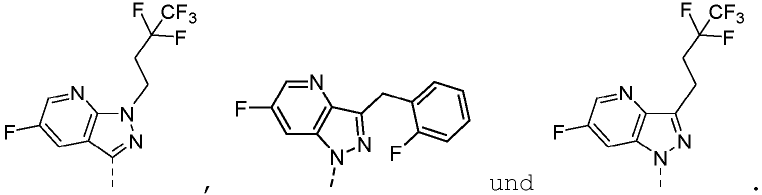

7. Verbindung, Stereoisomer davon oder pharmazeutisch akzeptables Salz davon gemäß Anspruch 1, wobei E₁ aus der Gruppe ausgewählt ist, bestehend aus einer Einfachbindung, -CH₂- und -(CH₂)₂-.

8. Verbindung, Stereoisomer davon oder pharmazeutisch akzeptables Salz davon gemäß Anspruch 1, wobei E₂ aus der Gruppe ausgewählt ist, bestehend aus -CH₂-, -(CH₂)₂-, -(CH₂)₃-, -CH₂CO-, -(CH₂)₂CO-, -O(CH₂)₂-, -OCH₂C(O)- und -CH₂NHC(O)-, wobei jeder der CH₂ optional mit 1 oder 2 R_{b} substituiert ist; optional E₂ aus der Gruppe ausgewählt ist, bestehend aus -CH₂-, -(CH₂)₂-, -CF₂CH₂-, -(CH₂)₃-, -CH₂CH=CH-, -CH₂CO-, -CO-, -C(CH₃)₂CO-, -CF₂CO-, -(CH₂)₂CO-, -O(CH₂)₂-, -OCH₂C(O)- und -CH₂NHC(O)-.

9. Verbindung, Stereoisomer davon oder pharmazeutisch akzeptables Salz davon gemäß Anspruch 1, wobei E₃ aus der Gruppe ausgewählt ist, bestehend aus einer Einfachbindung, NH, N(CH₃) und O.

10. Verbindung, Stereoisomer davon oder pharmazeutisch akzeptables Salz davon gemäß Anspruch 1, wobei T₁ aus der Gruppe ausgewählt ist, bestehend aus N, CH, C(OH), C(OC(=O)NHEt), C(CO₂Et), C(NHCO₂CH₃), C[C(=O)NH(CH₂)₂OCH₃] und C(CH₃).

11. Verbindung, Stereoisomer davon oder pharmazeutisch akzeptables Salz davon gemäß Anspruch 1, wobei die Struktureinheit 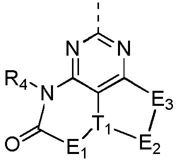 aus der Gruppe ausgewählt ist, bestehend aus 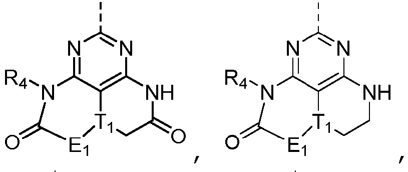 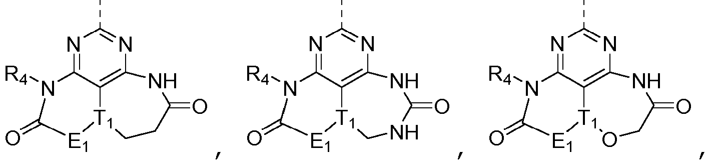 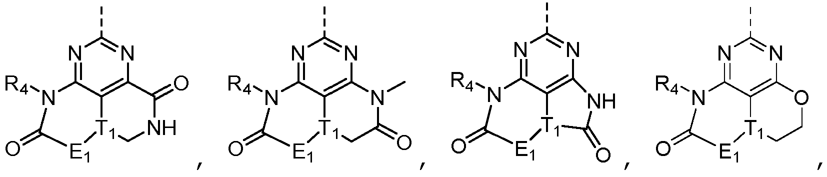 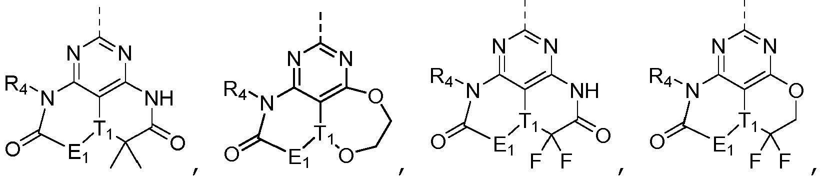 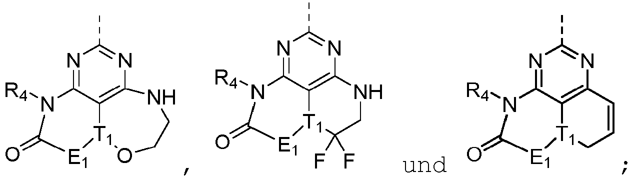 optional die Struktureinheit 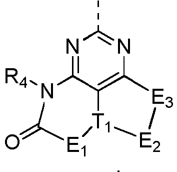 aus der Gruppe ausgewählt ist, bestehend aus

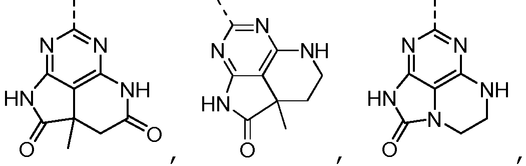

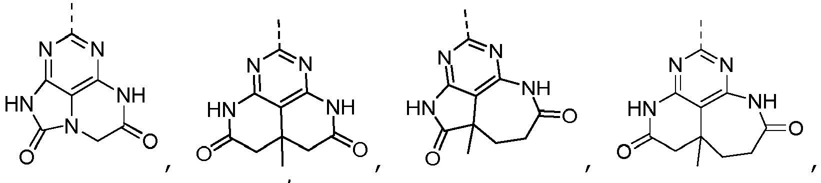

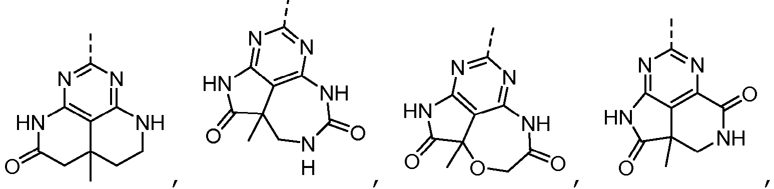

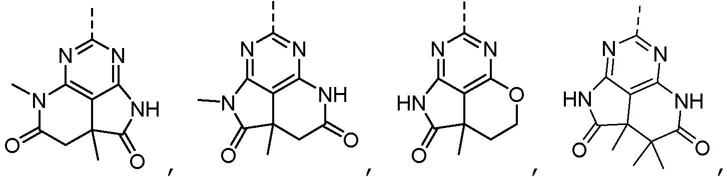

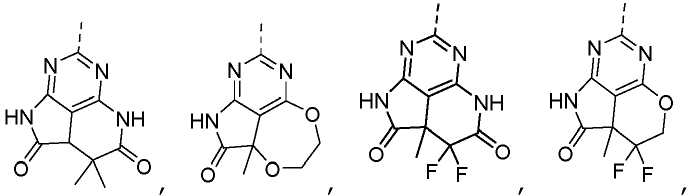

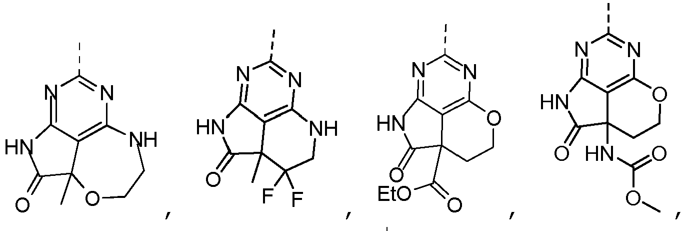

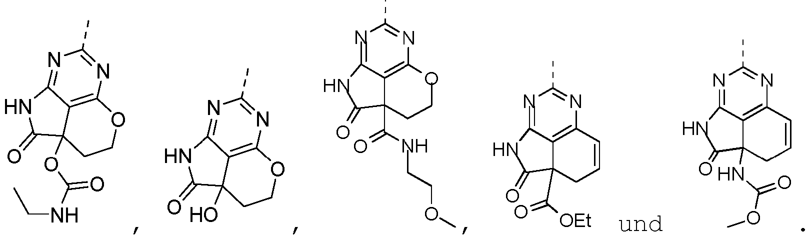

12. Verbindungen, Stereoisomere davon oder pharmazeutisch akzeptable Salze davon gemäß den Ansprüchen 1 bis 11, wobei die Verbindungen aus der Gruppe ausgewählt sind, bestehend aus 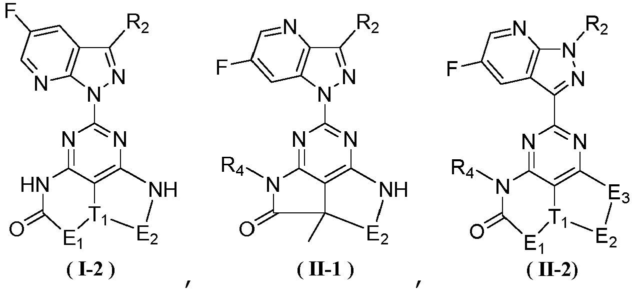 und 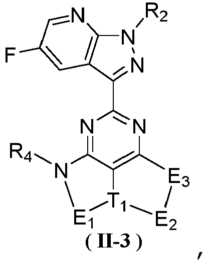 wobei R₂, R₄, T₁, E₁, E₂ und E₃ wie in irgendeinem der Ansprüche 1 bis 11 definiert sind.

13. Verbindungen gemäß Anspruch 1 der folgenden Formeln, Stereoisomere davon oder pharmazeutisch akzeptable Salze davon: 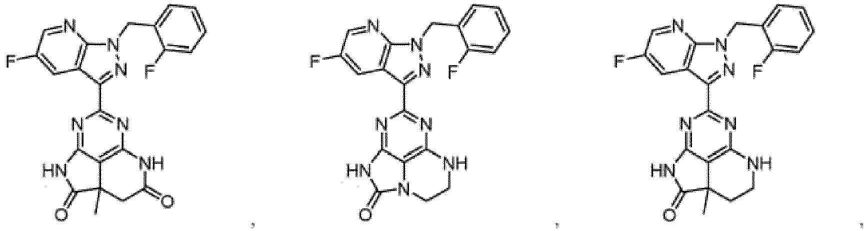 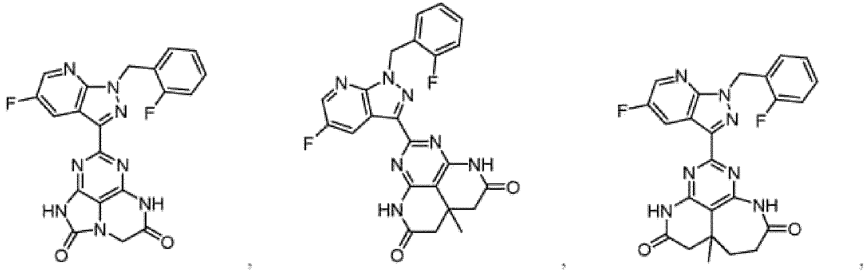 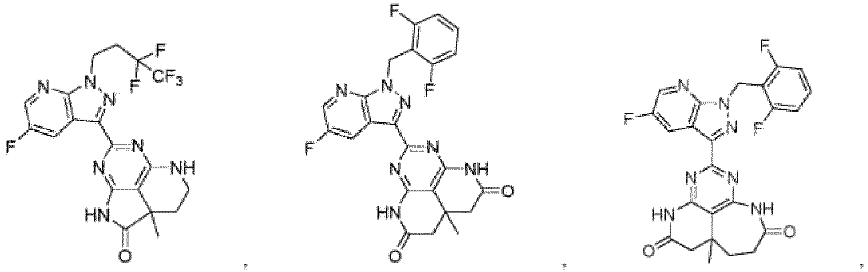 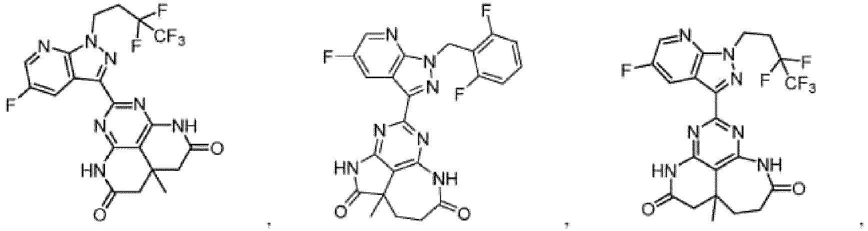 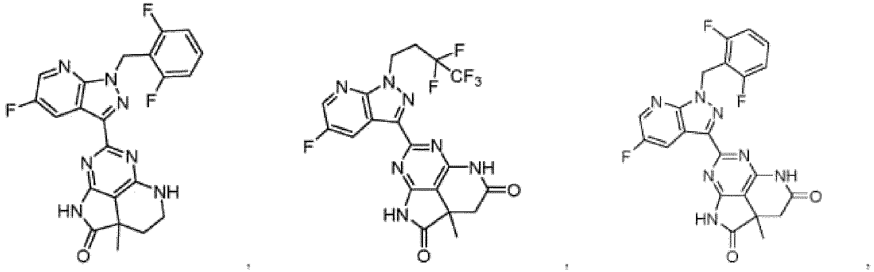 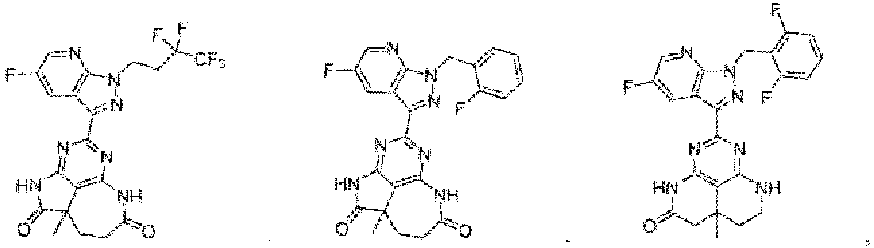 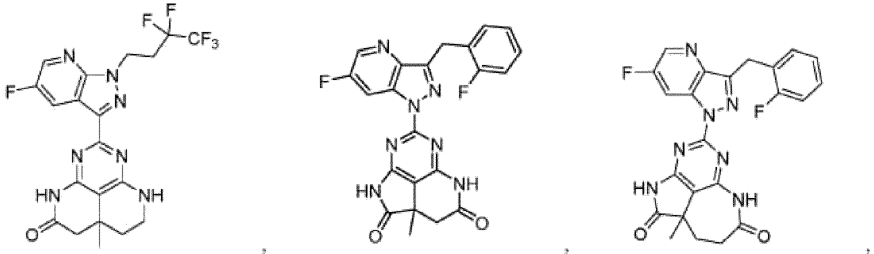 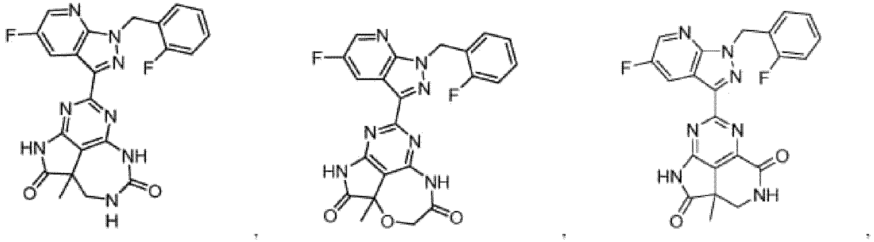 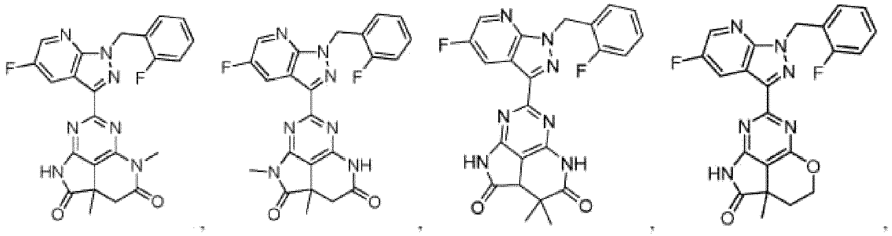 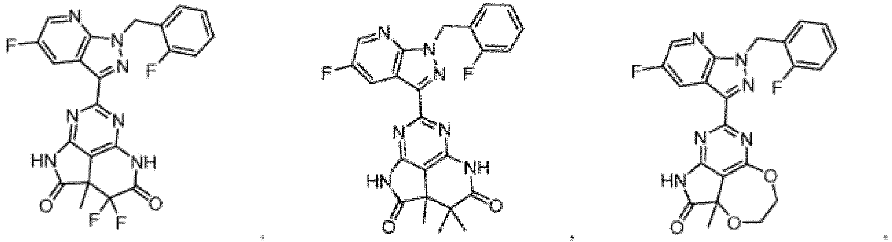 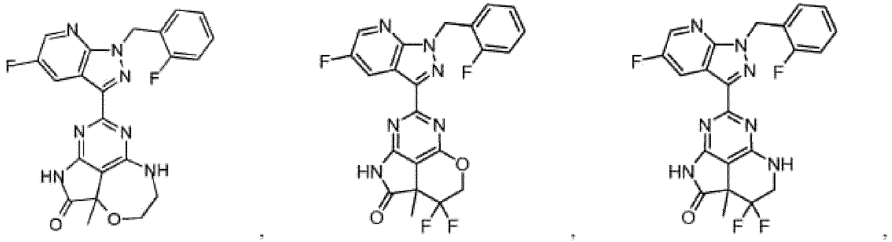 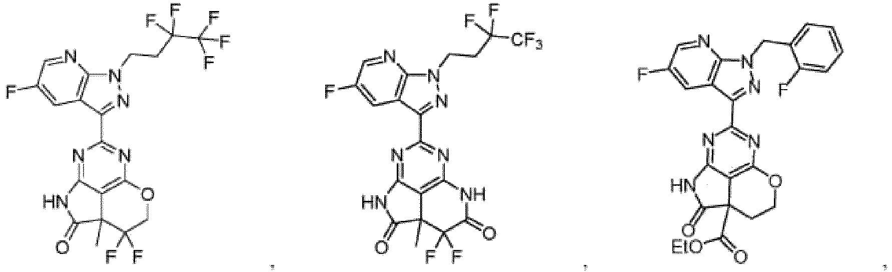 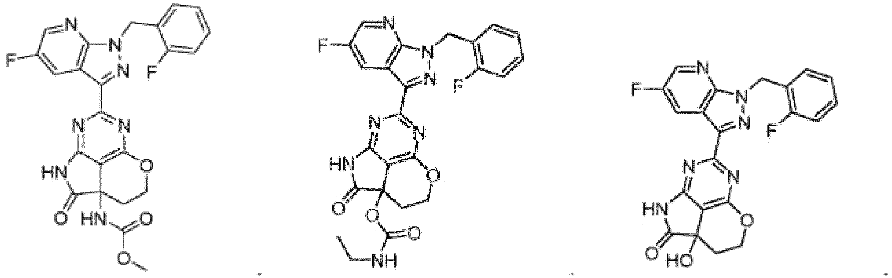 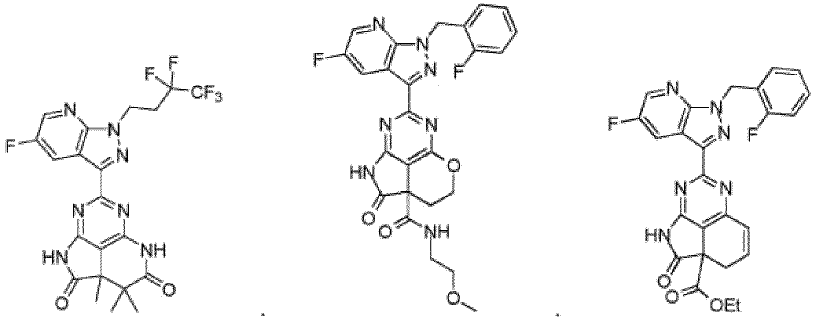 und

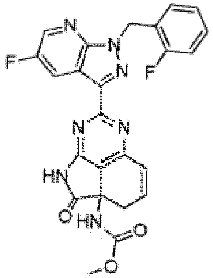

14. Verbindungen gemäß Anspruch 1 der folgenden Formeln oder pharmazeutisch akzeptable Salze oder Stereoisomere davon:

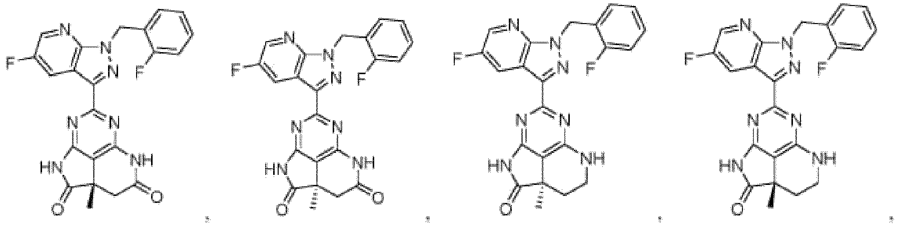

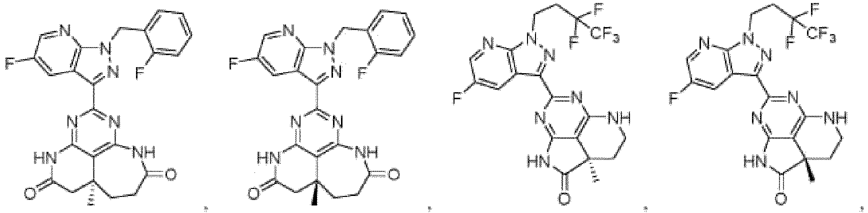

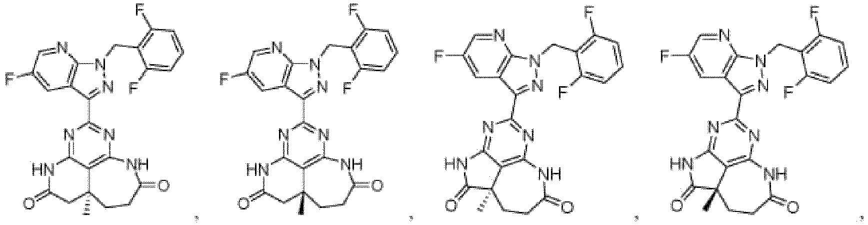

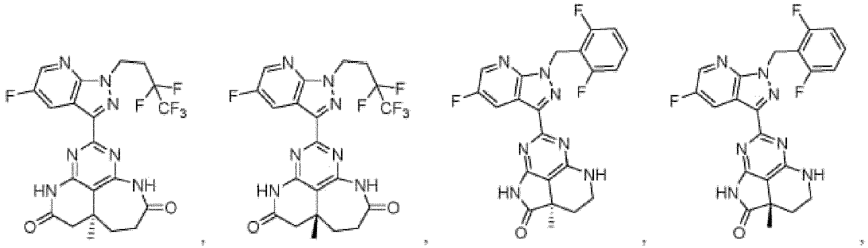

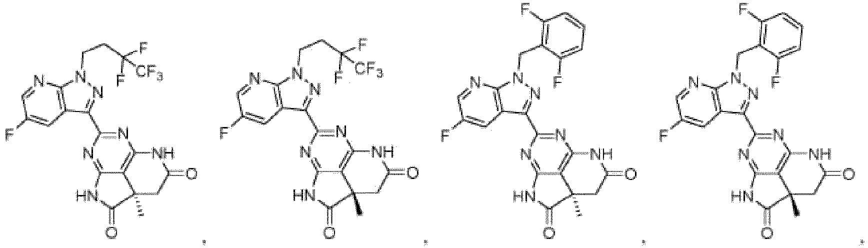

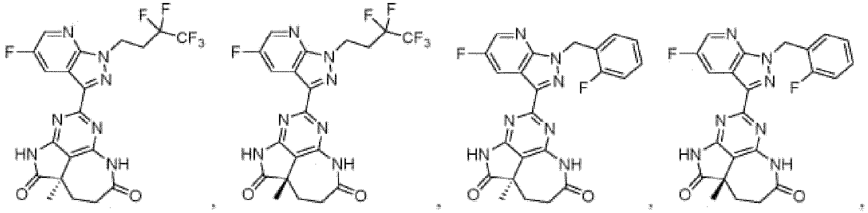

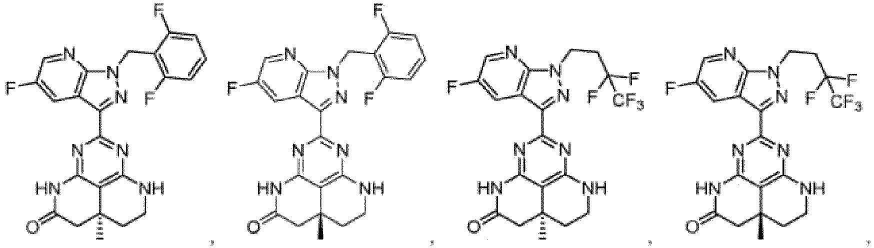

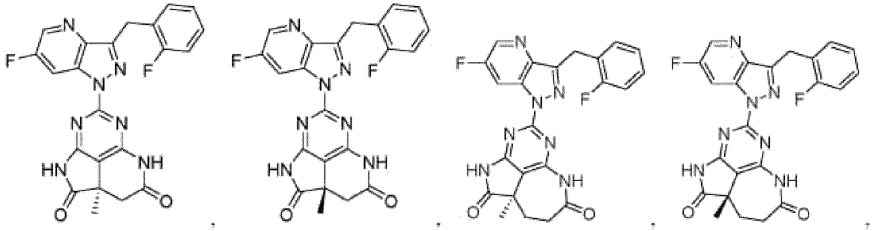

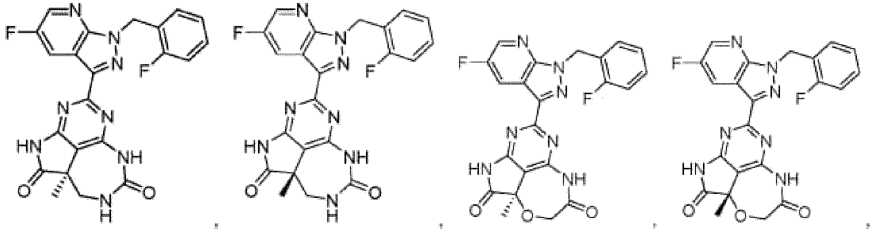

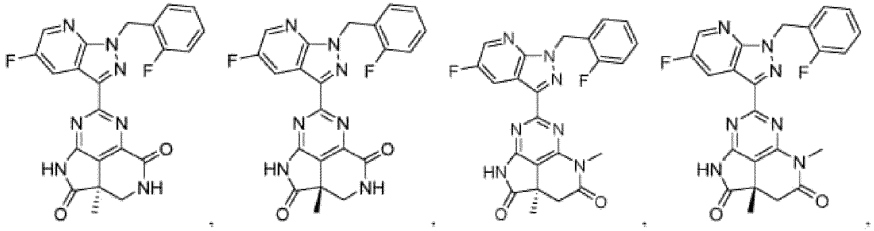

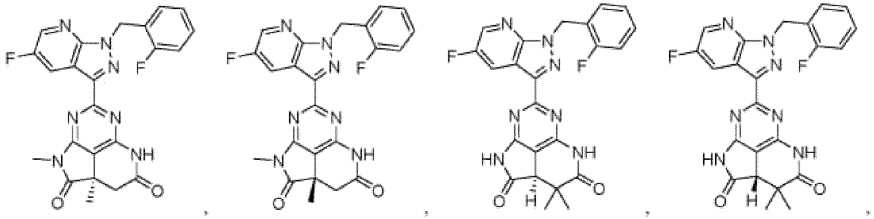

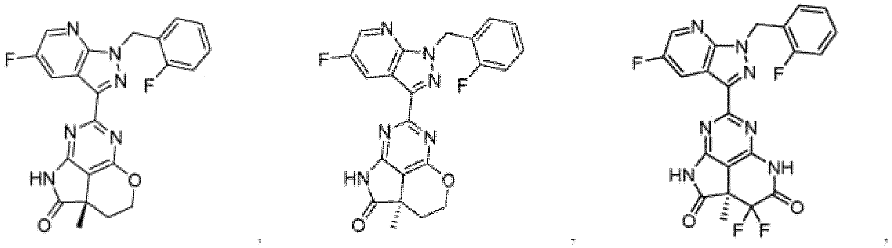

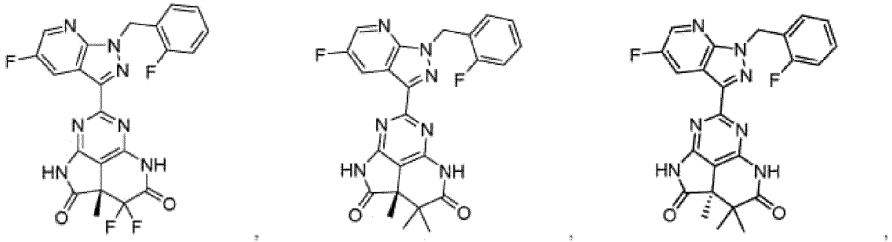

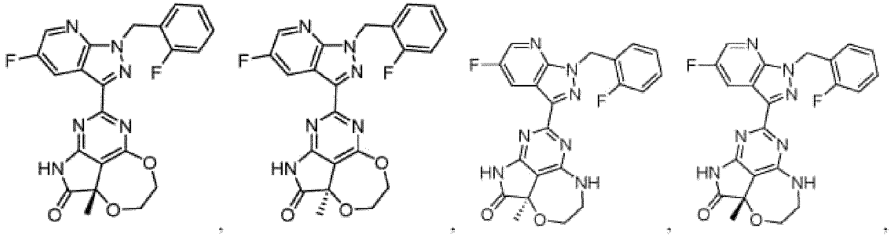

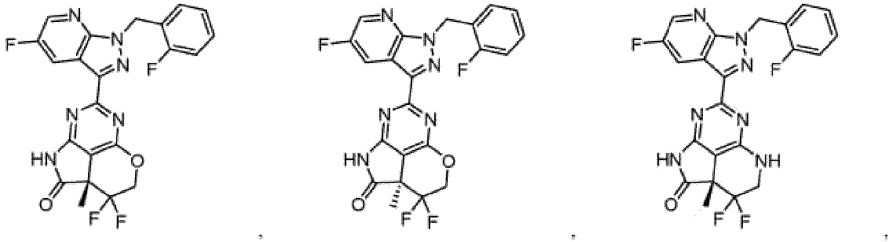

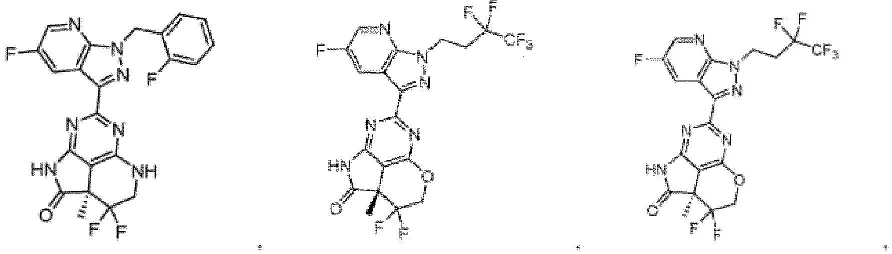

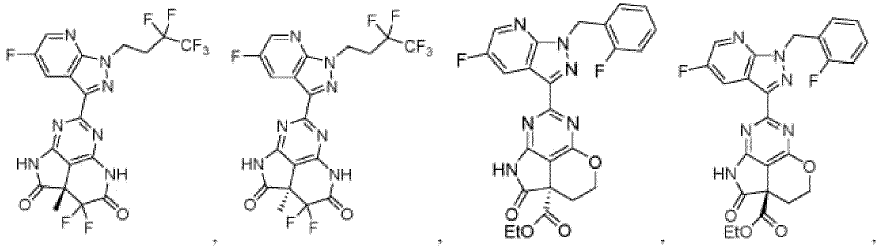

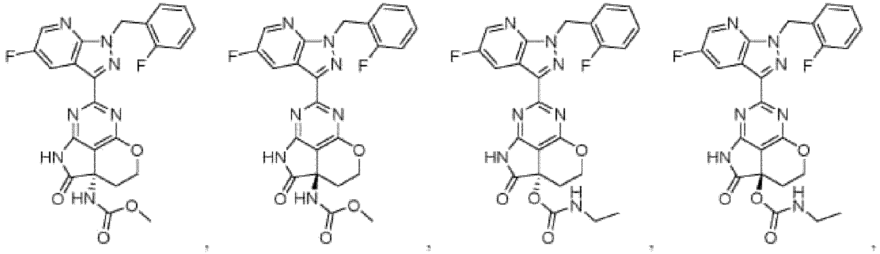

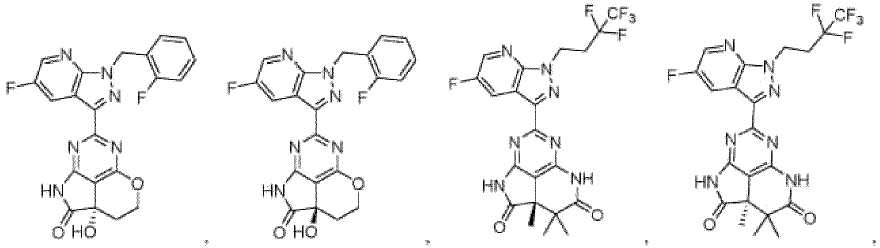

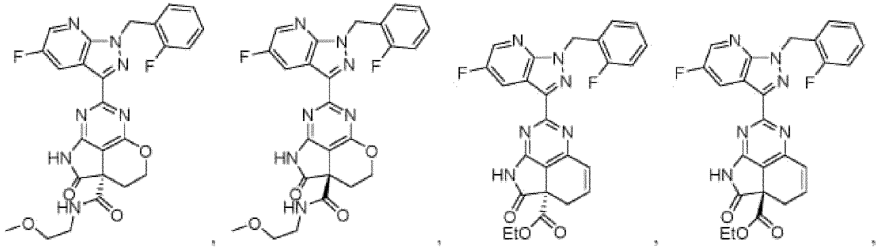

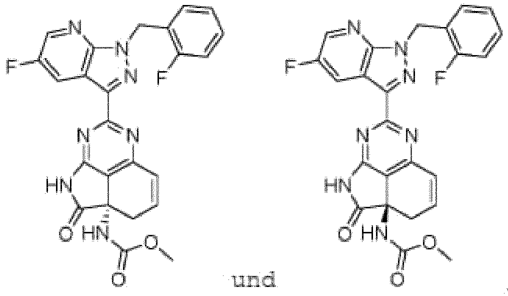

15. Pharmazeutische Zusammensetzung, welche die Verbindung, das Stereoisomer davon oder das pharmazeutisch akzeptable Salz davon gemäß irgendeinem der Ansprüche 1 bis 14 umfasst und optional ferner einen pharmazeutisch akzeptablen Hilfsstoff umfasst.

16. Verbindung, Stereoisomer davon oder pharmazeutisch akzeptables Salz davon gemäß irgendeinem der Ansprüche 1 bis 14 oder pharmazeutische Zusammensetzung gemäß Anspruch 15 zur Verwendung bei der Behandlung einer sGC-Agonisten-assoziierten Erkrankung; wobei die sGC-Agonisten-assoziierte Erkrankung optional Herzversagen oder Bluthochdruck ist.

## Revendications

1. Composé de formule (II), stéréoisomère de celui-ci ou sel pharmaceutiquement acceptable de celui-ci, 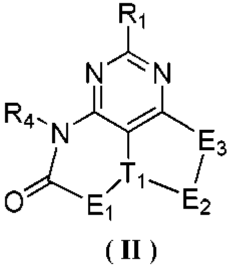 dans lequel,
R₁ est sélectionné dans le groupe consistant en 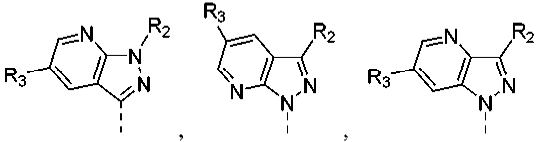 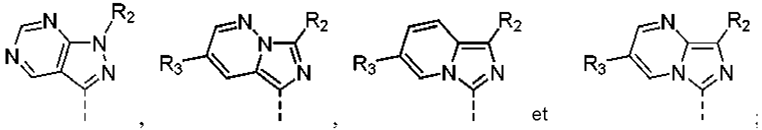
chaque R₂ est sélectionné indépendamment dans le groupe consistant en 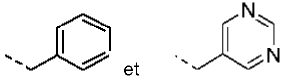 substitués avec 1 ou 2 R_{d} et un alkyle en C₁₋₃ substitué avec 1, 2 ou 3 R_{d} ;
chaque R₃ est sélectionné indépendamment dans le groupe consistant en H et un halogène ;
R₄ est sélectionné dans le groupe consistant en H et un alkyle en C₁₋₃ ;
E₁ est sélectionné parmi -(CH₂)ₘ- ;
m est sélectionné dans le groupe consistant en 0, 1 et 2 ;
E₂ est sélectionné dans le groupe consistant en -(CH₂)ₙ-, -(CH₂)ₚC(O)-, -O(CH₂)_{q}-, -O(CH2)ᵣC(O)-, -CH₂CH=CH- et -(CH₂)ₛNHC(O)-, dans lequel chacun des CH₂ est facultativement substitué avec 1 ou 2 R_{b} ;
E₃ est sélectionné dans le groupe consistant en une liaison simple, NR_{c} et 0 ;
n est sélectionné dans le groupe consistant en 1, 2 et 3 ;
p est sélectionné dans le groupe consistant en 0, 1 et 2 ;
q est sélectionné dans le groupe consistant en 1 et 2 ;
r est sélectionné dans le groupe consistant en 1 et 2 ;
s est sélectionné dans le groupe consistant en 1 et 2 ;
T₁ est sélectionné dans le groupe consistant en N et CRₐ ;
chaque Rₐ est sélectionné indépendamment dans le groupe consistant en H, OH, -OC(=O)NHEt, -CO₂Et, -NHCO₂CH₃, -C(=O)NH(CH₂)₂OCH₃ et un alkyle en C₁₋₃ ;
chaque Rb est sélectionné indépendamment dans le groupe consistant en F et CH₃ ;
chaque Rc est sélectionné indépendamment dans le groupe consistant en H et CH₃ ;
chaque Rd est sélectionné indépendamment dans le groupe consistant en un halogène et CF₃.

2. Composé, stéréoisomère de celui-ci ou sel pharmaceutiquement acceptable de celui-ci selon la revendication 1, dans lequel chaque Rₐ est sélectionné indépendamment dans le groupe consistant en H, OH, -OC(=O)NHEt, -CO₂Et, -C(=O)NH(CH₂)₂OCH₃, -NHCO₂CH₃ et CH₃.

3. Composé, stéréoisomère de celui-ci ou sel pharmaceutiquement acceptable de celui-ci selon la revendication 1, dans lequel chaque R_{d} est sélectionné indépendamment dans le groupe consistant en F et CF₃.

4. Composé, stéréoisomère de celui-ci ou sel pharmaceutiquement acceptable de celui-ci selon la revendication 1, dans lequel chaque R₂ est sélectionné indépendamment dans le groupe consistant en : 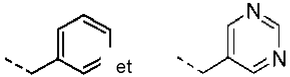 substitués avec 1 ou 2 R_{d} et un alkyle en C₁₋₃ substitué avec 1, 2 ou 3 R_{d} ; facultativement, R₂ est sélectionné indépendamment dans le groupe consistant en 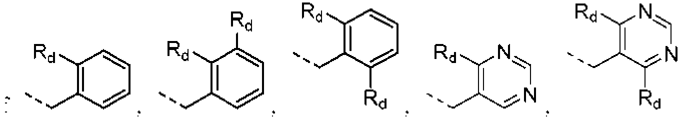 et 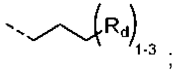 facultativement, chaque R₂ est sélectionné indépendamment dans le groupe consistant en

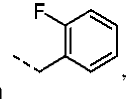

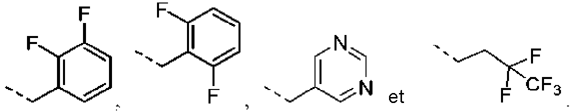

5. Composé, stéréoisomère de celui-ci ou sel pharmaceutiquement acceptable de celui-ci selon la revendication 1, dans lequel chaque R₃ est sélectionné indépendamment dans le groupe consistant en H et F.

6. Composé, stéréoisomère de celui-ci ou sel pharmaceutiquement acceptable de celui-ci selon la revendication 1,
dans lequel R₁ est sélectionné dans le groupe consistant en, 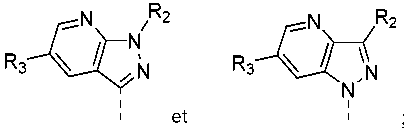
facultativement, R₁ est sélectionné dans le groupe consistant en 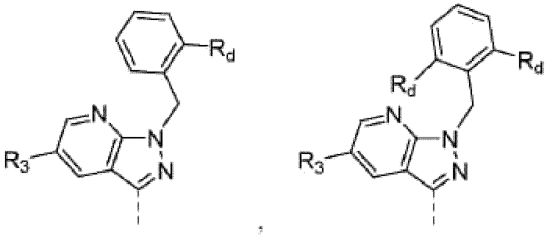 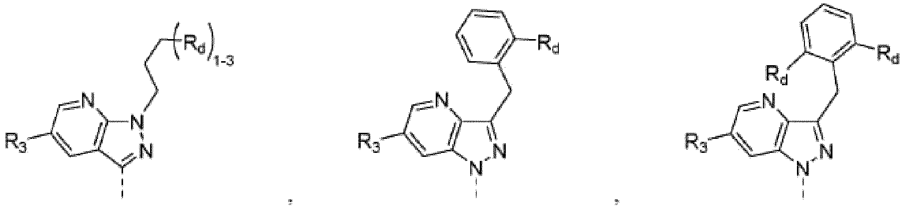 et 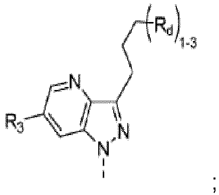 facultativement, R₁ est sélectionné dans le groupe consistant en

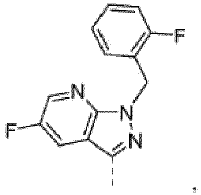

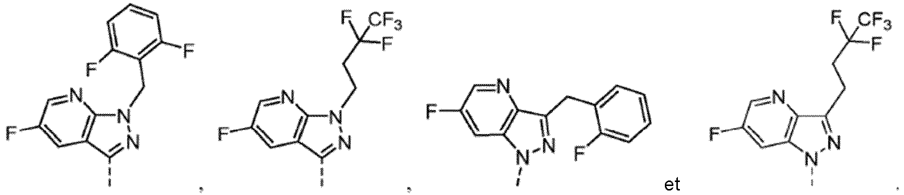

7. Composé, stéréoisomère de celui-ci ou sel pharmaceutiquement acceptable de celui-ci selon la revendication 1, dans lequel E₁ est sélectionné dans le groupe consistant en une liaison simple, -CH₂- et -(CH₂)₂-.

8. Composé, stéréoisomère de celui-ci ou sel pharmaceutiquement acceptable de celui-ci selon la revendication 1, dans lequel E₂ est sélectionné dans le groupe consistant en -CH₂-, -(CH₂)₂-, -(CH₂)₃-, -CH₂CO-, -(CH₂)₂CO-, -O(CH₂)₂-, -OCH₂(CO)- et -CH₂NHC(O)-, dans lequel chacun des CH₂ est facultativement substitué avec 1 ou 2 R_{b} ; facultativement, E₂ est sélectionné dans le groupe consistant en -CH₂-, -(CH₂)₂-, -CF₂CH₂-, -(CH₂)₃-, -CH₂CH=CH-, CH₂CO-, -CO-, -C(CH₃)₂CO-, -CF₂CO-, -(CH₂)₂CO-, -O(CH₂)₂-, -OCH₂C(O)- et -CH₂NHC(O)-.

9. Composé, stéréoisomère de celui-ci ou sel pharmaceutiquement acceptable de celui-ci selon la revendication 1, dans lequel E₃ est sélectionné dans le groupe consistant en une liaison simple, NH, N(CH₃) et O.

10. Composé, stéréoisomère de celui-ci ou sel pharmaceutiquement acceptable de celui-ci selon la revendication 1, dans lequel Ti est sélectionné dans le groupe consistant en N, CH, C(OH), C(OC(=O)NHEt), C(CO₂Et), C(NHCO₂CH₃ ), C[C(=O)NH(CH₂)₂ OCH₃] et C(CH₃).

11. Composé, stéréoisomère de celui-ci ou sel pharmaceutiquement acceptable de celui-ci selon la revendication 1, dans lequel le motif de structure 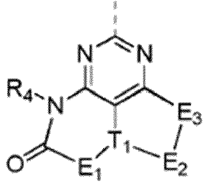 est sélectionné dans le groupe consistant en 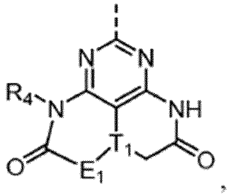 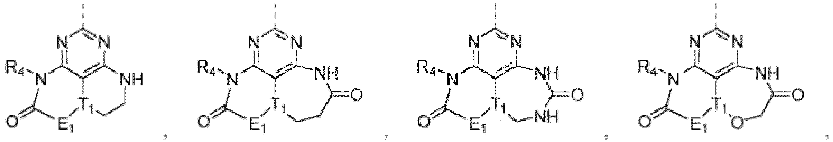 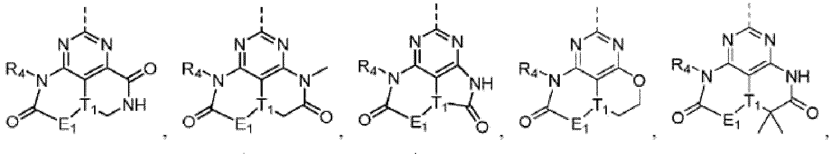 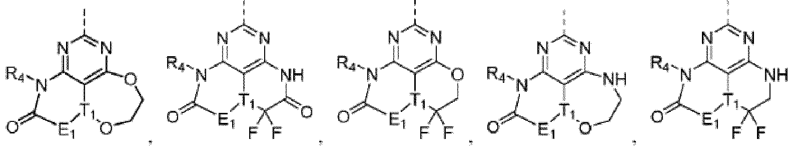 et 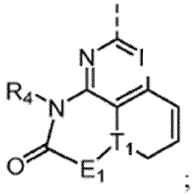 facultativement, le motif de structure 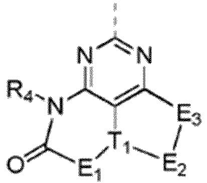 est sélectionné dans le groupe consistant en 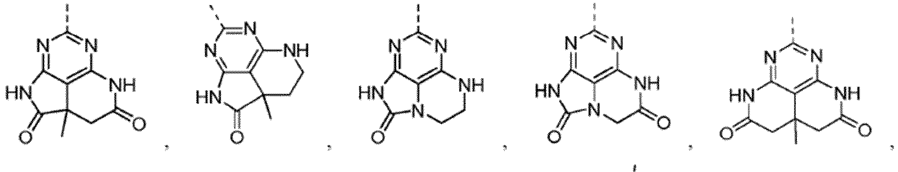 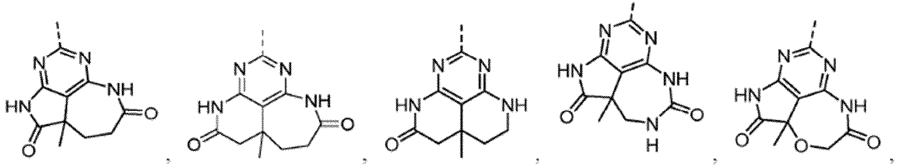 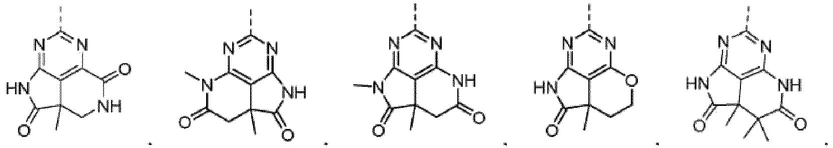 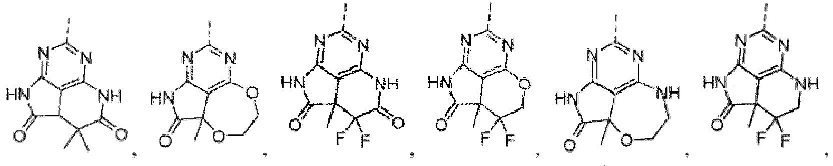 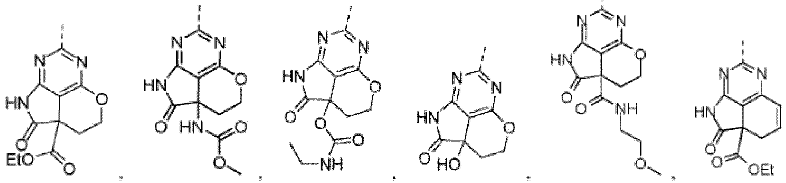 et

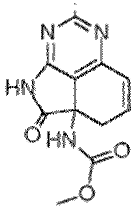

12. Composés, stéréoisomères de ceux-ci ou sels pharmaceutiquement acceptable de ceux-ci selon les revendications 1 à 11, dans lesquels les composés sont sélectionnés dans le groupe consistant en 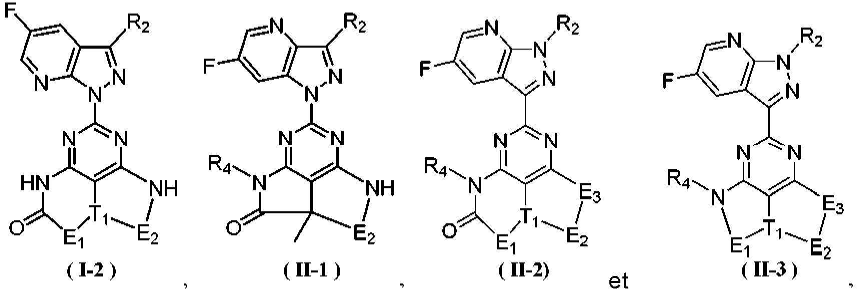 dans lesquels R₂, R₄, T₁, E₁, E₂ et E₃ sont tels que définis dans l'une quelconque des revendications 1 à 11.

13. Composés selon la revendication 1 de formules suivantes, stéréoisomères de ceux-ci ou sels pharmaceutiquement acceptables de ceux-ci : 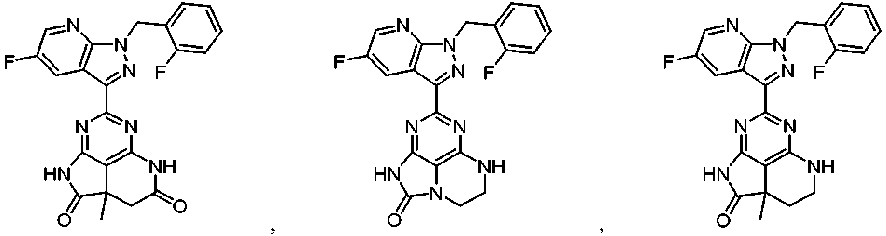 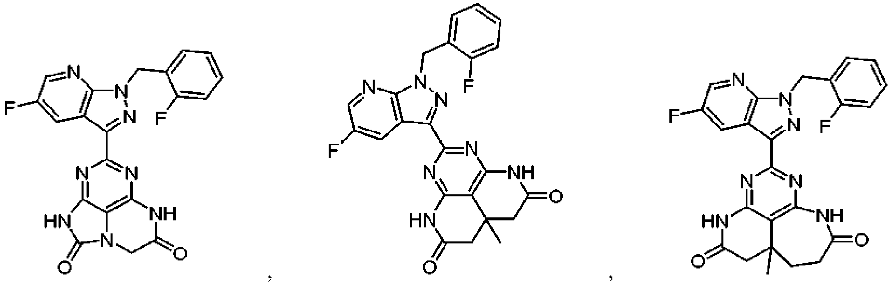 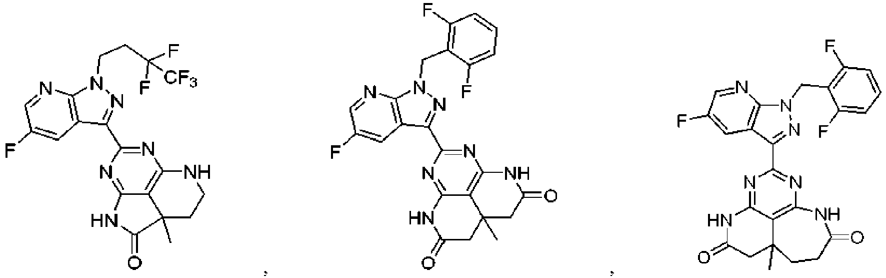 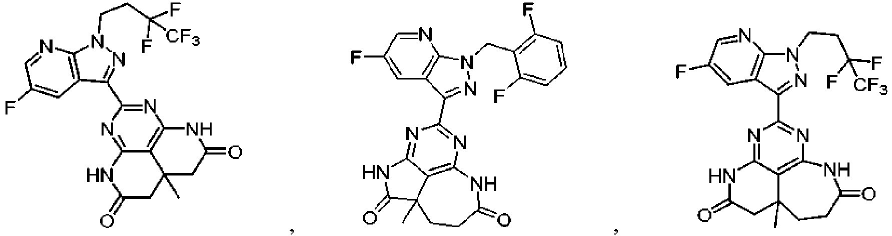 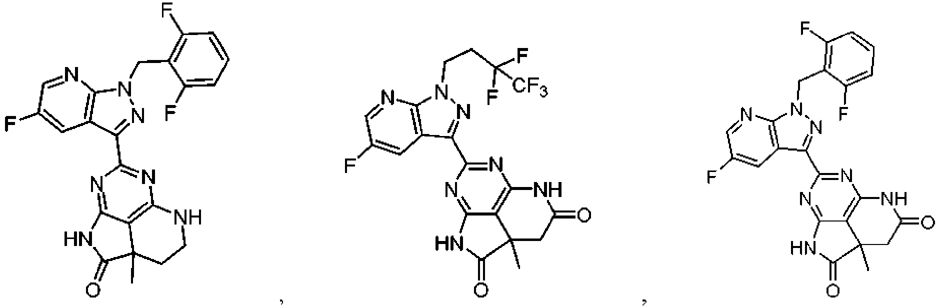 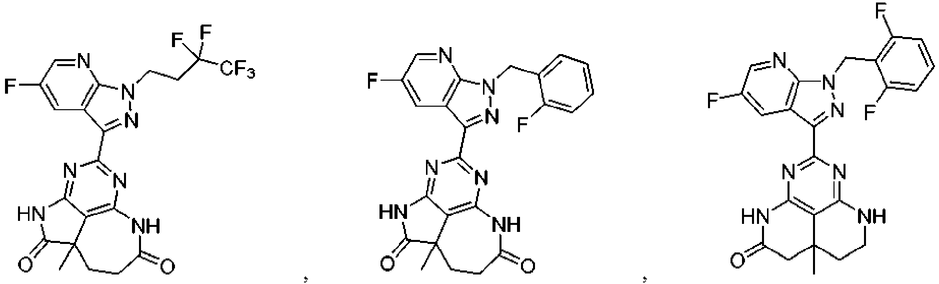 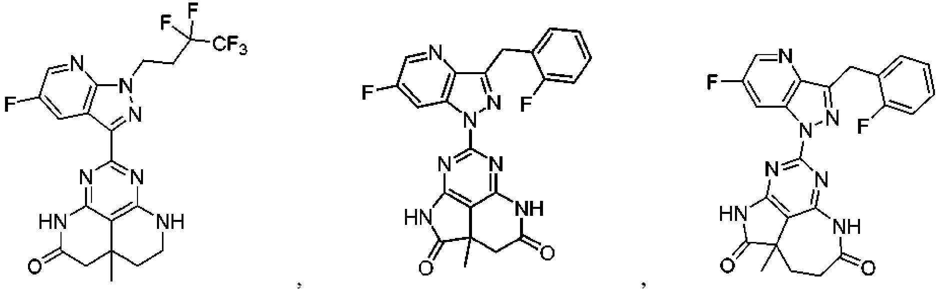 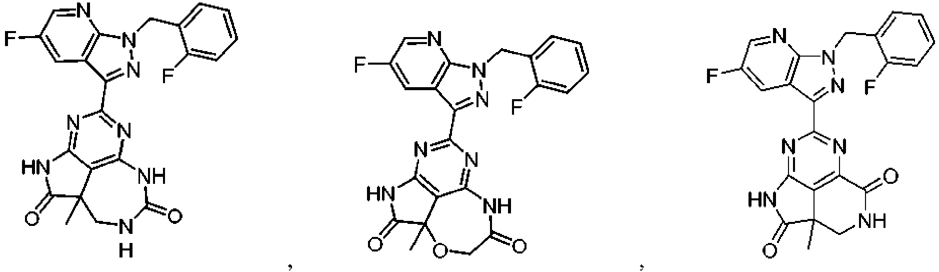 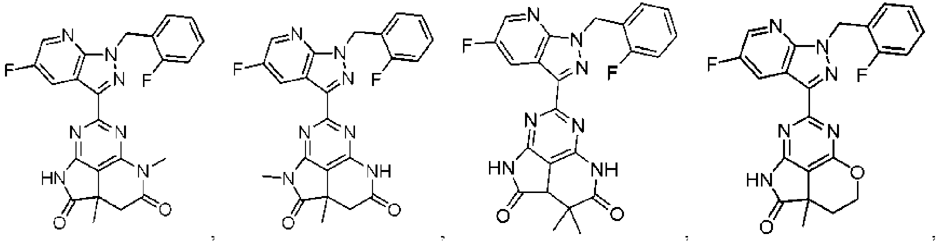 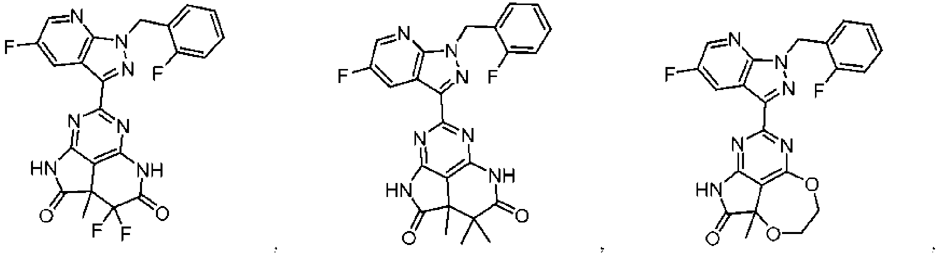 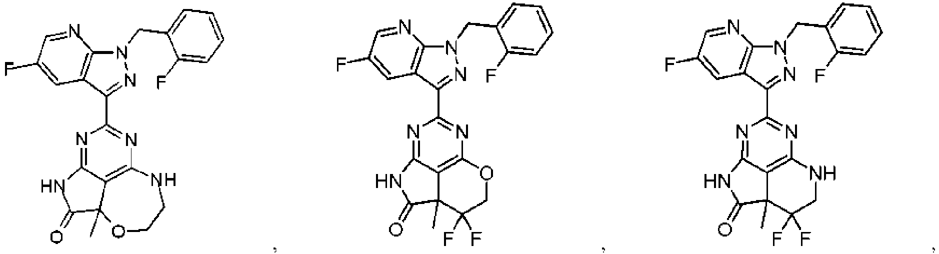 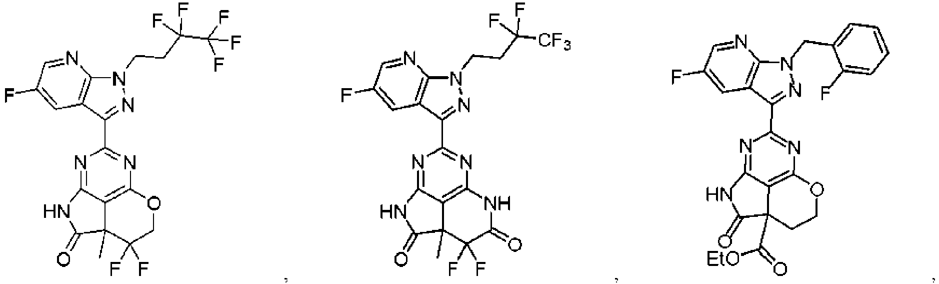 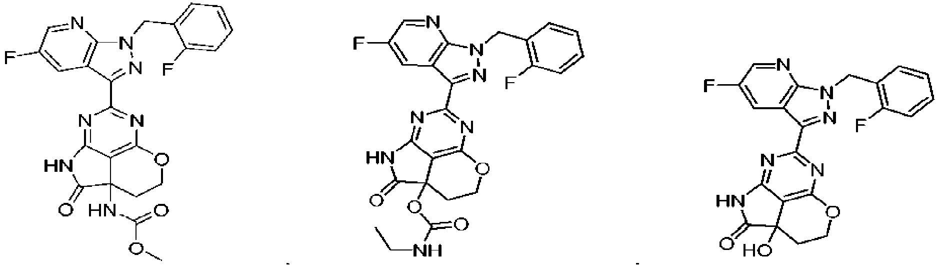 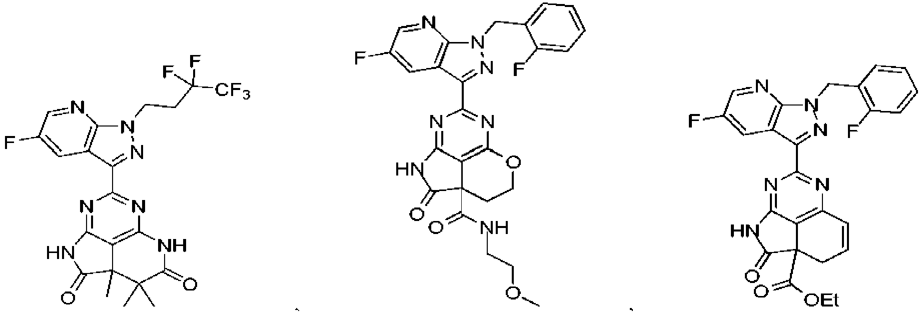 et

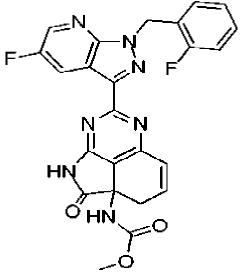

14. Composés selon la revendication 1 de formules suivantes ou sels ou stéréoisomères pharmaceutiquement acceptables de ceux-ci

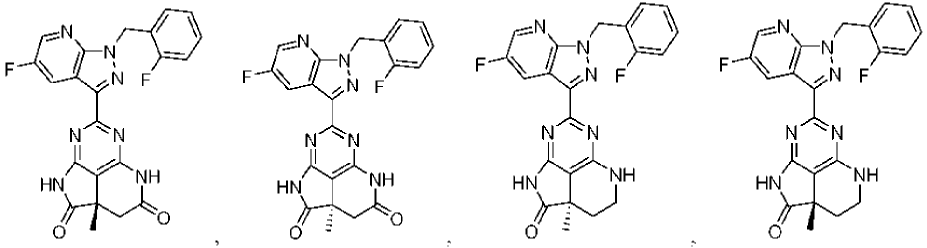

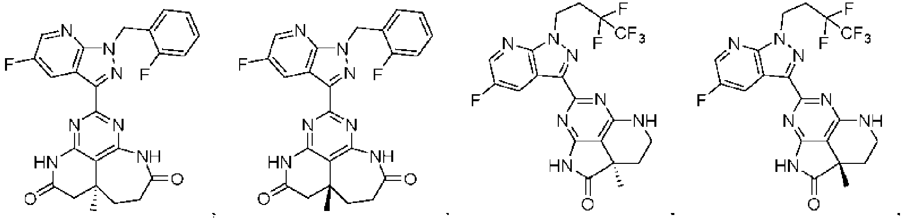

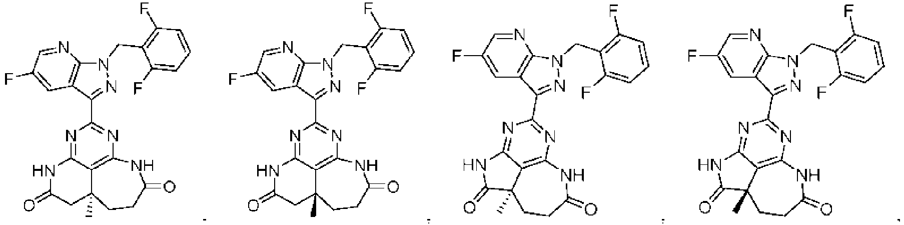

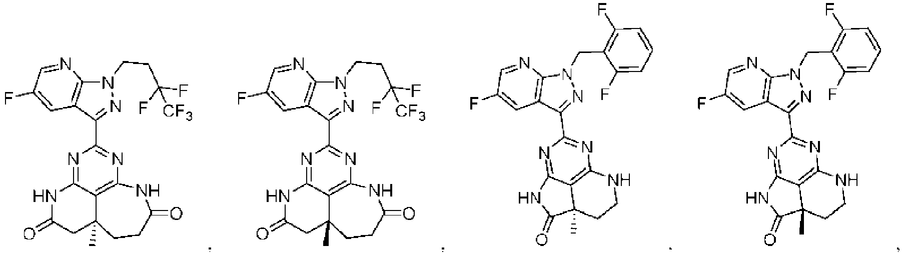

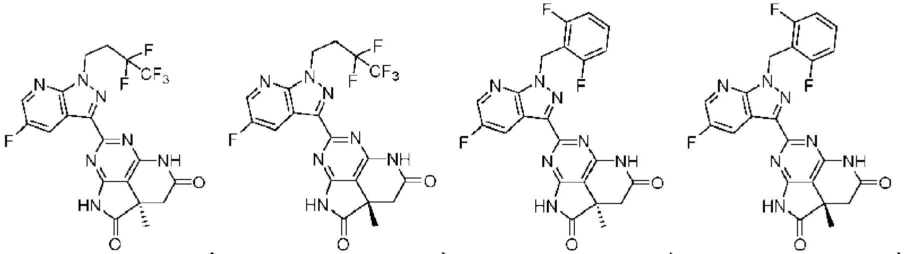

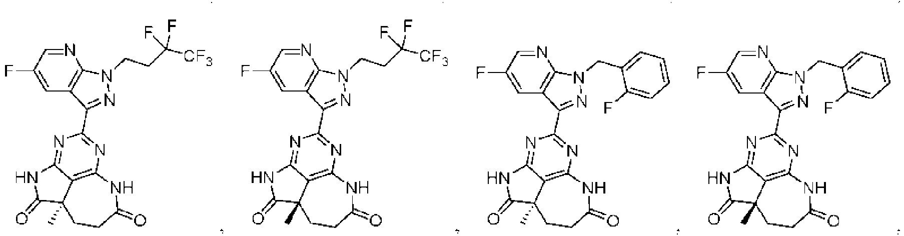

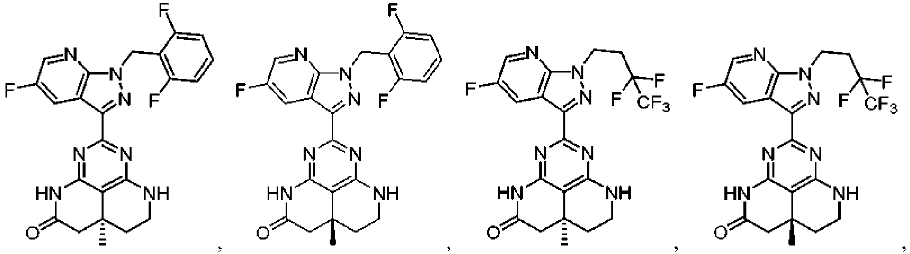

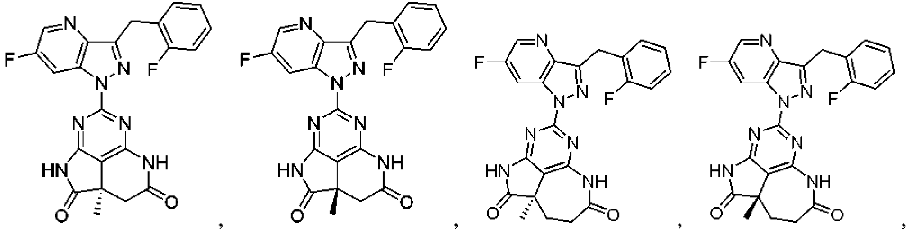

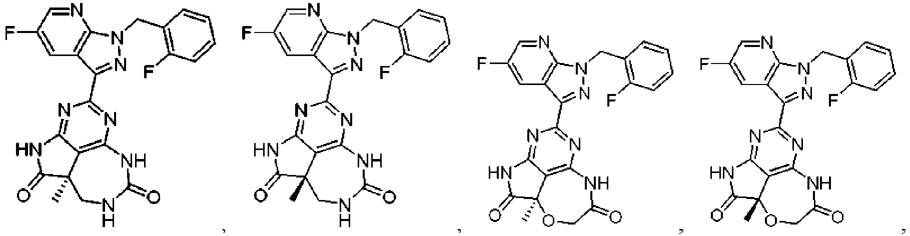

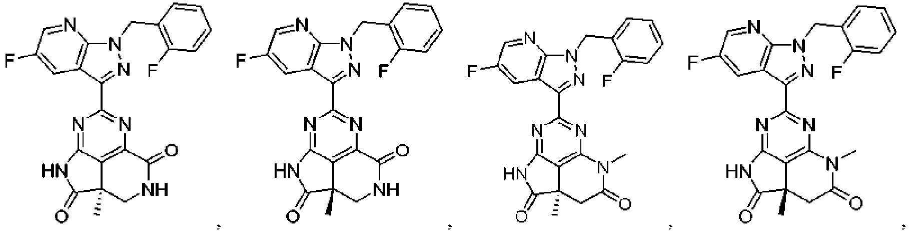

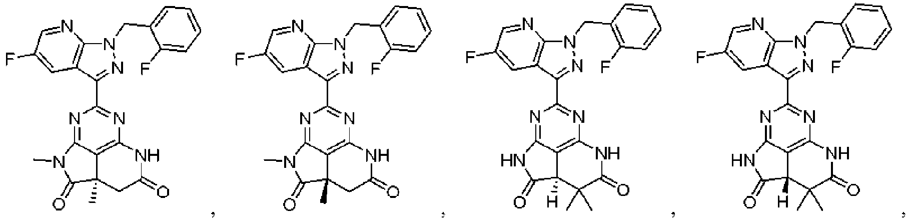

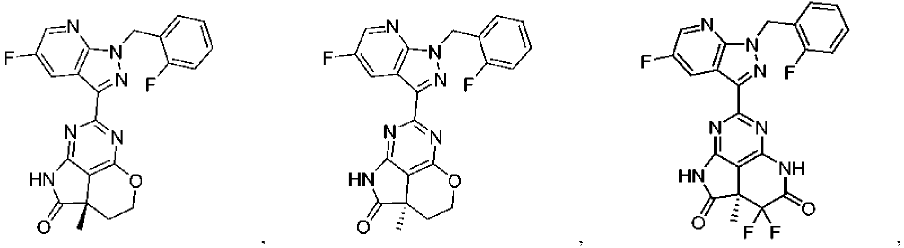

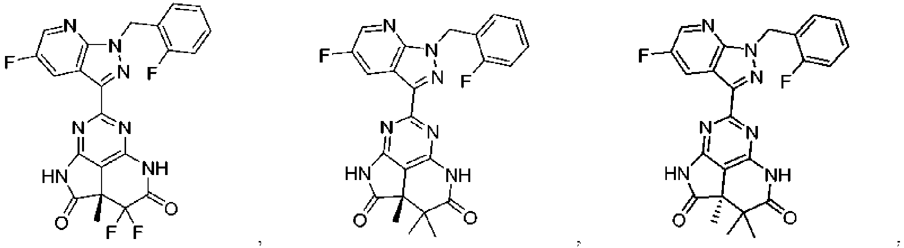

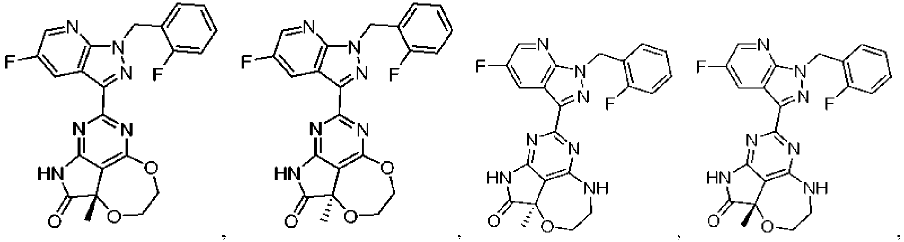

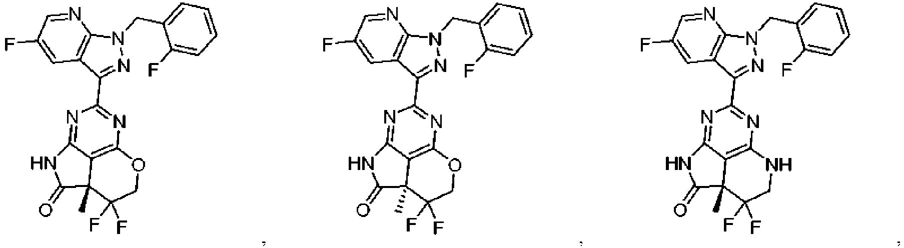

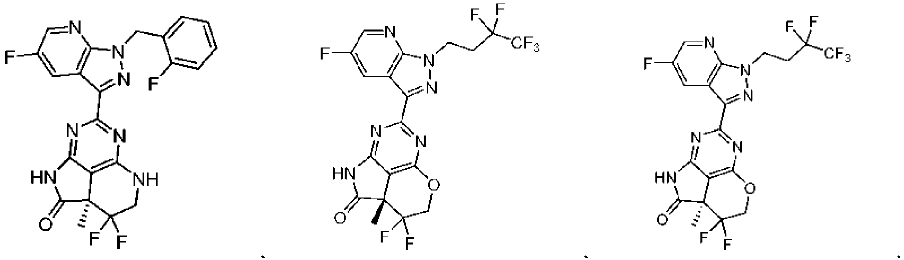

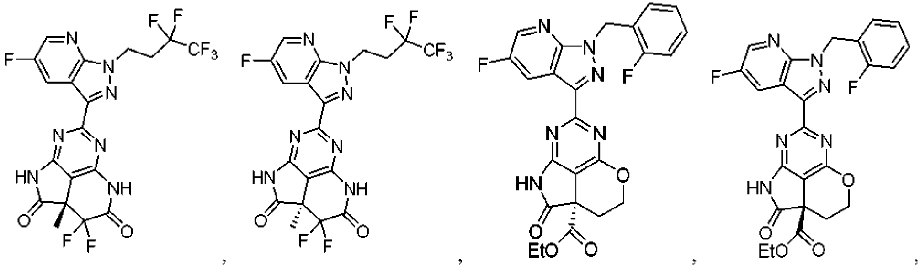

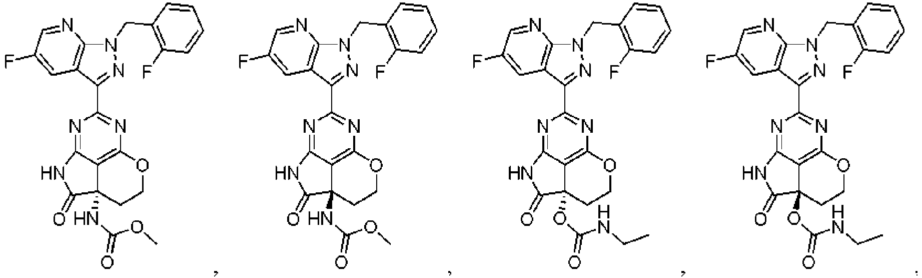

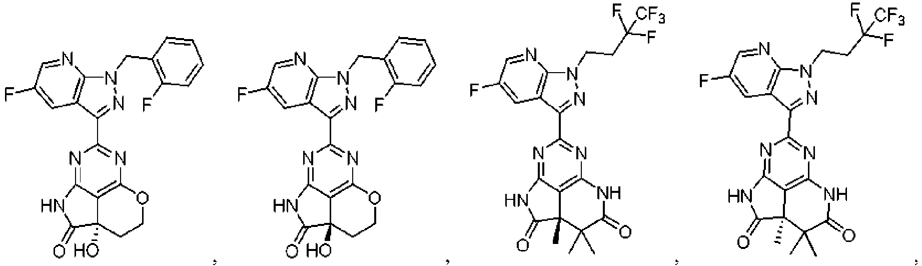

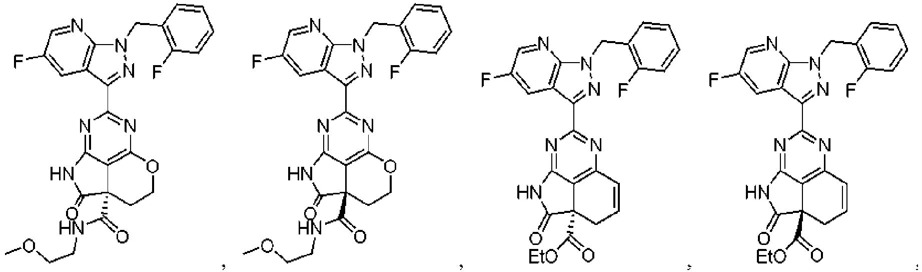

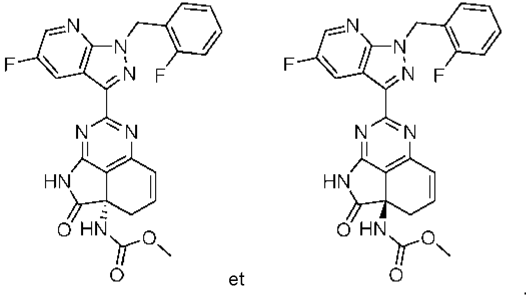

15. Composition pharmaceutique comprenant le composé, le stéréoisomère de celui-ci ou le sel pharmaceutiquement acceptable de celui-ci selon l'une quelconque des revendications 1 à 14, et facultativement comprenant en outre un excipient pharmaceutiquement acceptable.

16. Composé, stéréoisomère de celui-ci ou sel pharmaceutiquement acceptable de celui-ci selon l'une quelconque des revendications 1 à 14 ou composition pharmaceutique selon la revendication 15, pour une utilisation dans le traitement d'une maladie associée à un agoniste de la sGC ; facultativement, dans lequel la maladie associée à un agoniste de la sGC est une insuffisance cardiaque ou une hypertension.
